# EUROPEAN PATENT SPECIFICATION

(11) **EP 2 261 366 B1**
(45) Date of publication and mention of the grant of the patent: **27.01.2016**
(21) Application number: 10006026.8
(22) Date of filing: 11.11.2008
(51) Int. Cl.: C12Q 1/68

(54) **Methods and nucleic acids for the analysis of CpG methylation associated with the development of prostate cell proliferative disorders**
Verfahren und Nukleinsäuren zur Analyse der CpG Methylierung, die mit der Entwicklung von mit der prostatischen Zellproliferation assozierten Krankheiten einhergeht
Procédé et acides nucléiques pour l'analyse de la methylation CpG associé aux maladies prolifiques des cellules prostatiques

(30) Priority: 23.11.2007 EP 07121408; 13.10.2008 EP 08166448
(43) Date of publication of application: 15.12.2010
(62) Divisional of application: 08851452.6
(73) Proprietor: Epigenomics AG, 10829 Berlin (DE)
(72) Inventor: Sledziewski, Andrew, Shoreline, WA 98177 (US); Payne, Shannon, Seattle, WA 98177 (US); Schuster, Matthias, 13156 Berlin (DE); Lewin, Jörn, 10115 Berlin (DE); Schlegel, Thomas, 10407 Berlin (DE); Morotti, Andrew M., Seattle, WA 98102 (US)
(74) Representative: Zwicker, Jörk

(56) References cited:
- EP-A1- 1 748 083
- EP-A1- 1 764 107
- WO-A2-01/77384
- US-B1- 6 331 413
- WANG X ET AL: "KLF8 transcription factor participates in oncogenic transformation" ONCOGENE, NATURE PUBLISHING GROUP, GB LNKD- DOI:10.1038/SJ.ONC.1209796, vol. 26, no. 3, 18 January 2007 (2007-01-18), pages 456-461, XP002540311 ISSN: 0950-9232 [retrieved on 2007-07-10]
- SCHULZ W A ET AL: "Epigenetics of prostate cancer: beyond DNA methylation" JOURNAL OF CELLULAR AND MOLECULAR MEDICINE, vol. 10, no. 1, January 2006 (2006-01), pages 100-125, XP002602398 ISSN: 1582-1838
- PAYNE S ET AL: "Urine is the preferred remote body fluid for early identification of prostate cancer using real-time PCR detection of DNA methylation markers" EJC SUPPLEMENTS, vol. 5, no. 4, September 2007 (2007-09), page 282, XP002517341 & 14TH EUROPEAN CANCER CONFERENCE (ECCO 14); BARCELONA, SPAIN; SEPTEMBER 23 -27, 2007 ISSN: 1359-6349
- JERÓNIMO CARMEN ET AL: "A quantitative promoter methylation profile of prostate cancer" CLINICAL CANCER RESEARCH, THE AMERICAN ASSOCIATION FOR CANCER RESEARCH, US, vol. 10, no. 24, 15 December 2004 (2004-12-15), pages 8472-8478, XP002485532 ISSN: 1078-0432
- BASTIAN P J ET AL: "Molecular Biomarker in Prostate Cancer: The Role of CpG Island Hypermethylation" EUROPEAN UROLOGY, ELSEVIER BV, NL, vol. 46, no. 6, 1 December 2004 (2004-12-01), pages 698-708, XP004645485 ISSN: 0302-2838
- DHANASEKARAN S M ET AL: "Delineation of prognostic biomarkers in prostate cancer" NATURE 20010823 MACMILLAN MAGAZINES LTD GB, vol. 412, no. 6849, 23 August 2001 (2001-08-23), pages 822-826, XP002517408

## Description

### FIELD OF THE INVENTION

The present invention relates to genomic DNA sequences that exhibit altered expression patterns in disease states relative to normal. Particular embodiments provide methods, nucleic acids, nucleic acid arrays and kits useful for detecting, or for diagnosing prostate carcinoma.

### PRIOR ART

Prostate cancer is the most common cancer and the third leading cause of death in American men (Jemal et al., Cancer statistics, 2006. CA Cancer J Clin. 2006;56(2): 106-30). Incidence and mortality rates for this disease increase greatly with age, with more than 65% of all prostate cancer cases diagnosed in men older than 65 (Jemal et al., 2006; supra). Stage of disease at diagnosis also affects overall survival rates. Due to widespread use of the prostate-specific antigen (PSA) screening test, nearly 90% of new patients are diagnosed with local or regional disease (Jemal et al., 2006; supra). Patients with local or regional disease when diagnosed have a five-year relative survival rate approaching 100% (Jemal et al., 2006; supra).

The current guidelines for prostate cancer screening, according to the American Cancer Society, advises testing for elevated PSA and digital rectal examination annually beginning at age 50. For men at high risk of developing prostate cancer (African American men and men with one or more first degree relatives diagnosed at an early age), screening should begin at age 45. Positive findings on either of these exams is confirmed by prostate biopsy. The advent of PSA screening has changed the landscape of prostate cancer diagnosis. Incidence rates in prostate cancer have increased dramatically in the last 20 years, while diagnosis in males older than 65 has levelled off. PSA testing suffers from two disadvantages. The first is its low specificity as PSA is elevated in a number of benign conditions in addition to prostate cancer. This results in a large number of prostate biopsies being conducted in men who do not have prostate cancer. The second disadvantage is that despite the relatively high sensitivity of PSA, there are men who harbor prostate cancer in the absence of elevated PSA (> 4 ng/ml) (Thompson et al., Prevalence of prostate cancer among men with a prostate-specific antigen level < or =4.0 ng per milliliter. N Engl J Med. 2004 May 27;350(22):2239-46. Erratum in: N Engl J Med. 2004 30;351(14): 1470). It has also been estimated that up to 10% of prostate biopsies under currrent guidelines are falsely negative, resulting in decreased sensitivity even with biopsy (Djavan et al., Optimal predictors of prostate cancer on repeat prostate biopsy: a prospective study of 1,051 men. J Urol. 2000; 163(4): 1144-8; discussion 1148-9.; Mian et al., Predictors of cancer in repeat extended multisite prostate biopsy in men with previous negative extended multisite biopsy. Urology. 2002;60(5):836-40; Gupta et al., Transrectal ultrasound guided biopsy for detecting early prostate cancer: An Indian experience., Indian J Cancer. 2005;42(3): 151-4; Hanley et al., DNA integrity assay: a plasma-based screening tool for the detection of prostate cancer. Clin Cancer Res. 20061;12(15):4569-74). Improved tests with increased specificity and sensitivity are clearly needed. Krupple-like factor 8 (KLF8) has been shown (Wang and Zhao, 2007, Oncogene 26: 456-461) to be overexpressed in several caners such as ovarian, renal and breast cancer. Induction of KLF8 expression induced features of malignant transformation in cell culture.

Multifactorial approach. Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states. The present invention describes a plurality of particularly efficient and unique panels of genes, the methylation analysis of one or a combination of the members of the panel enabling the detection of prostate cell proliferative disorders with a particularly high sensitivity, specificity and/or predictive value.

Development of medical tests. Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. An example of a test that has high specificity is a gene-based test that can detect a p53 mutation. Specificity is important when the cost or risk associated with further diagnostic procedures or further medical intervention are very high.

### BRIEF DESCRIPTION OF THE DRAWINGS

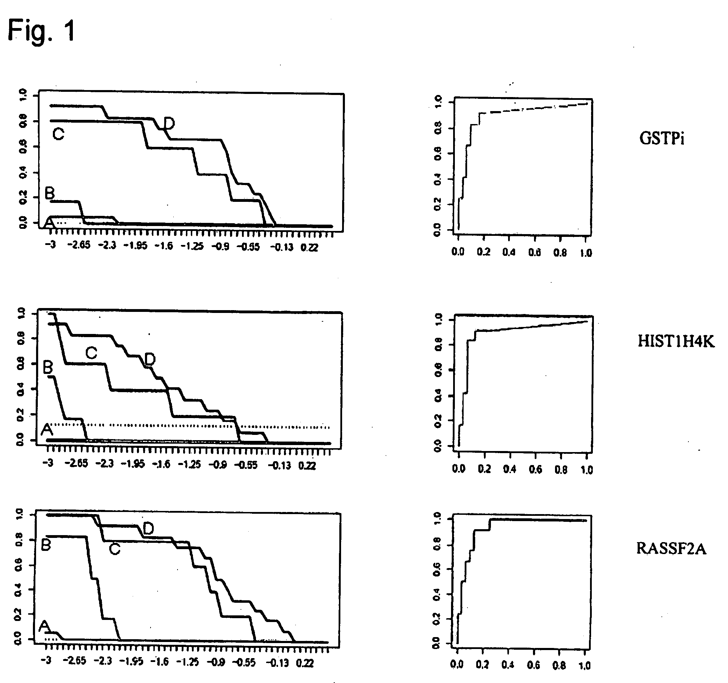

Figure 1 provides an overview of the log mean methylation measured by means of the HM assay according to Example 1. For each analysed gene (as labeled to the right of the figures), two plots are provided, the left hand side plots provide the multiclass analysis, sensitivity is shown on the Y-axis, DNA methylation measured in (log 10 ng/ml) is shown on the X-axis. The right hand plot provides an ROC wherein sensitivity is shown on the Y-axis and 1 -specificity is shown on the X-axis. (A -Normal blood, B - Normal prostate, C - NAT prostate, D - Tumor prostate).

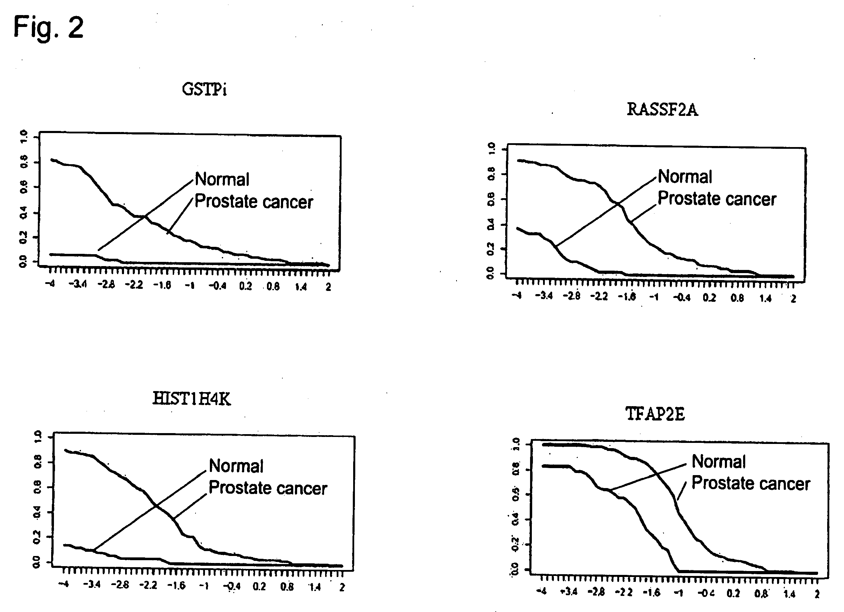

Figure 2 provides an overview of the log mean methylation measured by means of HM real-time PCR assays of post-prostatic massage urine of PCa and negative class I (young asymptomatic individuals) according to Example 1 . For each analysed gene (as labeled above each plot), sensitivity is shown on the Y-axis, DNA methylation measured in (log 10 ng/ml) is shown on the X-axis.

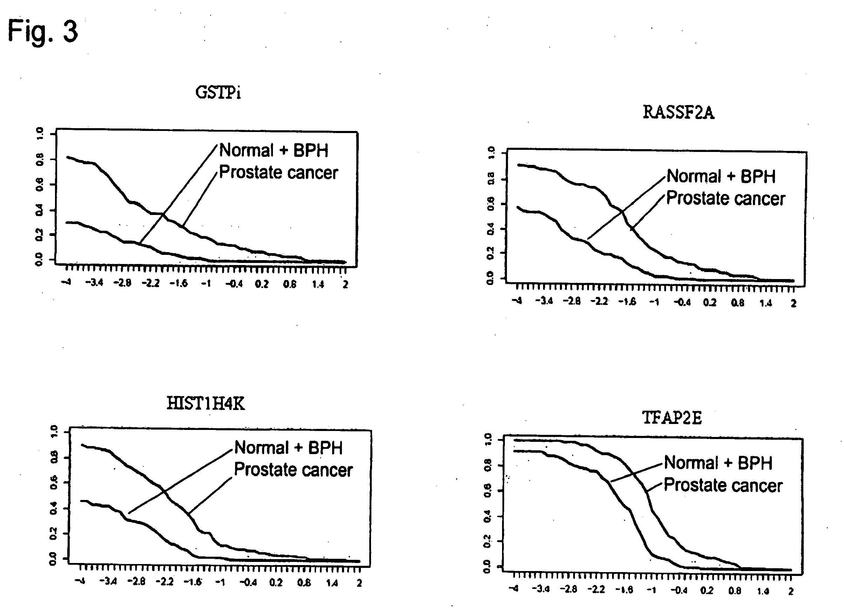

Figure 3 provides an overview of the log mean methylation measured by means of HM real-time PCR assays of post-prostatic massage urine of PCa and negative class II (young asymptomatic plus biopsy negative individuals) according to Example 1 . For each analysed gene (as labeled above each plot), sensitivity is shown on the Y-axis, DNA methylation measured in (log 10 ng/ml) is shown on the X-axis.

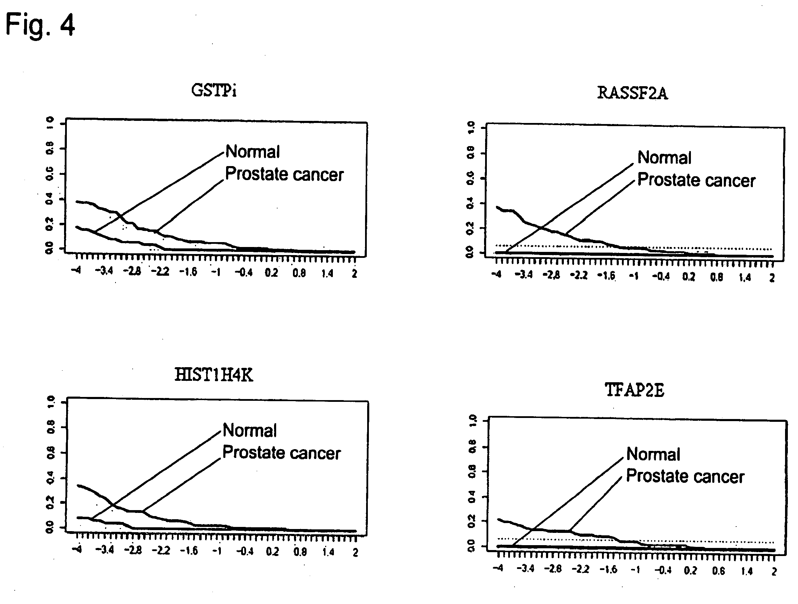

Figure 4 shows the log mean methylation measured by means of HM real-time PCR assays of plasma of PCa and negative class I (young asymptomatic individuals). For each analysed gene (as labeled above each plot), sensitivity is shown on the Y-axis, DNA methylation measured in (log 10 ng/ml) is shown on the X-axis.

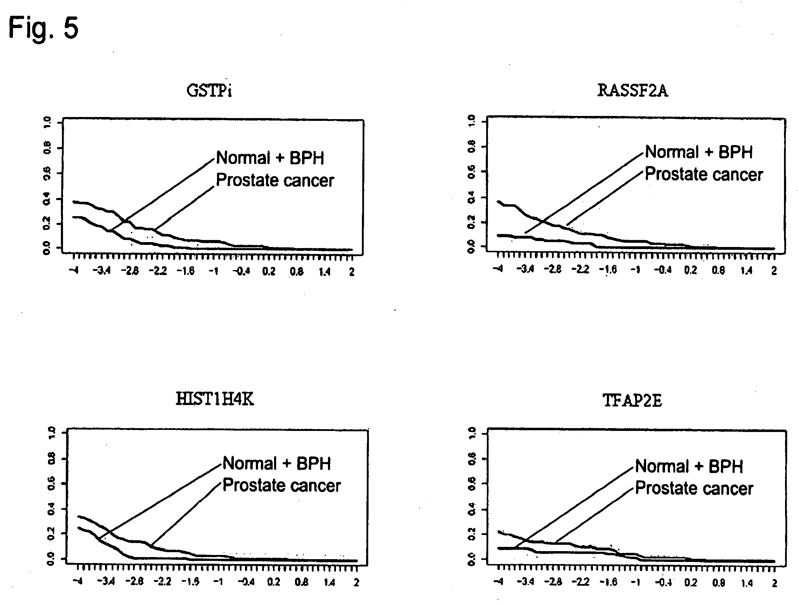

Figure 5 shows the log mean methylation measured by means of HM real-time PCR assays of plasma of PCa and negative class II (young asymptomatic plus biopsy negative individuals). For each analysed gene (as labeled above each plot), sensitivity is shown on the Y-axis, DNA methylation measured in (log 10 ng/ml) is shown on the X-axis.

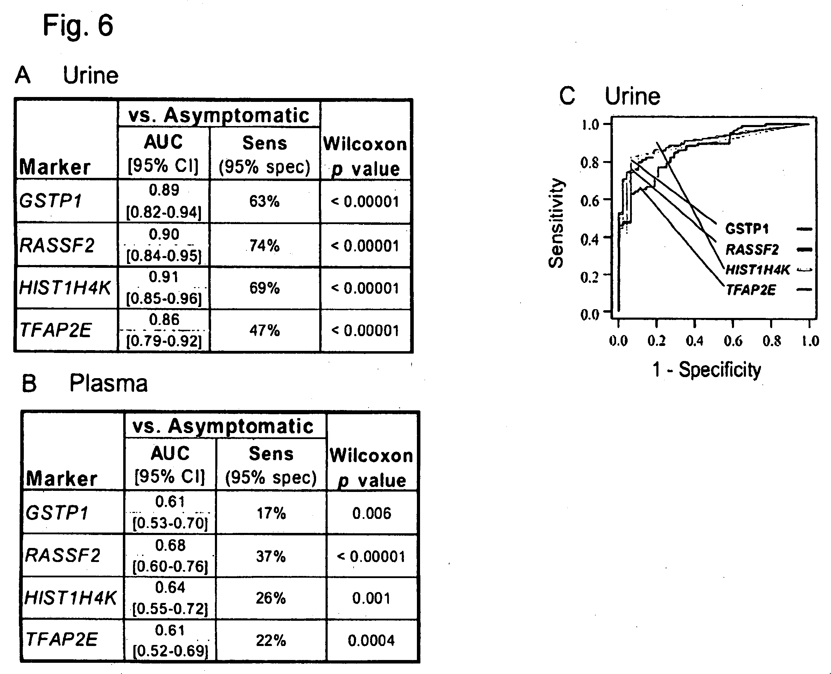

Figure 6 shows biomarker performance in discrimination of PCa patients from asymptomatic males (Example 1 : Performance of biomarkers for Pea vs. negative class I (asymptomatic males)). A, Table compares the AUC, sensitivity and Wilcoxon p values of the four biomarkers for PCa versus asymptomatic males in urine DNA. B, Table compares the AUC, sensitivity and Wilcoxon values of the four biomarkers for PCa versus asymptomatic males in plasma DNA. C, ROC curves for individual PCa biomarkers in urine DNA.

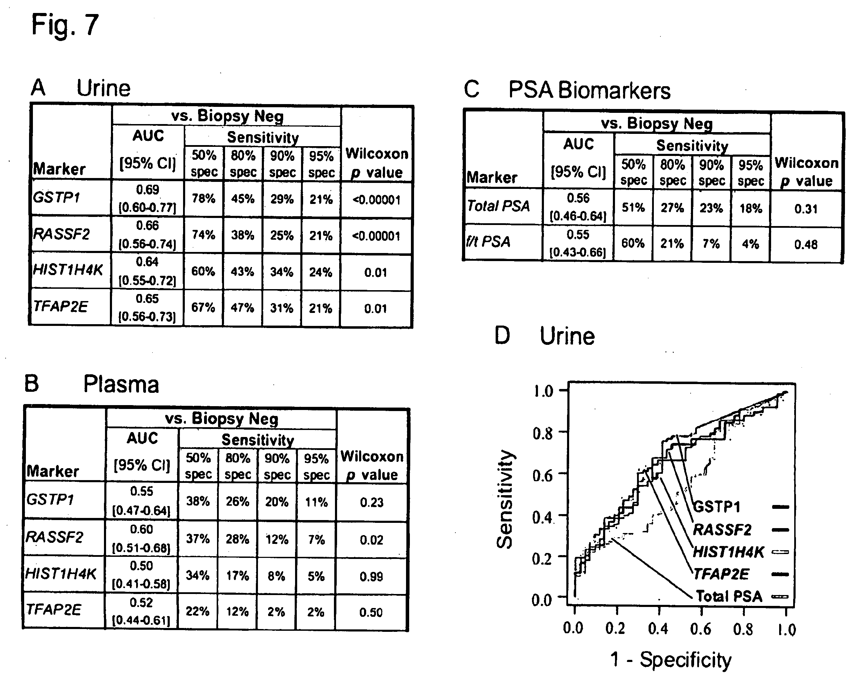

Figure 7 shows biomarker performance in discrimination of PCa from biopsy negative patients (Example 1 : Perfomance of biomarkers for PCa vs. biopsy negative males). A, Table compares .the AUC and Wilcoxon p values of the four biomarkers for PCa versus biopsy negative patients in urine DNA. Sensitivities are given for 50%, 80%, 90% and 95% specificities. B, Table compares the AUC and Wilcoxon p values of the four biomarkers for PCa versus biopsy negative patients in plasma DNA. Sensitivities are given for 50%, 80%, 90% and 95% specificities. C, Table compares the AUC and Wilcoxon p values of PSA-related biomarkers for PCa versus biopsy negative patients. Sensitivities are given for 50%, 80%, 90% and 95% specificities. D, ROC curves for individual PCa biomarkers in urine DNA.

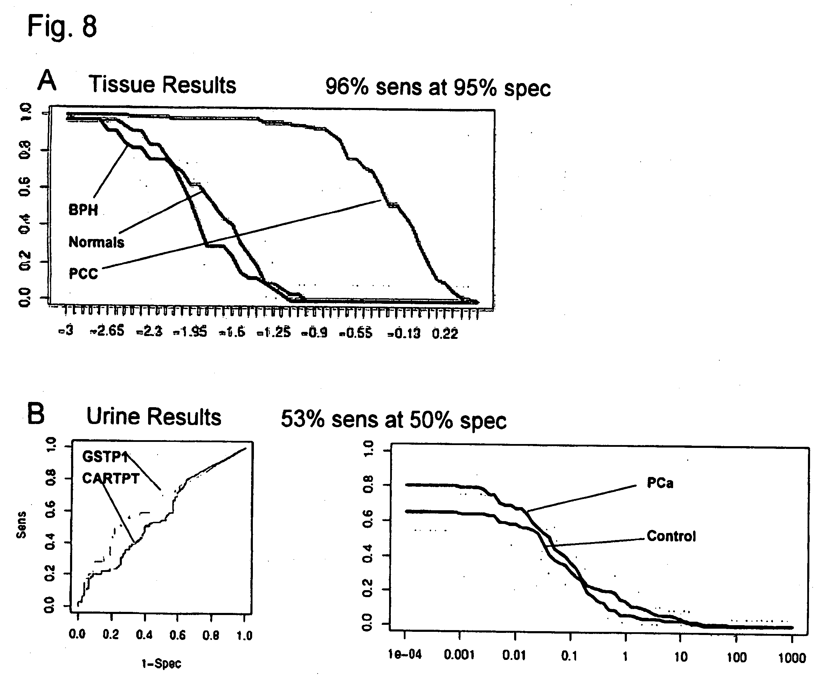

Figure 8 shows CARTPT biomarker performance. Figure 8A shows CARTPT biomarker performance in discrimination of PCa from benign tissues (log mean methylation measured by means of real-time quantitative PCR (qPCR); Example 2) and Figure 8B shows CARTPT biomarker performance in discrimination of PCa from biopsy negative patient urine (left ROC curve, right log mean methylation measured by means of real-time qPCR; Example 3).

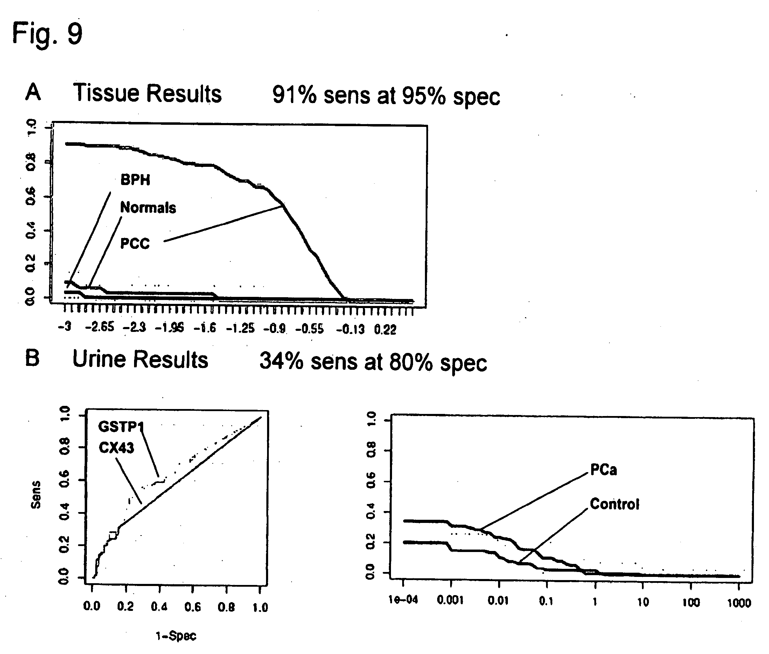

Figure 9 shows CX43/GJA1 biomarker performance. Figure 9A shows CX43/GJA1 biomarker performance in discrimination of PCa from benign tissues (log mean methylation measured by means of real-time qPCR; Example 2) and Figure 9B shows CX43/GJA1 biomarker performance in discrimination of PCa from biopsy negative patient urine (left ROC curve, right log mean methylation measured by means of real-time qPCR; Example 3).

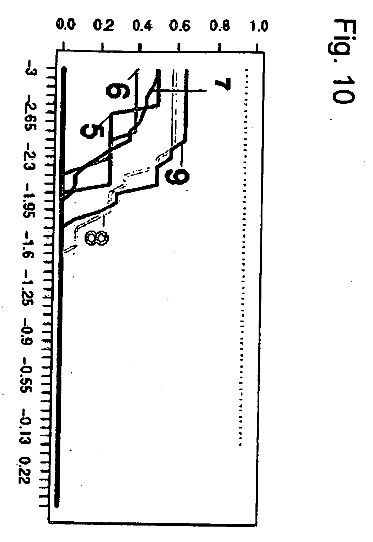

Figure 10 shows GPR68 biomarker performance in discrimination of high Gleason sum PCa from low Gleason sum PCa tissues (log mean methylation measured by means of real-time qPCR; Example 2). 15/26 (58%) Gleason 8/9; 3/12 (25%) Gleason 5/6; AUC =0.66; Wilcoxon p =0.05 Gleason 9-10 vs. 5-8 (The numbers 5-9 within the diagram indicate the Gleason scores 5, 6, 7, 8, and 9).

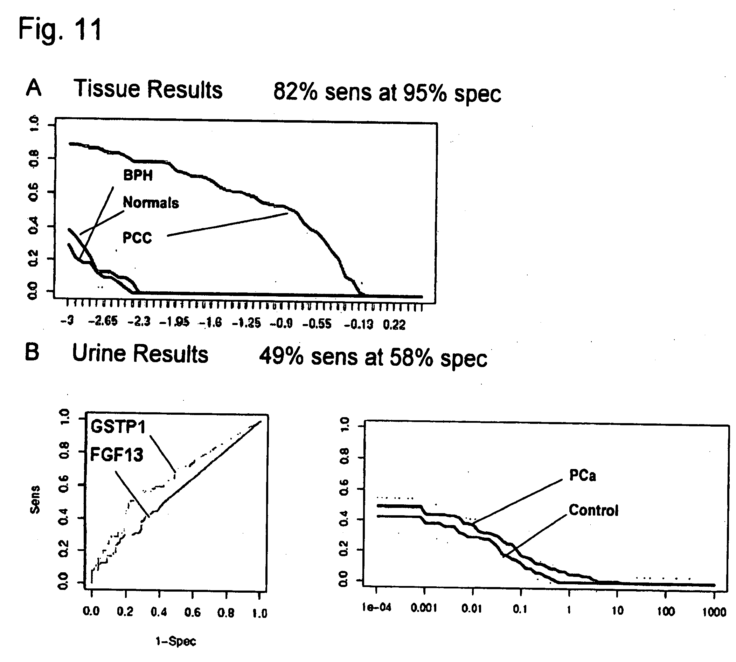

Figure 11 shows FGF 13 biomarker performance. Figure 11A shows FGF 13 biomarker performance in discrimination of PCa from benign tissues (log mean methylation measured by means of real-time qPCR; Example 2) and Figure 11B shows FGF 13 biomarker performance in discrimination of PCa from biopsy negative patient urine (left ROC curve, right log mean methylation measured by means of real-time qPCR; Example 3).

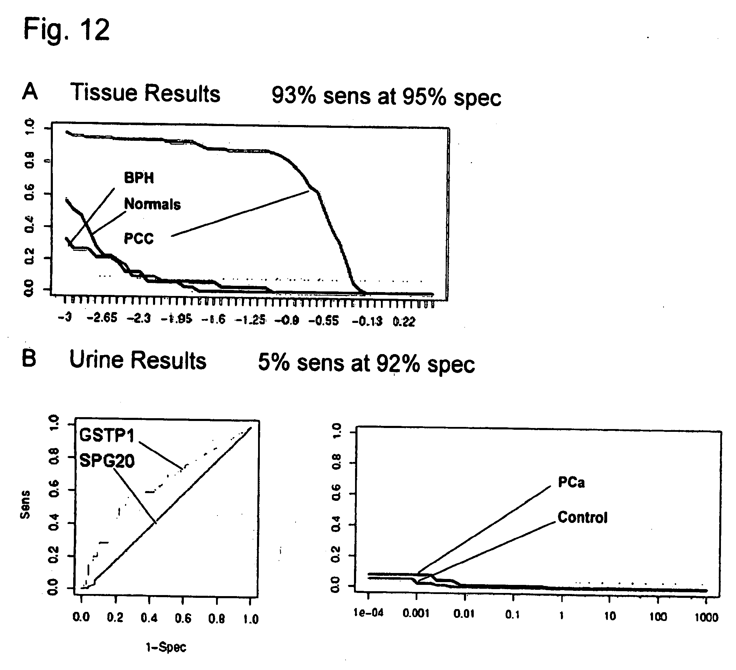

Figure 12 shows SPG20 biomarker performance. Figure 12A shows SPG20 biomarker performance in discrimination of PCa from benign tissues (log mean methylation measured by means of real-time qPCR, Example 2) and Figure 12B shows SPG20 biomarker performance of PCa from biopsy negative patient urine (left ROC curve, right log mean methylation measured by means of real-time qPCR; Example 3).

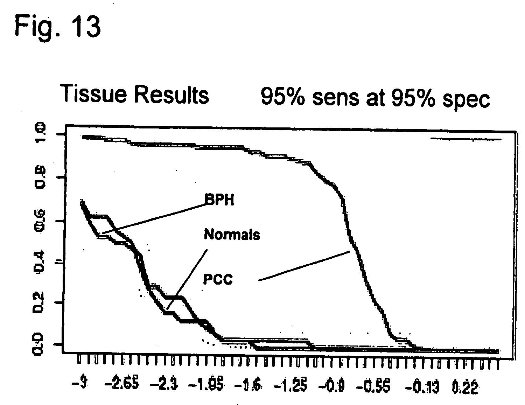

Figure 13 shows NKX2-6 biomarker performance in discrimination of PCa from benign tissues (log mean methylation measured by means of real-time qPCR, Example 2).

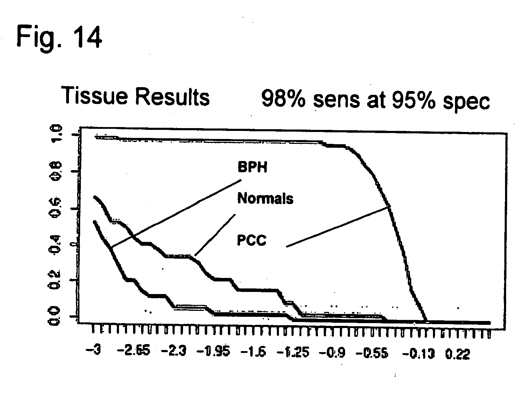

Figure 14 shows RARB biomarker performance in discrimination of PCa from benign tissues (log mean methylation measured by means of real-time qPCR, Example 2).

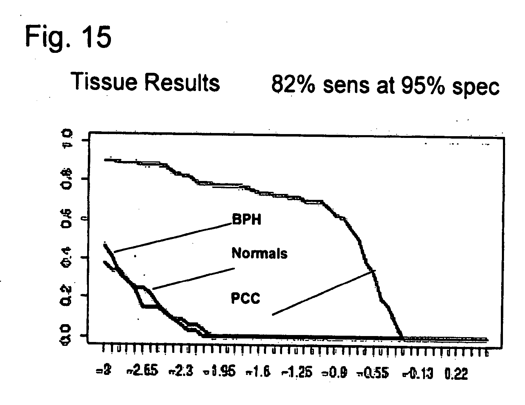

Figure 15 shows PDE4D biomarker performance in discrimination of PCa from benign tissues (log mean methylation measured by means of real-time qPCR, Example 2).

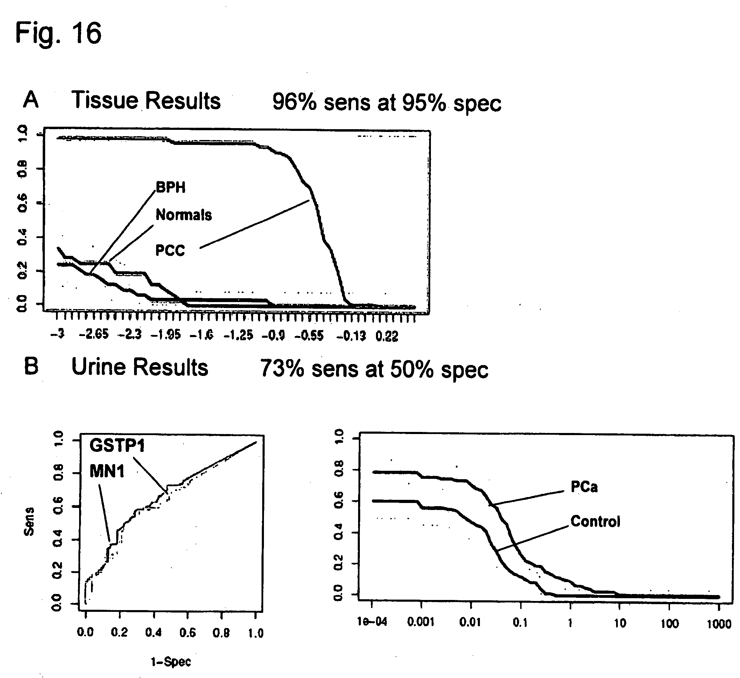

Figure 16 shows MN1 biomarker performance. Figure 16A shows MN1 biomarker performance in discrimination of PCa from benign tissues (log mean methylation measured by means of real-time qPCR, Example 2) and Figure 16B shows MN1 biomarker performance in discrimination of PCa from biopsy negative patient urine (left ROC curve, right log mean methylation measured by means of real-time qPCR; Example 3).

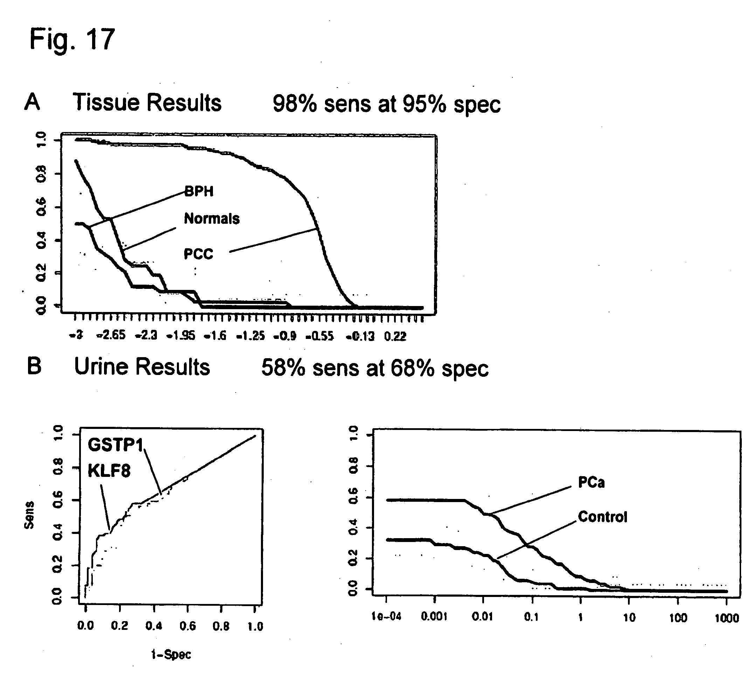

Figure 17 shows KLF8 biomarker performance. Figure 17A shows KLF8 biomarker performance in discrimination of PCa from benign tissues (log mean methylation measured by means of real-time qPCR, Example 2) and Figure 17B shows KLF8 biomarker performance in discrimination of PCa from biopsy negative patient urine (left ROC curve, right log mean methylation measured by means of real-time qPCR; Example 3).

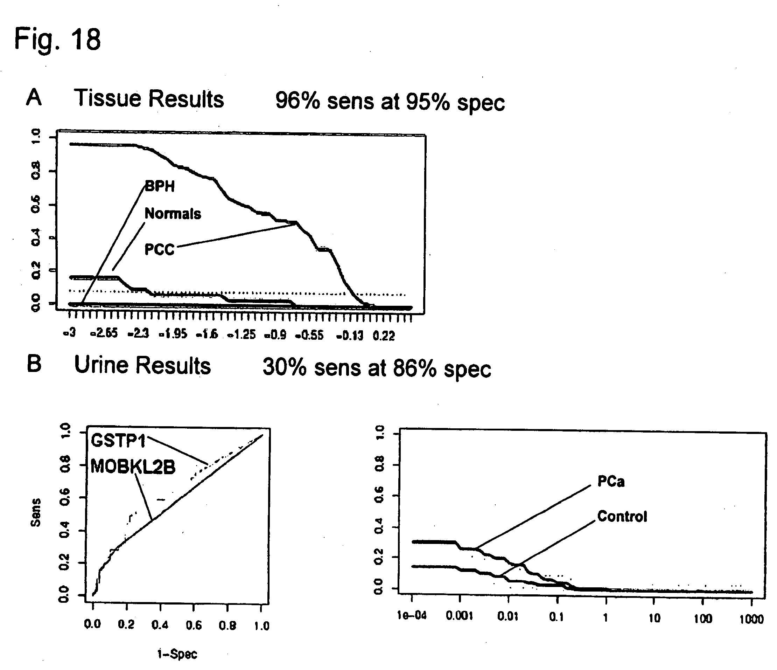

Figure 18 shows M0BKL2B biomarker performance. Figure 18A shows MOBKL2B biomarker performance in discrimination of PCa from benign tissues (log mean methylation measured by means of real-time qPCR, Example 2) and Figure 18B shows M0BKL2B biomarker performance of PCa from biopsy negative patient urine (left ROC curve, right log mean methylation measured by means of real-time qPCR; Example 3).

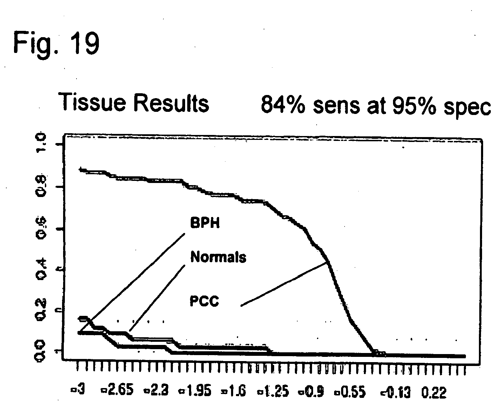

Figure 19 shows HIST1H2BD biomarker performance in discrimination of PCa from benign tissues (log mean methylation measured by means of real-time qPCR, Example 2).

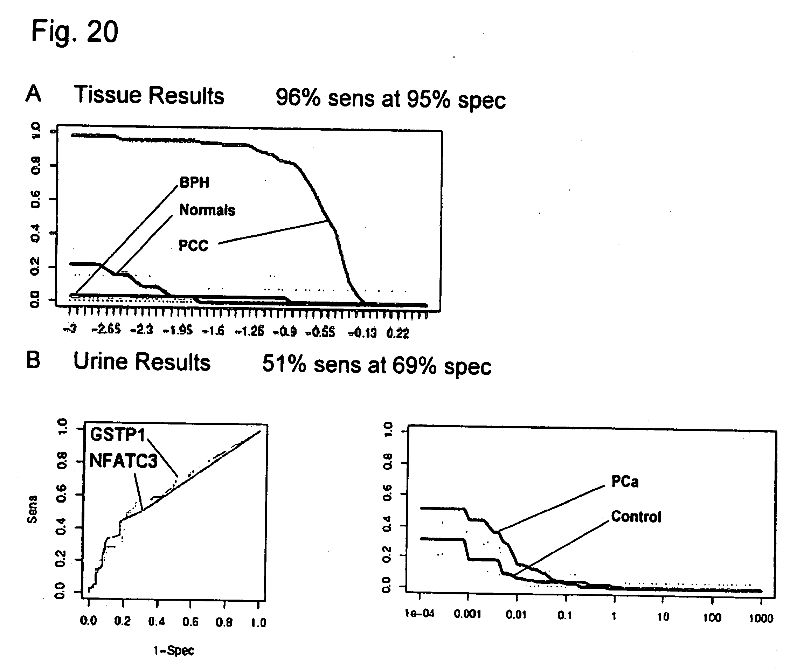

Figure 20 shows NFATC3 biomarker performance. Figure 20A shows NFATC3 biomarker performance in discrimination of PCa from benign tissues (log mean methylation measured by means of real-time qPCR, Example 2) and Figure 20B shows NFATC3 biomarker performance in discrimination of PCa from biopsy negative patient urine (left ROC curve, right log mean methylation measured by means of real-time qPCR; Example 3).

### SUMMARY OF THE INTENTION

The present invention provides a method for detecting prostate cell proliferative disorders, most preferably, prostate carcinoma in a subject comprising determining the expression levels of the KLF8 gene or the KLF8 gene and at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1 ; M0BKL2B; IFLTD1 (KRAS); ANXA2; NFATC3; MN1; FGF13; NKX2-6; SPG20; DSE/SART2; GJA1 (CX43); SPATA6; MCC; GPR68 (INTRON 1 AND/OR EXON 2 REGION) in a biological sample isolated from said subject wherein underexpression and/or CpG methylation is indicative of the presence of said disorder.

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB;
NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1 ; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
- HIST1H4K +RASSF2A or HIST1H4K +GSTPi

Various aspects of the present invention provide an efficient and unique genetic marker, whereby expression analysis of said marker enables the detection of prostate cell proliferative disorders, most preferably, prostate carcinoma with a particularly high sensitivity, specificity and/or predictive value.

In one embodiment the invention provides a method for detecting prostate cell proliferative disorders, most preferably, prostate carcinoma in a subject comprising determining the expression levels of the KLF8 gene or the KLF8 gene and at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1;FGF13; NKX2-6; SPG20; DSE/SART2; GJA1 (CX43); SPATA6; MCC; GPR68 (INTRON 1 AND/OR EXON 2 REGION); in a biological sample isolated from said subject wherein under-expression and/or CpG methylation is indicative of the presence of said disorder. In one embodiment said expression level is determined by detecting the presence, absence or level of mRNA transcribed from said gene. In a further embodiment said expression level is determined by detecting the presence, absence or level of a polypeptide encoded by said gene or sequence thereof

In a further preferred embodiment said expression is determined by detecting the presence or absence of CpG methylation within said gene, wherein the presence of methylation indicates the presence of prostate cell proliferative disorders, more specifically, prostate carcinoma.

Said method comprises the following steps: i) contacting genomic DNA isolated from a biological sample (preferably selected from the group consisting of ejaculate, blood plasma, blood serum, whole blood, isolated blood cells, cells isolated from the blood) obtained from the subject with at least one reagent, or series of reagents that distinguishes between methylated and non-methylated CpG dinucleotides within at least one and more preferably a plurality of target regions of the genomic DNA, wherein the nucleotide sequence of said target region comprises at least one CpG dinucleotide sequence of the KLF8 gene or the KLF8 gene and at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1; FGF 13; NKX2-6; SPG20;
DSE/SART2; GJA1 (CX43); SPATA6; MCC; GPR68 (INTRON 1 AND/OR EXON 2 REGION); and ii) detecting carcinoma , at least in part.

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1 H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

Preferably the target region comprises, or hybridizes under stringent conditions to a sequence of at least 16, 50, 100 or 500 contiguous nucleotides of at least one sequence selected from the group consisting of SEQ ID NO: 1-4; 37-65.

Said use of the gene may be enabled by means of any analysis of the expression of the gene, by means of mRNA expression analysis or protein expression analysis. However, in the most preferred embodiment of the invention the detection of detecting prostate cell proliferative disorders, most preferably, prostate carcinomas is enabled by means of analysis of the methylation status of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or its promoter or regulatory elements.

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

The invention provides a method for the analysis of biological samples for features associated with the development of cancer, the method characterized in that the nucleic acid, or a fragment thereof of SEQ ID NO: 53 or SEQ ID NO: 53 and at least one sequence selected from the group consisting of SEQ ID NO: 1-4; 37-52 and 54-65 is contacted with a reagent or series of reagents capable of distinguishing between methylated and non methylated CpG dinucleotides within the genomic sequence.

The present invention provides a method for ascertaining epigenetic parameters of genomic DNA associated with the development of prostate cancer. The method has utility for the improved detection and diagnosis of said prostate cell proliferative disorders, in particular prostate cancer.

Preferably, the source of the test sample is selected from the group consisting of cells or cell lines, histological slides, biopsies, paraffin-embedded tissue, body fluids, ejaculate, urine, blood, and combinations thereof. More preferably, the source is selected from the group consisting of ejaculate, urine, blood plasma, blood serum, whole blood, isolated blood cells, cells isolated from the blood obtained from the subject.

Specifically, the present invention provides a method for detecting prostate cancer suitable for use in a diagnostic tool, comprising: obtaining a biological sample comprising genomic nucleic acid(s); contacting the nucleic acid(s), or a fragment thereof, with a reagent or a plurality of reagents sufficient for distinguishing between methylated and non methylated CpG dinucleotide sequences within at least one and more preferably a plurality of target sequence(s) of the subject nucleic acid, wherein each of said target sequences comprises, or hybridises under stringent conditions to, a sequence comprising at least 16, 50, 100 or 500 contiguous nucleotides of a sequence of SEQ ID NO: 53 or SEQ ID NO: 53 and a sequence selected from the group consisting of SEQ ID NO: 1-4; 37-52 and 54-65, said contiguous nucleotides comprising at least one CpG dinucleotide sequence; and determining, based at least in part on said distinguishing, the methylation state of at least one and more preferably a plurality of target CpG dinucleotide sequence, or an average, or a value reflecting an average methylation state of a plurality of target CpG dinucleotide sequences.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 5-20; 66-181, and contiguous regions thereof corresponding to the target sequence.

Additional embodiments provide a method for the detection of detecting prostate cell proliferative disorders, most preferably, prostate cancer comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 5-20; 66-181, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of a sequence selected from the group consisting of SEQ ID NO: 1-4; 37-65.

Preferably, determining comprises use of at least one method selected from the group consisting of: I) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 5-20; 66-181, and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 5-20; 66-181, and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 5-20; 66-181, and complements thereof, and extending at least one such hybridized nucleic acid molecule by at least one nucleotide base; and iv) sequencing of the amplificate.

Further embodiments provide a method for the analysis (i.e. detection of classification) of carcinoma, comprising: obtaining a biological sample having subject genomic DNA; extracting the genomic DNA; contacting the genomic DNA, or a fragment thereof, comprising one or more sequences selected from the group consisting of SEQ ID NO: 1-4; 37-65 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence at least one sequence selected from the group consisting of SEQ ID NO: 1-4; 37-65, or an average, or a value reflecting an average methylation state of a plurality of CpG dinucleotide sequences thereof. Preferably, the digested or undigested genomic DNA is amplified prior to said determining.
Additional embodiments provide novel genomic and chemically modified nucleic acid sequences, as well as oligonucleotides and/or PNA-oligomers for analysis of cytosine methylation patterns within sequences from the group consisting of SEQ ID NO: 1-4; 37-65.

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an increased presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a decreased presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and'DNA chip(s),'as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific blocking probes (also referred to herein as blockers) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific blocking probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401 A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 600C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5.

"Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The terms "RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); " shall be taken to include all transcript variants thereof and all promoter and regulatory elements thereof. Furthermore as a plurality of SNPs are known within said genes the term shall be taken to include all sequence variants thereof.

The present invention provides a method for detecting carcinoma in a subject comprising determining the expression levels of the KLF8 gene or the KLF8 gene and at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1 ; M0BKL2B; IFLTDI (KRAS); ANXA2; NFATC3; MN1; FGF13; NKX2-6; SPG20; DSE/SART2; GJA1 (CX43); SPATA6; MCC; GPR68 (INTRON 1 AND/OR EXON 2 REGION); in a biological sample isolated from said subject wherein underexpression and/or CpG methylation is indicative of the presence or class of said disorder. Said markers may be used for the diagnosis of prostate cancer including early detection during the pre-cancerous stages of the disease. The markers of the present invention are particularly efficient in detecting malignant prostate cell proliferative disorders such as prostate carcinoma, thereby providing improved means for the early detection, classification and treatment of said disorders.

In addition to the embodiments above wherein the methylation analysis of the KLF8 gene or the KLF8 gene and at least one gene selected from the group consisting of RASSF2A; TFAP2E; HIST1H4K; GSTPi; MME; RARB; NKX3-1;
NPAL3; ACVR2A; ARL4C; PDE4D; CARTPT; ff1ST1H2BD; CUL1; MOBKL2B; IFLTDI (KRAS); ANXA2; NFATC3; MN1; FGF13; NKX2-6; SPG20; DSE/SART2; GJA1 (CX43); SPATA6; MCC; GPR68 (INTRON 1 AND/OR EXON 2 REGION); is analysed, the invention presents further panels of genes comprising at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); with novel utility for the detection of cell proliferative disorders, in particular prostate cancer. Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

Bisulfite modification of DNA is an art-recognized tool used to assess CpG methylation status. 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is converted in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98,1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31,1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum MoI Genet., 6:387-95, 1997; Feil R, et al., Nucleic Acids Res., 22:695-, 1994; Martin V, et al., Gene, 157:261-4, 1995; WO 97/46705 and WO 95/15373).

The present invention provides for the use of the bisulfite technique, in combination with one or more methylation assays, for determination of the methylation status of CpG dinucleotide sequences within SEQ ID NO: 1-4; 37-65. Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood or ejaculate. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) the genes RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION).

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

According to the present invention, determination of the methylation status of CpG dinucleotide sequences within SEQ ID NO: 1-4; 37-65 has utility in the diagnosis of prostate cancer.

Methylation Assay Procedures. Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA. COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89: 1827-1831 , 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (e.g., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific blocking probes (also referred to herein as blockers) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific blocking probes covering CpG positions between the amplification primers. The HeavyMethyl™ assay may also be used in combination with methylation specific amplification primers.

Typical reagents (e.g., as might be found in a typical MethyLight D -based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

MSP. MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

MethyLight™. The MethyLight™ assay is a high-throughput quantitative methylation assay that utilizes fluorescence-based real-time PCR (TaqMantH) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight™ assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight™ process can by used with any suitable probes e.g. "TaqMan®" , Lightcycler® etc. For example, double-stranded genomic DNA is treated with sodium bisulfite and subjected to one of two sets of PCR reactions using TaqMan® probes; e.g., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 100C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the Taqman® probe by digesting it to release the fluorescent reporter molecule for quantitative detection of its now unquenched signal using a real-time fluorescent detection system.

Typical reagents (e.g., as might be found in a typical MethyLightD -based kit) for MethyLight™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM™ (quantitative methylation) assay is an alternative quantitative test for methylation patterns in genomic DNA samples, wherein sequence discrimination occurs at the level of probe hybridization.In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM™ process can by used with any suitable probes e.g. "TaqMan®" , Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.
Typical reagents (e.g., as might be found in a typical QM™ -based kit) for QM™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

Ms-SNuPE. The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

The genomic sequences according to SEQ ID NO: 1-4; 37-65, and non-naturally occurring treated variants thereof according to SEQ ID NO: 5-20; 66-181, were determined to have novel utility for the early detection, classification and/or treatment of cell proliferative disorders, in particular prostate carcinoma

In one embodiment the invention of the method comprises the following steps: i) contacting genomic DNA (preferably isolated from body fluids) obtained from the subject with at least one reagent, or series of reagents that distinguishes between methylated and non-methylated CpG dinucleotides within the KLF8 gene or the KLF8 gene and at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; FGF 13; NKX2-6; SPG20; DSE/SART2; GJA1 (CX43); SPATA6; MCC; GPR68 (INTRON 1 AND/OR EXON 2 REGION);
(including promoter and regulatory regions); and ii) detecting prostate cell proliferative disorders, most preferably, prostate carcinoma.

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic matter or preneoplastic matter are suitable for use in the present method, preferred are cell lines, histological slides, biopsies, paraffin-embedded tissue, body fluids, ejaculate, urine, blood plasma, blood serum, whole blood, isolated blood cells, cells isolated from the blood and combinations thereof. Body fluids are the preferred source of the DNA; particularly preferred are ejaculate, urine, blood plasma, blood serum, whole blood, isolated blood cells and cells isolated from the blood.

The genomic DNA sample is then treated with at least one reagent, or series of reagents that distinguishes between methylated and non-methylated CpG dinucleotides within at least one and more preferably a plurality of target region(s) of the genomic DNA, wherein each target region comprises, or hybridizes under stringent conditions to a sequence of at least 16, 50, 100 or 500 contiguous nucleotides of at least one sequence selected from the group consisting of SEQ ID NO: 1-4; 37-65 respectively, wherein said contiguous nucleotides comprise at least one CpG dinucleotide sequence.

It is particularly preferred that said reagent converts cytosine bases which are unmethylated at the 5'-position to uracil, thymine, or another base which is dissimilar to cytosine in terms of hybridisation behaviour. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behavior It is preferred that this treatment is carried out with bisulfite (hydrogen sulfite, disulfite) and subsequent alkaline hydrolysis. Such a treatment results in the conversion of SEQ ID NO: 1-4; 37-65 to SEQ ID NO: 5-12; 66-123 (see Table 1) wherein said CpG dinucleotide are methylated or SEQ ID NO: 13-20; 124-181 wherein said CpG dinucleotides are unmethylated (see Table 1).

The treated DNA is then analysed in order to determine the methylation state of the KLF8 gene or the KLF8 gene and at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; FGF13; NKX2-6; SPG20; DSE/SART2; GJA1 (CX43); SPATA6; MCC; GPR68 (INTRON 1 AND/OR EXON 2 REGION); prior to the treatment.

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

It is particularly preferred that each target region comprises, or hybridizes under stringent conditions to at least 16, 50, 100 or 500 contiguous nucleotides of the KLF8 or the KLF8 gene and a gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; FGF 13; NKX2-6; SPG20; DSE/SART2; GJA1 (CX43); SPATA6; MCC; GPR68 (INTRON 1 AND/OR EXON 2 REGION); . It is exemplified that the sequence of said genes according to SEQ ID NO: 1-4; 37-65 is analysed as provided in Table 1 and the accompanying sequence listing. The method of analysis may be selected from those known in the art, including those listed herein. Particularly preferred are MethyLight™, MSP and the use of blocking oligonucleotides

(HeavyMethyl™) as described herein. It is further preferred that any oligonucleotides used in such analysis (including primers, blocking oligonucleotides and detection probes) should be reverse complementary, identical, or hybridise under stringent or highly stringent conditions to an at least 16-base-pair long segment of the base sequences of one or more of SEQ ID NO: 5-20; 66-181 and sequences complementary thereto.

Aberrant methylation, more specifically hypermethylation of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); (as well as promoter and/or regulatory regions thereof) is associated with the presence of prostate cell proliferative disorders, in particular, prostate cancer. Accordingly wherein a biological sample presents within any degree of methylation, said sample should be determined as being of a cell proliferative disorder, in particular cancer.

Said method may be enabled by means of any analysis of the expression of an RNA transcribed therefrom or polypeptide or protein translated from said RNA, preferably by means of mRNA expression analysis or polypeptide expression analysis. Accordingly the present invention also provides diagnostic assays and methods, both quantitative and qualitative for detecting the expression of at least one gene selected from the group consisting of RASSF2A; TFAP2E; HIST1H4K; GSTPi; MIME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); in a subject and determining therefrom upon the presence or absence of prostate cell proliferative disorders, most preferably, prostate cancer in said subject.

Aberrant expression of mRNA transcribed from at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); is associated with the presence of prostate cell proliferative disorders, in particular, prostate cancer in a subject. According to the present invention, under expression (and/or presence methylation) is associated with the presence of cancer, and vice versa over-expression (and/or absence of methylation) is associated with the absence of cancer.

Preferably the mRNA expression of plurality of genes (herein also referred to as a "gene panel") are analysed. 2, 3 or 4 genes may be analysed. In one embodiment of the method said panel comprises at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF 13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1 H4K.

Particularly exemplified are the following combinations of genes:
HIST1H4K +RASSF2A or HIST1H4K +GSTPi

To detect the presence of mRNA encoding a gene or genomic sequence, a sample is obtained from a patient. The sample may be any suitable sample comprising cellular matter of the tumor. Suitable sample types include cell lines, histological slides, biopsies, paraffin-embedded tissue, body fluids, ejaculate, urine, blood plasma, blood serum, whole blood, isolated blood cells, cells isolated from the blood and all possible combinations thereof. It is preferred that said sample types are ejaculate or body fluids selected from the group consisting ejaculate, urine, blood plasma, blood serum, whole blood, isolated blood cells, cells isolated from the blood.

The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridisation probes (e.g. TaqMan™, Lightcycler™, Molecular Beacons™ & Scorpion™ primer) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels, chemiluminescent labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used.

In an RNase protection assay (RPA), the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel et al. 2003), particularly preferred are probes with high specific activities.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression.

DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of the genes RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy®3- or Cy®5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridisation using a fluorescent microarray scanner. The fluorescent intensity of each spot indicates the level of expression of the analysed gene; bright spots correspond to strongly expressed genes, while dim spots indicate weak expression..

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.

Another aspect of the application disclose a kit for use in diagnosis of prostate cell proliferative disorders, most preferably, prostate cancer in a subject according to the methods of the present invention, said kit comprising: a means for measuring the level of transcription of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF 13; NKX2-6; SPG20; DSE/SART2; GJA1 (CX43); SPATA6; MCC; GPR68 (INTRON 1 AND/OR EXON 2 REGION). The means for measuring the level of transcription comprise oligonucleotides or polynucleotides which may be able to hybridise under stringent or moderately stringent conditions to the transcription products of a gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION). The level of transcription may be determined by techniques selected from the group of Northern Blot analysis, reverse transcriptase PCR, real-time

PCR, RNAse protection, and microarray. The kit may further comprises means for obtaining a biological sample of the patient. Exemplified is a kit, which further comprises a container which is most preferably suitable for containing the means for measuring the level of transcription and the biological sample of the patient, and most preferably further comprises instructions for use and interpretation of the kit results. In an example the kit comprises (a) a plurality of oligonucleotides or polynucleotides able to hybridise under stringent or moderately stringent conditions to the transcription products of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); (b) a container , preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further preferred that said kit further contains an Rnase reagent.

The present application further discloses methods for the detection of the presence of the polypeptide encoded by said gene sequences in a sample obtained from a patient.

Aberrant levels of polypeptide expression of the polypeptides encoded by at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); are associated with the presence of cancer.

Under expression of said polypeptides is associated with the presence of prostate cell proliferative disorders, in particular, prostate cancer.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn, pp 217-262,1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide(s) encoded by the RASSF2A; TFAP2E; HIST1H4K; GSTPi; MME; RARB; NKX3-1; NPAL3; ACVR2A; ARL4C; PDE4D; CARTPT; HIST1H2BD; CUL1 ; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION) genes.

Such antibodies are useful for cancer diagnosis. In certain embodiments production of monoclonal or polyclonal antibodies can be induced by the use of an epitope encoded by a polypeptide of a gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION) as an antigene. Such antibodies may in turn be used to detect expressed polypeptides as markers for cancer diagnosis. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzyme's, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

The proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase) . The location of the antibody on the membrane is then detected.

The proteins may be detected by means of immunochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference in its entirety). Methods for preparation of the polypeptides or epitopes thereof include, but are not limited to chemical synthesis, recombinant DNA techniques or isolation from biological samples.

In the final step of the method the diagnosis of the patient is determined, whereby under-expression (of RASSF2A; TFAP2E; HIST1H4K; GSTPi; MME; RARB; NKX3-1; NPAL3; ACVR2A; ARL4C; PDE4D; CARTPT; HIST1 H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); niRNA or polypeptides) is indicative of the presence of cancer. The term under-expression shall be taken to mean expression at a detected level less than a pre-determined cut off which may be selected from the group consisting of the mean, median or an optimised threshold value.

Another aspect of the application discloses a kit for use in diagnosis of cancer in a subject according to the methods of the present invention, comprising: a means for detecting polypeptides of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION). The means for detecting the polypeptides comprise preferably antibodies, antibody derivatives, or antibody fragments. The polypeptides are most preferably detected by means of Western Blotting utilizing a labelled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit comprises: (a) a means for detecting polypeptides of at least one gene selected from the group consisting of RASSF2A; TFAP2E; HIST1 H4K; GSTPi; MME; RARB; NKX3-1 ; NPAL3; ACVR2A; ARL4C; PDE4D; CARTPT; HIST1H2BD; CUL1; MOBKL2B; IFLTDI (KRAS); ANXA2; NFATC3; MN1; KLF8; FGF1 3; NKX2-6; SPG20; DSE/SART2; GJA1 (CX43); SPATA6; MCC; GPR68 (INTRON 1 AND/OR EXON 2 REGION); (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating, packaged in a separate container.

Particular embodiments of the present invention provide a novel application of the analysis of methylation levels and/or patterns within said sequences that enables a precise detection, characterisation and/or treatment of prostate carcinoma. Early detection of cancer is directly linked with disease prognosis, and the disclosed method thereby enables the physician and patient to make better and more informed treatment decisions.

The present invention provides novel uses for the genomic sequences SEQ ID NO: 1-4; 37-65. Additional embodiments provide modified variants of SEQ ID NO: 1-4; 37-65, as well as oligonucleotides and/or PNA-oligomers for analysis of cytosine methylation patterns within SEQ ID NO: 1-4; 37-65 or within the modified variants of SEQ ID NO: 1-4; 37-65.

An objective of the invention comprises analysis of the methylation state of one or more CpG dinucleotides within SEQ ID NO: 53 or SEQ ID NO: 53 and SEQ ID NO: 1-4; 37-52 and 54-65 and sequences complementary thereto.

The disclosure provides treated nucleic acids, derived from genomic SEQ ID NO: 1 -4; 37-65, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. The application discloses a non-naturally occurring modified nucleic acid comprising a sequence of at least 16 contiguous nucleotide bases in length of a sequence selected from the group consisting of SEQ ID NO: 5-20; 66-181. Said nucleic acid may be at least 50, 100, 150, 200, 250 or 500 base pairs in length of a segment of the nucleic acid sequence disclosed in SEQ ID NO: 5-20; 66-181. Exemplified is a nucleic acid molecule that is identical or complementary to all or a portion of the sequences SEQ ID NO: 5-20; 66-181 but not SEQ ID NO: 1-4; 37-65 or other naturally occurring DNA.

Said sequence may comprise at least one CpG, TpA or CpA dinucleotide and sequences complementary thereto. The sequences of SEQ ID NO: 5-20; 66-181 provide non-naturally occurring modified versions of the nucleic acid according to SEQ ID NO: 1-4; 37-65, wherein the modification of each genomic sequence results in the synthesis of a nucleic acid having a sequence that is unique and distinct from said genomic sequence as follows. For each sense strand genomic DNA, e.g., SEQ ID NO: 1 , four converted versions are disclosed. A first version wherein "C" is converted to "T," but "CpG" remains "CpG" (i.e., corresponds to case where, for the sense strand genomic sequence, all "C" residues of CpG dinucleotide sequences are methylated and are thus not converted); a second version discloses the complement of the disclosed genomic DNA sequence (i.e. antisense strand), wherein "C" is converted to "T," but "CpG" remains "CpG" (i.e., corresponds to case where, for all "C" residues of CpG dinucleotide sequences are methylated and are thus not converted). The 'upmethylated' converted sequences of SEQ ID NO: 1-4; 37-65 correspond to SEQ ID NO: 5-12; 66-123. A third chemically converted version of each genomic sequences is provided, wherein "C" is converted to "T" for all "C" residues, including those of "CpG" dinucleotide sequences (i.e., corresponds to case where, for the sense strand genomic sequences, all "C" residues of CpG dinucleotide sequences are unmethylated); a fourth chemically converted version of each sequence, discloses the complement of the disclosed genomic DNA sequence (i.e. antisense strand), wherein "C" is converted to "T" for all "C" residues, including those of "CpG" dinucleotide sequences (i.e., corresponds to case where, for the complement (antisense strand) of each genomic sequence, all "C" residues of CpG dinucleotide sequences are unmethylated). The 'downmethylated' converted sequences of SEQ ID NO: 1-4; 37-65 corresponds to SEQ ID NO: 13-20; 124-181. See Table 1 for further details.

Significantly, heretofore, the nucleic acid sequences and molecules according SEQ ID NO: 5-20; 66-181 were not implicated in or connected with the detection, classification or treatment of cancer.

In an alternative preferred embodiment, the application further discloses oligonucleotides or oligomers suitable for use in the methods of the invention for detecting the cytosine methylation state within genomic or treated (chemically modified) DNA, according to SEQ ID NO: 1-4; 37-65 or SEQ ID NO: 5-20; 66-181. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 5-20; 66-181 and/or sequences complementary thereto, or to a genomic sequence according to SEQ ID NO: 1-4; 37- 65 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 1-4; 37-65 or to the complements thereof. Particularly preferred is a nucleic acid molecule that hybridizes under moderately stringent and/or stringent hybridization conditions to all or a portion of the sequences SEQ ID NO: 5-20; 66-181 but not SEQ ID NO: 1-4; 37-65 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 5-20; 66-181, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 1 -4; 37-65 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.50C and 1.5°C per 1% mismatch.

Examples of inventive oligonucleotides of length X (in nucleotides), as indicated by polynucleotide positions with reference to, e.g., SEQ ID NO: 2, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-I));
where n=1, 2, 3,...(Y-(X-I));
where Y equals the length (nucleotides or base pairs) of SEQ ID NO: 2 (6096);
where X equals the common length (in nucleotides) of each oligonucleotide in the set (e.g., X=20 for a set of consecutively overlapping 20-mers); and
where the number (Z) of consecutively overlapping oligomers of length X for a given SEQ ID NO 1 of length Y is equal to Y- (X-I). For example Z= 6096
   - 19= 6077 for either sense or antisense sets of SEQ ID NO: 2, where X=20.

Preferably, the set is limited to those oligomers that comprise at least one CpG, TpG or CpA dinucleotide.

Examples of inventive 20-mer oligonucleotides include the following set of 2,261 oligomers (and the antisense set complementary thereto), indicated by polynucleotide positions with reference to SEQ ID NO: 1-4; 37-65:
1-20, 2-21 , 3-22, 4-23, 5-24, and 6077- 6096.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO: 2:
1 -25, 2-26, 3-27, 4-28, 5-29, and 6072- 6096.

Preferably, the set is limited to those oligomers that comprise at least one CpG, TpG or CpA dinucleotide.

The present invention encompasses, for each of SEQ ID NO: 1-4; 37-65 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequences corresponding to SEQ ID NO: 1-4; 37-65. Preferred sets of such oligonucleotides or modified oligonucleotides of length X are those consecutively overlapping sets of oligomers corresponding to SEQ ID NO: 1-4; 37-65 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5 '-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to the present application are typically used in 'sets,' which contain at least one oligomer for analysis of each of the CpG dinucleotides of a genomic sequence selected from the group consisting SEQ ID NO: 1-4; 37-65 and sequences complementary thereto, or to the corresponding CpG, TpG or CpA dinucleotide within a sequence of the treated nucleic acids according to SEQ ID NO: 5-20; 66-181 and sequences complementary thereto. However, it is anticipated that for economic or other factors it may be preferable to analyse a limited selection of the CpG dinucleotides within said sequences, and the content of the set of oligonucleotides is altered accordingly.

Therefore, in particular embodiments, the present application discloses a set of at least two (2) oligomers (oligonucleotides and/or PNA-oligomers) that are useful for detecting the cytosine methylation state in treated genomic DNA (SEQ ID NO: 5-20; 66-181), or in genomic DNA (SEQ ID NO: 1-4; 37-65 and sequences complementary thereto). These oligomers may also be named probes and enable diagnosis and detection of prostate cell proliferative disorders, most preferably, prostate carcinoma. The set of oligomers may also be used for detecting single nucleotide polymorphisms (SNPs) in treated genomic DNA (SEQ ID NO: 5-20; 66-181 and sequences complementary thereto), or in genomic DNA (SEQ ID NO: 1 -4; 37-65 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO: 1-4, 37-65; SEQ ID NO: 5-20, 66-181 ; sequences complementary thereto; or segments thereof.

It is anticipated that the oligonucleotides may constitute all or part of an "array" or "DNA chip" (i.e., an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21 , January 1999, and from the literature cited therein). Fluorescently labelled probes are often used for the scanning of immobilized DNA arrays. The simple attachment of Cy 3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital read-out of each molecular species in the mixture).

It is particularly preferred that the oligomers according to the invention are utilised for detecting, or for diagnosing prostate cell proliferative disorders, most preferably, prostate carcinoma.

In the most preferred embodiment of the method, the presence or absence of prostate cell proliferative disorders, most preferably, prostate cancer is determined. This is achieved by analysis of the methylation status of at least one and more preferably a plurality of, target sequence(s) comprising at least one CpG position said sequence comprising, or hybridizing under stringent conditions to at least 16, 50, 100 or 500 contiguous nucleotides of SEQ ID NO: 53 or SEQ ID NO: 53 and a sequence selected from the group consisting SEQ ID NO: 1-4; 37-52 and 54-65 and complements thereof.

Preferably a plurality of target regions (herein also referred to as a "gene panel") are analysed. Preferably target regions of 2, 3 or 4 genes are analysed. In one embodiment of the method said panel comprises the target regions of at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20: DSE/SART2; GJA1 (CX43); SPATA6; MCC; GPR68 (INTRON 1 AND/OR EXON 2 REGION); and/or their promoter or regulatory regions. Preferably said panel comprises a target region of the gene HIST1H4K.

Particularly exemplified are the following combinations of target regions of genes:
HIST1H4K +RASSF2A or HIST1H4K +GSTPi

The present invention further provides a method for ascertaining genetic and/or epigenetic parameters of the genomic sequences according to SEQ ID NO: 1-4; 37-65 within a subject by analysing cytosine methylation and single nucleotide polymorphisms. Said method comprising contacting a nucleic acid comprising at least one genomic sequence selected from the group consisting SEQ ID NO: 1-4; 37-65 in a biological sample obtained from said subject with at least one reagent or a series of reagents, wherein said reagent or series of reagents, distinguishes between methylated and non-methylated CpG dinucleotides within the target nucleic acid(s).

In a preferred embodiment, said method comprises the following steps: In the first step, a sample of the tissue to be analysed is obtained. The source may be any suitable source, such as cell lines, histological slides, biopsies, paraffin-embedded tissue, body fluids, ejaculate, urine, blood plasma, blood serum, whole blood, isolated blood cells, cells isolated from the blood and all possible combinations thereof. It is preferred that said sources of DNA are ejaculate or body fluids selected from the group consisting ejaculate, urine, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample), methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g. ultrafiltration, silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the second step of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/01 1715, which is incorporated by reference in its entirety)^ It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2,5,7,8, -tetramethylchromane 2-carboxylic acid or trihydroxybenzoe acid and derivates thereof, e.g. gallic acid (see: PCT/EP2004/01 1715 which is incorporated by reference in its entirety). The bisulfite conversion is preferably carried out at a reaction temperature between 30°C and 70°C, whereby the temperature is increased to over 85°C for short periods of times during the reaction (see: PCT/EP2004/011715 which is incorporated by reference in its entirety). The bisulfite treated DNA is preferably purified priori to the quantification. This may be conducted by any means known in the art, such as but not limited to ultrafiltration, preferably carried out by means of Microcon™ columns (manufactured by Millipore™). The purification is carried out according to a modified manufacturer's protocol (see: PCT/EP2004/01 1715 which is incorporated by reference in its entirety).

In the third step of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NO: 5-20; 66-181 and sequences complementary thereto.

In an alternate embodiment of the method, the methylation status of pre-selected CpG positions within at least one genomic sequence selected from the group consisting SEQ ID NO: 1-4; 37-65, may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridises to a bisulfite treated CpG dinucleotide. Therefore, the sequence of said primers comprises at least one CpG dinucleotide. MSP primers specific for non-methylated DNA contain a "T" at the position of the C position in the CpG. Preferably, therefore, the base sequence of said primers is required to comprise a sequence having a length of at least 9 nucleotides which hybridises to a treated nucleic acid sequence according to one of SEQ ID NO: 5-20; 66-181 and sequences complementary thereto, wherein the base sequence of said oligomers comprises at least one CpG dinucleotide. A further preferred embodiment of the method comprises the use of blocker oligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes maybe designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5 '-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5 '-3' direction) the blocker - a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said blocking oligonucleotides is required to comprise a sequence having a length of at least 9 nucleotides which hybridises to a treated nucleic acid sequence according to one of SEQ ID NO: 5-20; 66-181 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragment ed manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1 : 147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the fourth step of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analysed by means of oligomer-based methods such as, but not limited to, array technology and probe-based technologies as well as by means of techniques such as sequencing and template directed extension.

In one embodiment of the method, the amplificates synthesised in step three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG, TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence selected from the group consisting SEQ ID NO: 1-4; 37-65, and the equivalent positions within SEQ ID NO: 5-20; 66-181. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; also see United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the fourth step of the method comprises the use of template-directed oligonucleotide extension, such as MS-SNuPE as described by Gonzalgo & Jones, Nucleic Acids Res. 25:2529-2531, 1997.

In yet a further embodiment of the method, the fourth step of the method comprises sequencing and subsequent sequence analysis of the amplificate generated in the third step of the method (Sanger F., et al., Proc Natl Acad Sci USA 74:5463-5467, 1977).

### Best mode

In the most preferred embodiment of the method the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or blocking oligonucleotides, and further wherein
e)e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NO: 5-20; 66-18 and sequences complementary thereto, wherein the base sequence of said oligomers comprise at least one CpG dinucleotide.

Step e) of the method, namely the detection of the specific amplificates indicative of the methylation status of one or more CpG positions of at least one genomic sequence selected from the group consisting SEQ ID NO: 1-4; 37-65 is carried out by means of real-time detection methods as described above.

Additional embodiments of the invention provide a method for the analysis of the methylation status of genomic DNA according to the invention (SEQ ID NO: 1-4; 37-65, and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH (see for example but not limited to US6,605,432 or WO 2006/088978).

In the first step of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. AU clinical sample types comprising neoplastic or potentially neoplastic matter are suitable for use in the present method, preferred are cell lines, histological slides, biopsies, paraffin-embedded tissue, body fluids, ejaculate, urine, blood plasma, blood serum, whole blood, isolated blood cells, cells isolated from the blood and combinations thereof. Body fluids are the preferred source of the DNA; particularly preferred are urine, blood plasma, blood serum, whole blood, isolated blood cells and cells isolated from the blood.

Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of Msel, Bfal, Cspól, Trull, Tvull, Tru9I, Tvu9I, Mael and Xspl. Particularly preferred is the use of two or three such enzymes. Particularly preferred is the use of a combination of Msel, Bfal and Cspól.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the third step, the DNA (or fragments thereof) is then digested with one or more methylation sensitive restriction enzymes. The digestion is carried out such that hydrolysis of the DNA at the restriction site is informative of the methylation status of a specific CpG dinucleotide of at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION).

Preferably a plurality of genes (herein also referred to as a "gene panel") are analysed. Preferably 2, 3 or 4 genes are analysed. In one embodiment of the method said panel comprises at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTDI (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or their promoter or regulatory regions. Preferably said group comprises the gene HIST1H4K.

Particularly exemplified are the following combinations of genes:
HIST1H4K +RASSF2A or HIST1H4K +GSTPi

Preferably, the methylation-specific restriction enzyme is selected from the group consisting of Bsi El , Hga I, HinPl, Hpy99I, Ava I, Bee AI, Bsa HI, Bisl, BstUI, BshI236I, AccII, BstFNI, McrBC, GIaI, Mvnl, HpaII (HapII), Hhal, Acil, Smal, HinPlI, HpyCH4IV, Eagl and mixtures of two or more of the above enzymes. Preferred is a mixture containing the restriction enzymes BstUI, HpaII, HpyCH4IV and HinPlI.

In the fourth step, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from the group consisting SEQ ID NO: 1-4; 37-65, and complements thereof. Preferably said contiguous sequence is at least 16, 20 or 25 nucleotides in length. In an alternative embodiment said primers may be complementary to any adaptors linked to the fragments.

In the fifth step the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 1-4; 37-65, and complements thereof. Preferably said contiguous sequence is at least 16, 20 or 25 nucleotides in length.

Subsequent to the determination of the methylation state or level of the genomic nucleic acids, the presence or absence of prostate cell proliferative disorders, most preferably, prostate carcinoma is deduced based upon the methylation state or level of at least one CpG dinucleotide sequence of SEQ ID NO: 1-4; 37-65, or an average, or a value reflecting an average methylation state of a plurality of CpG dinucleotide sequences of SEQ ID NO: 1-4; 37-65 wherein methylation is associated with the presence of prostate cell proliferative disorders, most preferably, prostate cancer. Wherein said methylation is determined by quantitative means the cut-off point for determining said the presence of methylation is preferably zero (i.e. wherein a sample displays any degree of methylation it is determined as having a methylated status at the analysed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

### Kits

Moreover, an additional aspect of the presentapplication discloses a kit comprising: a means for determining methylation of at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION). The means for determining said methylation comprise preferably a bisulfite-containing reagent; one or a plurality of oligonucleotides consisting whose sequences in each case are identical, are complementary, or hybridise under stringent or highly stringent conditions to a 9 or more preferably 18 base long segment of a sequence selected from SEQ ID NO: 5-20; 66-181 ; and optionally instructions for carrying out and evaluating the described method of methylation analysis. In one embodiment the base sequence of said oligonucleotides comprises at least one CpG, CpA or TpG dinucleotide.

Said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit along the lines of the present invention can also contain only part of the aforementioned components.

The kit may comprise additional bisulfite conversion reagents selected from the group consisting: DNA denaturation buffer; sulfonation buffer; DNA recovery reagents or kits (e.g., precipitation, ultrafiltration, affinity column); desulfonation buffer; and DNA recovery components.

The kit may also contain, packaged in separate containers, a polymerase and a reaction buffer optimised for primer extension mediated by the polymerase, such as PCR. The kit may further comprise means for obtaining a biological sample of the patient. Preferred is a kit, which further comprises a container suitable for containing the means for determining methylation of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1 H2BD; CUL1; MOBKL2B; IFLTD1 (KRAS); ANXA2; NFATC3; MN1; KLF8; FGF 13; NKX2-6; SPG20; DSE/SART2; GJA1 (CX43); SPATA6; MCC;
GPR68 (INTRO 1 AND/OR EXON 2 REGION) in the biological sample of the patient, and most preferably further comprises instructions for use and interpretation of the kit results. In a preferred example the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 5-20; 66-181 ; and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 5-20; 66-181 and sequences complementary thereto; and optionally (d) instructions for use and interpretation of the kit results.

In an alternative example the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 5-20; 66-181; (d) at least one oligonucleotides and/or PNA-oligomer having a length of at least 9 or 16 nucleotides which is identical to or hybridises to a pre-treated nucleic acid sequence according to one of SEQ ID NO: 5-20; 66-181 and sequences complementary thereto; and optionally (e) instructions for use and interpretation of the kit results.

The kit may also contain other components such as buffers or solutions suitable for blocking, washing or coating, packaged in a separate container.

Another aspect of the application discloses a kit for use in determining the presence of and/or diagnosing prostate cell proliferative disorders, most preferably, prostate carcinoma, said kit comprising: a means for measuring the level of transcription of at least one gene selected from the group consisting of RASSF2A; TFAP2E; HIST1 H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); and/or a means for determining methylation of at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1;
MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION).

Typical reagents (e.g., as might be found in a typical COBRA™-based kit) for COBRA™ analysis may include, but are not limited to: PCR primers for at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); restriction enzyme and appropriate buffer: gene-hybridization oligo; control hybridization oligo; kinase labeling kit for oligo probe; and labeled nucleotides. Typical reagents (e.g., as might be found in a typical MethyLight ™ -based kit) for

MethyLight™ analysis may include, but are not limited to: PCR primers for the bisulfite converted sequence of the at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION); bisulfite specific probes (e.g. TaqMan ™ or Lightcycler ™); optimized PCR buffers and deoxynucleotides; and Taq polymerase.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for the bisulfite converted sequence of at least one gene selected from the group consisting of RASSF2A; TFAP2E; FHST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1 ; KLF8; FGF 13; NKX2-6; SPG20; DSE/SART2; GJA1 (CX43); SPATA6; MCC; GPR68 (INTRON 1 AND/OR EXON 2 REGION); reaction buffer (for the Ms-SNuPE reaction); and labelled nucleotides.

Typical reagents (e.g., as might be found in a typical MSP-based kit) for MSP analysis may include, but are not limited to: methylated and unmethylated PCR primers for the bisulfite converted sequence of at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION), optimized PCR buffers and deoxynucleotides, and specific probes.

Moreover, an additional aspect of the present application is an alternative kit comprising a means for determining methylation of at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF 13; NKX2-6; SPG20; DSE/SART2; GJA1 (CX43); SPATA6; MCC; GPR68 (INTRON 1 AND/OR EXON 2 REGION), wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from the group consisting of SEQ ID NO: 1-4; 37-65; and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence selected from the group consisting of SEQ ID NO: 1-4; 37-65.

Said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from the group consisting of SEQ ID NO: 1-4; 37-65.

The kit may comprise additional reagents selected from the group consisting: buffer (e.g. restriction enzyme, PCR, storage or washing buffers); DNA recovery reagents or kits (e.g., precipitation, ultrafiltration, affinity column) and DNA recovery components.

The kit may contain, packaged in separate containers, a polymerase and a reaction buffer optimised for primer extension mediated by the polymerase, such as PCR. In another embodiment of the invention the kit further comprise means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of at least one sequence selected from the group consisting of SEQ ID NO: 1-4; 37-65; and optionally (d) instructions for use and interpretation of the kit results.

In an alternative preferred embodiment the kit may comprise: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from the group consisting of SEQ ID NO: 1-4; 37-65; and optionally (d) instructions for use and interpretation of the kit results.

In an alternative embodiment the kit may comprise: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from the group consisting of SEQ ID NO: 1-4; 37-65; (d) at least one set of oligonucleotides one or a plurality of nucleic acids or peptide nucleic acids which are identical, are complementary, or hybridise under stringent or highly stringent conditions to an at least 9 base long segment of a sequence selected from the group consisting of SEQ ID NO: 1-4; 37-65 and optionally (e) instructions for use and interpretation of the kit results.

The kit may also contain other components such as buffers or solutions suitable for blocking, washing or coating, packaged in a separate container.

The application further discloses a kit for use in providing a diagnosis of the presence of a prostate cell proliferative disorders, most preferably, prostate carcinoma in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF 13; NKX2-6; SPG20; DSE/SART2; GJA1 (CX43); SPATA6; MCC; GPR68 (INTRON 1 AND/OR EXON 2 REGION) and comprises a sequence of at least 15 base pairs in length but no more than 200 bp of a sequence selected from the group consisting of SEQ ID NO: 1-4; 37-65. Preferably said sequence is at least 15 base pairs in length but no more than 80 bp of a sequence selected from the group consisting of SEQ ID NO: 1-4; 37-65. It is further preferred that said sequence is at least 20 base pairs in length but no more than 30 bp of a sequence selected from the group consisting of SEQ ID NO: 1-4; 37-65.

Said test kit preferably further comprises a restriction enzyme component comprising one or a plurality of methylation-sensitive restriction enzymes.

In a further embodiment said test kit is further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The application further discloses a composition of matter useful for detecting, or for diagnosing prostate carcinoma. Said composition comprising at least one nucleic acid 18 base pairs in length of a segment of the nucleic acid sequence disclosed in SEQ ID NO: 5-20; 66-181, and one or more substances taken from the group comprising : 1-5 mmol/l Magnesium Chloride, 100-500 µmol/l dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 5-20; 66-181 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

In further examples said at least one nucleic acid is at least 50, 100, 150, 200, 250 or 500 base pairs in length of a segment of the nucleic acid sequence disclosed in SEQ ID NO: 5-20; 66-181.

Subject matter of the invention is a method for detecting prostate cell proliferative disorders in a subject comprising determining the expression levels of the KLF8 gene or the KLF8 gene and at least one gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; FGF13; NKX2-6; SPG20; DSE/SART2; GJA1 (CX43); SPATA6; MCC; GPR68 (INTRON 1 AND/OR EXON 2 REGION) in a biological sample isolated from said subject wherein underexpression and/or CpG methylation is indicative of the presence of said disorder.

A preferred embodiment further comprises determining the expression level of PSA in said biological sample isolated from said subject wherein underexpression and/or CpG methylation is indicative of the presence of said disorder.

According to a preferred embodiment, said expression level is determined by detecting the presence, absence or level of mRNA transcribed from said gene.

According to a preferred embodiment, said expression level is determined by detecting the presence, absence or level of a polypeptide encoded by said gene or sequence thereof.

According to a preferred embodiment, said polypeptide is detected by one or more means selected from the group comprising western blot analysis, chromatography, immunoassay, ELISA immunoassay, radioimmunoassay, antibody and combinations thereof.

According to a preferred embodiment, said expression is determined by detecting the presence or absence of CpG methylation within said gene, wherein the presence of methylation indicates the presence of a carcinoma.

Subject matter of the invention is further a method for detecting prostate cell proliferative disorders in a subject, comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of a sequence selected from the group consisting of SEQ ID NO: 1-4; 37-65 respectively, wherein said contiguous nucleotides comprise at least one CpG dinucleotide sequence, and whereby detecting carcinoma is, at least in part, afforded.

A preferred embodiment comprises further determining the expression level of PSA in said biological sample isolated from said subject wherein underexpression and/or CpG methylation is indicative of the presence of said disorder.

Subject matter of the invention is further a method for detecting prostate cell proliferative disorders, comprising:
a) extracting or otherwise isolating genomic DNA from a biological sample obtained from the subject; b) treating the genomic DNA of a), or a fragment thereof, with one or more reagents to convert cytosine bases that are unmethylated in the 5-position thereof to uracil or to another base that is detectably dissimilar to cytosine in terms of hybridization properties;
c) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 5-20; 66-181, and complements thereof, wherein the treated genomic DNA or the fragment thereof is either amplified to produce at least one amplificate, or is not amplified; and
d) determining, based on a presence or absence of, or on a property of said amplificate, the methylation state or level of at least one CpG dinucleotide of a sequence selected from the group consisting SEQ ID NO: 1-4; 37-65, or an average, or a value reflecting an average methylation state or level of a plurality of CpG dinucleotides of a sequence selected from the group consisting of SEQ ID NO: 1-4; 37-65, whereby at least one of detecting and diagnosing cancer is, at least in part, afforded.

A preferred embodiment comprises further determining the expression level of PSA in said biological sample isolated from said subject wherein underexpression and/or CpG methylation is indicative of the presence of said disorder.

According to a preferred embodiment, treating the genomic DNA, or the fragment thereof in b), comprises use of a reagent selected from the group comprising of bisulfite, hydrogen sulfite, disulfite, and combinations thereof.

According to a preferred embodiment, contacting or amplifying in c) comprises use of at least one method selected from the group comprising: use of a heat-resistant DNA polymerase as the amplification enzyme; use of a polymerase lacking 5'-3' exonuclease activity; use of a polymerase chain reaction (PCR); generation of an amplificate nucleic acid molecule carrying a detectable label.

According to a preferred embodiment, the biological sample obtained from the subject is selected from the group comprising cell lines, histological slides, biopsies, paraffin-embedded tissue, body fluids, ejaculate, ejaculate, urine, blood plasma, blood serum, whole blood, isolated blood cells, cells isolated from the blood and combinations thereof.

A preferred embodiment comprises further in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 5-20; 66-181, and complements thereof, wherein said nucleic acid molecule or peptide nucleic acid molecule suppresses amplification of the nucleic acid to which it is hybridized.

According to a preferred embodiment, determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NO: 5-20; 66-181 , and complements thereof.

According to a preferred embodiment, at least one such hybridizing nucleic acid molecule or peptide nucleic acid molecule is bound to a solid phase.

A preferred embodiment comprises further extending at least one such hybridized nucleic acid molecule by at least one nucleotide base.

According to a preferred embodiment, determining in d), comprises sequencing of the amplificate.

According to a preferred embodiment, contacting or amplifying in c), comprises use of methylation-specific primers.

Subject matter of the invention is further a method for detecting prostate cell proliferative disorders, comprising:
a) extracting or otherwise isolating genomic DNA from a biological sample obtained from the subject; b) digesting the genomic DNA of a), or a fragment thereof, with one or more methylation sensitive restriction enzymes;
c) contacting the DNA restriction enzyme digest of b), with an amplification enzyme and at least two primers suitable for the amplification of a sequence comprising at least one CpG dinucleotide of a sequence selected from the group consisting of SEQ ID NO: 1-4, 37-65;
d) determining, based on a presence or absence of an amplificate the methylation state or level of at least one CpG dinucleotide of a sequence selected from the group consisting SEQ ID NO: 1-4, 37-65, whereby at least one of detecting and classifying cancer is, at least in part, afforded.

A preferred embodiment comprises further determining the expression level of PSA in said biological sample isolated from said subject wherein underexpression and/or CpG methylation is indicative of the presence of said disorder.

According to a preferred embodiment, the presence or absence of an amplificate is determined by means of hybridization to at least one nucleic acid or peptide nucleic acid which is identical, complementary, or hybridizes under stringent or highly stringent conditions to an at least 16 base long segment of a sequence selected from the group consisting of SEQ ID NO: 1-4; 37-65.

Subject matter of the invention is further a treated nucleic acid for use in the detection of prostate cell proliferative disorders derived from genomic SEQ ID NO: 1-4, 37-65 wherein the treatment is suitable to convert at least one unmethylated cytosine base of the genomic DNA sequence to uracil or another base that is detectably dissimilar to cytosine in terms of hybridization.

The applicationfurther discloses a nucleic acid for use in the detection of prostate cell proliferative disorders, comprising at least 9 or at least 16 contiguous nucleotides of a treated genomic DNA sequence selected from the group consisting of SEQ ID NO: 5-20, 66-181, and sequences complementary thereto, wherein the treatment is suitable to convert at least one unmethylated cytosine base of the genomic DNA sequence selected from the group consisting of SEQ ID NO: 1-4, 37-65 to uracil or another base that is detectably dissimilar to cytosine in terms of hybridization.

The applicationfurther discloses a nucleic acid for use in the detection of prostate cell proliferative disorders, comprising at least 50 contiguous nucleotides of a DNA sequence selected from the group consisting of SEQ ID NO: 5-20, 66-181, and sequences complementary thereto.

According to a preferred embodiment, the contiguous base sequence comprises at least one CpG, TpG or CpA dinucleotide sequence.

Subject matter of the invention is further a nucleic acid for use in the detection of prostate cell proliferative disorders, comprising at least 9 or at least 16 contiguous nucleotides of a treated genomic DNA sequence selected from the group consisting of SEQ ID NO: 5-20, 66-181 and sequences complementary thereto as a diagnostic means.

The applicationfurther discloses a kit suitable for determining the expression level by detecting the presence, absence or level of mRNA transcribed from a gene selected from the group consisting of RASSF2A; TFAP2E; HIST1H4K; GSTPi; MME; RARB; NKX3-1 ; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF 13; NKX2-6; SPG20; DSE/SART2; GJA1 (CX43); SPATA6; MCC; GPR68 (INTRON 1 AND/OR EXON 2 REGION), comprising
a) a plurality of oligonucleotides or polynucleotides able to hybridise under stringent or moderately stringent conditions to the transcription products of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION);
(b) a container suitable for containing the oligonucleotides or polynucleotides and a biological sample of the patient comprising the transcription products wherein the oligonucleotides or polynucleotides can hybridise under stringent or moderately stringent conditions to the transcription products;
(c) means to detect the hybridisation of (b); and optionally
(d) instructions for use and interpretation of the kit results.

The application further discloses a kit suitable for determining the expression level by detecting the presence, absence or level of a polypeptide encoded by a gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF1 3; NKX2-6; SPG20; DSE/SART2; GJA1 (CX43); SPATA6; MCC; GPR68 (INTRON 1 AND/OR EXON 2 REGION), or sequence thereof, comprising
(a) a means for detecting polypeptides of a gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; M0BKL2B; IFLTD1 (KRAS); ANXA2; NFATC3; MN1; KLF8; FGF13; NKX2-6; SPG20; DSE/SART2; GJA1 (CX43); SPATA6; MCC; GPR68 (INTRON 1 AND/OR EXON 2 REGION);
(b) a container suitable for containing the said means and the biological sample of the patient comprising the polypeptides wherein the means can form complexes with the polypeptides; (c) a means to detect the complexes of (b).

The application further discloses a kit suitable for determing the expression level by detecting the presence or absence of CpG methylation within a gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; KLF8; FGF1 3; NKX2-6; SPG20; DSE/SART2; GJA1 (CX43); SPATA6; MCC; GPR68 (INTRON 1 AND/OR EXON 2 REGION), wherein the presence of methylation indicates the presence of a carcinoma, comprising
(a) a bisulfite reagent;
(b) a container suitable for containing the said bisulfite reagent and the biological sample of the patient;
(c) at least one set of oligonucleotides containing two oligonucleotides whose sequences in each case are identical , are complementary, or hybridize under stringent or highly stringent conditions to a 9 or more preferably 18 base long segment of a sequence selected from SEQ ID NO: 5-20, 66-181.

Subject matter of the invention is further a kit suitable for determing the expression level by detecting the presence or absence of CpG methylation within a gene selected from the group consisting of RASSF2A; TFAP2E; HIST1H4K; GSTPi; MME; RARB; NKX3-1; NPAL3; ACVR2A; ARL4C;
PDE4D; CARTPT; HIST1H2BD; CUL1; M0BKL2B; IFLTD1 (KRAS); ANXA2; NFATC3; MN1; KLF8; FGF 13; NKX2-6; SPG20; DSE/SART2; GJA1 (CX43); SPATA6; MCC; GPR68 (INTRON 1 AND/OR EXON 2 REGION), wherein the presence of methylation indicates the presence of a carcinoma, comprising
(a) a methylation sensitive restriction enzyme reagent;
(b) a container suitable for containing the said reagent and the biological sample of the patient;
(c) at least one set of oligonucleotides one or a plurality of nucleic acids or peptide nucleic acids which are identical, are complementary, or hybridize under stringent or highly stringent conditions to an at least 9 base long segment of a sequence selected from the group consisting of SEQ ID NO: 1-4, 37-65; and optionally
(d) instructions for use and interpretation of the kit results.

Subject matter of the invention is further the use of a method of the invention, an inventive nucleic acid and/or a kit of the invention in the diagnosis and/or detection of prostate cell proliferative disorders.

While the present invention has been described with specificity in accordance with certain of its preferred embodiments, the following examples serve only to illustrate the invention and are not intended to limit the invention within the principles and scope of the broadest interpretations and equivalent configurations thereof.

### Example 1

The aim of the present study was to determine the feasibility of measuring DNA methylation markers for prostate cancer (hereinafter also referred to as PCa) in remote body fluids. In this process a high quality workflow flow for urine was utilized, candidate markers were analyzed by HeavyMethyl™ (HM) technology (Cottrell et al., Nucleic Acids Res. 2004 Jan 13;32(1):elO.) and it was demonstrated that PCa sheds DNA that can be detected by means of methylation analysis in both plasma and urine with high sensitivity. It was thus established that the analyzed markers were suitable for the development of a screening test for PCa based on DNA methylation analysis.

### Study Objectives

The purpose of the present study was to conduct an investigation into whether DNA methylation markers of PCa can be measured in a remote body fluid. The study was designed to identify the optimal analyte for such a test and to generate specificity and analytical performance data for marker candidates.

### Candidate markers and location of assays

The markers RASSF2 and TFAP2E were identified on the basis of their methylation in prostate cancer tissues, as determined in a preliminary study (not described herein). The markers GSTPi and HIST1H4K had been previously identified in a study by the applicant as published in patent application WO 2005/054517.

Methylation analysis was performed by means of the HeavyMethyl™. Isolated genomic is bisulfite treated to convert non-methylated cytosines to uracil, wherein methylated cyctosines are conserved. Fragments of the bisulfite treated DNA comprising potentially methylated CpG dinucleotides are then amplified by means of PCR. The primers do not cover any potentially methylated cytosine positions (i.e. do not hybridise to genomic CpG dinucleotides). Amplification of fragments comprising unmethylated CpG dinucleotides is suppressed by means of a blocking oligonucleotide that hybridises to TG dinucleotides. Accordingly only DNA that was methylated in the genomic sample is amplified. Amplificate fragments are detected by means of detectably labelled probes suitable for use in PCR reactions such as RealTime detection probes. Assay primer and probes are provided in the accompanying sequence listing as according to Table 2.

### GSTPi

Chromosomal Location: 11q13
Nearby Gene(s): GSTPi HM forward primer is just upstream of exon 1 and the reverse primer is just downstream of exon I of GSTP1.
RASSF2A
Chromosomal Location: 20pter-p12.1
Nearby Gene(s): w/i the CpG island of intron 1 of the v. 1 transcript of RASSF2.
HIST1H4K
Chromosomal Location: 6p22-21.3
Nearby Gene(s): overlaps intronless H1ST1H4K
TFAP2E
Chromosomal Location: 1p34.3
Nearby Gene(s): w/i intron 3 of TFAP2E (∼11 kb downstream of txn start) and ~20 kb upstream of KIAA0319L txn start (PKD-1 like gene).

### Tissue Study

Assays were initially tested in normal tissues, NAT and PCa. The marker candidates that were analyzed in the HM tissue test were all very specific for normal blood and normal prostate tissue. In contrast to previous studies it was observed that DNA from prostate normal adjacent tumor (NAT) is nearly as methylated as prostate tumor DNA. NAT (which may contain BPH) is clearly distinct from BPH tissue that has been derived from non-prostate-tumor-bearing patients without elevated PSA (the origin of many BPH tissue samples in the MSP tissue test). In fact, there is evidence in the literature that GSTP1 in NAT is methylated (Hanson et al., 2006).

Performance of the markers in normal + BPH as compared to PCa is provided in Table 3 and Figure 1.

### Remote analyte analysis

In order to maximize the analyte equivalent in real-time PCR assays (1.5 ml equivalents), the maximum number of assays in the study was capped at four, with each assay run in duplicate for each sample.

### Sample collection

For this study, the inventors collected matched plasma and urine from a total of 191 men, including 91 males with biopsy-confirmed prostate cancer, 51 males with no cancer detected by biopsy (subsequently diagnosed with BPH), and 50 young asymptomatic males. In all analyses, the positive class is comprised of the PCa samples. In designing the present study, the definition of the negative class was an issue as there is no detection method that excludes presence of PCa with 100% certainty. Biopsy has a false negative diagnosis rate of at least 10% (Djavan et al., 2000; Mian et al., 2002; Gupta et al., 2005; Hanley et al., 2006) while PSA measurement is prone to both false negatives and false positives. Because the primary objective of the study was to demonstrate the feasibility of measuring methylated markers of PCa in a remote body fluid, the inventors focused on a negative class that minimized the probability of false positives. Consequently, young asymptomatic males were chosen as the "true" negative class. It was reasoned that young asymptomatic males with no family history of prostate cancer should be truly negative for PCa. Because one embodiment of the PCa test is as a diagnostic follow-on to PSA, the inventors also included a second negative class of biopsy negative, BPH samples. A potentially confounding factor in this class is the likely presence of false negative biopsies. In five PCa cases, only a plasma sample was collected and in ten additional cases only a urine sample was collected. The samples were collected at multiple sites. The urine was collected after a prostatic massage, both plasma and urine samples were obtained before any treatment for PCa. Inclusion and exclusion criteria were designed to ensure that the patients analyzed reflect the potential patients who would use PCa screening tests.

The following inclusion and exclusion criteria applied to the patients undergoing biopsy:
Inclusion criteria:
   - Indication for biopsy (elevated PSA and/or suspicious DRE)
   - Biopsy scheduled within 1 week after sample collection
   - Age 40-80

### Exclusion criteria:

Any prior treatment for prostate cancer
History of cancer or serious illness in the past 5 years
Symptoms of urinary tract infection

The following criteria applied to the asymptomatic men of the control group:
Inclusion criteria:
   - Male
   - Age 18-30

### Exclusion criteria:

Any prior treatment for or symptoms of prostate cancer or prostate disease
History of cancer or serious illness in the past 5 years
Symptoms of urinary tract infection
Patient data and tumor characteristics
The Gleason score (where appropriate) of the patient samples are listed Table 4. The mean PSA values for the prostate cancer, HGPIN and biopsy negative samples were 18.2 ± 33.1, 7.0 ± 3.0 and 8.8 ± 5.2 respectively. The prostate cancer, HGPIN and biopsy negative classes were diagnosed after sample collection via prostate biopsy. The mean number of biopsy cores for all sample classes was 8, although there was some variation between providers.

DNA extraction and bisulfite treatment was carried out according to standardised protocols. For each assay, 1.5 ml analyte equivalent was run in duplicate.

### Marker Performance, General Considerations

The initial objective of the study was to develop a panel of markers targeted as a diagnostic follow-on to PSA tests of 2.5 ng/ml or more for men over 50 years of age to discriminate prostate cancer from non-cancerous conditions. Such a test could be further expanded as a more specific prostate cancer screening test that would compete with PSA testing because of superior performance. In the present study the inventors analyzed the data in two different ways: (i) the inventors used prostate cancer and biopsy-negative samples to assess markers performance in the follow-on to PSA test (diagnostic application) and (ii) the inventors used prostate cancer and all the non-cancer (biopsy-negative and asymptomatic) samples to measure markers performance in screening test (screening application). The inventors report marker performance for plasma and urine separately, the inventors also provide data analysis for individual markers and marker panels. All data are reported as logmean raw mathylation values.

As a primary screening test, the marker panel would preferably identify PCa in men over age 50 years with improved specificity relative to PSA. All screening application analyses use the PCa samples as the positive class. For the purposes of the present study, the inventors analyzed data for the screening application with two alternative negative classes. The first negative class analyzed the 50 young asymptomatic males with minimal likelihood of undetected PCa. While this negative class represents a "true" test negative, it is not age-matched to the target PCa screening population and does not include any likely false positive classes, e.g. BPH. Therefore, the inventors performed a second analysis in which all 50 asymptomatic young controls and all 51 biopsy negative controls were analyzed as a 101 sample size negative class.

On average, approximately 20,000,000 PSA tests are performed every year in the US with only approximately 1,000,000 cases moving forward to biopsy (of which approximately 750,000 biopsies are unnecessary). Therefore, less than 5% of individuals that are currently screened by PSA fall in the negative class that is represented by elevated-PSA-BPH-positive whereas as the vast majority of the target screening population fall into the PSA-low negative class. Whereas the negative class of only asymptomatic young males may represent an overestimation of the discriminatory capacity of our markers, the combined negative class of asymptomatic young males plus age-matched biopsy negative males may represent an underestimation of the discriminatory capacity of our markers.

Sensitivity and specificity of individual (single) markers tested by real-time PCR in post-prostatic massage urine from prostate cancer patients vs. biopsy negative patients and asymptomatic control individualsis shown in Table 5. Figure 2 shows the HM real-time PCR assays of post-prostatic massage urine of PCa and negative class I (asymptomatic individuals). Figure 3 shows the HM real-time PCR assays of post-prostatic massage urine of PCa and negative class II (asymptomatic plus biopsy negative individuals).

Sensitivity and specificity of individual (single) markers tested by real-time PCR in plasma from prostate cancer patients vs. biopsy negative patients and asymptomatic control individualsis shown in Table 6. Figure 4 shows the HM real-time PCR assays of plasma of PCa and negative class I (asymptomatic individuals). Figure 5 shows the HM real-time PCR assays of plasma of PCa and negative class II (asymptomatic plus biopsy negative individuals). Figure 6 shows the performance of the biomarker assays for discrimination of PCa patients from asymptomatic young males in both urine and plasma. Figure 7 shows the performance of the biomarker assays for discrimination of PCa from biopsy negative patients in both urine and plasma.

As illustrated in Table 7, in all negative class comparisons and for all markers, urine was the more sensitive analyte.

### Correlation of markers with Gleason score

Increasing amounts of methylated marker DNA correlated with increasing Gleason score for all markers in plasma. This was true for samples with high amounts of methylated marker DNA in urine (see especially markers TFAP2E and RASSF2A), but in general the correlation was less strong in DNA from urine than in DNA from plasma. PSA as a marker of PCa in patients with elevated PSA (> 4 ng/ml) also correlated with increasing Gleason score.
Performance of screening marker panels to distinguish PCa from negative class I (asymptomatic males) in urine is provided in Table 8.
Performance of screening marker panels to distinguish PCa from negative class II (asymptomatic males plus biopsy negative) in urine is provided in Table 9.
Performance of screening marker panels to distinguish PCa from negative class I (asymptomatic males) in plasma is provided in Table 10.
Performance of screening marker panels to distinguish PCa from negative class II (asymptomatic males plus biopsy negative) in plasma is provided in in Table 11.

### Marker performance in diagnostic application: Follow-on to PSA

As a diagnostic follow-on to PSA testing, the markers would preferably identify PCa in men over age 50 years who have been classified as high-risk individuals due to elevated PSA (> 2.5 ng/ml). This is a distinct application and analysis and requires increased discrimination as compared to the screening test. False positives in this application arise from the elevated PSA, biopsy negative BPH class. Again, the PCa samples represent the positive class. For the purposes of a diagnostic follow-on application, the inventors analyzed the data using a single negative class comprised of the 51 biopsy negative samples. AUC of markers tested by real-time PCR in post-prostatic massage urine and plasma from prostate cancer patients and biopsy negative patients is provided in Table 12.
From said table it can be seen that for all methylation markers analyzed, urine was the more sensitive analyte. For total. PSA (treated here as an additional marker to determine if there is any further information provided past the > 4 ng/ml indication for biopsy), there was no difference in sensitivity between urine and plasma.

### Performance of marker panels

In order to provide improved accuracy, combinations of markers were assessed both qualitatively and quantitatively.
Table 13 provides the performance of diagnostic marker panels to distinguish PCa from biopsy negative in urine.
Table 14 provides the performance of diagnostic marker panels to distinguish PCa from biopsy negative in plasma.

### Discussion

The study was conducted on plasma and/or urine samples collected from 91 PCa patients, 51 biopsy-negative patients (diagnosed with BPH) and 50 young asymptomatic males. HM™ real-time PCR assays were used to measure DNA methylation of the candidate markers. The amount of methylated marker DNA was correlated with PCa in both plasma and urine, with urine DNA showing greater sensitivity. As a screening test (discrimination of PCa cancer from asymptomatic controls using urine analyte), anchor marker candidates GSTPi, RASSF2A, HIST1H4K and TFAP2E have 63%, 74%, 69% and 47% sensitivity at 96% specificity respectively. As a diagnostic follow-on to PSA test (discrimination of PCa from biopsy negative controls, all with elevated PSA), the markers have 23%, 18%, 28% & 23% sensitivity at 95% specificity, respectively. A quantitative screening panel of markers RASSF2A and HIST1H4K yielded 94% sensitivity at 88% specificity against asymptomatic individuals. A quantitative diagnostic panel of markers GSTPi and PSA yielded 83% sensitivity at 45% specificity. The performance of these marker compares well with the performance of PSA (18% sensitivity at 98% specificity for men < 60 years and 19% sensitivity at 94% specificity for men > 60 years) in the screening population (Punglia et al., 2003). Methylation of all markers correlated well with Gleason score in plasma DNA, but the correlation was less strong in urine DNA.

### Conclusions

At the completion of the present investigation, it was demonstrated that prostate cancer biomarkers based on methylated DNA can be measured in plasma and urine, with urine DNA showing greater sensitivity than plasma. Additionally, DNA methylation markers that discriminate PCa patients from asymptomatic controls and those with benign prostatic hyperplasia (BPH) were identified. The major conclusions of the present study are as follows:
1. Methylated markers of prostate cancer can be measured in both plasma and urine from PCa patients.
2. Identification of markers that discriminate PCa patients from those without PCa.

### Example 2

The aim of the present study was to determine the sensitivity and specificity of DNA methylation markers for prostate cancer (hereinafter also referred to as PCa) in tissues. In this study, 84 PCa, 34 BPH and 35 normal prostate tissues were analyzed using high sensitivity real-time PCR assays. It was thus established that the analyzed markers were suitable for the development of a screening test for PCa based on DNA methylation analysis.

### Study Objectives

The purpose of the present study was to validate on an independent sample set the DNA methylation markers of PCa identified by our microarray discovery technology. The study was designed to generate specificity and analytical performance data for the marker candidates.

Introduction: A PCa screening biomarker with the diagnostic ability to discriminate PCa from benign prostatic hyperplasia (BPH) in patients with elevated PSA would offer a valuable tool for the public health management of PCa. Aberrant DNA methylation occurs early in tumorigenesis, is stable, and can be assayed in tissues and body fluids, making targets of aberrant DNA methylation attractive biomarker candidates. The inventors have previously demonstrated that hypermethylation of GSTP1 can be detected in urine from PCa patients and serves as a sensitive marker for early identification of PCa. The inventors have now conducted a genome-wide screen to identify markers that augment the specificity of remote sample PCa detection in a diagnostic setting.

Methods: Custom Affymetrix microarrays with >50,000 features were used to explore methylation differences between PCa (n=20), BPH (n=17) and age-matched normal (n=12) tissues. Markers with >25% median methylation in bladder cancer were discarded. The markers were ranked by p statistic, median % PCa methylation and median % BPH methylation. Top candidates were chosen for real-time PCR assay development and validated using PCa (n=84), BPH (n=34) and age-matched normal (n=35) tissues.

Results: Comparison yielded a large number of statistically significant markers after Bonferroni correction: 145 markers for PCa vs. BPH and normal, 148 markers for PCa vs. normal, 121 markers for PCa vs. BPH, and 61 markers for PCa vs. BPH associated with PSA values of > 4ng/ml. The top 38 markers were chosen for real-time PCR assay development. Results of real-time PCR analysis on prostate tissue (Table 16) validated the markers and revealed 4 marker categories: high sensitivity and high specificity for PCa, high specificity but low sensitivity due to low median PMR in PCa, high specificity but low sensitivity due to background methylation in BPH and finally markers that may correlate with features of aggressive PCa. Performance results for selected markers are shown in Figures 8-20.

### Conclusions

At the completion of the present investigation, it was demonstrated that the inventors identified prostate cancer biomarkers based on methylated DNA that are highly specific and sensitive for PCa. Additionally, at least one DNA methylation marker that discriminates high Gleason score PCa patients (Gleason 8-10) from benign tissues was identified (GPR68, see Figure 10). The major conclusions of the present study are as follows:
1. The inventors have identified several previously undescribed DNA methylation markers of PCa.
2. Several of these markers show performance at least equivalent to GSTP1 for discriminating PCa tissues from BPH tissues.
3. The inventors have identified marker(s) that may distinguish pathologically aggressive PCa from indolent PCa.

**Table 1: Genes and sequences according to the present invention**

| Gene | Genomics SEQ ID NO: | Methylated bisulfite converted sense strand | Methylated bisulfite converted antsense strand | Unmethylated bisulfite converted sense strand | Unmethylated bisulfite converted antisense strand |
|---|---|---|---|---|---|
| RASSF2A | 1 | 5 | 6 | 13 | 14 |
| TFAP2E | 2 | 7 | 8 | 15 | 16 |
| HIST1H4K | 3 | 9 | 10 | 17 | 18 |
| GSTPi | 4 | 11 | 12 | 19 | 20 |
| MME | 37 | 66 | 67 | 124 | 125 |
| RARB | 38 | 68 | 69 | 126 | 127 |
| NKX3-1 | 39 | 70 | 71 | 128 | 129 |
| NPAL3 | 40 | 72 | 73 | 130 | 131 |
| ACVR2A | 41 | 74 | 75 | 132 | 133 |
| ARL4C | 42 | 76 | 77 | 134 | 135 |
| PDE4D | 43 | 78 | 79 | 136 | 137 |
| CARTPT | 44 | 80 | 81 | 138 | 139 |
| HIST1H2BD | 45 | 82 | 83 | 140 | 141 |
| CUL1 | 46 | 84 | 85 | 142 | 143 |
| MOBKL2B | 47 | 86 | 87 | 144 | 145 |
| IFLTD1; KRAS 48 | 48 | 88 | 89 | 146 | 147 |
| ANXA2 | 49 | 90 | 91 | 148 | 149 |
| NFATC3 | 50 | 92 | 93 | 150 | 151 |
| NFATC3 | 51 | 94 | 95 | 152 | 153 |
| MN1 | 52 | 96 | 97 | 154 | 155 |
| KLF8 | 53 | 98 | 99 | 156 | 157 |
| FGF13 | 54 | 100 | 101 | 158 | 159 |
| NKX2-6 | 55 | 102 | 103 | 160 | 161 |
| SPG20 | 56 | 104 | 105 | 162 | 163 |
| DSE/SART2 | 57 | 106 | 107 | 164 | 165 |
| GJA1 (aka CX43) | 58 | 108 | 109 | 166 | 167 |
| GJA1 (aka CX43) | 59 | 110 | 111 | 168 | 169 |
| SPARTA6 | 60 | 112 | 113 | 170 | 171 |
| MCC | 61 | 114 | 115 | 172 | 173 |
| GPR68 (Intron 1 region) | 62 | 116 | 117 | 174 | 175 |

**Table 2:Assay components according to Example 1**

| Gene | Forward Primer | Reverse Primer | Blocker | Detection Oligo |
|---|---|---|---|---|
| GSTPi | 21 | 22 | 23 | 24 |
| HIST1H4K | 25 | 26 | 27 | 28 |
| RASSF2A | 29 | 30 | 31 | 32 |
| TFAP2E | 34 | 35 | 36 | 37 |

**Table 3: Performance analysis of markers (normal plus BPH vs. PCa) in tissue test according to Example 1.**

| Marker | AUC | Sensitivity | Specificity |
|---|---|---|---|
| GSTPi | 0.90 | 0.83 | 0.91 |
| HIST1H4K | 0.91 | 0.83 | 0.91 |
| RASSF2A | 0.93 | 0.75 | 0.91 |
| TFAP2E | NA | NA | NA |

**Table 4: Remote samples according to Example 1.**

| Sample type | No. of samples |
|---|---|
| *Prostate Cancer - Gleason Score* | |
| 4 | 1 |
| 5 | 5 |
| 6 | 33 |
| 7 | 39 |
| 8 | 8 |
| 9 | 4 |
| No score available | 1 |
| Total *Prostate Cancer:* | 91 |
| *Biopsy Negative* | 51 |
| *Asymptomatic Control* | 50 |

**Table 5: Sensitivity and specificity of individual markers tested by real-time PCR in post-prostatic massage urine from prostate cancer patients, biopsy negative patients and asymptomatic control individuals.**

| Marker | Negative Class I: | | | Negative Class II: | | |
|---|---|---|---|---|---|---|
| | Asymptomatic | | | Asymptomatic + Biopsy (-) | | |
| | AUC | Sens / Spec | Wilcoxon p value | AUC | Sens / Spec | Wilcoxon p value |
| GSTPi | 0.89 | 0.63 / 0.96 | 0 | 0.79 | 0.31 / 0.96 | 0 |
| RASSF2A | 0.90 | 0.74 / 0.96 | 0 | 0.79 | 0.24 / 0.96 | 0 |
| HIST1H4K | 0.91 | 0.69 / 0.96 | 0 | 0.77 | 0.36 / 0.96 | 0 |
| TFAP2E | 0.86 | 0.47 / 0.96 | 0 | 0.77 | 0.27 / 0.96 | 0 |

**Table 6: Sensitivity and specificity of individual markers tested by real-time PCR in plasma from prostate cancer patients, biopsy negative patients and asymptomatic control individuals.**

| Marker | Negative Class I: | | | Negative Class II: | | |
|---|---|---|---|---|---|---|
| | Asymptomatic | | | Asymptomatic + Biopsy (-) | | |
| | AUC | Sens / Spec | Wilcoxon p value | AUC | Sens / Spec | Wilcoxon p value |
| GSTPi/ GSTP1 | 0.61 | 0.17 / 0.96 | 0.0063 | 0.58 | 0.17 / 0.95 | 0.0183 |
| RASSF2A | 0.68 | 0.37 / 1.00 | 0 | 0.64 | 0.20 / 0.95 | 0 |
| HIST1H4K | 0.64 | 0.26 / 0.96 | 5e⁻⁰⁴ | 0.56 | 0.16 / 0.95 | 0.0572 |
| TFAP2E | 0.61 | 0.22/1.00 | 4e⁻⁰⁴ | 0.56 | 0.09 / 0.95 | 0.0128 |

**Table 7**

| Marker | Negative Class I: | | Negative Class II: | |
|---|---|---|---|---|
| | Asymptomatic | | Asymptomatic + Biopsy (-) | |
| | Urine AUC | Plasma AUC | Urine AUC | Plasma AUC |
| GSTPi/ GSTP1 | 0.89 | 0.61 | 0.69 | 0.55 |
| RASSF2A | 0.90 | 0.68 | 0.66 | 0.60 |
| HIST1H4K | 0.91 | 0.64 | 0.64 | 0.50 |
| TFAP2E | 0.86 | 0.61 | 0.65 | 0.52 |

**Table 8: Performance of screening marker panels to distinguish PCa from negative class I (asymptomatic males) in urine**

| Marker Panel | % Sens PCa | % Spec Asymptomatic |
|---|---|---|
| Quantitative Single Markers: | | |
| RASSF2A | 74 | 96 |
| HIST1H4K | 69 | 96 |
| GSTPi | 63 | 96 |
| TFAP2E | 46 | 100 |

| Qualitative Panels: | | |
|---|---|---|
| GSTPi+HIST1H4K | 79 | 98 |
| RASSF2A+HIST1H4K | 94 | 88 |

| Quantitative Panels: | | |
|---|---|---|
| RASSF2A+HIST1H4K | 94 | 88 |
| quadSVM (all markers, no PSA) | 79 | 98 |

**Table 9**

| Marker Panel | % Sens PCa | % Spec Asymptomatic + Biopsy (-) |
|---|---|---|
| Quantitative Single Markers: | | |
| RASSF2A | 74 | 76 |
| HIST1H4K | 69 | 68 |
| GSTPi | 63 | 80 |
| TFAP2E | 46 | 88 |

| Qualitative Panels: | | |
|---|---|---|
| GSTPi+HIST1H4K | 79 | 72 |
| RASSF2A+HIST1H4K | 94 | 54 |

| Quantitative Panels: | | |
|---|---|---|
| RASSF2A+HIST1H4K | 94 | 58 |
| quadSVM (all markers, no PSA) | 79 | 76 |

**Table 10: Performance of screening marker panels to distinguish PCa from negative class I (asymptomatic males) in plasma**

| Marker Panel | % Sens PCa | % Spec Asymptomatic |
|---|---|---|
| Quantitative Single Markers: | | |
| RASSF2A | 37 | 100 |
| HIST1H4K | 26 | 96 |
| GSTPi | 17 | 94 |
| TFAP2E | 22 | 100 |
| Qualitative Panels: | | |
| RASSF2A+HIST1H4K | 41 | 98 |
| Quantitative Panels: | | |
| RASSF2A+TFAP2E (TFAP2E used to normalize) | 32 | 100 |
| quadSVM (all markers, no PSA) | 39 | 96 |

**Table 11**

| Marker Panel | % Sens PCa | % Spec Asymptomatic + Biopsy (-) |
|---|---|---|
| Quantitative Single Markers: | | |
| RASSF2A | 37 | 91 |
| HIST1H4K | 26 | 88 |
| GSTPi | 17 | 95 |
| TFAP2E | 22 | 92 |
| Qualitative Panels: | | |
| RASSF2A+HIST1H4K | 41 | 88 |
| Quantitative Panels: | | |
| RASSF2A+TFAP2E (TFAP2E used to normalize) | 32 | 92 |
| quadSVM (all markers, no PSA) | 39 | 94 |

**Table 12**

| Marker | PCa vs. Biopsy (-) | |
|---|---|---|
| | Urine AUC | Plasma AUC |
| GSTPi/ GSTP1 | 0.69 | 0.55 |
| RASSF2A | 0.66 | 0.60 |
| HIST1H4 K | 0.64 | 0.50 |
| TFAP2E | 0.65 | 0.52 |
| ***PSA | 0.56 | 0.56 |

| | | |
|---|---|---|
| ***Tests whether PSA contains further information beyond what was contributed by the > 4 ng/ml cut-off indication for prostate biopsy. | | |

**Table 13**

| Marker Panel | % Sens PCa | % Spec Biopsy (-) |
|---|---|---|
| Quantitative Single Markers: | | |
| RASSF2A | 74 | 55 |
| HIST1H4K | 69 | 41 |
| GSTPi | 63 | 64 |
| TFAP2E | 46 | 77 |

| Qualitative Panels: | | |
|---|---|---|
| GSTPi+HIST1H4K | 79 | 46 |
| RASSF2A+HIST1H4K | 94 | 21 |

| Quantitative Panels: | | |
|---|---|---|
| RASSF2A+HIST1H4K | 94 | 27 |
| GSTPi+PSA | 83 | 45 |
| quadSVM (all markers, no PSA) | 79 | 55 |

**Table 14**

| Marker Panel | % Sens PCa | % Spec Biopsy (-) |
|---|---|---|
| Quantitative Single Markers: | | |
| RASSF2A | 37 | 82 |
| HIST1H4K | 26 | 79 |
| GSTPi | 17 | 96 |
| TFAP2E | 22 | 84 |

| Qualitative Panels: | | |
|---|---|---|
| RASSF2A+HIST1H4K | 41 | 79 |

| Quantitative Panels: | | |
|---|---|---|
| RASSF2A+TFAP2E (TFAP2E used to normalize) | 32 | 85 |
| RASSF2A+TFAP2E+PSA (TFAP2E used to normalize) | 94 | 22 |
| quadSVM (all markers, no PSA) | 39 | 91 |
| quadSVM (all markers+PSA) | 48 | 87 |

**Table 15:Assay components according to Example 2**

| Gene | Genomics SEQ ID NO: | Forward Primer SEQ ID NO: | Reverse Primer SEQ ID NO: | Detection Probe SEQ ID NO: |
|---|---|---|---|---|
| MME | 37 | 182 | 183 | 184 |
| RARB | 38 | 185 | 186 | 187 |
| NKX3-1 | 39 | 188 | 189 | 190 |
| NPAL3 | 40 | 191 | 192 | 193 |
| ACVR2A | 41 | 194 | 195 | 196 |
| ARL4C | 42 | 197 | 198 | 199 |
| PDE4D | 43 | 200 | 201 | 202 |
| CARTPT | 44 | 203 | 204 | 205 |
| HIST1H2BD | 45 | 206 | 207 | 208 |
| CUL1 | 46 | 209 | 210 | 211 |
| MOBKL2B | 47 | 212 | 213 | 214 |
| IFLTD1; KRAS | 48 | 215 | 216 | 217 |
| ANXA2 | 49 | 218 | 219 | 220 |
| NFATC3 | 50 | 221 | 222 | 223 |
| NFATC3 | 51 | 224 | 225 | 226 |
| MN1 | 52 | 227 | 228 | 229 |
| KLF8 | 53 | 230 | 231 | 232 |
| FGF13 | 54 | 233 | 234 | 235 |
| NKX2-6 | 55 | 236 | 237 | 238 |
| SPG20 | 56 | 239 | 240 | 241 |
| DSE/SART2 | 57 | 242 | 243 | 244 |
| GJA1 (aka CX43) | 58 | 245 | 246 | 247 |
| GJA1 (aka CX43) | 59 | 248 | 49 | 250 |
| SPATA6 | 60 | 251 | 52 | 253 |
| MCC | 61 | 254 | 255 | 256 |
| GPR68 (Intron 1 region) | 62 | 257 | 258 | 259 |

**Table 16: AUC of assay according to Example 2**

| Forward Primer SEQ ID NO: | Reverse Primer SEQ ID NO: | Detection Probe SEQ ID NO: | AUC | Sensitivity | Specificity | Median PCa PMR | Median BPH PMR |
|---|---|---|---|---|---|---|---|
| 182 | 183 | 184 | 0.82 [0.75, 0.88] | 0.66 | 0.97 | 0,004 | 0 |
| 185 | 186 | 187 | 0.98 [0.94, 1.00] | 0,98 | 0,95 | 0,523 | 0,001 |
| 188 | 189 | 190 | 0.76 [0.68, 0.82] | 0.51 | 1.00 | 0.002 | 0 |
| 191 | 192 | 193 | 0.76 [0.68, 0.82] | 0,51 | 1,00 | 0,002 | 0 |
| 194 | 195 | 196 | 0.92 [0.87, 0.96] | 0.85 | 0.95 | 0.025 | 0 |
| 197 | 198 | 199 | 0.95 [0.90, 0.98] | 0.90 | 0.98 | 0.013 | 0 |
| 200 | 201 | 202 | 0.92 [0.85,0.95] | 0.82 | 0.95 | 0.212 | 0,001 |
| 203 | 204 | 205 | 0.98 [0.94,1.00] | 0,96 | 0,95 | 0,718 | 0,012 |
| 206 | 207 | 208 | 0.93 [0.87,0.96] | 0.84 | 0.95 | 0.154 | 0 |
| 209 | 210 | 211 | 0.71 [0.63,0.78] | 0.41 | 1.00 | 0,000 | 0 |
| 212 | 213 | 214 | 0.97 [0.92,0.99] | 0.96 | 0.95 | 0,218 | 0 |
| 215 | 216 | 217 | 0.75 [0.67, 0.82] | 0,50 | 1,00 | 0,001 | 0 |
| 218 | 219 | 220 | 0.77 [0.69, 0.84] | 0.55 | 0.98 | 0.004 | 0.000 |
| 221 | 222 | 223 | 0.98 [0.95,1.00] | 0,96 | 0,95 | 0.329 | 0 |
| 224 | 225 | 226 | 0.98 [0.94,1.00] | 0,96 | 0.95 | 0.317 | 0 |
| 227 | 228 | 229 | 0.98 [0.95,1.00] | 0.96 | 0,95 | 0.260 | 0,001 |
| 230 | 231 | 232 | 0.98 [0.94,1.00] | 0,98 | 0,95 | 0.357 | 0 |
| 233 | 234 | 235 | 0.92 [0.86, 0.96] | 0.82 | 0,95 | 0.173 | 0 |
| 236 | 237 | 238 | [0.95, 0.99] | 0.95 | 0,95 | 0.187 | 0,002 |
| 239 | 240 | 241 | 0.97 [0.92,0.99] | 0,93 | 0,95 | 0.297 | 0,001 |
| 242 | 243 | 244 | 0.96 [0.92,0.99] | 0.94 | 0.97 | 0.089 | 0 |
| 245 | 246 | 247 | 0.95 [0.90,0.98] | 0.91 | 0,95 | 0.171 | 0 |
| 248 | 249 | 250 | 0.97 [0.93, 0.99] | 0.93 | 0,95 | 0.173 | 0,001 |
| 251 | 252 | 253 | 0.94 [0.89,0.97] | 0.88 | 0.98 | 0.033 | 0 |
| 254 | 255 | 256 | 0.77 [0.70,0.84] | 0.55 | 1.00 | 0.003 | 0 |
| 257 | 258 | 259 | 0.76 [0.68,0.82] | 0.51 | 1.00 | 0.001 | 0.000 |

### Example 3

The aim of the present study was to determine the feasibility of measuring DNA methylation biomarkers for prostate cancer (hereinafter also referred to as PCa) in post-prostatic massage urine. In this process a well-characterized workflow for urine was used and 8 candidate biomarkers plus GSTP1 were analyzed by real-time PCR using both hybridization probes and HeavyMethyl™ (HM) (Cottrell et al., Nucleic Acids Res. 2004 Jan 13;32(1):e10.) technology following bisulfite treatment of genomic DNA (see Table 1).

It was demonstrated that PCa sheds DNA that can be detected by means of methylation analysis in urine with high sensitivity. It was also demonstrated that the analyzed biomarkers were suitable for the development of screening and diagnostic tests for PCa.

### Study Objectives

The purpose of the present study was to conduct an investigation into whether the candidate PCa-specific DNA methylation biomarkers identified by differential methylation hybridization (DMH) and validated in prostate tissues can be measured in post-prostatic massage urine. The study was designed to generate performance data for the biomarker candidates when analyzed individually and in panels.

### Candidate Biomarkers and Location of qPCR Assays

The PCa biomarker GSTP1 had been previously identified in a study by the applicant as published in patent application WO 2005/054517. The 8 candidate biomarkers were identified as described in WO 2005/054517 (P185WO) and subsequently validated in prostate tissues as described in Example 2. This was the first analysis of these candidate biomarkers in post-prostatic massage urine.

Methylation analysis was performed by means of real-time PCR using both hybridization probes (8 newly identified candidate biomarkers) and HeavyMethyl™ (GSTP1 biomarker) technology. Isolated genomic DNA was bisulfite treated to convert non-methylated cytosines to uracil, wherein methylated cyctosines are conserved. Fragments of the bisulfite treated DNA comprising potentially methylated CpG dinucleotides are then amplified by means of PCR. The primers do not cover any potentially methylated cytosine positions (i.e. do not hybridise to genomic CpG dinucleotides). Amplification of fragments comprising unmethylated CpG dinucleotides is suppressed by means of a blocking oligonucleotide that hybridises to TG dinucleotides. Accordingly only DNA that was methylated in the genomic sample is amplified. Amplificate fragments are detected by means of detectably labelled probes suitable for use in PCR reactions such as real-time detection probes.
Assay primer and probe oligos are provided in Table 2.

### GSTP1

Chromosomal Location: 11q13
Nearby Gene(s): GSTP1 HM forward primer is just upstream of exon 1 and the reverse primer is just downstream of exon 1 of *GSTP1.*
KLF8
Chromosomal Location: Xp11.21
Nearby Gene(s): w/i exon 1 of KLF8
NFATC3
Chromosomal Location: 16q22.1
Nearby Gene(s): w/i promoter of NFATC3
MOBKL2B
Chromosomal Location: 9p21.2
Nearby Gene(s): w/i intron 1 of MOBKL2B
GJA1 (aka CX43)
Chromosomal Location: 6q22.31
Nearby Gene(s): w/i intron 1 of GJA1
MN1
Chromosomal Location: 22q12.1
Nearby Gene(s): w/i the promoter of MN1
FGF 13
Chromosomal Location: Xq26.3
Nearby Gene(s): w/i intron 1 of FGF 13
CARTPT
Chromosomal Location: 5q13.2
Nearby Gene(s): w/i exon 1 of CARTPT
SPG20
Chromosomal Location: 13q13.3
Nearby Gene(s): w/i intron 1 of SPG20

### Study Design

In order to maximize the urine volume interrogated by a single real-time PCR assay (1.4 ml equivalents), the maximum number of candidate biomarkers previously validated in prostate tissues that could be analyzed in this study was nine, with each assay run once for each sample.

### Sample Collection

For this study, the inventors collected urine from a total of 159 men, including 81 males with biopsy-confirmed prostate cancer and 78 males with no cancer detected by prostate biopsy. In designing the present study, the definition of the negative class was an issue as there is no detection method that excludes presence of PCa with 100% certainty. Biopsy has a false negative diagnosis rate of at least 10% (Djavan et al., 2000; Mian et al., 2002; Gupta et al., 2005; Hanley et al., 2006) while PSA measurement is prone to both false negatives and false positives.

The samples were collected at six sites in the United States and Germany. Urine was collected after a 20-60 s prostatic massage prior to any treatment for PCa. Inclusion and exclusion criteria were designed to ensure that the patients analyzed reflect the potential patients who would use PCa screening and diagnostic tests.

The following inclusion and exclusion criteria applied to the patients undergoing prostate biopsy: Inclusion criteria:
- Indication for biopsy (elevated PSA and/or suspicious DRE)
- Biopsy scheduled within 1 week after sample collection
- Age 40-80

Exclusion criteria:
- Any prior treatment for prostate cancer
- History of cancer or serious illness in the past 5 years
- Symptoms of urinary tract infection

### Patient data and tumor characteristics

The Gleason scores (where appropriate) of the patient samples are listed Table 3. The median total PSA values for the prostate cancer and biopsy negative patients were 8.3 ± 1.6 and 5.4 ± 0.6, respectively. The prostate cancer and biopsy negative classes were defined after sample collection via prostate biopsy. The median number of previous biopsies for prostate cancer and biopsy negative patients were 0.5 ± 0.1 and 0.0 ± 0.1, respectively.

### Workflow

DNA extraction and bisulfite treatment was carried out according to standardised protocols (Lofton-Day et al., Clin. Chem. 2008 54(2):1-10.). For each qPCR assay, 1.4 ml urine equivalent was analyzed once.

### Biomarker Performance, General Considerations

The objective of the study was to determine the performance of the candidate biomarkers in men over 50 years of age in discriminating prostate cancer from non-cancerous conditions. The data was analyzed using prostate cancer patients as the positive class and biopsy negative patients as the negative class. In this example, the inventors report biomarker performance for urine only (data on tissue samples is provided in a Example 2). The inventors provide performance data for the biomarkers individually and in panels.

Performance data (AUC, sensitivity and specificity) of the individual biomarkers GSTPi, KLF8, NFATC3, MOBKL2B, GJA1/CX23, MN1, FGF13, CARTPT, and SPG20 is shown in Table 4. Performance data (AUC, sensitivity and specificity) for the biomarker panels is shown in Table 5. ROC curves for the markers compared to GSTP1 are shown in Figures 8-9, 11-12, 16-18 and 20.

### Discussion

The study was conducted on urine samples collected from 81 PCa and 78 biopsy negative patients. Real-time PCR assays were used to measure DNA methylation of the candidate biomarkers. The amount of methylated biomarker DNA was significantly correlated with PCa for GSTP1 and five of the newly identified candidate biomarkers (KLF8, NFATC3, MOBKL2B, GJA1 and MN1). The performances of these biomarkers compare well with the performance of PSA (18% sensitivity at 98% specificity for men < 60 years and 19% sensitivity at 94% specificity for men > 60 years) in the screening population (Punglia et al., 2003). Sensitivity of the six significant biomarkers at 95% specificity ranges from 12% for NFATC3 to 26% for KLF8(Table 6).

### Conclusions

The present investigation has demonstrated that GSTP1 and all of the newly identified prostate cancer biomarkers are measurable in urine from prostate cancer patients. Further, GSTP1 and five of the newly identified candidate biomarkers significantly discriminate PCa patients from biopsy negative patients. The major conclusion of the present study is: The DNA methylation-based biomarkers of prostate cancer KLF8, NFATC3, MOBKL2B, GJA1 and MN1 are measurable in urine from PCa patients and can significantly discriminate PCa from biopsy negative patients.

**Table 17: Genes and sequences**

| Gene | Genomics SEQ ID NO: | Methylated bisulfite converted sense strand | Methylated bisulfite converted antsense strand | Unmethylated bisulfite converted sense strand | Unmethylated bisulfite converted antisense strand |
|---|---|---|---|---|---|
| GSTP1 | 4 | 11 | 12 | 19 | 20 |
| KLF8 | 53 | 98 | 99 | 156 | 157 |
| NFATC3 | 50 | 92 | 93 | 150 | 151 |
| MOBKL2B | 47 | 86 | 87 | 144 | 145 |
| GJA1 (aka CX43) | 58 | 108 | 109 | 166 | 167 |
| MN1 | 52 | 96 | 97 | 154 | 155 |
| FGF13 | 54 | 100 | 101 | 158 | 159 |
| CARTPT | 44 | 80 | 81 | 138 | 139 |
| SPG20 | 56 | 104 | 105 | 162 | 163 |

**Table 18:Assay components**

| Gene | Forward Primer | Reverse Primer | Blocker | Detection Oligo |
|---|---|---|---|---|
| GSTP1 | | | | |
| KLF8 | | | NA | |
| NFATC3 | | | NA | |
| MOBKL2B | | | NA | |
| GJA1 (aka CX43) | | | NA | |
| MN1 | | | NA | |
| FGF13 | | | NA | |
| CARTPT | | | NA | |
| SPG20 | | | NA | |

**Table 19: Patient samples.**

| Sample type | No. of samples |
|---|---|
| *Prostate Cancer - Gleason Score* | |
| 4 | 2 |
| 5 | 3 |
| 6 | 14 |
| 7 | 32 |
| 8 | 3 |
| 9 | 4 |
| 10 | 3 |
| No score available | 20 |
| Total *Prostate Cancer:* | 81 |
| *Biopsy Negative* | 78 |

**Table 20: Performance analysis of individual biomarkers analysed in post-prostatic massage urine from prostate cancer and biopsy negative patients.**

| Biomarker | AUC | Sensitivity | Specificity | Wilcoxon *p* value |
|---|---|---|---|---|
| GSTP1 | 0.64 [0.56-0.71] | 0.67 | 0.50 | 0.0027 |
| KLF8 | 0.67 [0.59-0.75] | 0.58 | 0.68 | 0.0000 |
| NFATC3 | 0.62 [0.54-0.70] | 0.51 | 0.69 | 0.0026 |
| MOBKL2B | 0.58 [0.50-0.66] | 0.30 | 0.86 | 0.0150 |
| GJA1 (aka CX43) | 0.58 [0.50-0.66] | 0.34 | 0.80 | 0.0385 |
| MN1 | 0.66 [0.58-0.73] | 0.73 | 0.50 | 0.0003 |
| FGF13 | 0.55 [0.47-0.63] | 0.49 | 0.58 | 0.2406 |
| CARTPT | 0.57 [0.49-0.64] | 0.53 | 0.50 | 0.1345 |
| SPG20 | 0.6494 [0.40-0.56] | 0.05 | 0.92 | 0.4415 |

**Table 21: Performance of biomarker panels to distinguish PCa from biopsy negative patients in post-prostatic massage urine**

| Biomarker Panel | AUC | Sensitivity | Specificity | Wilcoxon *p* value |
|---|---|---|---|---|
| GSTP1 + Total PSA | 0.76 [0.68-0.83] | 0.88 | 0.52 | 0.0000 |
| KLF8 + Total PSA | 0.79 [0.71-0.86] | 0.88 | 0.52 | 0.0000 |
| MN1 + Total PSA | 0.77 [0.69-0.84] | 0.84 | 0.52 | 0.0000 |
| GSTP1 + KLF8 | 0.67 [0.57-0.72] | 0.57 | 0.72 | 0.0001 |
| KLF8 + GJA1 (aka CX43) | 0.71 [0.63-0.78] | 0.57 | 0.73 | 0.0000 |
| GSTP1 + KLF8 + GJA1 (aka CX43) | 0.70 [0.62-0.78] | 0.56 | 0.75 | 0.0000 |
| KLF8 + MOBKL2B | 0.66 [0.57-0.73] | 0.60 | 0.60 | 0.0003 |
| GSTP1 + KLF8 + NFATC3 | 0.68 [0.60-0.75] | 0.64 | 0.64 | 0.0001 |
| KLF8 + NFATC3 | 0.69 [0.61-0.76] | 0.74 | 0.53 | 0.0000 |
| MN1 + MOBKL2B | 0.67 [0.59-0.74] | 0.73 | 0.51 | 0.0002 |
| KLF8 + MN1 | 0.68 [0.60-0.75] | 0.73 | 0.51 | 0.0001 |
| MN1 + GJA1 (aka CX43) | 0.68 [0.60-0.75] | 0.73 | 0.51 | 0.0002 |
| GSTP1 + MN1 | 0.66 [0.59-0.74] | 0.72 | 0.51 | 0.0003 |

**Table 22: Performance analysis of individual biomarkers at high fixed specificity (95%).**

| Biomarker | AUC | Sensitivity | Specificity | Wilcoxon *p* value |
|---|---|---|---|---|
| GSTP1 | 0.64 [0.56-0.71] | 0.15 | 0.95 | 0.0027 |
| KLF8 | 0.67 [0.59-0.75] | 0.26 | 0.95 | 0.0000 |
| NFATC3 | 0.62 [0.54-0.70] | 0.12 | 0.95 | 0.0026 |
| MOBKL2B | 0.58 [0.50-0.66] | 0.16 | 0.95 | 0.0150 |
| GJA1 (aka CX43) | 0.58 | 0.14 | 0.95 | 0.0385 |
| MN1 | 0.66 [0.58-0.73] | 0.19 | 0.95 | 0.0003 |
| FGF13 | 0.55 [0.47-0.63] | 0.11 | 0.95 | 0.2406 |
| CARTPT | 0.57 [0.49-0.64] | 0.10 | 0.95 | 0.1345 |
| SPG20 | 0.6494 [0.40-0.56] | 0.01 | 0.95 | 0.4415 |

## Claims

1. A method for detecting prostate cell proliferative disorders in a subject comprising detecting the presence or absence of CpG methylation within at least the KLF8 gene or the KLF8 gene and of at least one gene selected from the group consisting of RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; FGF13; NKX2-6; SPG20; DSE/SART2; GJA1 (CX43); SPATA6; MCC; GPR68 (INTRON 1 AND/OR EXON 2 REGION) in a biological sample isolated from said subject, wherein the presence of methylation indicates the presence of a carcinoma.

2. The method according to claim 1 further comprising determining the expression level of PSA in said biological sample isolated from said subject wherein underexpression and/or CpG methylation is indicative of the presence of said disorder.

3. A method for detecting prostate cell proliferative disorders in a subject, comprising contacting genomic DNA isolated from a biological sample obtained from said subject with at least one reagent, or series of reagents that distinguishes between methylated and non- methylated CpG dinucleotides within at least one target region of the genomic DNA, wherein the target region comprises, or hybridizes under stringent conditions to a sequence of at least 16 contiguous nucleotides of SEQ ID NO: 53, wherein said contiguous nucleotides comprise at least one CpG dinucleotide sequence, and whereby detecting carcinoma is, at least in part, afforded.

4. A method for detecting prostate cell proliferative disorders, comprising:
a) extracting or otherwise isolating genomic DNA from a biological sample obtained from the subject;
b) treating the genomic DNA of a), or a fragment thereof, with one or more reagents to convert cytosine bases that are unmethylated in the 5-position thereof to uracil or to another base that is detectably dissimilar to cytosine in terms of hybridization properties;
c) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 98; 99; 156; 157; and complements thereof, wherein the treated genomic DNA or the fragment thereof is either amplified to produce at least one amplificate, or is not amplified; and
d) determining, based on a presence or absence of, or on a property of said amplificate, the methylation state or level of at least one CpG dinucleotide of SEQ ID NO: 53, or an average, or a value reflecting an average methylation state or level of a plurality of CpG dinucleotides of SEQ ID NO: 53, whereby at least one of detecting and diagnosing cancer is, at least in part, afforded.

5. A method for detecting prostate cell proliferative disorders, comprising:
a) extracting or otherwise isolating genomic DNA from a biological sample obtained from the subject;
b) digesting the genomic DNA of a), or a fragment thereof, with one or more methylation sensitive restriction enzymes;
c) contacting the DNA restriction enzyme digest of b), with an amplification enzyme and at least two primers suitable for the amplification of a sequence comprising at least one CpG dinucleotide of SEQ ID NO: 53;
d) determining, based on a presence or absence of an amplificate the methylation state or level of at least one CpG dinucleotide of SEQ ID NO: 53, whereby at least one of detecting and classifying cancer is, at least in part, afforded.

6. A treated nucleic acid of at least 50 base pairs in length, derived from genomic SEQ ID NO: 53, wherein the treatment of SEQ ID NO: 53 is suitable to convert at least one unmethylated cytosine base of the genomic DNA sequence to uracil or another base that is detectably dissimilar to cytosine in terms of hybridization, wherein said treated nucleic acid comprises at least one CpG, CpA or TpG dinucleotide.

7. A nucleic acid for use in the detection of prostate cell proliferative disorders by means of methylation analysis, comprising at least 16 contiguous nucleotides of a DNA sequence selected from the group consisting of SEQ ID NO: 98; 99; 156; 157; and sequences complementary thereto, wherein said contiguous nucleotides comprise at least one CpG, CpA or TpG dinucleotide.

8. A nucleic acid, comprising at least 50 contiguous nucleotides of a DNA sequence selected from the group consisting of SEQ ID NO: 98; 99; 156; 157; and sequences complementary thereto, wherein said nucleic acid comprises at least one CpG, CpA or TpG dinucleotide.

9. Use of a kit, the kit comprising
(a) a bisulfite reagent;
(b) a container suitable for containing the said bisulfite reagent and the biological sample of the patient;
(c) at least one set of oligonucleotides containing two oligonucleotides whose sequences in each case are identical, are complementary, or hybridize under stringent or highly stringent conditions to a 9 or more preferably 18 base long segment of a sequence selected from SEQ ID NO: 98; 99; 156; 157
for the diagnosis and/or detection of prostate cell proliferative disorders by methylation analysis of the KLF8 gene.

10. Use of a kit, the kit comprising
(a) a methylation sensitive restriction enzyme reagent;
(b) a container suitable for containing the said reagent and the biological sample of the patient;
(c) at least one set of oligonucleotides one or a plurality of nucleic acids or peptide nucleic acids which are identical, are complementary, or hybridize under stringent or highly stringent conditions to an at least 9 base long segment of SEQ ID NO: 53; and, optionally,
(d) instructions for use and interpretation of the kit results
for the diagnosis and/or detection of prostate cell proliferative disorders by methylation analysis of the KLF8 gene.

11. Use of a nucleic acid according to claims 6 or 8 in the diagnosis and/or detection of prostate cell proliferative disorders by methylation analysis of the KLF8 gene.

## Patentansprüche

1. Verfahren zur Detektion von Prostata-Zellproliferationserkrankungen in einem Subjekt, umfassend das Bestimmen der Anwesenheit oder Abwesenheit einer CpG-Methylierung in mindestens dem KLF8 Gen oder dem KLF8 Gen und mindestens einem Gen ausgewählt aus der Gruppe bestehend aus RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; FGF13; NKX2-6; SPG20; DSE/SART2; GJA1 (CX43); SPATA6; MCC; GPR68 (INTRON 1 und/oder EXON 2 Region) in einer biologischen Probe, welche aus dem besagten Subjekt isoliert wurde, wobei die Anwesenheit der Methylierung die Anwesenheit eines Karzinoms anzeigt.

2. Verfahren gemäß Anspruch 1, weiterhin die Bestimmung des Expressionsniveaus von PSA in besagter biologischer Probe umfassend, welche von besagtem Subjekt isoliert wurde, wobei die Unterexpression und/oder die CpG-Methylierung auf die Anwesenheit von besagter Erkrankung hinweist.

3. Verfahren zur Detektion von Prostata-Zellproliferationserkrankungen in einem Subjekt, umfassend das Inkontaktbringen von genomischer DNA, welche aus einer biologischen Probe, die von besagtem Subjekt erhalten wurde, mit mindestens einem Reagenz, oder einer Serie von Reagenzien, welches zwischen metyhlierten und nicht-methylierten CpG-Dinukleotiden in mindestens einer Zielregion der genomischen DNA unterscheidet, wobei die Zielregion eine Sequenz von mindestens jeweils 16 zusammenhängenden Nukleotiden einer Sequenz ausgewählt aus der Gruppe bestehend aus SEQ ID NO: 53 umfasst oder unter stringenten Bedingungen mit dieser hybridisiert, wobei besagte zusammenhängende Nukleotide mindestens eine CpG-Dinukleotidsequenz umfassen und wodurch die Detektion eines Karzinoms, zumindest teilweise, ermöglicht wird.

4. Verfahren zur Detektion von Prostata-Zellproliferationserkrankungen, umfassend:
a) Extrahieren oder anderweitiges Isolieren genomischer DNA aus einer biologischen Probe, welche von einem Subjekt erhalten wurde;
b) Behandeln der genomischen DNA aus a), oder eines Fragmentes davon, mit einem oder mehreren Reagentien, um Cytosinbasen, welche an ihrer 5' Position unmethyliert sind, in Uracil, oder eine andere Base, welche detektierbar unähnlich zu Cytosin im Hinblick auf ihre Hybridisierungseigenschaften ist, zu konvertieren;
c) Inkontaktbringen der behandelten genomischen DNA, oder dem behandelten Fragment davon, mit einem Amplifikationsenzym und mindestens einem Primer, welcher eine zusammenhängende Sequenz von mindestens 9 Nukleotiden umfasst, welche komplementär zu, unter moderat stringenten Bedingungen hybridisiert oder stringenten Bedingungen mit einer Sequenz hybridisiert, ausgewählt aus der Gruppe bestehend aus SEQ ID NO: 98; 99; 156; 157; und Komplementen davon, wobei die behandelte genomische DNA oder das Fragment davon entweder amplifiziert wird, um mindestens ein Amplifikat zu produzieren, oder nicht amplifiziert wird; und
d) Bestimmen, basierend auf der Anwesenheit oder Abwesenheit von, oder einer Eigenschaft von besagtem Amplifikat, des Methylierungsstatus oder -niveaus von mindestens einem CpG-Dinukleotid der SEQ ID NO: 53, oder einem Mittelwert oder Wert, der einen mittleren Methylierungsstatus oder -niveau von einer Vielzahl von CpG-Dinukleotiden der SEQ ID NO: 53 reflektiert, wobei mindestens eines, Detektieren und Diagnostizieren von Krebs, zumindest teilweise, ermöglicht wird.

5. Verfahren zur Detektion von Prostata-Zellproliferationserkrankungen, umfassend
a) Extrahieren oder anderweitiges Isolieren genomischer DNA aus einer biologischen Probe, welche von einem Subjekt erhalten wurde;
b) Verdauen der genomischen DNA aus a), oder eines Fragmentes davon, mit einem oder mehreren methylierungssensitiven Restriktionsenzymen;
c) Inkontaktbringen des DNA Restriktionsenzymverdaus aus b) mit einem Amplifikationsenzym und mindestens zwei Primern, welche zur Amplifikation einer Sequenz, welche mindestens ein CpG-Dinukleotid der SEQ ID NO: 53 umfasst, geeignet sind;
e) Bestimmen, basierend auf der Anwesenheit oder Abwesenheit eines Amplifikates, des Methylierungsstatus oder -niveaus von mindestens einem CpG-Dinukleotid der SEQ ID NO: 53, wobei mindestens eines, Detektieren und Klassifizieren von Krebs, zumindest teilweise, ermöglicht wird.

6. Behandelte Nukleinsäure von mindestens 50 Basenpaaren Länge, welche von der genomischen DNA SEQ ID NO: 53 stammt, wobei die Behandlung der SEQ ID NO: 53 geeignet ist, um mindestens eine unmethylierte Cytosinbase der genomischen DNA-Sequenz zu Uracil oder einer anderen Base, welche detektierbar unähnlich zu Cytosin in Bezug auf Hybridisierung ist, zu konvertieren, wobei besagte behandelte Nukleinsäure mindestens ein CpG-, CpA- oder TpG -Dinukleotid umfasst.

7. Nukleinsäure zur Verwendung in der Detektion von Prostata-Zellproliferationserkrankungen durch Mittel der Methylierungsanalyse, welche mindestens 16 zusammenhängende Nukleotide einer DNA-Sequenz, ausgewählt aus der Gruppe bestehend aus SEQ ID NO: 98; 99; 156; 157; und dazu komplementären Sequenzen umfasst, wobei besagte zusammenhängende Nukleotide mindestens ein CpG-, CpA- oder TpG-Dinukleotid umfassen.

8. Nukleinsäure, welche mindestens 50 zusammenhängende Nukleotide einer DNA-Sequenz umfasst, ausgewählt aus der Gruppe bestehend aus SEQ ID NO: 98; 99; 156; 157; und dazu komplementären Sequenzen, wobei besagte Nukleinsäure mindestens ein CpG-, CpA- oder TpG-Dinukleotid umfasst.

9. Verwendung eines Kits, das Kit umfassend:
(a) ein Bisulfitreagenz;
(b) einen Behälter, welcher geeignet ist, um besagtes Reagenz und die biologische Probe des Patienten zu beinhalten;
(c) mindestens ein Set von Oligonukleotiden, welches zwei Oligonukleotide beinhaltet, deren Sequenzen in jedem Fall entweder identisch sind mit, komplementär sind, oder unter stringenten oder hochstringenten Bedingungen mit einem 9 oder bevorzugter 18 Basenpaare langen Segment einer Sequenz ausgewählt aus SEQ ID NO: 98; 99; 156; 157 hybridisieren,
zur Diagnose und/oder Detektion von Prostata Zellproliferationserkrankungen durch Methylierungsanalyse des KLF8 Gens.

10. Verwendung eines Kits, das Kit umfassend:
(a) ein methylierungssensitives Restriktionsenzym Reagenz;
(b) ein Behälter, welcher geeignet ist, um besagtes Reagenz und die biologische Probe des Patienten zu beinhalten;
(c) mindestens ein Set von Oligonukleotiden, eine oder eine Vielzahl von Nukleinsäuren oder Peptidnukleinsäuren, welche identisch sind mit oder komplementär sind oder unter stringenten oder hoch stringenten Bedingungen mit einem mindestens 9 Basen langen Segment der SEQ ID NO: 53 hybridisieren; und, optional,
(d) Instruktionen zur Verwendung und Interpretation der Kitergebnisse
zur Diagnose und/oder Detektion von Prostata Zellproliferationserkrankungen durch Methylierungsanalyse des KLF8 Gens.

11. Verwendung einer Nukleinsäure gemäß den Ansprüchen 6 oder 8 zur Diagnose und/oder Detektion von Prostata Zellproliferationserkrankungen durch Methylierungsanalyse des KLF8 Gens.

## Revendications

1. Procédé pour la détection de désordres prolifératifs des cellules prostatiques chez un sujet, comprenant la détection de la présence ou de l'absence de méthylation de CpG dans au moins le gène KLF8 ou le gène KLF8 et au moins un gène choisi dans le groupe consistant en RASSF2A; TFAP2E; HIST1H4K; GSTPi; MME; RARB; NKX3-1; NPAL3; ACVR2A; ARL4C; PDE4D; CARTPT; HIST1H2BD; CUL1; MOBKL2B; IFLTD1 (KRAS); ANXA2; NFATC3; MN1; FGF13; NKX2-6; SPG20; DES/SART2; GJA1 (CX43); SPATA6; MCC; GPR68 (REGION de l'INTRON 1 ET/OU de l'EXON 2) dans un échantillon biologique isolé à partir dudit sujet, tandis que la présence de méthylation indique la présence d'un carcinome.

2. Procédé selon la revendication 1, comprenant en outre la détermination du niveau d'expression du PSA dans ledit échantillon biologique isolé à partir dudit sujet, tandis que la sous-expression et/ou la méthylation de CpG est indicatrice de la présence dudit désordre.

3. Procédé pour la détection de désordres prolifératifs des cellules prostatiques chez un sujet, comprenant la mise en contact d'ADN génomique isolé à partir d'un échantillon biologique obtenu à partir dudit sujet avec au moins un réactif, ou des séries de réactifs faisant la distinction entre dinucléotides CpG méthylés et non-méthylés dans au moins une région cible de l'ADN génomique, tandis que la région cible comprend, ou hybride dans des conditions stringentes avec une séquence d'au moins 16 nucléotides contigus de SEQ ID NO: 53, lesdits nucléotides contigus comprenant au moins une séquence dinucléotidique CpG, procurant ainsi, au moins pour partie, une détection de carcinome.

4. Procédé pour la détection de désordres prolifératifs des cellules prostatiques, comprenant:
a) l'extraction ou l'isolement d'une autre manière d'ADN génomique à partir d'un échantillon biologique obtenu à partir du sujet;
b) le traitement de l'ADN génomique de a), ou d'un fragment de celui-ci, avec un ou plusieurs réactifs pour convertir les bases cytosine qui sont non-méthylées dans leur position 5 en uracile ou en une autre base qui se révèle par détection être dissemblable vis-à-vis de la cytosine en termes de propriétés d'hybridation;
c) la mise en contact de l'ADN génomique traité, ou du fragment traité de celui-ci, avec une enzyme d'amplification et au moins une amorce comprenant une séquence contiguë d'au moins 9 nucléotides qui est complémentaire de, ou hybride dans des conditions modérément stringentes ou stringentes à une séquence choisie dans le groupe consistant en SEQ ID NO: 98; 99; 156; 157; et des compléments de celles-ci, tandis que l'ADN génomique traité ou le fragment de celui-ci soit est amplifié pour produire au moins un produit d'amplification, soit n'est pas amplifié; et
d) la détermination, sur la base d'une présence ou d'une absence de, ou d'une propriété dudit produit d'amplification, de l'état ou du niveau de méthylation d'au moins un dinucléotide CpG de SEQ ID NO: 53, ou d'une moyenne, ou d'une valeur reflétant un état ou un niveau de méthylation moyen d'une pluralité de dinucléotides CpG de SEQ ID NO: 53, procurant ainsi, au moins pour partie, au moins l'un parmi une détection et un diagnostic de cancer.

5. Procédé pour la détection de désordres prolifératifs des cellules prostatiques, comprenant:
a) l'extraction ou l'isolement d'une autre manière d'ADN génomique à partir d'un échantillon biologique obtenu à partir du sujet;
b) la digestion de l'ADN génomique de a), ou d'un fragment de celui-ci, avec une ou plusieurs enzymes de restriction sensibles à la méthylation;
c) la mise en contact du produit de digestion d'enzyme de restriction d'ADN de b), avec une enzyme d'amplification et au moins deux amorces appropriées pour l'amplification d'une séquence comprenant au moins un dinucléotide CpG de SEQ ID NO: 53;
d) la détermination, sur la base d'une présence ou d'une absence d'un produit d'amplification, de l'état ou du niveau de méthylation d'au moins un dinucléotide CpG de SEQ ID NO: 53, procurant ainsi, au moins pour partie, au moins l'un parmi une détection et une classification d'un cancer.

6. Acide nucléique traité ayant une longueur d'au moins 50 paires de bases dérivé de SEQ ID NO: 53 génomique, tandis que le traitement de SEQ ID NO: 53 est approprié pour convertir au moins une base cytosine non-méthylée de la séquence d'ADN génomique en uracile ou en une autre base qui par détection n'est pas semblable à la cytosine en termes d'hybridation, tandis que ledit acide nucléique traité comprend au moins un dinucléotide CpG, CpA ou TpG.

7. Acide nucléique pour utilisation dans la détection de désordres prolifératifs de cellules prostatiques au moyen d'une analyse par méthylation, comprenant au moins 16 nucléotides contigus d'une séquence d'ADN choisie dans le groupe consistant en SEQ ID NO: 98; 99; 156; 157; et des séquences complémentaires de celles-ci, tandis que lesdits nucléotides contigus comprennent au moins un dinucléotide CpG, CpA ou TpG.

8. Acide nucléique, comprenant au moins 50 nucléotides contigus d'une séquence d'ADN choisie dans le groupe consistant en SEQ ID NO: 98; 99; 156; 157; et des séquences complémentaires de celles-ci, tandis que ledit acide nucléique comprend au moins un dinucléotide CpG, CpA ou TpG.

9. Utilisation d'un kit, le kit comprenant:
(a) un réactif au bisulfite;
(b) un récipient approprié pour contenir ledit réactif au bisulfite et l'échantillon biologique du patient;
(c) au moins un ensemble d'oligonucléotides contenant deux oligonucléotides dont les séquences dans chaque cas sont identiques, sont complémentaires, ou hybrident dans des conditions stringentes ou fortement stringentes à un segment ayant une longueur de 9 ou plus préférablement 18 bases d'une séquence choisie parmi SEQ ID NO: 98; 99; 156; 157
pour le diagnostic et/ou la détection de désordres prolifératifs des cellules prostatiques par une analyse par méthylation du gène KLF8.

10. Utilisation d'un kit, le kit comprenant:
(a) un réactif enzyme de restriction sensible à la méthylation;
(b) un récipient approprié pour contenir ledit réactif et l'échantillon biologique du patient;
(c) au moins un ensemble d'oligonucléotides, un ou une pluralité d'acides nucléiques ou d'acides nucléiques de peptide qui sont identiques, sont complémentaires, ou hybrident dans des conditions stringentes ou fortement stringentes à un segment ayant une longueur d'au moins 9 bases de SEQ ID NO: 53; et, en option,
(d) des instructions pour l'utilisation et l'interprétation des résultats du kit pour le diagnostic et/ou la détection de désordres prolifératifs des cellules prostatiques par analyse par méthylation du gène KLF8.

11. Utilisation d'un acide nucléique selon les revendications 6 ou 8 dans le diagnostic et/ou la détection de désordres prolifératifs des cellules prostatiques par une analyse par méthylation du gène KLF8.


### Sequence listing

<110> Epigenomics AG
<120> Methods and nucleic acids for the analysis of gene expression associated with the development of prostate cell proliferative disorders
<130> E31117PCT
<160> 269
<210> 1
   <211> 1920
   <212> DNA
   <213> Homo Sapiens
<400> 1
   ccaggctgcc gtagacacag cctttgctct cccgaaaaac acgttctagg cgccgggatt      60
   ccagatacct gggaaataga gtgcacgcag ctgttgagag gcctcgcgct tggcttctcc     120
   tatcactgag gcgcagaggt gctgtggaca gcccagaccc acacggcgcc cgaggtgaaa     180
   cagaaccctc agtctcccta tgaggccact ggcactctcg gctgtcccca gagctctccg     240
   acttagagct gaatgcaaag taagcgctcg aaatgcagaa gtagccgggg ccgcccacgg     300
   cacctgcctc gctcggggcg agagaagacg ccaggctgag gtcccagcga cctcaggcac     360
   cagctccgaa ggagggcggg gagaccgcaa aggggaagtg cccggagggc caacggcccc     420
   cgcgcaccct gcgcccctct gaagcgcgcc gcctccccgc gccggggact gggacctgcc     480
   tctggggaat ccgcctagaa gacggcggcg gactggggtc gggcactctc cagggctgtc     540
   aggccctccc cagccctgca cctgccgcgc cgccccacct cgccaggaag tctcagagac     600
   cccggggatg gggtgggagc gccttcccat cgcgggctca aaaagaagga aggacgcccc     660
   caggggtcgt agaaggagga ctagctccaa gccacaactt tcttcggacc caaggcaggc     720
   cggctggggc tccgcgccta cacggcccct ggcgggggtc cgcgcgcccc gggagccccg     780
   cggctcgggg aggaaagagg agacaagaga caggcgagga ttacggggct gacccagccg     840
   gggtagggac catcgtggaa aaactttggc gaggtggggg gacgcggaaa gagagcggcc     900
   cgcgccctgc accttgcgcc gggcatcccg cgccagtgcc tcgctcccag tgccccgcgc     960
   cccgcgcccc gcgccttgcc ttcaccccgg gccagctgca tcgcgcccgc gccgcaggaa    1020
   ccgtggagtt ggaaagtggg ggcgccgcgg ctggggggct gcttcagctg cgcctcggcc    1080
   agcgatcggc gggccgggct caaatccagc caggctgggc aggcggtggc cgcgcgactg    1140
   gggaccgggc gccccgccct cctcgctccc ctcctccttc ctctccctcc ctccagcccc    1200
   ttggcctttt tcagccccta ccggatctgc tcgtccgctg tcctctcttt tctctcgctc    1260
   ttcatatcac tctccacccc ttcgccttgc cttcgccttt cttcctcccc ttgtctcctg    1320
   ccccctcctc ttctcccctc ccctctaggg gcggagcttc tcccctccct cccagacaat    1380
   gctgtggctg cgtccccttc cccgccagct cgtccaggct cccgccgcca gcgattcttc    1440
   cgggctgggg gtggggaggt ggggggggag tgcagggttg gggaggatga gctggctccc    1500
   ctcacctcct tgctgctgcc ctctccaaga gggatggaga cttggcccaa gctcctcggt    1560
   tcacccggag ctgtgacagc cactcccagg gaacagtcac gctgccctac caagcccacc    1620
   tccagcggcc tggattcccc aggcagaggt tgtgggattt tgttttttct aacatcccag    1680
   cttattccca aaagggtttg agccggacag gggctaaaca ggccccttcg acttggcggg    1740
   ccggccagac gtgacagcaa tgccaaggag gccaagtttc tttgtccatt tctcacctcc    1800
   cccttttcca tccctggacc tcctggcgcc cccagtacac agaggccctt gagcagcccg    1860
   gctgcaggtt ccctatctac tcagagttct ccccctcacg tgcctatccc caaccctgca    1920
<210> 2
   <211> 6096
   <212> DNA
   <213> Homo Sapiens
<400> 2
   taccagtgta agattcaaaa ttcccttttt gcactgcaca gtgagatgcc cagggctcca      60
   gctcagtgcc tggacatagc gattcctggg cctgcccgtc gccgccccaa gcgaagctgg     120
   tgcgccttgg gcggagcaga cagagaccct gggtggcagg ggcttgggaa gacatgggcg     180
   gctagggctt tatgcgccct caccgctgcc ctctgctatt tgcaggcaat ggacgagccg     240
   ggaatgagcc tcctagacca gtccgtgatc aagaaaggta aggaatggtc tgtcagggca     300
   gagcccggcg agatggtgca ggcccttggt gcacagatcc attttcttca ccggccgtgc     360
   ctcctgtgtg tcgccaggct gggtgtccac caggcactct tcctggccca gccagatgtt     420
   aggcagacgt gcgggcttgg tgagtttgcc cagcaccctg tggcctgggg tgggcctcag     480
   cggatcagca ttcactgggc tgcagcactg ggagcctggc ctctccccgc cgagggggag     540
   ggcactcttg tggatctgga gttgatttgc agaacgagtt aaaccacttc cctgtttccc     600
   taagagatgg gaatggaagt gctgttccca cggagttggg gaaatgattt tcactttaca     660
   gtgccttagc atttcggtgc ctggcgggca ctttcttcct cttccttcca ggcagggcct     720
   tggaggcctc tgggggaatt ttctttctgt gggagtctct tgcggcattt agacttaggg     780
   gagcttgtgt gtgagtactg tgtgttaggc tgtgtgcacc tgagtcaggg cccacctgct     840
   cctgggtgtc tgtgtccatg tgagttcagg gtcctgtgca tgtctgaaat gttcccttca     900
   tgggtgtctt agtatttctt ggagtgtgag tgtgtctgtt tctgtgaatg tgtttgtgag     960
   gtgtgtctct gtatgttggt gtgcatttct ctgcatttgg gggatgtaca catttctcaa    1020
   tatgtacagt atctctgttg tgtcctgcac tttgttcttt ggtatctgag gatttccaag    1080
   catgcgcggg ccctctctgt gtatatatag gagtatttat gtgactcctg gcattagtaa    1140
   aatccaggga cacgggatcc accttttctg gcctgaggac caagtactgg ccatgacagg    1200
   ggaaggtgag agacgacaaa aacagagaga cagccagaga ggagcagaga gtcagagggg    1260
   cccaggcatt gggtagcagc ctctttacat ttggggcagg tgcccgaaag aattcagagg    1320
   tgcacatgag cctgaggtgc cccaggcagg cactgctccc acagggtttg gcttgagttg    1380
   tttttcaaac gagtgaattc aagcctgggc tctatttgcc ctccacttgt tctcagggga    1440
   ggccaaggtg gaagtggtgg tagcagggct ggggctggac ttccaggagc tggggctgag    1500
   ttaccaggag ctgggggttg ggtggatgac ttggagtgtg tagcagggaa gatgaggcaa     1560
   cagggcagga agtgggtggg gggaggtgga attggggctg tgtcctgtgt cgcttggaac    1620
   tgggagtgtg ggaaagacac taggaacctg gttgcagcgc agctctgctg gtggggcttg    1680
   gttggcttac tgtacagagc ctttcttgac ccctgaagaa agagatccgt ctgcagtggg    1740
   caaaagcctg cctggacttc ctggccacca gaaatatgag catggtggtg gtccccagtt    1800
   ccctattcat gcttgggctc aagagactgg gagtctaggt tcactgactc cctgagaaag    1860
   actaagaccc tgcattttag aaagaggttt ggggatctct gccctgcgca agggtagaag    1920
   gatcagctgt tcctctgagc accttaaccc ggaaccccgg tccgaagccg agacaggaga    1980
   ctggatgcga ggccctccca gagctggttt ctctcaaaca acttccaaaa ctcctagatc    2040
   ctaggggtac gccgaaatcc cccaaagcag tccaaagaac acaacgagag tcctaacatc    2100
   ccaggtggcg gcgcgctggc tccctggagc ggggcgggac gcggccgcgc ggactcacgt    2160
   gcacaaccgc gcgggacggg gccacgcgga ctcacgtgca caaccgcggg accccagcgc    2220
   cagcgggacc ccagcgccag cgggacccca gcgccagcgg gaccccagcg ccagcgggac    2280
   cccagcgcca gcgggacccc agcgccagcg ggaccccagc gccagcgggt ctgtggccca    2340
   gtggagcgag tggagcgctg gcgacctgag cggagactgc gccctggacg ccccagccta    2400
   gacgtcaagt tacagcccgc gcagcagcag caaaggggaa ggggcaggag ccgggcacag    2460
   ttggatccgg aggtcgtgac ccaggggaaa gcgtgggcgg tcgacccagg gcagctgcgg    2520
   cggcgaggca ggtgggctcc ttgctccctg gagccgcccc tccccacacc tgccctcggc    2580
   gcccccagca gttttcacct tggccctccg cggtcactgc gggattcggc gttgccgcca    2640
   gcccagtggg gagtgaatta gcgccctcct tcgtcctcgg cccttccgac ggcacgagga    2700
   actcctgtcc tgccccacag accttcggcc tccgccgagt gcggtactgg agcctgcccc    2760
   gccagggccc tggaatcaga gaaagtcgct ctttggccac ctgaagcgtc ggatccctac    2820
   agtgcctccc agcctgggcg ggagcggcgg ctgcgtcgct gaaggttggg gtccttggtg    2880
   cgaaagggag gcagctgcag cctcagcccc accccagaag cggccttcgc atcgctgcgg    2940
   tgggcgttct cgggcttcga cttcgccagc gccgcggggc agaggcacct ggagctcgca    3000
   gggcccagac ctgggttgga aaagcttcgc tgactgcagg caagcgtccg ggaggggcgg   3060
   ccaggcgaag ccccggcgct ttaccacaca cttccgggtc ccatgccagt tgcatccgcg    3120
   gtattgggca ggaaatggca gggctgaggc cgaccctagg agtataaggg agccctccat    3180
   ttcctgccca catttgtcac ctccagtttt gcaacctatc ccagacacac agaaagcaag    3240
   caggactggt ggggagacgg agcttaacag gaatattttc cagcagtgag caggggctgt    3300
   atgggacgcg ggaggagctc agaggaggcg cggagagtgc ccgaggttgg gtgagtgcct    3360
   agaggggaga tagttgaacc gggttcaaga ggtgcttagt gggtgtttgt tgaatgaatg    3420
   agtgatgggc tttgaagtct gagtgcattg aaagaggggg tgtgtaaaaa gggctccttt    3480
   catcacacag gacacagcat atgcaaatcc tctccctgtg gaaaagccag acaggttaaa    3540
   aaggttacaa acaaattagc cgggcatggt ggtgcgcgtc tgtagtccca gctactaggg    3600
   aggctgagcc aggggaatcg cttgaacccg ggaggcggag attgcagtga gccaagatcg    3660
   cgccactgca ctccagcctg gaaacagagc gagactccgt ctcggaaaaa aaaaaaaaaa    3720
   gttacaaacc gtgtgtgggt ttcaggttat acaatcagag ctggagggga gtggtcaagg   3780
   atgagaactg agatggatcc ctcgttccct ctggaggaga gtgggtggtt gcctacttgg    3840
   gggtggggaa tccctctcca cgggctcagc tgtccaatct caggggatct ctaggacagg     3900
   agctgatgta aacagtcgcc ctattccttg ctgtctttgg ccctggagaa ggaggaggga    3960
   gctggggagg gtctccactt cccagacaat ctctaagcag ccaggacatg ggtgagatga    4020
   gtgagatact gacttctggg acagaatttg agagggtgcc aaaaaactca gtaatcaaga 4080
   taaataggcc gggcgcagtg gctcacgtct gtaatcccag cactttggga ggccggatca 4140
   cttgaggtca agagttcgag accagcctgg ccaagatggt gaaaccccat ctctactaaa 4200
   aatacaaaaa ttagcccagt gtggtggcgc tagcctgtaa tcccagccac tcaggaggct 4260
   gaggcaagag aattgcttga cccaggaggc agaggttgca gtgagccgag atcatgccac 4320
   tgtactccag cctggacaac agagggagac tatctcaaaa aaaaaaaaaa aaaaaaaaaa 4380
   aaaaaagagg ccgggcggcg gtggctcaca ccatgtgatc ccagcacttt gggaggccga 4440
   ggcgggtgga tcacctgagg tctggagttc gagaccagcc tggccaatat ggtgaaaccc 4500
   cgtttctact aaaaatacaa aaattagctg ggtggggtgg caggcacctg taatcccagc 4560
   tactccggag gctgaggcag gagaatccct tgaacctgcg ggcggaggtt tgcagtgaac 4620
   caagatcaca ccattgcact ccagcctgga caacaacagc aaaactctgt ctcaaaaaaa 4680
   aaaaaaatct tttttttcga gacacagttt tactctctcg cccaggttgg ggtgcagcac 4740
   cacgatctca gctcactgca acctctgcct ctcagattct cgtacctcag cctcccaagt 4800
   agctgggatt acaggtacct gtcaccacgc ccagctaatt tttgtatttt tagtaggggc 4860
   gtggtttcac catgttggcc aggctggtct tgaactcctg acctcaagtg acctgcccgc 4920
   ttcagccacc caaagtgctg ggattacagg cgtgagccac cacgcttggc ctttttaaat 4980
   gaaaatagtg caaaaatcca cgataaacaa aatatcaaaa atttactgaa cttgcacttc 5040
   cacaaccctt tctcacctgc ctcccaggct actctctgcc ccagaaagca acttaaaaaa 5100
   tgtgcagatg gagtttggac tttacctgaa aatggtggga gctatggaaa accttggagc 5160
   aggggagtga aggatagaaa ttatatgtaa aagaaaccct gggccgggcg cagtggctta 5220
   tgcctgtaat cccagcactt tgggaggccg aggcaggtgg attacctgag gtcaggagat 5280
   tgagaccagc ctgaccaaca tggtgaaatg tcatctctac taaaaataca aaaaaaatta 5340
   gccaggcatg gtggtgcacg cctgtagtcc cagctactcc ggaggctgag acaggaaaat 5400
   cgcttgaacc cgggaggcgg aggttgcagt gagccaagat tgtgccattg cactccagcc 5460
   tgggcaacaa gagcaaaact ccatcttaaa aaaaaagaaa gaaagaaacc ctctggcagt 5520
   tgatgagaag gaaacttaat cggcaggtcc cagcagggga gatgaggaga ctctagggag 5580
   ggcatttgca catgctgtgc cccagtgtgg gccagggagc aggtcactac tcctcccgtc 5640
   taccttcctc ttgctccaac cccttcaagc tttggaccag tggtacccta agtgtagtcc 5700
   aaggaaccac atgcatcagg acccccaggg ggtgcttgtt aaaaatgcaa attttggcca 5760
   ggtgcagtgg ctcacacctg taatcccagc actttgggag gccgaggcgg gtggatcacg 5820
   aggtcaggag atcgagacca tcctggcaaa cacggtgaaa ccccatctct actaaaaaaa 5880
   caaaaacaaa ccaaaaaaaa cattagctgg gcgtggtggc gggcgcctgt agtcccagct 5940
   actcgggagg ctgaggcagg agaatggcgt gaacccggga ggcggagctt gcagtgagct 6000
   gagattgcgc cactgcaccc cagcctgggc gacagagcga gactctgcct caaaaaaaaa 6060
   aaagcaaatt tcttgggcac caccccacat tgactg 6096
<210> 3
   <211> 2501
   <212> DNA
   <213> Homo Sapiens
<400> 3
   tttgcaaatg gagacatctt cattattcct atagtatcat atgtttttaa agtttgtact     60
   cacactttgg gtgataaatg aaggacaaga tccttcccta tccttgtgag gatgactaca    120
   gcatgactgg atgggcttgc tatgattttt atctttccct gtgttctcac taccgtttta    180
   ttaatctcag ttctttttca cagggtagca cagaatttaa ctagcagaaa gagatccagc    240
   catgtagacc agagatttgt ctaagtgacg gcatgtaaga atcaggaagg aaagtttttt    300
   gtttaaatac caacaggttc cttccttaaa gcaattatta tttttcaaat ctaacccaca    360
   aggtgatagt atccttaaac caattaaatc agaatctcgg gttggataac ctcaaatatg    420
   acttattagc acttcccatt aatcactggt ccttcaggcc tttaagttta cttactagga    480
   atctcacttt taataccatc ttatcaactt cagttgtaaa taagagaaca ctcaaaggct    540
   gaggaattct cagcggtaaa gctctgccca cgttaagtaa caaaggataa gttagtcttt    600
   gttgtgatca ctttgttgta ctgataagct acgtatttct actcaaggat tcaaattctc    660
   acctttctca agaattgggc caaaaccgat aaactaaact tatttacggt ccactgatta    720
   aaggttgttg cataataagt tcttgctatg ttcagcagtt ggattcacag cgccagaaac    780
   ctataactgc ttgactttcc tccccactac actgcgaaaa ttgcccctta aatgtaacta    840
   accctaaaac ctcaacagta tcgtggccag gcgtggtggc tcactactgt aataccaaca    900
   ttaggcatag gcgaggggat tgaggccagg atatcgaaac tagcctggga aacacacgga    960
   gacccggtct ttggaaaaat aattagcctt gcgtggtggt gggcgcgagg ttccggctaa   1020
   tcgggaggct acagtgagcc atgatgacac tgcactacag tctgcgcgac ggcccatgtc   1080
   agtaagctct ggagcacctg aaacaagttg tgttgggtat tttatttact ggagagcgat   1140
   tagtgactga tgcctactta cagcgactag agacgcatgc tccgatagca gcacaaactc   1200
   agcaggcgcg aacaaatggt aaagagaaac tgggcaaaca agcatcacgg ctcctcagct   1260
   gagaaagtgg gggccctaaa aagggccttt tgttgataga aagggacgct caaccaccga   1320
   aaccgtagag ggtgcggccc tggcgcttga gcgcgtagac cacatccatg gcggtgaccg   1380
   tcttgcgctt ggcgtgctct gtataggtca cggcgtcccg gatcacgttc tccaggaaca   1440
   ccttcagcac cccgcgagtc tcctcgtaga tgaggccgga gatgcgcttc acgccgccgc   1500
   ggcgagcaag gcgccggatg gccggcttgg tgatgccctg gatattgtcg cgcagtactt   1560
   tacggtggcg cttagcgccg cctttgccaa gacccttccc gcctttgccg cggccagaca   1620
   tgacgagcaa gaggagtctc acccaacgct ttgtgaggac tctggcctga ggcagcgcct   1680
   ttatacgaca gttggcggac cgaactgaga acctgaaaga agtcggcggg aagtcccgcc   1740
   ccggtggggg aggggaaatc taaagggcca aaccgaaata gggggaaaaa aaaagcgagc   1800
   ttcttgtttc cgtgttctga attttgtaac gtgcatagta ttttgttacc acgttatgag   1860
   gctttaaaaa attgcttttg aacgcagaag atatacatca atactgtggg aaatacaaga   1920
   aaggacaaga aattaagaaa ctacaatgtt atcccatcac acaggctagt taatcatgta   1980
   ttttgcagag cagttgcaca tatttttcca agaaaatgta tacagtgttg tatatggagt   2040
   tttgtaacct ccttatattg attataattt aaccaatttc tattaaagag ataaaagtga   2100
   tgttttggtg tctatgtttc ttaggaatta tcaatagtta taatcagttc cccagcaatt   2160
   ttttaatcgg ctgtatttta aaaataatgt tttccacatt caacataaat gtactttttc   2220
   tctatacttg ggaccaatat tgaaatttat gattttatta caccaaaatt taaattttat   2280
   tacattaata tttaaaattg tattagaggt ctcatgattt ggtactacgg gtctccgcat   2340
   tatttccttt ccaaatttcc taatctgttt caccaaggtt tctggacaac tttagagacc   2400
   ttttgtgaag tttgaataaa atctcttcga gattttgata attgcattag ctttaggact   2460
   taattggaat agaattaaaa tccttaaaac aagctcttat a                        2501
<210> 4
   <211> 2501
   <212> DNA
   <213> Homo Sapiens
<400> 4
   ttgttgtaca gaatatttca tcacccaggt attatgccga gtacccaata gttctctttt 60
   ctgctcctct ccttcctccc atcctgcacc ctggagtcaa ccacagtgtc tgttgtttcc 120
   ttgtttgtgt tataagttct catcatttag ctcccactta caagtgagaa catccagtat 180
   ttggatttct gttcctgcat tagtttgcta aggataatag cctctagctc catccatgtt 240
   cccacaaaag acatgatcta gttcttttta atggctgcat taaatgaagt tttaaagata 300
   caacataaac accaacctct tccccaccac aaaaatccct tgctgaattt gattacactt 360
   aaattaacga gttttgtttc atgaaagact ccttggacaa acttgacagt tgatggaata 420
   ggagaagctg tctgtcatgt ctaaagccaa caagagatca atatctagaa taaatggaga 480
   tctgcaaatc aacagaaagt aggcagcaaa gccaaagaaa atagcctaag gcacagccac 540
   taaaaggaac gtgatcatgt cctttgcagg gacatgggtg gagctggaag ccgttagcct 600
   cagcaaactc acacaggaac agaaaaccag cgagaccgca tggtctcact tataagtggg 660
   agctgaacaa tgagaacaca tggtcacatg gcggcgatca acacacactg gtgcctgttg 720
   agcggggtgc tggggaggga gagtaccagg aagaatagct aagggatact gggcttaata 780
   cctgggtgat gggatgatct gtacagcaaa ccatcatggc gcacacacct atgtaacaaa 840
   cctgcacatc ctctacatgt accccagaac ttcaaataaa agttggacgg ccaggcgtgg 900
   tggctcacgc ctgtaatccc agcactttgg gaagccgagg cgtgcagatc acctaaggtc 960
   aggagttcga gaccagcccg gccaacatgg tgaaaccccg tctctactaa aaatacaaaa 1020
   atcagccaga tgtggcacgc acctataatt ccacctactc gggaggctga agcagaattg 1080
   cttgaacccg agaggcggag gttgcagtga gccgccgaga tcgcgccact gcactccagc 1140
   ctgggccaca gcgtgagact acgtcataaa ataaaataaa ataacacaaa ataaaataaa 1200
   ataaaataaa ataaaataaa ataaaataaa ataaaataaa ataaaaaaat aaaataaaat 1260
   aaaataaaat aaagcaattt cctttcctct aagcggcctc cacccctctc ccctgccctg 1320
   tgaagcgggt gtgcaagctc cgggatcgca gcggtcttag ggaatttccc cccgcgatgt 1380
   cccggcgcgc cagttcgctg cgcacacttc gctgcggtcc tcttcctgct gtctgtttac 1440
   tccctaggcc ccgctgggga cctgggaaag agggaaaggc ttccccggcc agctgcgcgg 1500
   cgactccggg gactccaggg cgcccctctg cggccgacgc ccggggtgca gcggccgccg 1560
   gggctggggc cggcgggagt ccgcgggacc ctccagaaga gcggccggcg ccgtgactca 1620
   gcactggggc ggagcggggc gggaccaccc ttataaggct cggaggccgc gaggccttcg 1680
   ctggagtttc gccgccgcag tcttcgccac cagtgagtac gcgcggcccg cgtccccggg 1740
   gatggggctc agagctccca gcatggggcc aacccgcagc atcaggcccg ggctcccggc 1800
   agggctcctc gcccacctcg agacccggga cgggggccta ggggacccag gacgtcccca 1860
   gtgccgttag cggctttcag gggcccggga gcgcctcggg gagggatggg accccggggg 1920
   cggggagggg gggcagactg cgctcaccgc gccttggcat cctcccccgg gctccagcaa 1980
   acttttcttt gttcgctgca gtgccgccct acaccgtggt ctatttccca gttcgaggta 2040
   ggagcatgtg tctggcaggg aagggaggca ggggctgggg ctgcagccca cagcccctcg 2100
   cccacccgga gagatccgaa cccccttatc cctccgtcgt gtggctttta ccccgggcct 2160
   ccttcctgtt ccccgcctct cccgccatgc ctgctccccg ccccagtgtt gtgtgaaatc 2220
   ttcggaggaa cctgtttccc tgttccctcc ctgcactcct gacccctccc cgggttgctg 2280
   cgaggcggag tcggcccggt ccccacatct cgtacttctc cctccccgca ggccgctgcg 2340
   cggccctgcg catgctgctg gcagatcagg gccagagctg gaaggaggag gtggtgaccg 2400
   tggagacgtg gcaggagggc tcactcaaag cctcctgcgt aagtgaccat gcccgggcaa 2460
   ggggaggggg tgctgggcct tagggggctg tgactaggat c 2501
<210> 5
   <211> 1920
   <212> DNA
   <213> Artificial Sequence
<220>
   <223> chemically treated genomic DNA (Homo sapiens)
<400> 5
   ttaggttgtc gtagatatag tttttgtttt ttcgaaaaat acgttttagg cgtcgggatt      60
   ttagatattt gggaaataga gtgtacgtag ttgttgagag gtttcgcgtt tggttttttt     120
   tattattgag gcgtagaggt gttgtggata gtttagattt atacggcgtt cgaggtgaaa     180
   tagaattttt agttttttta tgaggttatt ggtattttcg gttgttttta gagtttttcg      240
   atttagagtt gaatgtaaag taagcgttcg aaatgtagaa gtagtcgggg tcgtttacgg     300
   tatttgtttc gttcggggcg agagaagacg ttaggttgag gttttagcga ttttaggtat     360
   tagtttcgaa ggagggcggg gagatcgtaa aggggaagtg ttcggagggt taacggtttt     420
   cgcgtatttt gcgttttttt gaagcgcgtc gttttttcgc gtcggggatt gggatttgtt     480
   tttggggaat tcgtttagaa gacggcggcg gattggggtc gggtattttt tagggttgtt     540
   aggttttttt tagttttgta tttgtcgcgt cgttttattt cgttaggaag ttttagagat     600
   ttcggggatg gggtgggagc gtttttttat cgcgggttta aaaagaagga aggacgtttt      660
   taggggtcgt agaaggagga ttagttttaa gttataattt ttttcggatt taaggtaggt     720
   cggttggggt ttcgcgttta tacggttttt ggcgggggtt cgcgcgtttc gggagtttcg     780
   cggttcgggg aggaaagagg agataagaga taggcgagga ttacggggtt gatttagtcg     840
   gggtagggat tatcgtggaa aaattttggc gaggtggggg gacgcggaaa gagagcggtt     900
   cgcgttttgt attttgcgtc gggtatttcg cgttagtgtt tcgtttttag tgtttcgcgt     960
   ttcgcgtttc gcgttttgtt tttatttcgg gttagttgta tcgcgttcgc gtcgtaggaa    1020
   tcgtggagtt ggaaagtggg ggcgtcgcgg ttggggggtt gttttagttg cgtttcggtt    1080
   agcgatcggc gggtcgggtt taaatttagt taggttgggt aggcggtggt cgcgcgattg    1140
   gggatcgggc gtttcgtttt tttcgttttt tttttttttt tttttttttt ttttagtttt    1200
   ttggtttttt ttagttttta tcggatttgt tcgttcgttg tttttttttt tttttcgttt    1260
   tttatattat tttttatttt ttcgttttgt tttcgttttt tttttttttt ttgttttttg   1320
   tttttttttt tttttttttt ttttttaggg gcggagtttt tttttttttt tttagataat   1380
   gttgtggttg cgtttttttt ttcgttagtt cgtttaggtt ttcgtcgtta gcgatttttt    1440
   cgggttgggg gtggggaggt ggggggggag tgtagggttg gggaggatga gttggttttt     1500
   tttatttttt tgttgttgtt ttttttaaga gggatggaga tttggtttaa gtttttcggt     1560
   ttattcggag ttgtgatagt tatttttagg gaatagttac gttgttttat taagtttatt     1620
   tttagcggtt tggatttttt aggtagaggt tgtgggattt tgtttttttt aatattttag    1680
   tttattttta aaagggtttg agtcggatag gggttaaata ggttttttcg atttggcggg     1740
   tcggttagac gtgatagtaa tgttaaggag gttaagtttt tttgtttatt ttttattttt    1800
   ttttttttta tttttggatt ttttggcgtt tttagtatat agaggttttt gagtagttcg    1860
   gttgtaggtt ttttatttat ttagagtttt tttttttacg tgtttatttt taattttgta    1920
<210> 6
   <211> 1920
   <212> DNA
   <213> Artificial Sequence
<220>
   <223> chemically treated genomic DNA (Homo sapiens)
<400> 6
   tgtagggttg gggataggta cgtgaggggg agaattttga gtagataggg aatttgtagt 60
   cgggttgttt aagggttttt gtgtattggg ggcgttagga ggtttaggga tggaaaaggg 120
   ggaggtgaga aatggataaa gaaatttggt ttttttggta ttgttgttac gtttggtcgg 180
   ttcgttaagt cgaaggggtt tgtttagttt ttgttcggtt taaatttttt tgggaataag 240
   ttgggatgtt agaaaaaata aaattttata atttttgttt ggggaattta ggtcgttgga 300
   ggtgggtttg gtagggtagc gtgattgttt tttgggagtg gttgttatag tttcgggtga 360
   atcgaggagt ttgggttaag tttttatttt ttttggagag ggtagtagta aggaggtgag 420
   gggagttagt ttattttttt taattttgta tttttttttt atttttttat ttttagttcg 480
   gaagaatcgt tggcggcggg agtttggacg agttggcggg gaaggggacg tagttatagt     540
   attgtttggg agggagggga gaagtttcgt ttttagaggg gaggggagaa gaggaggggg     600
   taggagataa ggggaggaag aaaggcgaag gtaaggcgaa ggggtggaga gtgatatgaa 660
   gagcgagaga aaagagagga tagcggacga gtagattcgg taggggttga aaaaggttaa 720
   ggggttggag ggagggagag gaaggaggag gggagcgagg agggcggggc gttcggtttt 780
   tagtcgcgcg gttatcgttt gtttagtttg gttggatttg agttcggttc gtcgatcgtt 840
   ggtcgaggcg tagttgaagt agttttttag tcgcggcgtt tttatttttt aattttacgg 900
   tttttgcggc gcgggcgcga tgtagttggt tcggggtgaa ggtaaggcgc ggggcgcggg 960
   gcgcggggta ttgggagcga ggtattggcg cgggatgttc ggcgtaaggt gtagggcgcg 1020
   ggtcgttttt ttttcgcgtt tttttatttc gttaaagttt ttttacgatg gtttttattt 1080
   cggttgggtt agtttcgtaa ttttcgtttg ttttttgttt tttttttttt tttcgagtcg 1140
   cggggttttc ggggcgcgcg gattttcgtt aggggtcgtg taggcgcgga gttttagtcg 1200
   gtttgttttg ggttcgaaga aagttgtggt ttggagttag tttttttttt acgatttttg 1260
   ggggcgtttt tttttttttt tgagttcgcg atgggaaggc gtttttattt tattttcggg 1320
   gtttttgaga ttttttggcg aggtggggcg gcgcggtagg tgtagggttg gggagggttt 1380
   gatagttttg gagagtgttc gattttagtt cgtcgtcgtt ttttaggcgg attttttaga 1440
   ggtaggtttt agttttcggc gcggggaggc ggcgcgtttt agaggggcgt agggtgcgcg 1500
   ggggtcgttg gtttttcggg tatttttttt ttgcggtttt ttcgtttttt ttcggagttg 1560
   gtgtttgagg tcgttgggat tttagtttgg cgtttttttt cgtttcgagc gaggtaggtg 1620
   tcgtgggcgg tttcggttat ttttgtattt cgagcgttta ttttgtattt agttttaagt 1680
   cggagagttt tggggatagt cgagagtgtt agtggtttta tagggagatt gagggttttg 1740
   ttttatttcg ggcgtcgtgt gggtttgggt tgtttatagt atttttgcgt tttagtgata 1800
   ggagaagtta agcgcgaggt tttttaatag ttgcgtgtat tttatttttt aggtatttgg 1860
   aatttcggcg tttagaacgt gtttttcggg agagtaaagg ttgtgtttac ggtagtttgg 1920
<210> 7
   <211> 6096
   <212> DNA
   <213> Artificial Sequence
<220>
   <223> chemically treated genomic DNA (Homo sapiens)
<400> 7
   tattagtgta agatttaaaa tttttttttt gtattgtata gtgagatgtt tagggtttta      60
   gtttagtgtt tggatatagc gatttttggg tttgttcgtc gtcgttttaa gcgaagttgg     120
   tgcgttttgg gcggagtaga tagagatttt gggtggtagg ggtttgggaa gatatgggcg     180
   gttagggttt tatgcgtttt tatcgttgtt ttttgttatt tgtaggtaat ggacgagtcg     240
   ggaatgagtt ttttagatta gttcgtgatt aagaaaggta aggaatggtt tgttagggta     300
   gagttcggcg agatggtgta ggtttttggt gtatagattt atttttttta tcggtcgtgt     360
   tttttgtgtg tcgttaggtt gggtgtttat taggtatttt ttttggttta gttagatgtt     420
   aggtagacgt gcgggtttgg tgagtttgtt tagtattttg tggtttgggg tgggttttag     480
   cggattagta tttattgggt tgtagtattg ggagtttggt tttttttcgt cgagggggag      540
   ggtatttttg tggatttgga gttgatttgt agaacgagtt aaattatttt tttgtttttt      600
   taagagatgg gaatggaagt gttgttttta cggagttggg gaaatgattt ttattttata     660
   gtgttttagt atttcggtgt ttggcgggta tttttttttt ttttttttta ggtagggttt     720
   tggaggtttt tgggggaatt ttttttttgt gggagttttt tgcggtattt agatttaggg      780
   gagtttgtgt gtgagtattg tgtgttaggt tgtgtgtatt tgagttaggg tttatttgtt     840
   tttgggtgtt tgtgtttatg tgagtttagg gttttgtgta tgtttgaaat gtttttttta    900
   tgggtgtttt agtatttttt ggagtgtgag tgtgtttgtt tttgtgaatg tgtttgtgag     960
   gtgtgttttt gtatgttggt gtgtattttt ttgtatttgg gggatgtata tattttttaa   1020
   tatgtatagt atttttgttg tgttttgtat tttgtttttt ggtatttgag gatttttaag    1080
   tatgcgcggg ttttttttgt gtatatatag gagtatttat gtgatttttg gtattagtaa      1140
   aatttaggga tacgggattt attttttttg gtttgaggat taagtattgg ttatgatagg    1200
   ggaaggtgag agacgataaa aatagagaga tagttagaga ggagtagaga gttagagggg 1260
   tttaggtatt gggtagtagt ttttttatat ttggggtagg tgttcgaaag aatttagagg 1320
   tgtatatgag tttgaggtgt tttaggtagg tattgttttt atagggtttg gtttgagttg 1380
   ttttttaaac gagtgaattt aagtttgggt tttatttgtt ttttatttgt ttttagggga 1440
   ggttaaggtg gaagtggtgg tagtagggtt ggggttggat ttttaggagt tggggttgag 1500
   ttattaggag ttgggggttg ggtggatgat ttggagtgtg tagtagggaa gatgaggtaa 1560
   tagggtagga agtgggtggg gggaggtgga attggggttg tgttttgtgt cgtttggaat 1620
   tgggagtgtg ggaaagatat taggaatttg gttgtagcgt agttttgttg gtggggtttg 1680
   gttggtttat tgtatagagt tttttttgat ttttgaagaa agagattcgt ttgtagtggg 1740
   taaaagtttg tttggatttt ttggttatta gaaatatgag tatggtggtg gtttttagtt 1800
   ttttatttat gtttgggttt aagagattgg gagtttaggt ttattgattt tttgagaaag 1860
   attaagattt tgtattttag aaagaggttt ggggattttt gttttgcgta agggtagaag 1920
   gattagttgt ttttttgagt attttaattc ggaatttcgg ttcgaagtcg agataggaga 1980
   ttggatgcga ggttttttta gagttggttt tttttaaata atttttaaaa tttttagatt 2040
   ttaggggtac gtcgaaattt tttaaagtag tttaaagaat ataacgagag ttttaatatt 2100
   ttaggtggcg gcgcgttggt tttttggagc ggggcgggac gcggtcgcgc ggatttacgt 2160
   gtataatcgc gcgggacggg gttacgcgga tttacgtgta taatcgcggg attttagcgt 2220
   tagcgggatt ttagcgttag cgggatttta gcgttagcgg gattttagcg ttagcgggat 2280
   tttagcgtta gcgggatttt agcgttagcg ggattttagc gttagcgggt ttgtggttta 2340
   gtggagcgag tggagcgttg gcgatttgag cggagattgc gttttggacg ttttagttta 2400
   gacgttaagt tatagttcgc gtagtagtag taaaggggaa ggggtaggag tcgggtatag 2460
   ttggattcgg aggtcgtgat ttaggggaaa gcgtgggcgg tcgatttagg gtagttgcgg 2520
   cggcgaggta ggtgggtttt ttgttttttg gagtcgtttt tttttatatt tgttttcggc 2580
   gtttttagta gtttttattt tggtttttcg cggttattgc gggattcggc gttgtcgtta 2640
   gtttagtggg gagtgaatta gcgttttttt tcgttttcgg ttttttcgac ggtacgagga 2700
   atttttgttt tgttttatag attttcggtt ttcgtcgagt gcggtattgg agtttgtttc 2760
   gttagggttt tggaattaga gaaagtcgtt ttttggttat ttgaagcgtc ggatttttat 2820
   agtgtttttt agtttgggcg ggagcggcgg ttgcgtcgtt gaaggttggg gtttttggtg 2880
   cgaaagggag gtagttgtag ttttagtttt attttagaag cggttttcgt atcgttgcgg 2940
   tgggcgtttt cgggtttcga tttcgttagc gtcgcggggt agaggtattt ggagttcgta 3000
   gggtttagat ttgggttgga aaagtttcgt tgattgtagg taagcgttcg ggaggggcgg 3060
   ttaggcgaag tttcggcgtt ttattatata ttttcgggtt ttatgttagt tgtattcgcg 3120
   gtattgggta ggaaatggta gggttgaggt cgattttagg agtataaggg agttttttat 3180
   tttttgttta tatttgttat ttttagtttt gtaatttatt ttagatatat agaaagtaag 3240
   taggattggt ggggagacgg agtttaatag gaatattttt tagtagtgag taggggttgt 3300
   atgggacgcg ggaggagttt agaggaggcg cggagagtgt tcgaggttgg gtgagtgttt 3360
   agaggggaga tagttgaatc gggtttaaga ggtgtttagt gggtgtttgt tgaatgaatg 3420
   agtgatgggt tttgaagttt gagtgtattg aaagaggggg tgtgtaaaaa gggttttttt 3480
   tattatatag gatatagtat atgtaaattt tttttttgtg gaaaagttag ataggttaaa 3540
   aaggttataa ataaattagt cgggtatggt ggtgcgcgtt tgtagtttta gttattaggg 3600
   aggttgagtt aggggaatcg tttgaattcg ggaggcggag attgtagtga gttaagatcg 3660
   cgttattgta ttttagtttg gaaatagagc gagatttcgt ttcggaaaaa aaaaaaaaaa 3720
   gttataaatc gtgtgtgggt tttaggttat ataattagag ttggagggga gtggttaagg 3780
   atgagaattg agatggattt ttcgtttttt ttggaggaga gtgggtggtt gtttatttgg 3840
   gggtggggaa ttttttttta cgggtttagt tgtttaattt taggggattt ttaggatagg 3900
   agttgatgta aatagtcgtt ttattttttg ttgtttttgg ttttggagaa ggaggaggga 3960
   gttggggagg gtttttattt tttagataat ttttaagtag ttaggatatg ggtgagatga 4020
   gtgagatatt gatttttggg atagaatttg agagggtgtt aaaaaattta gtaattaaga 4080
   taaataggtc gggcgtagtg gtttacgttt gtaattttag tattttggga ggtcggatta 4140
   tttgaggtta agagttcgag attagtttgg ttaagatggt gaaattttat ttttattaaa 4200
   aatataaaaa ttagtttagt gtggtggcgt tagtttgtaa ttttagttat ttaggaggtt 4260
   gaggtaagag aattgtttga tttaggaggt agaggttgta gtgagtcgag attatgttat 4320
   tgtattttag tttggataat agagggagat tattttaaaa aaaaaaaaaa aaaaaaaaaa 4380
   aaaaaagagg tcgggcggcg gtggtttata ttatgtgatt ttagtatttt gggaggtcga 4440
   ggcgggtgga ttatttgagg tttggagttc gagattagtt tggttaatat ggtgaaattt 4500
   cgtttttatt aaaaatataa aaattagttg ggtggggtgg taggtatttg taattttagt 4560
   tatttcggag gttgaggtag gagaattttt tgaatttgcg gggcggaggt tgtagtgaat 4620
   taagattata ttattgtatt ttagtttgga taataatagt aaaattttgt tttaaaaaaa 4680
   aaaaaaattt tttttttcga gatatagttt tattttttcg tttaggttgg ggtgtagtat 4740
   tacgatttta gtttattgta atttttgttt tttagatttt cgtattttag ttttttaagt 4800
   agttgggatt ataggtattt gttattacgt ttagttaatt tttgtatttt tagtaggggc 4860
   gtggttttat tatgttggtt aggttggttt tgaatttttg attttaagtg atttgttcgt 4920
   tttagttatt taaagtgttg ggattatagg cgtgagttat tacgtttggt ttttttaaat 4980
   gaaaatagtg taaaaattta cgataaataa aatattaaaa atttattgaa tttgtatttt   5040
   tataattttt ttttatttgt tttttaggtt attttttgtt ttagaaagta atttaaaaaa   5100
   tgtgtagatg gagtttggat tttatttgaa aatggtggga gttatggaaa attttggagt   5160
   aggggagtga aggatagaaa ttatatgtaa aagaaatttt gggtcgggcg tagtggttta   5220
   tgtttgtaat tttagtattt tgggaggtcg aggtaggtgg attatttgag gttaggagat   5280
   tgagattagt ttgattaata tggtgaaatg ttatttttat taaaaatata aaaaaaatta   5340
   gttaggtatg gtggtgtacg tttgtagttt tagttatttc ggaggttgag ataggaaaat   5400
   cgtttgaatt cgggaggcgg aggttgtagt gagttaagat tgtgttattg tattttagtt   5460
   tgggtaataa gagtaaaatt ttattttaaa aaaaaagaaa gaaagaaatt ttttggtagt   5520
   tgatgagaag gaaatttaat cggtaggttt tagtagggga gatgaggaga ttttagggag   5580
   ggtatttgta tatgttgtgt tttagtgtgg gttagggagt aggttattat ttttttcgtt   5640
   tatttttttt ttgttttaat ttttttaagt tttggattag tggtatttta agtgtagttt   5700
   aaggaattat atgtattagg atttttaggg ggtgtttgtt aaaaatgtaa attttggtta   5760
   ggtgtagtgg tttatatttg taattttagt attttgggag gtcgaggcgg gtggattacg   5820
   aggttaggag atcgagatta ttttggtaaa tacggtgaaa ttttattttt attaaaaaaa   5880
   taaaaataaa ttaaaaaaaa tattagttgg gcgtggtggc gggcgtttgt agttttagtt   5940
   attcgggagg ttgaggtagg agaatggcgt gaattcggga ggcggagttt gtagtgagtt   6000
   gagattgcgt tattgtattt tagtttgggc gatagagcga gattttgttt taaaaaaaaa   6060
   aaagtaaatt ttttgggtat tattttatat tgattg                             6096
<210> 8
   <211> 6096
   <212> DNA
   <213> Artificial Sequence
<220>
   <223> chemically treated genomic DNA (Homo sapiens)
<400> 8
   tagttaatgt ggggtggtgt ttaagaaatt tgtttttttt tttttgaggt agagtttcgt     60
   tttgtcgttt aggttggggt gtagtggcgt aattttagtt tattgtaagt ttcgtttttc    120
   gggtttacgt tatttttttg ttttagtttt tcgagtagtt gggattatag gcgttcgtta    180
   ttacgtttag ttaatgtttt ttttggtttg tttttgtttt tttagtagag atggggtttt    240
   atcgtgtttg ttaggatggt ttcgattttt tgatttcgtg atttattcgt ttcggttttt    300
   taaagtgttg ggattatagg tgtgagttat tgtatttggt taaaatttgt atttttaata    360
   agtatttttt gggggttttg atgtatgtgg ttttttggat tatatttagg gtattattgg    420
   tttaaagttt gaaggggttg gagtaagagg aaggtagacg ggaggagtag tgatttgttt    480
   tttggtttat attggggtat agtatgtgta aatgtttttt ttagagtttt tttatttttt    540
   ttgttgggat ttgtcgatta agtttttttt ttattaattg ttagagggtt tttttttttt    600
   ttttttttta agatggagtt ttgtttttgt tgtttaggtt ggagtgtaat ggtataattt    660
   tggtttattg taattttcgt ttttcgggtt taagcgattt ttttgtttta gttttcggag    720
   tagttgggat tataggcgtg tattattatg tttggttaat tttttttgta tttttagtag    780
   agatgatatt ttattatgtt ggttaggttg gttttaattt tttgatttta ggtaatttat    840
   ttgtttcggt tttttaaagt gttgggatta taggtataag ttattgcgtt cggtttaggg    900
   ttttttttat atataatttt tattttttat ttttttgttt taaggttttt tatagttttt    960
   attattttta ggtaaagttt aaattttatt tgtatatttt ttaagttgtt ttttggggta   1020
   gagagtagtt tgggaggtag gtgagaaagg gttgtggaag tgtaagttta gtaaattttt   1080
   gatattttgt ttatcgtgga tttttgtatt atttttattt aaaaaggtta agcgtggtgg   1140
   tttacgtttg taattttagt attttgggtg gttgaagcgg gtaggttatt tgaggttagg   1200
   agtttaagat tagtttggtt aatatggtga aattacgttt ttattaaaaa tataaaaatt   1260
   agttgggcgt ggtgataggt atttgtaatt ttagttattt gggaggttga ggtacgagaa   1320
   tttgagaggt agaggttgta gtgagttgag atcgtggtgt tgtattttaa tttgggcgag   1380
   agagtaaaat tgtgtttcga aaaaaaagat tttttttttt tttgagatag agttttgttg   1440
   ttgttgttta ggttggagtg taatggtgtg attttggttt attgtaattt tcgtttcgta   1500
   ggtttaaggg atttttttgt tttagttttc ggagtagttg ggattatagg tgtttgttat   1560
   tttatttagt taatttttgt atttttagta gaaacggggt tttattatat tggttaggtt   1620
   ggtttcgaat tttagatttt aggtgattta ttcgtttcgg ttttttaaag tgttgggatt   1680
   atatggtgtg agttatcgtc gttcggtttt tttttttttt tttttttttt tttttttttt   1740
   gagatagttt ttttttgttg tttaggttgg agtatagtgg tatgatttcg gtttattgta   1800
   atttttgttt tttgggttaa gtaatttttt tgttttagtt ttttgagtgg ttgggattat   1860
   aggttagcgt tattatattg ggttaatttt tgtattttta gtagagatgg ggttttatta   1920
   ttttggttag gttggtttcg aatttttgat tttaagtgat tcggtttttt aaagtgttgg   1980
   gattatagac gtgagttatt gcgttcggtt tatttatttt gattattgag ttttttggta 2040
   tttttttaaa ttttgtttta gaagttagta ttttatttat tttatttatg ttttggttgt 2100
   ttagagattg tttgggaagt ggagattttt tttagttttt tttttttttt ttagggttaa 2160
   agatagtaag gaatagggcg attgtttata ttagtttttg ttttagagat tttttgagat 2220
   tggatagttg agttcgtgga gagggatttt ttatttttaa gtaggtaatt atttattttt 2280
   ttttagaggg aacgagggat ttattttagt ttttattttt gattattttt ttttagtttt 2340
   gattgtataa tttgaaattt atatacggtt tgtaattttt tttttttttt ttcgagacgg 2400
   agtttcgttt tgtttttagg ttggagtgta gtggcgcgat tttggtttat tgtaattttc 2460
   gtttttcggg tttaagcgat ttttttggtt tagttttttt agtagttggg attatagacg 2520
   cgtattatta tgttcggtta atttgtttgt aattttttta atttgtttgg tttttttata 2580
   gggagaggat ttgtatatgt tgtgttttgt gtgatgaaag gagttttttt tatatatttt 2640
   tttttttaat gtatttagat tttaaagttt attatttatt tatttaataa atatttatta 2700
   agtatttttt gaattcggtt taattatttt ttttttaggt atttatttaa tttcgggtat 2760
   ttttcgcgtt ttttttgagt ttttttcgcg ttttatatag tttttgttta ttgttggaaa 2820
   atatttttgt taagtttcgt ttttttatta gttttgtttg ttttttgtgt gtttgggata 2880
   ggttgtaaaa ttggaggtga taaatgtggg taggaaatgg agggtttttt tatattttta 2940
   gggtcggttt tagttttgtt attttttgtt taatatcgcg gatgtaattg gtatgggatt 3000
   cggaagtgtg tggtaaagcg tcggggtttc gtttggtcgt ttttttcgga cgtttgtttg 3060
   tagttagcga agttttttta atttaggttt gggttttgcg agttttaggt gtttttgttt 3120
   cgcggcgttg gcgaagtcga agttcgagaa cgtttatcgt agcgatgcga aggtcgtttt 3180
   tggggtgggg ttgaggttgt agttgttttt ttttcgtatt aaggatttta atttttagcg 3240
   acgtagtcgt cgttttcgtt taggttggga ggtattgtag ggattcgacg ttttaggtgg 3300
   ttaaagagcg attttttttg attttagggt tttggcgggg taggttttag tatcgtattc 3360
   ggcggaggtc gaaggtttgt ggggtaggat aggagttttt cgtgtcgtcg gaagggtcga 3420
   ggacgaagga gggcgttaat ttatttttta ttgggttggc ggtaacgtcg aatttcgtag 3480
   tgatcgcgga gggttaaggt gaaaattgtt gggggcgtcg agggtaggtg tggggagggg 3540
   cggttttagg gagtaaggag tttatttgtt tcgtcgtcgt agttgttttg ggtcgatcgt 3600
   ttacgttttt ttttgggtta cgattttcgg atttaattgt gttcggtttt tgtttttttt 3660
   tttttgttgt tgttgcgcgg gttgtaattt gacgtttagg ttggggcgtt tagggcgtag 3720
   ttttcgttta ggtcgttagc gttttattcg ttttattggg ttatagattc gttggcgttg 3780
   gggtttcgtt ggcgttgggg tttcgttggc gttggggttt cgttggcgtt ggggtttcgt 3840
   tggcgttggg gtttcgttgg cgttggggtt tcgttggcgt tggggtttcg cggttgtgta 3900
   cgtgagttcg cgtggtttcg tttcgcgcgg ttgtgtacgt gagttcgcgc ggtcgcgttt 3960
   cgtttcgttt tagggagtta gcgcgtcgtt atttgggatg ttaggatttt cgttgtgttt 4020
   tttggattgt tttgggggat ttcggcgtat ttttaggatt taggagtttt ggaagttgtt 4080
   tgagagaaat tagttttggg agggtttcgt atttagtttt ttgtttcggt ttcggatcgg 4140
   ggtttcgggt taaggtgttt agaggaatag ttgatttttt tatttttgcg tagggtagag 4200
   atttttaaat ttttttttaa aatgtagggt tttagttttt tttagggagt tagtgaattt 4260
   agatttttag ttttttgagt ttaagtatga atagggaatt ggggattatt attatgttta 4320
   tatttttggt ggttaggaag tttaggtagg tttttgttta ttgtagacgg attttttttt 4380
   ttaggggtta agaaaggttt tgtatagtaa gttaattaag ttttattagt agagttgcgt 4440
   tgtaattagg tttttagtgt tttttttata tttttagttt taagcgatat aggatatagt 4500
   tttaatttta ttttttttta tttatttttt gttttgttgt tttatttttt ttgttatata 4560
   ttttaagtta tttatttaat ttttagtttt tggtaattta gttttagttt ttggaagttt 4620
   agttttagtt ttgttattat tatttttatt ttggtttttt ttgagaataa gtggagggta 4680
   aatagagttt aggtttgaat ttattcgttt gaaaaataat ttaagttaaa ttttgtggga 4740
   gtagtgtttg tttggggtat tttaggttta tgtgtatttt tgaatttttt cgggtatttg 4800
   ttttaaatgt aaagaggttg ttatttaatg tttgggtttt tttgattttt tgtttttttt 4860
   tggttgtttt tttgtttttg tcgtttttta tttttttttg ttatggttag tatttggttt 4920
   ttaggttaga aaaggtggat ttcgtgtttt tggattttat taatgttagg agttatataa 4980
   atatttttat atatatatag agagggttcg cgtatgtttg gaaattttta gatattaaag 5040
   aataaagtgt aggatataat agagatattg tatatattga gaaatgtgta tattttttaa 5100
   atgtagagaa atgtatatta atatatagag atatatttta taaatatatt tatagaaata 5160
   gatatattta tattttaaga aatattaaga tatttatgaa gggaatattt tagatatgta 5220
   taggattttg aatttatatg gatatagata tttaggagta ggtgggtttt gatttaggtg 5280
   tatatagttt aatatatagt atttatatat aagttttttt aagtttaaat gtcgtaagag 5340
   atttttatag aaagaaaatt tttttagagg tttttaaggt tttgtttgga aggaagagga 5400
   agaaagtgtt cgttaggtat cgaaatgtta aggtattgta aagtgaaaat tattttttta 5460
   atttcgtggg aatagtattt ttatttttat tttttaggga aatagggaag tggtttaatt 5520
   cgttttgtaa attaatttta gatttataag agtgtttttt ttttcggcgg ggagaggtta 5580
   ggtttttagt gttgtagttt agtgaatgtt gattcgttga ggtttatttt aggttatagg 5640
   gtgttgggta aatttattaa gttcgtacgt ttgtttaata tttggttggg ttaggaagag 5700
   tgtttggtgg atatttagtt tggcgatata taggaggtac ggtcggtgaa gaaaatggat 5760
   ttgtgtatta agggtttgta ttatttcgtc gggttttgtt ttgatagatt attttttatt 5820
   ttttttgatt acggattggt ttaggaggtt tattttcggt tcgtttattg tttgtaaata 5880
   gtagagggta gcggtgaggg cgtataaagt tttagtcgtt tatgtttttt taagtttttg 5940
   ttatttaggg tttttgtttg tttcgtttaa ggcgtattag tttcgtttgg ggcggcgacg 6000
   ggtaggttta ggaatcgtta tgtttaggta ttgagttgga gttttgggta ttttattgtg 6060
   tagtgtaaaa agggaatttt gaattttata ttggta 6096
<210> 9
   <211> 2501
   <212> DNA
   <213> Artificial Sequence
<220>
   <223> chemically treated genomic DNA (Homo sapiens)
<400> 9
   tttgtaaatg gagatatttt tattattttt atagtattat atgtttttaa agtttgtatt 60
   tatattttgg gtgataaatg aaggataaga ttttttttta tttttgtgag gatgattata 120
   gtatgattgg atgggtttgt tatgattttt attttttttt gtgtttttat tatcgtttta 180
   ttaattttag ttttttttta tagggtagta tagaatttaa ttagtagaaa gagatttagt 240
   tatgtagatt agagatttgt ttaagtgacg gtatgtaaga attaggaagg aaagtttttt 300
   gtttaaatat taataggttt tttttttaaa gtaattatta ttttttaaat ttaatttata 360
   aggtgatagt atttttaaat taattaaatt agaatttcgg gttggataat tttaaatatg 420
   atttattagt attttttatt aattattggt tttttaggtt tttaagttta tttattagga 480
   attttatttt taatattatt ttattaattt tagttgtaaa taagagaata tttaaaggtt 540
   gaggaatttt tagcggtaaa gttttgttta cgttaagtaa taaaggataa gttagttttt 600
   gttgtgatta ttttgttgta ttgataagtt acgtattttt atttaaggat ttaaattttt 660
   atttttttta agaattgggt taaaatcgat aaattaaatt tatttacggt ttattgatta 720
   aaggttgttg tataataagt ttttgttatg tttagtagtt ggatttatag cgttagaaat 780
   ttataattgt ttgatttttt tttttattat attgcgaaaa ttgtttttta aatgtaatta 840
   attttaaaat tttaatagta tcgtggttag gcgtggtggt ttattattgt aatattaata 900
   ttaggtatag gcgaggggat tgaggttagg atatcgaaat tagtttggga aatatacgga 960
   gattcggttt ttggaaaaat aattagtttt gcgtggtggt gggcgcgagg tttcggttaa 1020
   tcgggaggtt atagtgagtt atgatgatat tgtattatag tttgcgcgac ggtttatgtt 1080
   agtaagtttt ggagtatttg aaataagttg tgttgggtat tttatttatt ggagagcgat 1140
   tagtgattga tgtttattta tagcgattag agacgtatgt ttcgatagta gtataaattt 1200
   agtaggcgcg aataaatggt aaagagaaat tgggtaaata agtattacgg ttttttagtt 1260
   gagaaagtgg gggttttaaa aagggttttt tgttgataga aagggacgtt taattatcga 1320
   aatcgtagag ggtgcggttt tggcgtttga gcgcgtagat tatatttatg gcggtgatcg 1380
   ttttgcgttt ggcgtgtttt gtataggtta cggcgtttcg gattacgttt tttaggaata 1440
   tttttagtat ttcgcgagtt ttttcgtaga tgaggtcgga gatgcgtttt acgtcgtcgc 1500
   ggcgagtaag gcgtcggatg gtcggtttgg tgatgttttg gatattgtcg cgtagtattt 1560
   tacggtggcg tttagcgtcg tttttgttaa gatttttttc gtttttgtcg cggttagata 1620
   tgacgagtaa gaggagtttt atttaacgtt ttgtgaggat tttggtttga ggtagcgttt 1680
   ttatacgata gttggcggat cgaattgaga atttgaaaga agtcggcggg aagtttcgtt 1740
   tcggtggggg aggggaaatt taaagggtta aatcgaaata gggggaaaaa aaaagcgagt 1800
   tttttgtttt cgtgttttga attttgtaac gtgtatagta ttttgttatt acgttatgag 1860
   gttttaaaaa attgtttttg aacgtagaag atatatatta atattgtggg aaatataaga 1920
   aaggataaga aattaagaaa ttataatgtt attttattat ataggttagt taattatgta 1980
   ttttgtagag tagttgtata tattttttta agaaaatgta tatagtgttg tatatggagt 2040
   tttgtaattt ttttatattg attataattt aattaatttt tattaaagag ataaaagtga 2100
   tgttttggtg tttatgtttt ttaggaatta ttaatagtta taattagttt tttagtaatt 2160
   ttttaatcgg ttgtatttta aaaataatgt tttttatatt taatataaat gtattttttt 2220
   tttatatttg ggattaatat tgaaatttat gattttatta tattaaaatt taaattttat 2280
   tatattaata tttaaaattg tattagaggt tttatgattt ggtattacgg gttttcgtat 2340
   tatttttttt ttaaattttt taatttgttt tattaaggtt tttggataat tttagagatt 2400
   ttttgtgaag tttgaataaa attttttcga gattttgata attgtattag ttttaggatt 2460
   taattggaat agaattaaaa tttttaaaat aagtttttat a 2501
<210> 10
   <211> 2501
   <212> DNA
   <213> Artificial Sequence
<220>
   <223> chemically treated genomic DNA (Homo sapiens)
<400> 10
   tataagagtt tgttttaagg attttaattt tattttaatt aagttttaaa gttaatgtaa      60
   ttattaaaat ttcgaagaga ttttatttaa attttataaa aggtttttaa agttgtttag     120
   aaattttggt gaaatagatt aggaaatttg gaaaggaaat aatgcggaga ttcgtagtat     180
   taaattatga gatttttaat ataattttaa atattaatgt aataaaattt aaattttggt     240
   gtaataaaat tataaatttt aatattggtt ttaagtatag agaaaaagta tatttatgtt     300
   gaatgtggaa aatattattt ttaaaatata gtcgattaaa aaattgttgg ggaattgatt     360
   ataattattg ataattttta agaaatatag atattaaaat attattttta tttttttaat     420
   agaaattggt taaattataa ttaatataag gaggttataa aattttatat ataatattgt     480
   atatattttt ttggaaaaat atgtgtaatt gttttgtaaa atatatgatt aattagtttg     540
   tgtgatggga taatattgta gttttttaat tttttgtttt tttttgtatt ttttatagta    600
   ttgatgtata ttttttgcgt ttaaaagtaa ttttttaaag ttttataacg tggtaataaa    660
   atattatgta cgttataaaa tttagaatac ggaaataaga agttcgtttt tttttttttt     720
   ttatttcggt ttggtttttt agattttttt ttttttatcg gggcgggatt tttcgtcgat    780
   tttttttagg tttttagttc ggttcgttaa ttgtcgtata aaggcgttgt tttaggttag    840
   agtttttata aagcgttggg tgagattttt tttgttcgtt atgtttggtc gcggtaaagg     900
   cgggaagggt tttggtaaag gcggcgttaa gcgttatcgt aaagtattgc gcgataatat     960
   ttagggtatt attaagtcgg ttattcggcg ttttgttcgt cgcggcggcg tgaagcgtat    1020
   tttcggtttt atttacgagg agattcgcgg ggtgttgaag gtgtttttgg agaacgtgat    1080
   tcgggacgtc gtgatttata tagagtacgt taagcgtaag acggttatcg ttatggatgt    1140
   ggtttacgcg tttaagcgtt agggtcgtat tttttacggt ttcggtggtt gagcgttttt    1200
   ttttattaat aaaaggtttt ttttagggtt tttatttttt tagttgagga gtcgtgatgt    1260
   ttgtttgttt agtttttttt tattatttgt tcgcgtttgt tgagtttgtg ttgttatcgg   1320
   agtatgcgtt tttagtcgtt gtaagtaggt attagttatt aatcgttttt tagtaaataa    1380
   aatatttaat ataatttgtt ttaggtgttt tagagtttat tgatatgggt cgtcgcgtag    1440
   attgtagtgt agtgttatta tggtttattg tagtttttcg attagtcgga atttcgcgtt    1500
   tattattacg taaggttaat tattttttta aagatcgggt tttcgtgtgt tttttaggtt   1560
   agtttcgata ttttggtttt aattttttcg tttatgttta atgttggtat tatagtagtg   1620
   agttattacg tttggttacg atattgttga ggttttaggg ttagttatat ttaaggggta    1680
   attttcgtag tgtagtgggg aggaaagtta agtagttata ggtttttggc gttgtgaatt    1740
   taattgttga atatagtaag aatttattat gtaataattt ttaattagtg gatcgtaaat    1800
   aagtttagtt tatcggtttt ggtttaattt ttgagaaagg tgagaatttg aatttttgag    1860
   tagaaatacg tagtttatta gtataataaa gtgattataa taaagattaa tttatttttt    1920
   gttatttaac gtgggtagag ttttatcgtt gagaattttt tagtttttga gtgttttttt    1980
   atttataatt gaagttgata agatggtatt aaaagtgaga tttttagtaa gtaaatttaa    2040
   aggtttgaag gattagtgat taatgggaag tgttaataag ttatatttga ggttatttaa    2100
   ttcgagattt tgatttaatt ggtttaagga tattattatt ttgtgggtta gatttgaaaa    2160
   ataataattg ttttaaggaa ggaatttgtt ggtatttaaa taaaaaattt ttttttttga    2220
   tttttatatg tcgttattta gataaatttt tggtttatat ggttggattt ttttttgtta   2280
   gttaaatttt gtgttatttt gtgaaaaaga attgagatta ataaaacggt agtgagaata    2340
   tagggaaaga taaaaattat agtaagttta tttagttatg ttgtagttat ttttataagg    2400
   atagggaagg atttgttttt ttatttatta tttaaagtgt gagtataaat tttaaaaata    2460
   tatgatatta taggaataat gaagatgttt ttatttgtaa a                        2501
<210> 11
   <211> 2501
   <212> DNA
   <213> Artificial Sequence
<220>
   <223> chemically treated genomic DNA (Homo sapiens)
<400> 11
   ttgttgtata gaatatttta ttatttaggt attatgtcga gtatttaata gttttttttt      60
   ttgttttttt tttttttttt attttgtatt ttggagttaa ttatagtgtt tgttgttttt     120
   ttgtttgtgt tataagtttt tattatttag tttttattta taagtgagaa tatttagtat     180
   ttggattttt gtttttgtat tagtttgtta aggataatag tttttagttt tatttatgtt     240
   tttataaaag atatgattta gtttttttta atggttgtat taaatgaagt tttaaagata     300
   taatataaat attaattttt tttttattat aaaaattttt tgttgaattt gattatattt     360
   aaattaacga gttttgtttt atgaaagatt ttttggataa atttgatagt tgatggaata     420
   ggagaagttg tttgttatgt ttaaagttaa taagagatta atatttagaa taaatggaga     480
   tttgtaaatt aatagaaagt aggtagtaaa gttaaagaaa atagtttaag gtatagttat     540
   taaaaggaac gtgattatgt tttttgtagg gatatgggtg gagttggaag tcgttagttt     600
   tagtaaattt atataggaat agaaaattag cgagatcgta tggttttatt tataagtggg     660
   agttgaataa tgagaatata tggttatatg gcggcgatta atatatattg gtgtttgttg     720
   agcggggtgt tggggaggga gagtattagg aagaatagtt aagggatatt gggtttaata     780
   tttgggtgat gggatgattt gtatagtaaa ttattatggc gtatatattt atgtaataaa     840
   tttgtatatt ttttatatgt attttagaat tttaaataaa agttggacgg ttaggcgtgg     900
   tggtttacgt ttgtaatttt agtattttgg gaagtcgagg cgtgtagatt atttaaggtt     960
   aggagttcga gattagttcg gttaatatgg tgaaatttcg tttttattaa aaatataaaa    1020
   attagttaga tgtggtacgt atttataatt ttatttattc gggaggttga agtagaattg    1080
   tttgaattcg agaggcggag gttgtagtga gtcgtcgaga tcgcgttatt gtattttagt    1140
   ttgggttata gcgtgagatt acgttataaa ataaaataaa ataatataaa ataaaataaa    1200
   ataaaataaa ataaaataaa ataaaataaa ataaaataaa ataaaaaaat aaaataaaat    1260
   aaaataaaat aaagtaattt tttttttttt aagcggtttt tatttttttt ttttgttttg    1320
   tgaagcgggt gtgtaagttt cgggatcgta gcggttttag ggaatttttt ttcgcgatgt    1380
   ttcggcgcgt tagttcgttg cgtatatttc gttgcggttt tttttttgtt gtttgtttat    1440
   tttttaggtt tcgttgggga tttgggaaag agggaaaggt tttttcggtt agttgcgcgg    1500
   cgatttcggg gattttaggg cgtttttttg cggtcgacgt tcggggtgta gcggtcgtcg    1560
   gggttggggt cggcgggagt tcgcgggatt ttttagaaga gcggtcggcg tcgtgattta    1620
   gtattggggc ggagcggggc gggattattt ttataaggtt cggaggtcgc gaggttttcg    1680
   ttggagtttc gtcgtcgtag ttttcgttat tagtgagtac gcgcggttcg cgttttcggg    1740
   gatggggttt agagttttta gtatggggtt aattcgtagt attaggttcg ggttttcggt    1800
   agggtttttc gtttatttcg agattcggga cggggtttta ggggatttag gacgttttta    1860
   gtgtcgttag cggtttttag gggttcggag gcgtttcggg gagggatggg atttcggggg    1920
   cggggagggg gggtagattg cgtttatcgc gttttggtat tttttttcgg gttttagtaa    1980
   attttttttt gttcgttgta gtgtcgtttt atatcgtggt ttatttttta gttcgaggta     2040
   ggagtatgtg tttggtaggg aagggaggta ggggttgggg ttgtagttta tagtttttcg    2100
   tttattcgga gagattcgaa tttttttatt ttttcgtcgt gtggttttta tttcgggttt    2160
   tttttttgtt tttcgttttt ttcgttatgt ttgtttttcg ttttagtgtt gtgtgaaatt    2220
   ttcggaggaa tttgtttttt tgtttttttt ttgtattttt gatttttttt cgggttgttg    2280
   cgaggcggag tcggttcggt ttttatattt cgtatttttt ttttttcgta ggtcgttgcg    2340
   cggttttgcg tatgttgttg gtagattagg gttagagttg gaaggaggag gtggtgatcg    2400
   tggagacgtg gtaggagggt ttatttaaag ttttttgcgt aagtgattat gttcgggtaa    2460
   ggggaggggg tgttgggttt tagggggttg tgattaggat t                         2501
<210> 12
   <211> 2501
   <212> DNA
   <213> Artificial Sequence
<220>
   <223> chemically treated genomic DNA (Homo sapiens)
<400> 12
   gattttagtt atagtttttt aaggtttagt attttttttt tttgttcggg tatggttatt     60
   tacgtaggag gttttgagtg agtttttttg ttacgttttt acggttatta tttttttttt   120
   ttagttttgg ttttgatttg ttagtagtat gcgtagggtc gcgtagcggt ttgcggggag     180
   ggagaagtac gagatgtggg gatcgggtcg atttcgtttc gtagtaattc ggggaggggt     240
   taggagtgta gggagggaat agggaaatag gttttttcga agattttata taatattggg    300
   gcggggagta ggtatggcgg gagaggcggg gaataggaag gaggttcggg gtaaaagtta     360
   tacgacggag ggataagggg gttcggattt tttcgggtgg gcgaggggtt gtgggttgta     420
   gttttagttt ttgttttttt tttttgttag atatatgttt ttatttcgaa ttgggaaata    480
   gattacggtg tagggcggta ttgtagcgaa taaagaaaag tttgttggag ttcgggggag     540
   gatgttaagg cgcggtgagc gtagtttgtt tttttttttc gttttcgggg ttttattttt    600
   tttcgaggcg tttcgggttt tttgaaagtc gttaacggta ttggggacgt tttgggtttt     660
   ttaggttttc gtttcgggtt tcgaggtggg cgaggagttt tgtcgggagt tcgggtttga     720
   tgttgcgggt tggttttatg ttgggagttt tgagttttat tttcggggac gcgggtcgcg     780
   cgtatttatt ggtggcgaag attgcggcgg cgaaatttta gcgaaggttt cgcggttttc     840
   gagttttata agggtggttt cgtttcgttt cgttttagtg ttgagttacg gcgtcggtcg     900
   tttttttgga gggtttcgcg gattttcgtc ggttttagtt tcggcggtcg ttgtatttcg     960
   ggcgtcggtc gtagaggggc gttttggagt tttcggagtc gtcgcgtagt tggtcgggga    1020
   agtttttttt ttttttttag gtttttagcg gggtttaggg agtaaataga tagtaggaag   1080
   aggatcgtag cgaagtgtgc gtagcgaatt ggcgcgtcgg gatatcgcgg ggggaaattt    1140
   tttaagatcg ttgcgatttc ggagtttgta tattcgtttt atagggtagg ggagaggggt    1200
   ggaggtcgtt tagaggaaag gaaattgttt tattttattt tattttattt tattttttta    1260
   ttttatttta ttttatttta ttttatttta ttttatttta ttttatttta ttttgtgtta    1320
   ttttatttta ttttatgacg tagttttacg ttgtggttta ggttggagtg tagtggcgcg    1380
   atttcggcgg tttattgtaa ttttcgtttt tcgggtttaa gtaattttgt tttagttttt    1440
   cgagtaggtg gaattatagg tgcgtgttat atttggttga tttttgtatt tttagtagag    1500
   acggggtttt attatgttgg tcgggttggt ttcgaatttt tgattttagg tgatttgtac    1560
   gtttcggttt tttaaagtgt tgggattata ggcgtgagtt attacgtttg gtcgtttaat    1620
   ttttatttga agttttgggg tatatgtaga ggatgtgtag gtttgttata taggtgtgtg    1680
   cgttatgatg gtttgttgta tagattattt tattatttag gtattaagtt tagtattttt    1740
   tagttatttt ttttggtatt tttttttttt agtatttcgt ttaataggta ttagtgtgtg    1800
   ttgatcgtcg ttatgtgatt atgtgttttt attgtttagt ttttatttat aagtgagatt    1860
   atgcggtttc gttggttttt tgtttttgtg tgagtttgtt gaggttaacg gtttttagtt    1920
   ttatttatgt ttttgtaaag gatatgatta cgtttttttt agtggttgtg ttttaggtta    1980
   ttttttttgg ttttgttgtt tattttttgt tgatttgtag atttttattt attttagata   2040
   ttgatttttt gttggtttta gatatgatag atagtttttt ttattttatt aattgttaag    2100
   tttgtttaag gagtttttta tgaaataaaa ttcgttaatt taagtgtaat taaatttagt    2160
   aagggatttt tgtggtgggg aagaggttgg tgtttatgtt gtatttttaa aattttattt    2220
   aatgtagtta ttaaaaagaa ttagattatg ttttttgtgg gaatatggat ggagttagag    2280
   gttattattt ttagtaaatt aatgtaggaa tagaaattta aatattggat gtttttattt    2340
   gtaagtggga gttaaatgat gagaatttat aatataaata aggaaataat agatattgtg    2400
   gttgatttta gggtgtagga tgggaggaag gagaggagta gaaaagagaa ttattgggta    2460
   ttcggtataa tatttgggtg atgaaatatt ttgtataata a                        2501
<210> 13
   <211> 1920
   <212> DNA
   <213> Artificial Sequence
<220>
   <223> chemically treated genomic DNA (Homo sapiens)
<400> 13
   ttaggttgtt gtagatatag tttttgtttt tttgaaaaat atgttttagg tgttgggatt    60
   ttagatattt gggaaataga gtgtatgtag ttgttgagag gttttgtgtt tggttttttt   120
   tattattgag gtgtagaggt gttgtggata gtttagattt atatggtgtt tgaggtgaaa   180
   tagaattttt agttttttta tgaggttatt ggtatttttg gttgttttta gagttttttg  240
   atttagagtt gaatgtaaag taagtgtttg aaatgtagaa gtagttgggg ttgtttatgg   300
   tatttgtttt gtttggggtg agagaagatg ttaggttgag gttttagtga ttttaggtat   360
   tagttttgaa ggagggtggg gagattgtaa aggggaagtg tttggagggt taatggtttt   420
   tgtgtatttt gtgttttttt gaagtgtgtt gtttttttgt gttggggatt gggatttgtt   480
   tttggggaat ttgtttagaa gatggtggtg gattggggtt gggtattttt tagggttgtt   540
   aggttttttt tagttttgta tttgttgtgt tgttttattt tgttaggaag ttttagagat   600
   tttggggatg gggtgggagt gtttttttat tgtgggttta aaaagaagga aggatgtttt  660
   taggggttgt agaaggagga ttagttttaa gttataattt tttttggatt taaggtaggt   720
   tggttggggt tttgtgttta tatggttttt ggtgggggtt tgtgtgtttt gggagttttg   780
   tggtttgggg aggaaagagg agataagaga taggtgagga ttatggggtt gatttagttg   840
   gggtagggat tattgtggaa aaattttggt gaggtggggg gatgtggaaa gagagtggtt   900
   tgtgttttgt atttgtgttt gggtattttg tgttagtgtt ttgtttttag tgttttgtgt   960
   tttgtgtttt gtgttttgtt tttattttgg gttagttgta ttgtgtttgt gttgtaggaa  1020
   ttgtggagtt ggaaagtggg ggtgttgtgg ttggggggtt gttttagttg tgttttggtt  1080
   agtgattggt gggttgggtt taaatttagt taggttgggt aggtggtggt tgtgtgattg  1140
   gggattgggt gttttgtttt ttttgttttt tttttttttt tttttttttt ttttagtttt   1200
   ttggtttttt ttagttttta ttggatttgt ttgtttgttg tttttttttt ttttttgttt  1260
   tttatattat tttttatttt tttgttttgt ttttgttttt tttttttttt ttgttttttg  1320
   tttttttttt tttttttttt ttttttaggg gtggagtttt tttttttttt tttagataat     1380
   gttgtggttg tgtttttttt tttgttagtt tgtttaggtt tttgttgtta gtgatttttt  1440
   tgggttgggg gtggggaggt ggggggggag tgtagggttg gggaggatga gttggttttt 1500
   tttatttttt tgttgttgtt ttttttaaga gggatggaga tttggtttaa gttttttggt  1560
   ttatttggag ttgtgatagt tatttttagg gaatagttat gttgttttat taagtttatt  1620
   tttagtggtt tggatttttt aggtagaggt tgtgggattt tgtttttttt aatattttag 1680
   tttattttta aaagggtttg agttggatag gggttaaata ggtttttttg atttggtggg 1740
   ttggttagat gtgatagtaa tgttaaggag gttaagtttt tttgtttatt ttttattttt  1800
   ttttttttta tttttggatt ttttggtgtt tttagtatat agaggttttt gagtagtttg   1860
   gttgtaggtt ttttatttat ttagagtttt tttttttatg tgtttatttt taattttgta   1920
<210> 14
   <211> 1920
   <212> DNA
   <213> Artificial Sequence
<220>
   <223> chemically treated genomic DNA (Homo sapiens)
<400> 14
   tgtagggttg gggataggta tgtgaggggg agaattttga gtagataggg aatttgtagt 60
   tgggttgttt aagggttttt gtgtattggg ggtgttagga ggtttaggga tggaaaaggg 120
   ggaggtgaga aatggataaa gaaatttggt ttttttggta ttgttgttat gtttggttgg 180
   tttgttaagt tgaaggggtt tgtttagttt ttgtttggtt taaatttttt tgggaataag 240
   ttgggatgtt agaaaaaata aaattttata atttttgttt ggggaattta ggttgttgga 300
   ggtgggtttg gtagggtagt gtgattgttt tttgggagtg gttgttatag ttttgggtga 360
   attgaggagt ttgggttaag tttttatttt ttttggagag ggtagtagta aggaggtgag 420
   gggagttagt ttattttttt taattttgta tttttttttt atttttttat ttttagtttg 480
   gaagaattgt tggtggtggg agtttggatg agttggtggg gaaggggatg tagttatagt 540
   attgtttggg agggagggga gaagttttgt ttttagaggg gaggggagaa gaggaggggg 600
   taggagataa ggggaggaag aaaggtgaag gtaaggtgaa ggggtggaga gtgatatgaa 660
   gagtgagaga aaagagagga tagtggatga gtagatttgg taggggttga aaaaggttaa 720
   ggggttggag ggagggagag gaaggaggag gggagtgagg agggtggggt gtttggtttt 780
   tagttgtgtg gttattgttt gtttagtttg gttggatttg agtttggttt gttgattgtt 840
   ggttgaggtg tagttgaagt agttttttag ttgtggtgtt tttatttttt aattttatgg 900
   tttttgtggt gtgggtgtga tgtagttggt ttggggtgaa ggtaaggtgt ggggtgtggg 960
   gtgtggggta ttgggagtga ggtattggtg tgggatgttt ggtgtaaggt gtagggtgtg 1020
   ggttgttttt tttttgtgtt tttttatttt gttaaagttt ttttatgatg gtttttattt 1080
   tggttgggtt agttttgtaa tttttgtttg ttttttgttt tttttttttt ttttgagttg 1140
   tggggttttt ggggtgtgtg gatttttgtt aggggttgtg taggtgtgga gttttagttg 1200
   gtttgttttg ggtttgaaga aagttgtggt ttggagttag tttttttttt atgatttttg 1260
   ggggtgtttt tttttttttt tgagtttgtg atgggaaggt gtttttattt tatttttggg 1320
   gtttttgaga ttttttggtg aggtggggtg gtgtggtagg tgtagggttg gggagggttt 1380
   gatagttttg gagagtgttt gattttagtt tgttgttgtt ttttaggtgg attttttaga 1440
   ggtaggtttt agtttttggt gtggggaggt ggtgtgtttt agaggggtgt agggtgtgtg 1500
   ggggttgttg gttttttggg tatttttttt ttgtggtttt tttgtttttt tttggagttg 1560
   gtgtttgagg ttgttgggat tttagtttgg tgtttttttt tgttttgagt gaggtaggtg 1620
   ttgtgggtgg ttttggttat ttttgtattt tgagtgttta ttttgtattt agttttaagt 1680
   tggagagttt tggggatagt tgagagtgtt agtggtttta tagggagatt gagggttttg 1740
   ttttattttg ggtgttgtgt gggtttgggt tgtttatagt atttttgtgt tttagtgata 1800
   ggagaagtta agtgtgaggt tttttaatag ttgtgtgtat tttatttttt aggtatttgg 1860
   aattttggtg tttagaatgt gttttttggg agagtaaagg ttgtgtttat ggtagtttgg 1920
<210> 15
   <211> 6096
   <212> DNA
   <213> Artificial Sequence
<220>
   <223> chemically treated genomic DNA (Homo sapiens)
<400> 15
   tattagtgta agatttaaaa tttttttttt gtattgtata gtgagatgtt tagggtttta 60
   gtttagtgtt tggatatagt gatttttggg tttgtttgtt gttgttttaa gtgaagttgg 120
   tgtgttttgg gtggagtaga tagagatttt gggtggtagg ggtttgggaa gatatgggtg 180
   gttagggttt tatgtgtttt tattgttgtt ttttgttatt tgtaggtaat ggatgagttg 240
   ggaatgagtt ttttagatta gtttgtgatt aagaaaggta aggaatggtt tgttagggta 300
   gagtttggtg agatggtgta ggtttttggt gtatagattt atttttttta ttggttgtgt 360
   tttttgtgtg ttgttaggtt gggtgtttat taggtatttt ttttggttta gttagatgtt 420
   aggtagatgt gtgggtttgg tgagtttgtt tagtattttg tggtttgggg tgggttttag 480
   tggattagta tttattgggt tgtagtattg ggagtttggt ttttttttgt tgagggggag 540
   ggtatttttg tggatttgga gttgatttgt agaatgagtt aaattatttt tttgtttttt 600
   taagagatgg gaatggaagt gttgttttta tggagttggg gaaatgattt ttattttata 660
   gtgttttagt attttggtgt ttggtgggta tttttttttt ttttttttta ggtagggttt 720
   tggaggtttt tgggggaatt ttttttttgt gggagttttt tgtggtattt agatttaggg 780
   gagtttgtgt gtgagtattg tgtgttaggt tgtgtgtatt tgagttaggg tttatttgtt 840
   tttgggtgtt tgtgtttatg tgagtttagg gttttgtgta tgtttgaaat gtttttttta 900
   tgggtgtttt agtatttttt ggagtgtgag tgtgtttgtt tttgtgaatg tgtttgtgag 960
   gtgtgttttt gtatgttggt gtgtattttt ttgtatttgg gggatgtata tattttttaa 1020
   tatgtatagt atttttgttg tgttttgtat tttgtttttt ggtatttgag gatttttaag 1080
   tatgtgtggg ttttttttgt gtatatatag gagtatttat gtgatttttg gtattagtaa 1140
   aatttaggga tatgggattt attttttttg gtttgaggat taagtattgg ttatgatagg 1200
   ggaaggtgag agatgataaa aatagagaga tagttagaga ggagtagaga gttagagggg 1260
   tttaggtatt gggtagtagt ttttttatat ttggggtagg tgtttgaaag aatttagagg 1320
   tgtatatgag tttgaggtgt tttaggtagg tattgttttt atagggtttg gtttgagttg 1380
   ttttttaaat gagtgaattt aagtttgggt tttatttgtt ttttatttgt ttttagggga 1440
   ggttaaggtg gaagtggtgg tagtagggtt ggggttggat ttttaggagt tggggttgag 1500
   ttattaggag ttgggggttg ggtggatgat ttggagtgtg tagtagggaa gatgaggtaa 1560
   tagggtagga agtgggtggg gggaggtgga attggggttg tgttttgtgt tgtttggaat 1620
   tgggagtgtg ggaaagatat taggaatttg gttgtagtgt agttttgttg gtggggtttg 1680
   gttggtttat tgtatagagt tttttttgat ttttgaagaa agagatttgt ttgtagtggg 1740
   taaaagtttg tttggatttt ttggttatta gaaatatgag tatggtggtg gtttttagtt 1800
   ttttatttat gtttgggttt aagagattgg gagtttaggt ttattgattt tttgagaaag 1860
   attaagattt tgtattttag aaagaggttt ggggattttt gttttgtgta agggtagaag 1920
   gattagttgt ttttttgagt attttaattt ggaattttgg tttgaagttg agataggaga 1980
   ttggatgtga ggttttttta gagttggttt tttttaaata atttttaaaa tttttagatt 2040
   ttaggggtat gttgaaattt tttaaagtag tttaaagaat ataatgagag ttttaatatt 2100
   ttaggtggtg gtgtgttggt tttttggagt ggggtgggat gtggttgtgt ggatttatgt 2160
   gtataattgt gtgggatggg gttatgtgga tttatgtgta taattgtggg attttagtgt 2220
   tagtgggatt ttagtgttag tgggatttta gtgttagtgg gattttagtg ttagtgggat 2280
   tttagtgtta gtgggatttt agtgttagtg ggattttagt gttagtgggt ttgtggttta 2340
   gtggagtgag tggagtgttg gtgatttgag tggagattgt gttttggatg ttttagttta 2400
   gatgttaagt tatagtttgt gtagtagtag taaaggggaa ggggtaggag ttgggtatag 2460
   ttggatttgg aggttgtgat ttaggggaaa gtgtgggtgg ttgatttagg gtagttgtgg 2520
   tggtgaggta ggtgggtttt ttgttttttg gagttgtttt tttttatatt tgtttttggt 2580
   gtttttagta gtttttattt tggttttttg tggttattgt gggatttggt gttgttgtta 2640
   gtttagtggg gagtgaatta gtgttttttt ttgtttttgg tttttttgat ggtatgagga 2700
   atttttgttt tgttttatag atttttggtt tttgttgagt gtggtattgg agtttgtttt 2760
   gttagggttt tggaattaga gaaagttgtt ttttggttat ttgaagtgtt ggatttttat 2820
   agtgtttttt agtttgggtg ggagtggtgg ttgtgttgtt gaaggttggg gtttttggtg 2880
   tgaaagggag gtagttgtag ttttagtttt attttagaag tggtttttgt attgttgtgg 2940
   tgggtgtttt tgggttttga ttttgttagt gttgtggggt agaggtattt ggagtttgta 3000
   gggtttagat ttgggttgga aaagttttgt tgattgtagg taagtgtttg ggaggggtgg 3060
   ttaggtgaag ttttggtgtt ttattatata tttttgggtt ttatgttagt tgtatttgtg 3120
   gtattgggta ggaaatggta gggttgaggt tgattttagg agtataaggg agttttttat 3180
   tttttgttta tatttgttat ttttagtttt gtaatttatt ttagatatat agaaagtaag 3240
   taggattggt ggggagatgg agtttaatag gaatattttt tagtagtgag taggggttgt 3300
   atgggatgtg ggaggagttt agaggaggtg tggagagtgt ttgaggttgg gtgagtgttt 3360
   agaggggaga tagttgaatt gggtttaaga ggtgtttagt gggtgtttgt tgaatgaatg 3420
   agtgatgggt tttgaagttt gagtgtattg aaagaggggg tgtgtaaaaa gggttttttt 3480
   tattatatag gatatagtat atgtaaattt tttttttgtg gaaaagttag ataggttaaa 3540
   aaggttataa ataaattagt tgggtatggt ggtgtgtgtt tgtagtttta gttattaggg 3600
   aggttgagtt aggggaattg tttgaatttg ggaggtggag attgtagtga gttaagattg 3660
   tgttattgta ttttagtttg gaaatagagt gagattttgt tttggaaaaa aaaaaaaaaa 3720
   gttataaatt gtgtgtgggt tttaggttat ataattagag ttggagggga gtggttaagg 3780
   atgagaattg agatggattt tttgtttttt ttggaggaga gtgggtggtt gtttatttgg    3840
   gggtggggaa ttttttttta tgggtttagt tgtttaattt taggggattt ttaggatagg    3900
   agttgatgta aatagttgtt ttattttttg ttgtttttgg ttttggagaa ggaggaggga    3960
   gttggggagg gtttttattt tttagataat ttttaagtag ttaggatatg ggtgagatga    4020
   gtgagatatt gatttttggg atagaatttg agagggtgtt aaaaaattta gtaattaaga    4080
   taaataggtt gggtgtagtg gtttatgttt gtaattttag tattttggga ggttggatta    4140
   tttgaggtta agagtttgag attagtttgg ttaagatggt gaaattttat ttttattaaa    4200
   aatataaaaa ttagtttagt gtggtggtgt tagtttgtaa ttttagttat ttaggaggtt    4260
   gaggtaagag aattgtttga tttaggaggt agaggttgta gtgagttgag attatgttat    4320
   tgtattttag tttggataat agagggagat tattttaaaa aaaaaaaaaa aaaaaaaaaa    4380
   aaaaaagagg ttgggtggtg gtggtttata ttatgtgatt ttagtatttt gggaggttga    4440
   ggtgggtgga ttatttgagg tttggagttt gagattagtt tggttaatat ggtgaaattt    4500
   tgtttttatt aaaaatataa aaattagttg ggtggggtgg taggtatttg taattttagt    4560
   tattttggag gttgaggtag gagaattttt tgaatttgtg gggtggaggt tgtagtgaat    4620
   taagattata ttattgtatt ttagtttgga taataatagt aaaattttgt tttaaaaaaa    4680
   aaaaaaattt ttttttttga gatatagttt tatttttttg tttaggttgg ggtgtagtat    4740
   tatgatttta gtttattgta attttgtttt tttagatttt tgtattttag ttttttaagt    4800
   agttgggatt ataggtattt gttattatgt ttagttaatt tttgtatttt tagtaggggt    4860
   gtggttttat tatgttggtt aggttggttt tgaatttttg attttaagtg atttgtttgt    4920
   tttagttatt taaagtgttg ggattatagg tgtgagttat tatgtttggt ttttttaaat    4980
   gaaaatagtg taaaaattta tgataaataa aatattaaaa atttattgaa tttgtatttt    5040
   tataattttt ttttatttgt tttttaggtt attttttgtt ttagaaagta atttaaaaaa    5100
   tgtgtagatg gagtttggat tttatttgaa aatggtggga gttatggaaa attttggagt    5160
   aggggagtga aggatagaaa ttatatgtaa aagaaatttt gggttgggtg tagtggttta    5220
   tgtttgtaat tttagtattt tgggaggttg aggtaggtgg attatttgag gttaggagat    5280
   tgagattagt ttgattaata tggtgaaatg ttatttttat taaaaatata aaaaaaatta    5340
   gttaggtatg gtggtgtatg tttgtagttt tagttatttt ggaggttgag ataggaaaat    5400
   tgtttgaatt tgggaggtgg aggttgtagt gagttaagat tgtgttattg tattttagtt    5460
   tgggtaataa gagtaaaatt ttattttaaa aaaaaagaaa gaaagaaatt ttttggtagt    5520
   tgatgagaag gaaatttaat tggtaggttt tagtagggga gatgaggaga ttttagggag    5580
   ggtatttgta tatgttgtgt tttagtgtgg gttagggagt aggttattat tttttttgtt    5640
   tatttttttt ttgttttaat ttttttaagt tttggattag tggtatttta agtgtagttt    5700
   aaggaattat atgtattagg atttttaggg ggtgtttgtt aaaaatgtaa attttggtta    5760
   ggtgtagtgg tttatatttg taattttagt attttgggag gttgaggtgg gtggattatg    5820
   aggttaggag attgagatta ttttggtaaa tatggtgaaa ttttattttt attaaaaaaa    5880
   taaaaataaa ttaaaaaaaa tattagttgg gtgtggtggt gggtgtttgt agttttagtt    5940
   atttgggagg ttgaggtagg agaatggtgt gaatttggga ggtggagttt gtagtgagtt    6000
   gagattgtgt tattgtattt tagtttgggt gatagagtga gattttgttt taaaaaaaaa    6060
   aaagtaaatt ttttgggtat tattttatat tgattg                               6096
<210> 16
   <211> 6096
   <212> DNA
   <213> Artificial Sequence
<220>
   <223> chemically treated genomic DNA (Homo sapiens)
<400> 16
   tagttaatgt ggggtggtgt ttaagaaatt tgtttttttt tttttgaggt agagttttgt      60
   tttgttgttt aggttggggt gtagtggtgt aattttagtt tattgtaagt tttgtttttt     120
   gggtttatgt tatttttttg ttttagtttt ttgagtagtt gggattatag gtgtttgtta    180
   ttatgtttag ttaatgtttt ttttggtttg tttttgtttt tttagtagag atggggtttt     240
   attgtgtttg ttaggatggt tttgattttt tgattttgtg atttatttgt tttggttttt     300
   taaagtgttg ggattatagg tgtgagttat tgtatttggt taaaatttgt atttttaata     360
   agtatttttt gggggttttg atgtatgtgg ttttttggat tatatttagg gtattattgg    420
   tttaaagttt gaaggggttg gagtaagagg aaggtagatg ggaggagtag tgatttgttt     480
   tttggtttat attggggtat agtatgtgta aatgtttttt ttagagtttt tttatttttt     540
   ttgttgggat ttgttgatta agtttttttt ttattaattg ttagagggtt tttttttttt     600
   ttttttttta agatggagtt ttgtttttgt tgtttaggtt ggagtgtaat ggtataattt     660
   tggtttattg taatttttgt tttttgggtt taagtgattt ttttgtttta gtttttggag    720
   tagttgggat tataggtgtg tattattatg tttggttaat tttttttgta tttttagtag    780
   agatgatatt ttattatgtt ggttaggttg gttttaattt tttgatttta ggtaatttat    840
   ttgttttggt tttttaaagt gttgggatta taggtataag ttattgtgtt tggtttaggg    900
   ttttttttat atataatttt tattttttat ttttttgttt taaggttttt tatagttttt    960
   attattttta ggtaaagttt aaattttatt tgtatatttt ttaagttgtt ttttggggta   1020
   gagagtagtt tgggaggtag gtgagaaagg gttgtggaag tgtaagttta gtaaattttt   1080
   gatattttgt ttattgtgga tttttgtatt atttttattt aaaaaggtta agtgtggtgg   1140
   tttatgtttg taattttagt attttgggtg gttgaagtgg gtaggttatt tgaggttagg   1200
   agtttaagat tagtttggtt aatatggtga aattatgttt ttattaaaaa tataaaaatt   1260
   agttgggtgt ggtgataggt atttgtaatt ttagttattt gggaggttga ggtatgagaa   1320
   tttgagaggt agaggttgta gtgagttgag attgtggtgt tgtattttaa tttgggtgag   1380
   agagtaaaat tgtgttttga aaaaaaagat tttttttttt tttgagatag agttttgttg   1440
   ttgttgttta ggttggagtg taatggtgtg attttggttt attgtaattt ttgttttgta   1500
   ggtttaaggg atttttttgt tttagttttt ggagtagttg ggattatagg tgtttgttat   1560
   tttatttagt taatttttgt atttttagta gaaatggggt tttattatat tggttaggtt   1620
   ggttttgaat tttagatttt aggtgattta tttgttttgg ttttttaaag tgttgggatt   1680
   atatggtgtg agttattgtt gtttggtttt tttttttttt tttttttttt tttttttttt   1740
   gagatagttt ttttttgttg tttaggttgg agtatagtgg tatgattttg gtttattgta   1800
   atttttgttt tttgggttaa gtaatttttt tgttttagtt ttttgagtgg ttgggattat   1860
   aggttagtgt tattatattg ggttaatttt tgtattttta gtagagatgg ggttttatta   1920
   ttttggttag gttggttttg aatttttgat tttaagtgat ttggtttttt aaagtgttgg   1980
   gattatagat gtgagttatt gtgtttggtt tatttatttt gattattgag ttttttggta   2040
   tttttttaaa ttttgtttta gaagttagta ttttatttat tttatttatg ttttggttgt   2100
   ttagagattg tttgggaagt ggagattttt tttagttttt tttttttttt ttagggttaa   2160
   agatagtaag gaatagggtg attgtttata ttagtttttg ttttagagat tttttgagat   2220
   tggatagttg agtttgtgga gagggatttt ttatttttaa gtaggtaatt atttattttt   2280
   ttttagaggg aatgagggat ttattttagt ttttattttt gattattttt ttttagtttt   2340
   gattgtataa tttgaaattt atatatggtt tgtaattttt tttttttttt tttgagatgg   2400
   agttttgttt tgtttttagg ttggagtgta gtggtgtgat tttggtttat tgtaattttt   2460
   gttttttggg tttaagtgat ttttttggtt tagttttttt agtagttggg attatagatg   2520
   tgtattatta tgtttggtta atttgtttgt aattttttta atttgtttgg tttttttata   2580
   gggagaggat ttgtatatgt tgtgttttgt gtgatgaaag gagttttttt tatatatttt   2640
   tttttttaat gtatttagat tttaaagttt attatttatt tatttaataa atatttatta   2700
   agtatttttt gaatttggtt taattatttt ttttttaggt atttatttaa ttttgggtat   2760
   tttttgtgtt ttttttgagt tttttttgtg ttttatatag tttttgttta ttgttggaaa   2820
   atatttttgt taagttttgt ttttttatta gttttgtttg ttttttgtgt gtttgggata   2880
   ggttgtaaaa ttggaggtga taaatgtggg taggaaatgg agggtttttt tatattttta   2940
   gggttggttt tagttttgtt attttttgtt taatattgtg gatgtaattg gtatgggatt   3000
   tggaagtgtg tggtaaagtg ttggggtttt gtttggttgt tttttttgga tgtttgtttg   3060
   tagttagtga agttttttta atttaggttt gggttttgtg agttttaggt gtttttgttt   3120
   tgtggtgttg gtgaagttga agtttgagaa tgtttattgt agtgatgtga aggttgtttt   3180
   tggggtgggg ttgaggttgt agttgttttt tttttgtatt aaggatttta atttttagtg   3240
   atgtagttgt tgtttttgtt taggttggga ggtattgtag ggatttgatg ttttaggtgg   3300
   ttaaagagtg attttttttg attttagggt tttggtgggg taggttttag tattgtattt   3360
   ggtggaggtt gaaggtttgt ggggtaggat aggagttttt tgtgttgttg gaagggttga   3420
   ggatgaagga gggtgttaat ttatttttta ttgggttggt ggtaatgttg aattttgtag   3480
   tgattgtgga gggttaaggt gaaaattgtt gggggtgttg agggtaggtg tggggagggg   3540
   tggttttagg gagtaaggag tttatttgtt ttgttgttgt agttgttttg ggttgattgt   3600
   ttatgttttt ttttgggtta tgatttttgg atttaattgt gtttggtttt tgtttttttt   3660
   tttttgttgt tgttgtgtgg gttgtaattt gatgtttagg ttggggtgtt tagggtgtag   3720
   tttttgttta ggttgttagt gttttatttg ttttattggg ttatagattt gttggtgttg   3780
   gggttttgtt ggtgttgggg ttttgttggt gttggggttt tgttggtgtt ggggttttgt   3840
   tggtgttggg gttttgttgg tgttggggtt ttgttggtgt tggggttttg tggttgtgta   3900
   tgtgagtttg tgtggttttg ttttgtgtgg ttgtgtatgt gagtttgtgt ggttgtgttt   3960
   tgttttgttt tagggagtta gtgtgttgtt atttgggatg ttaggatttt tgttgtgttt   4020
   tttggattgt tttgggggat tttggtgtat ttttaggatt taggagtttt ggaagttgtt   4080
   tgagagaaat tagttttggg agggttttgt atttagtttt ttgttttggt tttggattgg   4140
   ggttttgggt taaggtgttt agaggaatag ttgatttttt tatttttgtg tagggtagag   4200
   atttttaaat ttttttttaa aatgtagggt tttagttttt tttagggagt tagtgaattt   4260
   agatttttag ttttttgagt ttaagtatga atagggaatt ggggattatt attatgttta   4320
   tatttttggt ggttaggaag tttaggtagg tttttgttta ttgtagatgg attttttttt   4380
   ttaggggtta agaaaggttt tgtatagtaa gttaattaag ttttattagt agagttgtgt   4440
   tgtaattagg tttttagtgt tttttttata tttttagttt taagtgatat aggatatagt   4500
   tttaatttta ttttttttta tttatttttt gttttgttgt tttatttttt ttgttatata   4560
   ttttaagtta tttatttaat ttttagtttt tggtaattta gttttagttt ttggaagttt    4620
   agttttagtt ttgttattat tatttttatt ttggtttttt ttgagaataa gtggagggta    4680
   aatagagttt aggtttgaat ttatttgttt gaaaaataat ttaagttaaa ttttgtggga    4740
   gtagtgtttg tttggggtat tttaggttta tgtgtatttt tgaatttttt tgggtatttg    4800
   ttttaaatgt aaagaggttg ttatttaatg tttgggtttt tttgattttt tgtttttttt    4860
   tggttgtttt tttgtttttg ttgtttttta tttttttttg ttatggttag tatttggttt    4920
   ttaggttaga aaaggtggat tttgtgtttt tggattttat taatgttagg agttatataa    4980
   atatttttat atatatatag agagggtttg tgtatgtttg gaaattttta gatattaaag    5040
   aataaagtgt aggatataat agagatattg tatatattga gaaatgtgta tattttttaa    5100
   atgtagagaa atgtatatta atatatagag atatatttta taaatatatt tatagaaata    5160
   gatatattta tattttaaga aatattaaga tatttatgaa gggaatattt tagatatgta    5220
   taggattttg aatttatatg gatatagata tttaggagta ggtgggtttt gatttaggtg    5280
   tatatagttt aatatatagt atttatatat aagttttttt aagtttaaat gttgtaagag    5340
   atttttatag aaagaaaatt tttttagagg tttttaaggt tttgtttgga aggaagagga    5400
   agaaagtgtt tgttaggtat tgaaatgtta aggtattgta aagtgaaaat tattttttta    5460
   attttgtggg aatagtattt ttatttttat tttttaggga aatagggaag tggtttaatt    5520
   tgttttgtaa attaatttta gatttataag agtgtttttt tttttggtgg ggagaggtta    5580
   ggtttttagt gttgtagttt agtgaatgtt gatttgttga ggtttatttt aggttatagg    5640
   gtgttgggta aatttattaa gtttgtatgt ttgtttaata tttggttggg ttaggaagag    5700
   tgtttggtgg atatttagtt tggtgatata taggaggtat ggttggtgaa gaaaatggat    5760
   ttgtgtatta agggtttgta ttattttgtt gggttttgtt ttgatagatt attttttatt    5820
   ttttttgatt atggattggt ttaggaggtt tatttttggt ttgtttattg tttgtaaata    5880
   gtagagggta gtggtgaggg tgtataaagt tttagttgtt tatgtttttt taagtttttg    5940
   ttatttaggg tttttgtttg ttttgtttaa ggtgtattag ttttgtttgg ggtggtgatg    6000
   ggtaggttta ggaattgtta tgtttaggta ttgagttgga gttttgggta ttttattgtg    6060
   tagtgtaaaa agggaatttt gaattttata ttggta                              6096
<210> 17
   <211> 2501
   <212> DNA
   <213> Artificial Sequence
<220>
   <223> chemically treated genomic DNA (Homo sapiens)
<400> 17
   tttgtaaatg gagatatttt tattattttt atagtattat atgtttttaa agtttgtatt      60
   tatattttgg gtgataaatg aaggataaga ttttttttta tttttgtgag gatgattata     120
   gtatgattgg atgggtttgt tatgattttt attttttttt gtgtttttat tattgtttta     180
   ttaattttag ttttttttta tagggtagta tagaatttaa ttagtagaaa gagatttagt     240
   tatgtagatt agagatttgt ttaagtgatg gtatgtaaga attaggaagg aaagtttttt     300
   gtttaaatat taataggttt tttttttaaa gtaattatta ttttttaaat ttaatttata     360
   aggtgatagt atttttaaat taattaaatt agaattttgg gttggataat tttaaatatg     420
   atttattagt attttttatt aattattggt tttttaggtt tttaagttta tttattagga      480
   attttatttt taatattatt ttattaattt tagttgtaaa taagagaata tttaaaggtt     540
   gaggaatttt tagtggtaaa gttttgttta tgttaagtaa taaaggataa gttagttttt     600
   gttgtgatta ttttgttgta ttgataagtt atgtattttt atttaaggat ttaaattttt     660
   atttttttta agaattgggt taaaattgat aaattaaatt tatttatggt ttattgatta      720
   aaggttgttg tataataagt ttttgttatg tttagtagtt ggatttatag tgttagaaat     780
   ttataattgt ttgatttttt tttttattat attgtgaaaa ttgtttttta aatgtaatta     840
   attttaaaat tttaatagta ttgtggttag gtgtggtggt ttattattgt aatattaata     900
   ttaggtatag gtgaggggat tgaggttagg atattgaaat tagtttggga aatatatgga     960
   gatttggttt ttggaaaaat aattagtttt gtgtggtggt gggtgtgagg ttttggttaa    1020
   ttgggaggtt atagtgagtt atgatgatat tgtattatag tttgtgtgat ggtttatgtt    1080
   agtaagtttt ggagtatttg aaataagttg tgttgggtat tttatttatt ggagagtgat    1140
   tagtgattga tgtttattta tagtgattag agatgtatgt tttgatagta gtataaattt    1200
   agtaggtgtg aataaatggt aaagagaaat tgggtaaata agtattatgg ttttttagtt     1260
   gagaaagtgg gggttttaaa aagggttttt tgttgataga aagggatgtt taattattga    1320
   aattgtagag ggtgtggttt tggtgtttga gtgtgtagat tatatttatg gtggtgattg    1380
   ttttgtgttt ggtgtgtttt gtataggtta tggtgttttg gattatgttt tttaggaata    1440
   tttttagtat tttgtgagtt tttttgtaga tgaggttgga gatgtgtttt atgttgttgt    1500
   ggtgagtaag gtgttggatg gttggtttgg tgatgttttg gatattgttg tgtagtattt    1560
   tatggtggtg tttagtgttg tttttgttaa gatttttttt gtttttgttg tggttagata     1620
   tgatgagtaa gaggagtttt atttaatgtt ttgtgaggat tttggtttga ggtagtgttt    1680
   ttatatgata gttggtggat tgaattgaga atttgaaaga agttggtggg aagttttgtt    1740
   ttggtggggg aggggaaatt taaagggtta aattgaaata gggggaaaaa aaaagtgagt    1800
   tttttgtttt tgtgttttga attttgtaat gtgtatagta ttttgttatt atgttatgag    1860
   gttttaaaaa attgtttttg aatgtagaag atatatatta atattgtggg aaatataaga     1920
   aaggataaga aattaagaaa ttataatgtt attttattat ataggttagt taattatgta    1980
   ttttgtagag tagttgtata tattttttta agaaaatgta tatagtgttg tatatggagt    2040
   tttgtaattt ttttatattg attataattt aattaatttt tattaaagag ataaaagtga    2100
   tgttttggtg tttatgtttt ttaggaatta ttaatagtta taattagttt tttagtaatt    2160
   ttttaattgg ttgtatttta aaaataatgt tttttatatt taatataaat gtattttttt    2220
   tttatatttg ggattaatat tgaaatttat gattttatta tattaaaatt taaattttat    2280
   tatattaata tttaaaattg tattagaggt tttatgattt ggtattatgg gtttttgtat    2340
   tatttttttt ttaaattttt taatttgttt tattaaggtt tttggataat tttagagatt     2400
   ttttgtgaag tttgaataaa atttttttga gattttgata attgtattag ttttaggatt    2460
   taattggaat agaattaaaa tttttaaaat aagtttttat a                        2501
<210> 18
   <211> 2501
   <212> DNA
   <213> Artificial Sequence
<220>
   <223> chemically treated genomic DNA (Homo sapiens)
<400> 18
   tataagagtt tgttttaagg attttaattt tattttaatt aagttttaaa gttaatgtaa      60
   ttattaaaat tttgaagaga ttttatttaa attttataaa aggtttttaa agttgtttag     120
   aaattttggt gaaatagatt aggaaatttg gaaaggaaat aatgtggaga tttgtagtat     180
   taaattatga gatttttaat ataattttaa atattaatgt aataaaattt aaattttggt     240
   gtaataaaat tataaatttt aatattggtt ttaagtatag agaaaaagta tatttatgtt     300
   gaatgtggaa aatattattt ttaaaatata gttgattaaa aaattgttgg ggaattgatt     360
   ataattattg ataattttta agaaatatag atattaaaat attattttta tttttttaat     420
   agaaattggt taaattataa ttaatataag gaggttataa aattttatat ataatattgt     480
   atatattttt ttggaaaaat atgtgtaatt gttttgtaaa atatatgatt aattagtttg     540
   tgtgatggga taatattgta gttttttaat tttttgtttt tttttgtatt ttttatagta     600
   ttgatgtata ttttttgtgt ttaaaagtaa ttttttaaag ttttataatg tggtaataaa     660
   atattatgta tgttataaaa tttagaatat ggaaataaga agtttgtttt tttttttttt     720
   ttattttggt ttggtttttt agattttttt ttttttattg gggtgggatt ttttgttgat     780
   tttttttagg tttttagttt ggtttgttaa ttgttgtata aaggtgttgt tttaggttag     840
   agtttttata aagtgttggg tgagattttt tttgtttgtt atgtttggtt gtggtaaagg     900
   tgggaagggt tttggtaaag gtggtgttaa gtgttattgt aaagtattgt gtgataatat     960
   ttagggtatt attaagttgg ttatttggtg ttttgtttgt tgtggtggtg tgaagtgtat    1020
   ttttggtttt atttatgagg agatttgtgg ggtgttgaag gtgtttttgg agaatgtgat    1080
   ttgggatgtt gtgatttata tagagtatgt taagtgtaag atggttattg ttatggatgt    1140
   ggtttatgtg tttaagtgtt agggttgtat tttttatggt tttggtggtt gagtgttttt    1200
   ttttattaat aaaaggtttt ttttagggtt tttatttttt tagttgagga gttgtgatgt    1260
   ttgtttgttt agtttttttt tattatttgt ttgtgtttgt tgagtttgtg ttgttattgg    1320
   agtatgtgtt tttagttgtt gtaagtaggt attagttatt aattgttttt tagtaaataa    1380
   aatatttaat ataatttgtt ttaggtgttt tagagtttat tgatatgggt tgttgtgtag    1440
   attgtagtgt agtgttatta tggtttattg tagttttttg attagttgga attttgtgtt    1500
   tattattatg taaggttaat tattttttta aagattgggt ttttgtgtgt tttttaggtt    1560
   agttttgata ttttggtttt aatttttttg tttatgttta atgttggtat tatagtagtg    1620
   agttattatg tttggttatg atattgttga ggttttaggg ttagttatat ttaaggggta    1680
   atttttgtag tgtagtgggg aggaaagtta agtagttata ggtttttggt gttgtgaatt    1740
   taattgttga atatagtaag aatttattat gtaataattt ttaattagtg gattgtaaat    1800
   aagtttagtt tattggtttt ggtttaattt ttgagaaagg tgagaatttg aatttttgag    1860
   tagaaatatg tagtttatta gtataataaa gtgattataa taaagattaa tttatttttt    1920
   gttatttaat gtgggtagag ttttattgtt gagaattttt tagtttttga gtgttttttt    1980
   atttataatt gaagttgata agatggtatt aaaagtgaga tttttagtaa gtaaatttaa    2040
   aggtttgaag gattagtgat taatgggaag tgttaataag ttatatttga ggttatttaa    2100
   tttgagattt tgatttaatt ggtttaagga tattattatt ttgtgggtta gatttgaaaa    2160
   ataataattg ttttaaggaa ggaatttgtt ggtatttaaa taaaaaattt ttttttttga    2220
   tttttatatg ttgttattta gataaatttt tggtttatat ggttggattt ttttttgtta    2280
   gttaaatttt gtgttatttt gtgaaaaaga attgagatta ataaaatggt agtgagaata    2340
   tagggaaaga taaaaattat agtaagttta tttagttatg ttgtagttat ttttataagg    2400
   atagggaagg attttgtttt ttatttatta tttaaagtgt gagtataaat tttaaaaata    2460
   tatgatatta taggaataat gaagatgttt ttatttgtaa a                        2501
<210> 19
   <211> 2501
   <212> DNA
   <213> Artificial Sequence
<220>
   <223> chemically treated genomic DNA (Homo sapiens)
<400> 19
   ttgttgtata gaatatttta ttatttaggt attatgttga gtatttaata gttttttttt 60
   ttgttttttt tttttttttt attttgtatt ttggagttaa ttatagtgtt tgttgttttt 120
   ttgtttgtgt tataagtttt tattatttag tttttattta taagtgagaa tatttagtat 180
   ttggattttt gtttttgtat tagtttgtta aggataatag tttttagttt tatttatgtt 240
   tttataaaag atatgattta gtttttttta atggttgtat taaatgaagt tttaaagata 300
   taatataaat attaattttt tttttattat aaaaattttt tgttgaattt gattatattt 360
   aaattaatga gttttgtttt atgaaagatt ttttggataa atttgatagt tgatggaata 420
   ggagaagttg tttgttatgt ttaaagttaa taagagatta atatttagaa taaatggaga 480
   tttgtaaatt aatagaaagt aggtagtaaa gttaaagaaa atagtttaag gtatagttat 540
   taaaaggaat gtgattatgt tttttgtagg gatatgggtg gagttggaag ttgttagttt 600
   tagtaaattt atataggaat agaaaattag tgagattgta tggttttatt tataagtggg 660
   agttgaataa tgagaatata tggttatatg gtggtgatta atatatattg gtgtttgttg 720
   agtggggtgt tggggaggga gagtattagg aagaatagtt aagggatatt gggtttaata 780
   tttgggtgat gggatgattt gtatagtaaa ttattatggt gtatatattt atgtaataaa 840
   tttgtatatt ttttatatgt attttagaat tttaaataaa agttggatgg ttaggtgtgg 900
   tggtttatgt ttgtaatttt agtattttgg gaagttgagg tgtgtagatt atttaaggtt 960
   aggagtttga gattagtttg gttaatatgg tgaaattttg tttttattaa aaatataaaa 1020
   attagttaga tgtggtatgt atttataatt ttatttattt gggaggttga agtagaattg 1080
   tttgaatttg agaggtggag gttgtagtga gttgttgaga ttgtgttatt gtattttagt 1140
   ttgggttata gtgtgagatt atgttataaa ataaaataaa ataatataaa ataaaataaa 1200
   ataaaataaa ataaaataaa ataaaataaa ataaaataaa ataaaaaaat aaaataaaat 1260
   aaaataaaat aaagtaattt tttttttttt aagtggtttt tatttttttt ttttgttttg 1320
   tgaagtgggt gtgtaagttt tgggattgta gtggttttag ggaatttttt tttgtgatgt 1380
   tttggtgtgt tagtttgttg tgtatatttt gttgtggttt tttttttgtt gtttgtttat 1440
   tttttaggtt ttgttgggga tttgggaaag agggaaaggt ttttttggtt agttgtgtgg 1500
   tgattttggg gattttaggg tgtttttttg tggttgatgt ttggggtgta gtggttgttg 1560
   gggttggggt tggtgggagt ttgtgggatt ttttagaaga gtggttggtg ttgtgattta 1620
   gtattggggt ggagtggggt gggattattt ttataaggtt tggaggttgt gaggtttttg 1680
   ttggagtttt gttgttgtag tttttgttat tagtgagtat gtgtggtttg tgtttttggg 1740
   gatggggttt agagttttta gtatggggtt aatttgtagt attaggtttg ggtttttggt 1800
   agggtttttt gtttattttg agatttggga tgggggttta ggggatttag gatgttttta 1860
   gtgttgttag tggtttttag gggtttggga gtgttttggg gagggatggg attttggggg 1920
   tggggagggg gggtagattg tgtttattgt gttttggtat ttttttttgg gttttagtaa 1980
   attttttttt gtttgttgta gtgttgtttt atattgtggt ttatttttta gtttgaggta 2040
   ggagtatgtg tttggtaggg aagggaggta ggggttgggg ttgtagttta tagttttttg 2100
   tttatttgga gagatttgaa tttttttatt tttttgttgt gtggttttta ttttgggttt 2160
   tttttttgtt ttttgttttt tttgttatgt ttgttttttg ttttagtgtt gtgtgaaatt 2220
   tttggaggaa tttgtttttt tgtttttttt ttgtattttt gatttttttt tgggttgttg 2280
   tgaggtggag ttggtttggt ttttatattt tgtatttttt tttttttgta ggttgttgtg 2340
   tggttttgtg tatgttgttg gtagattagg gttagagttg gaaggaggag gtggtgattg 2400
   tggagatgtg gtaggagggt ttatttaaag ttttttgtgt aagtgattat gtttgggtaa 2460
   ggggaggggg tgttgggttt tagggggttg tgattaggat t 2501
<210> 20
   <211> 2501
   <212> DNA
   <213> Artificial Sequence
<220>
   <223> chemically treated genomic DNA (Homo sapiens)
<400> 20
   gattttagtt atagtttttt aaggtttagt attttttttt tttgtttggg tatggttatt      60
   tatgtaggag gttttgagtg agtttttttg ttatgttttt atggttatta tttttttttt     120
   ttagttttgg ttttgatttg ttagtagtat gtgtagggtt gtgtagtggt ttgtggggag     180
   ggagaagtat gagatgtggg gattgggttg attttgtttt gtagtaattt ggggaggggt     240
   taggagtgta gggagggaat agggaaatag gtttttttga agattttata taatattggg     300
   gtggggagta ggtatggtgg gagaggtggg gaataggaag gaggtttggg gtaaaagtta     360
   tatgatggag ggataagggg gtttggattt ttttgggtgg gtgaggggtt gtgggttgta     420
   gttttagttt ttgttttttt tttttgttag atatatgttt ttattttgaa ttgggaaata     480
   gattatggtg tagggtggta ttgtagtgaa taaagaaaag tttgttggag tttgggggag     540
   gatgttaagg tgtggtgagt gtagtttgtt tttttttttt gtttttgggg ttttattttt     600
   ttttgaggtg ttttgggttt tttgaaagtt gttaatggta ttggggatgt tttgggtttt     660
   ttaggttttt gttttgggtt ttgaggtggg tgaggagttt tgttgggagt ttgggtttga     720
   tgttgtgggt tggttttatg ttgggagttt tgagttttat ttttggggat gtgggttgtg     780
   tgtatttatt ggtggtgaag attgtggtgg tgaaatttta gtgaaggttt tgtggttttt     840
   gagttttata agggtggttt tgttttgttt tgttttagtg ttgagttatg gtgttggttg     900
   tttttttgga gggttttgtg gatttttgtt ggttttagtt ttggtggttg ttgtattttg     960
   ggtgttggtt gtagaggggt gttttggagt ttttggagtt gttgtgtagt tggttgggga    1020
   agtttttttt ttttttttag gtttttagtg gggtttaggg agtaaataga tagtaggaag    1080
   aggattgtag tgaagtgtgt gtagtgaatt ggtgtgttgg gatattgtgg ggggaaattt    1140
   tttaagattg ttgtgatttt ggagtttgta tatttgtttt atagggtagg ggagaggggt    1200
   ggaggttgtt tagaggaaag gaaattgttt tattttattt tattttattt tattttttta    1260
   ttttatttta ttttatttta ttttatttta ttttatttta ttttatttta ttttgtgtta    1320
   ttttatttta ttttatgatg tagttttatg ttgtggttta ggttggagtg tagtggtgtg    1380
   attttggtgg tttattgtaa tttttgtttt ttgggtttaa gtaattttgt tttagttttt    1440
   tgagtaggtg gaattatagg tgtgtgttat atttggttga tttttgtatt tttagtagag    1500
   atggggtttt attatgttgg ttgggttggt tttgaatttt tgattttagg tgatttgtat    1560
   gttttggttt tttaaagtgt tgggattata ggtgtgagtt attatgtttg gttgtttaat    1620
   ttttatttga agttttgggg tatatgtaga ggatgtgtag gtttgttata taggtgtgtg    1680
   tgttatgatg gtttgttgta tagattattt tattatttag gtattaagtt tagtattttt    1740
   tagttatttt ttttggtatt tttttttttt agtattttgt ttaataggta ttagtgtgtg    1800
   ttgattgttg ttatgtgatt atgtgttttt attgtttagt ttttatttat aagtgagatt    1860
   atgtggtttt gttggttttt tgtttttgtg tgagtttgtt gaggttaatg gtttttagtt    1920
   ttatttatgt ttttgtaaag gatatgatta tgtttttttt agtggttgtg ttttaggtta    1980
   ttttttttgg ttttgttgtt tattttttgt tgatttgtag atttttattt attttagata    2040
   ttgatttttt gttggtttta gatatgatag atagtttttt ttattttatt aattgttaag    2100
   tttgtttaag gagtttttta tgaaataaaa tttgttaatt taagtgtaat taaatttagt    2160
   aagggatttt tgtggtgggg aagaggttgg tgtttatgtt gtatttttaa aattttattt    2220
   aatgtagtta ttaaaaagaa ttagattatg ttttttgtgg gaatatggat ggagttagag    2280
   gttattattt ttagtaaatt aatgtaggaa tagaaattta aatattggat gtttttattt    2340
   gtaagtggga gttaaatgat gagaatttat aatataaata aggaaataat agatattgtg    2400
   gttgatttta gggtgtagga tgggaggaag gagaggagta gaaaagagaa ttattgggta    2460
   tttggtataa tatttgggtg atgaaatatt ttgtataata a                        2501
<210> 21
   <211> 20
   <212> DNA
   <213> Artificial Sequence
<220>
   <223> chemically treated genomic DNA (Homo sapiens)
<400> 21
   gggattattt ttataaggtt 20
<210> 22
   <211> 21
   <212> DNA
   <213> Artificial Sequence
<220>
   <223> chemically treated genomic DNA (Homo sapiens)
<400> 22
   cccatactaa aaactctaaa c 21
<210> 23
   <211> 33
   <212> DNA
   <213> Artificial Sequence
<220>
   <223> chemically treated genomic DNA (Homo sapiens)
<400> 23
   ctaaacccca tccccaaaaa cacaaaccac aca 33
<210> 24
   <211> 24
   <212> DNA
   <213> Artificial Sequence
<220>
   <223> chemically treated genomic DNA (Homo sapiens)
<400> 24
   agtttcgtcg tcgtagtttt cgtt 24
<210> 25
   <211> 25
   <212> DNA
   <213> Artificial Sequence
<220>
   <223> chemically treated genomic DNA (Homo sapiens)
<400> 25
   ttttttagga atatttttag tattt 25
<210> 26
   <211> 17
   <212> DNA
   <213> Artificial Sequence
<220>
   <223> chemically treated genomic DNA (Homo sapiens)
<400> 26
   ccaaaacatc accaaac 17
<210> 27
   <211> 33
   <212> DNA
   <213> Artificial Sequence
<220>
   <223> chemically treated genomic DNA (Homo sapiens)
<400> 27
   caaaccaacc atccaacacc ttactcacca caa 33
<210> 28
   <211> 23
   <212> DNA
   <213> Artificial Sequence
<220>
   <223> chemically treated genomic DNA (Homo sapiens)
<400> 28
   cgtagatgag gtcggagatg cgt 23
<210> 29
   <211> 18
   <212> DNA
   <213> Artificial Sequence
<220>
   <223> chemically treated genomic DNA (Homo sapiens)
<400> 29
   ctaaaacctc aacctaac 18
<210> 30
   <211> 24
   <212> DNA
   <213> Artificial Sequence
<220>
   <223> chemically treated genomic DNA (Homo sapiens)
<400> 30
   gatttagagt tgaatgtaaa gtaa 24
<210> 31
   <211> 31
   <212> DNA
   <213> Artificial Sequence
<220>
   <223> chemically treated genomic DNA (Homo sapiens)
<400> 31
   cctaacatct tctctcaccc caaacaaaac a 31
<210> 32
   <211> 23
   <212> DNA
   <213> Artificial Sequence
<220>
   <223> chemically treated genomic DNA (Homo sapiens)
<400> 32
   aacgaaacaa ataccgtaaa cga 23
<210> 33
   <211> 20
   <212> DNA
   <213> Artificial Sequence
<220>
   <223> chemically treated genomic DNA (Homo sapiens)
<400> 33
   aaacccaaac ctaaattaaa 20
<210> 34
   <211> 17
   <212> DNA
   <213> Artificial Sequence
<220>
   <223> chemically treated genomic DNA (Homo sapiens)
<400> 34
   ggaagtgtgt ggtaaag 17
<210> 35
   <211> 28
   <212> DNA
   <213> Artificial Sequence
<220>
   <223> chemically treated genomic DNA (Homo sapiens)
<400> 35
   taaagtgttg gggttttgtt tggttgtt 28
<210> 36
   <211> 23
   <212> DNA
   <213> Artificial Sequence
<220>
   <223> chemically treated genomic DNA (Homo sapiens)
<400> 36
   aaacaaacgt ccgaaaaaaa cga 23
<210> 37
   <211> 1700
   <212> DNA
   <213> Homo Sapiens
<400> 37
   cccctcagga ctagagaatc ccgaagagcc cgtgtattgt tttcttgaaa gccccgaagc     60
   taagcaattc taaaaatgtt tagcaaagcc cttttgggaa tggtgagatg gggggtgggg    120
   aggatgggac attttcattg ccaccgcccg ccccccgtct ccccggcccc ctcacatgaa    180
   gtgactggat ttgggagacc tagcggaaac cttcaggtca cccagggaac tgctcctctc     240
   tctggtcctg cagcggcgag gcggggtagg gggtgggggg ggtgggccgt gagagcgccg     300
   agacgcgcgg ggcgcggaga tgtgcaagtg gcgaagcttg accgagagca ggctggagca     360
   gccgcccaac tcctggcgcg ggatctgctg aggggtcacg gtgagtctct gtgggtgcgg     420
   gtcggaggga tgcccaggtg cctcgtcgcc ccacggcccg ggcggaggcg ggtccggaac     480
   ctcccccaag tccggcctca gcctggagcc cgcgcgcctt gggcggatgc acggactgag     540
   aggcgcttgg ctgggctctc agcccgccac tgccacccgg ccccgtgcgc tcattggtcg     600
   ggatgtgtcg gctcagcagc ctccaacttc tcccgaatcc cactggtgag tcccaggaga     660
   gcgagctgag ggagaaaggt ccaaagggcg cgagcgccca gggcgctggg agcccgtggg     720
   acgggcacgg ggagggcaga gccagccgag gggagcggct gccgggaggt gcgctgagca     780
   gggtccgcag ctaaggtcca gcgcgatccg agcgcccagg accctgcggc cgtggaggag     840
   gcgtgccctg ggaaggagcc gctactggga cctgaagagt gaggggaggg agaggggccc     900
   agagggtgac ctggaggagg gctctggaag tcacgtcagg ttggctcttc aggttcattt     960
   ccatagttcc ctgcggcctc tgccttgggg agttatgttt tgttaccgag atccgcgcta    1020
   ccagattgca ccggggctga tttgggggct gggaatttgc cattctgctg tacagacact   1080
   gatttttttt tcttcttttt aaaaagcaag gtttgttttc attttggttt ctgtcggatt     1140
   ttctcatttg taactcactt ttctattttt acttttctta accggaagat cttttcatta    1200
   tcccacctct tcatcttgct tttaacctga tttcctatta tataattaat gcttaaatct    1260
   cacaagggtt ggtcggtgcg gggctggtgt gtccttttgt agatgaaagt ttaagacctc    1320
   ttaactggtt tcagtctatt tgtctggttt caaaataagg gtaattgcaa ccaaatatcc    1380
   ctctttagct tctcgatcaa gtcgattcct cccccatata tgattcaaat ggcaatttta    1440
   ttaaactgaa aactagtacc tgacttgcag cgtcagcatt tggaaagtgc ttgtcggcaa    1500
   atcgactgta tgaaggcgtg tagcttcatc ccctgccatt tttgtgggtt ctagcacttt    1560
   aaaaaattgt ctggtaattt cttggactta accctgtgtc tttggttgta gagcagcact    1620
   aaccagggga gtaatatggt tggcacttcg tgtccagaac tccgtatttc aggctgggaa    1680
   acacctaatt aatgtctggg                                                1700
<210> 38
   <211> 1700
   <212> DNA
   <213> Homo Sapiens
<400> 38
   cagctggtaa gtggcagacc tggaattgga atctagttca gtttgattcc ccaacccatg      60
   ctcttgacca ctatactgtt ttttcaagtc cagatctgaa atctcatttt ctgtgtggct     120
   gtgtgtttgg gacaggggta accaattcct gactactcta tatgctgcat agaacctgga     180
   gaggattttt caaagtaaat gaatctcgaa agctggattg cagagcaaac gagtgcagtc     240
   aattcagcca ggggcttgca agagggagaa agagaaaaag actgtggaat ggaaagtttc     300
   ccaacccaag cctttcccaa ggggtagcca ttctctgttc tacagtttag ggcttgcatg     360
   tgctttttct ggagtggaaa aatacataag ttataaggaa tttaacagac agaaaggcgc     420
   acagaggaat ttaaagtgtg ggctgggggg cgaggcggtg ggcgggaggc gagcgggcgc     480
   aggcggaaca ccgttttcca agctaagccg ccgcaaataa aaaggcgtaa agggagagaa     540
   gttggtgctc aacgtgagcc aggagcagcg tcccggctcc tcccctgctc attttaaaag     600
   cacttcttgt attgttttta aggtgagaaa taggaaagaa aacgccggct tgtgcgctcg     660
   ctgcctgcct ctctggctgt ctgcttttgc agggctgctg ggagttttta agctctgtga     720
   gaatcctggg agttggtgat gtcagactag ttgggtcatt tgaaggttag cagcccgggt     780
   agggttcacc gaaagttcac tcgcatatat taggcaattc aatctttcat tctgtgtgac     840
   agaagtagta ggaagtgagc tgttcagagg caggagggtc tattctttgc caaagggggg     900
   accagaattc ccccatgcga gctgtttgag gactgggatg ccgagaacgc gagcgatccg     960
   agcagggttt gtctgggcac cgtcggggta ggatccggaa cgcattcgga aggctttttg    1020
   caagcattta cttggaagga gaacttggga tctttctggg aaccccccgc cccggctgga    1080
   ttggccgagc aagcctggaa aatggtaaat gatcatttgg atcaattaca ggcttttagc    1140
   tggcttgtct gtcataattc atgattcggg gctgggaaaa agaccaacag cctacgtgcc    1200
   aaaaaagggg cagagtttga tggagttggg tggacttttc tatgccattt gcctccacac    1260
   ctagaggata agcacttttg cagacattca gtgcaaggga gatcatgttt gactgtatgg    1320
   atgttctgtc agtgagtcct gggcaaatcc tggatttcta cactgcgagt ccgtcttcct    1380
   gcatgctcca ggagaaagct ctcaaagcat gcttcagtgg attgacccaa accgaatggc    1440
   agcatcggca cactgctcaa tgtaggttta tttttttccc ttcttctacc aagaaaaaaa    1500
   aaattgtctc tcttgcatgc aataaagacg ttggaaataa actgcattgg tagcaagaca    1560
   aaggatttaa tgatttaatg ctgaaggggt gtgatatgct ggcatgcata ttgattccct    1620
   ttgctgaaaa tttcctgtta gatgttttct tcccaaataa cccctcccgc ccccatgctc    1680
   ctctccttac caaccttata                                                1700
<210> 39
   <211> 1700
   <212> DNA
   <213> Homo Sapiens
<400> 39
   tctctcctgg acaccggagg gaaccccgaa tataccgctt tactgcccac gggattggga     60
   agcacacgga ggctttgctg gtcaacccct gctttgtcac tgttaataag ttattattag    120
   ttctcataac taccgggggg ctggcgccga tcgcgggtgg cgcggggcgg gatccgctcc    180
   tgggacggtg gagggcggtg gcgctgcagt cgcgccggct tctgagcccc agggagcgga    240
   gggcacggag ccgggcgagt cctgcggtgt cagaatcctg cgccgccctg acgctggggc    300
   tcagggttca ttccgggggc gcgcgcgtgg ctgcggaggc gggaaggtcc ctctgcctgt    360
   cgccgcggag ccccgcgtcc ctgcaccccg agctctgggg gtccgccttg ggctgccccc    420
   cttccccggc ctcgccgctt acctggctcg gtctctgcca gcgtctcggc ctcctccggc    480
   gcggcgcggg gcccggtgct cagctggtcg ttctgcgccc cggcgcggct gcgtcctccc    540
   tctggctctg gctctggctc cggctccggg tcgcgctgtc tctggctgct cgtgcggccg    600
   ccttgccgct gcgcgccgtc ccgcaggatg tcctggatga ggaaggacgt gagcggcttg    660
   gacggggtcg gcggcgcggc cccctccgct ttcgcctccc cgggccgcgg ctccggaacc    720
   ctgagcatcc cggcggcccc gccttggcct gaatgcaccc gcccggcccg caccgctttc    780
   actttccgcc tcccggctcg cttatagccc aggggagcgg gccgcgccgc cctcccattg    840
   gccgcgggac gggccgcgcc gcggattggc cgccctgagc cccaggtgac cccgccgatc   900
   tgcgagccct gagcactgcc ccgctccgct cctccctagg ggattccttc cccagttccc    960
   tctccttccc caattccctc tccttcccca atttcctctc cttctccagc tctcctccct   1020
   ccgccctgag ccgttttccc aggccaggag cgcaccccaa agcggggctc ctctgcccca   1080
   acccacccgc aacagagaga agctcctggg gatgggacga tcaagacaaa cagtccagtt   1140
   agctcttcag gaccgtcaga gccaattaca ggggatgagc acgcagtcac tgcagccgcg   1200
   cggtaccgtg agagttctgg gtgctctctc tctctgggag agatggtgca aactcagatc   1260
   tgcaatatct acgacctcaa agaggcatga caagtaggtg cagccagacc ccaggctgaa   1320
   agaaggaaaa cacctttgcc aaagcctttt cccgttgcag aagtggtttt ctttggtggt   1380
   tcctttatcc gcaataacct gttaactttc cttcctcccc agacacatga cataccatcc   1440
   ttttgaaaag tttccagacg gatggaaggc gttttaggcc aggacaaatg caaaaactgc   1500
   atttaaaaaa gtagttcaaa agaacatcag catactcttt ttgcagatta ccaactgtta   1560
   acatgtaacc cattggctaa acaatgggtt agccagattg gtccaaccgg tactgtgact   1620
   ggactggacg gtaccgtgac aacagacgaa agtggaacag agatgatgga agtattttct   1680
   ttccttgtct tttctcttct                                                1700
<210> 40
   <211> 1700
   <212> DNA
   <213> Homo Sapiens
<400> 40
   ctggcctccc aaagtcctga gattacaggt gtgagccacc gcgccggccc ccaaatatgt     60
   tatttataaa ctgtccctgg cccgtccctc agatcgcagg cccaaccacg cccccagaaa    120
   aatctctcac cagccctgct ggccttcggg cagtttctac aatgcaccaa ctcgttcctc    180
   tcccatgttc ccaaggagct ttctctgccc cacgatctga gactagaact gcctctttct    240
   cattgttgat gtctaggccc aaatgacatt tcttcatacg taaaacgggc ttcaagtctc    300
   actcatggct gtgacgatct gaaagcaaat gcgtgtatat gacaacgtgt gagcgatgag    360
   aagcttgtca tctgtaaaac tccatgcgca tgttaattat catggccgtg ataattatgt    420
   acattataga aacattaacc aagcgctggg ctcgtttcag gaccttcagt attgctggga    480
   ggcttggtat ccgacaggtg ttccaggagg gcaggaaagg agagatatgt ttggtcccaa    540
   gtctcgcgag ggctctgggg agcgctgggt cttcaaggac gttttggatt tgaatacttg    600
   gtgagagcgt cgggtagggg aacagcagga gcaaagcccg gaaggtggct atgaggataa    660
   gaagggagga cacctggcag gctgctaaaa ccaggcccac ctgccgggca ggtggaggcg    720
   agcaaccacc taagctctct gggacagcgg gagagggggc aggggagtcg ccgcaccatt    780
   cttcctgtgg ccgctagggg gtccccgctc caggtgagcg ccaagctcta gccccaggtc    840
   gctcccttat gcttcaagca agctttgcag cgcagccaat tgccaggggc tgtcccgcct    900
   actccccgtc ctcgcagcca cttgcctgcg cgaaatgcct gccaacttct gactggcagg    960
   cagtctggca aatcaaatcg cgacctttga aagcaaaaca ctgcagcatc ttggcagctc   1020
   tgaattggga agggatgaag gaggctgtgc ctccgggttg cacgaagagt ccgagtcatt   1080
   tctcagaagg ttttgatagg tgggccttag aggagacgcc gccggtgagt agtgattaac    1140
   accgggagga aggggaattg aatttaacct tcgttttttt ctggaaaaag cgaagtcacc    1200
   taacgtcccc tagtgtacat acccttcctt cttactgtca ccagcctcgc caacctgggt    1260
   cccgttgcct tggaatgttc tttccagttt tgcatcgagg ccaagaggag cgggggcatg    1320
   ggctacctta ctaaaggtga tgccaggctc taccaaacca ggaagtgaca tggagttaac    1380
   tttgccagaa tttctcctct tcgtgccgag cggctcgggc ttcctggcgg cagcagatgg    1440
   tggagttagc aggtgggatg aggggaggcg ttcttggtct aagcccgctt ctggaacaga    1500
   ggtgctgtct cctcgagttg taagtttcca gctcagtggg acgggacgga agaatgtaac    1560
   cttctcgtga gccaaagccg aggaacggga agcttggcag ggaactggcg ctcacctcca    1620
   gaagccagat cgtcgggtgg tgggaaagag cgtgttttat tgatttgttc agaaagaggc    1680
   aaattcgaat acagacgcta                                                1700
<210> 41
   <211> 1700
   <212> DNA
   <213> Homo Sapiens
<400> 41
   catgaattag ccatattata cagttgtttt attaaaaacc aaaactttgt agagtacatt      60
   aacagagact gtatttaaga gaaaggcagt catcccttag tcatttatac aattatttat     120
   taagcactta tgtgctagtt gatacttctg ggtgctggaa taaaataagg aaaaaatcaa     180
   gatctctacc ttcacgaagc tcactaaagt gtggtaaatg acagagcaac taccttcaat     240
   agtctctagt ttgtattctg caaccaactg aatacagcaa atactaaaag ctacttgagg     300
   tagggatcca tcctattttt acctgttgga tcccattccg tgcctagcac accagcttgt     360
   aagcaatcga catggattaa ggggtaaaaa caggccctta aatagctaaa attttgacaa     420
   gtaatcactt gtctgatcat caaatatgtc gttcgaaatc ctcatttttt tcttcaaagc     480
   gcagtaaaca agtatttgcg cacctattac gtgcaaaaca gttttcaaca caatgctttg     540
   tgtgtaaaag gaacggcggg aatggattca agttgcgcat cgtgataaat ggcgtgaata     600
   tcctgagcgg gtggacagcc cttcctccgt gagttttttc tttttctttt ctagtcacca     660
   agacagcacg cgtgtgcaga agctgcagcg gtgggacgca gtgctaagtc tgggggcgca     720
   cacaccagct cgcgcgtcgc gagctcgcct gggcagctcg gcgggcgcgg cgaaacagct     780
   cccacgtccg ccttccaccg actcctcggt atcagagcgg acgaggcgtc cccccagagg     840
   aaagactggg cgccgcagac gccccttgga gaagtccctg gccactcgcc ccacctcggg     900
   gtcctcttcc tcttgggctt ctcctgaccc tttgggtggg cgctccagac aggcccgtcg     960
   gacgcgattt ggaaacgggt cgtgcccggc ctagcccgca tctactcgcc cggccacagc    1020
   ccagccacag cttagggact ttgaggcgcc cgcgcccctt tgtcgcggta tctctagccc    1080
   aagcaggccg gacgccgagg cctcgcggta tcgagcctcc gtccgccccg cctcctcgcc    1140
   ctccactccg ccccgccctc gctctctcac ccctcccccg ctcccgcctc gcttccccga    1200
   caatctcctc gcgctcccgg cccggcacgc gcgctgcgcc cggccgccgc tgccgccgcc    1260
   gccgccgccg ccgctgccgc cgagcgttcc tccgctgcgc ctggcttcca gcttcgggcc    1320
   ggaaccggaa gtttgggggg cggagcccgg cggaggccag gagaccgaaa acgcggccga    1380
   gcccggagcc cggagctgga gccagagcct ggaccagaac ttggccgccg cctgcaccgc    1440
   cgccgccgct gccgcccgcc gccccttccc cgcgccgcag ccgcctcgcc gccaccgccg    1500
   cgagctcggc cgccagtggt cctcggactt taggtgtctg ggttgaaggt cggtccggac    1560
   atcggaccca gtcagttccc agactgtccg ggcggcagcg gacgccgctg ccgccgccgc    1620
   ctcttcgtcg cctcagcctg gcgttttgtt ccgagagacg ggagaggcga gcggagctga    1680
   cagtgatttt gacagtgatt                                                1700
<210> 42
   <211> 1700
   <212> DNA
   <213> Homo Sapiens
<400> 42
   acctccgcgg ccatccccag ccaggccagc acagcgctcc gtgcccgctg aaccgtccca      60
   ggcagtcctc tgatgaccca aaccaccacc acctttgggc ccagagcagg acaaatgaaa     120
   tttcaacagg ggcctgccct gtgaccgaaa attactcagc ttcacagccc ctgaatgcaa     180
   ggagaggtgg tccccccaga accaacatgc cccccaaggt gggtacgccc acggttctgg     240
   gaaatgcata cctcaggtaa ttcacaggtg cgatccccca gtggaggcgg ggcagcctga     300
   acttctggcc ctgcaggagt gggaagaaat cgcttttgtc tttccgacaa ctgagccttc     360
   cacctggggc tgggagggcg gacagcagga ccggctcttc cactccccac cgcggccccc     420
   cgacctggtc agtagctctg gcgttcagac aaaaggtccc ctgagctggg ggtggggggc      480
   aggtcgagag taggccggta aatcagactt cgcagctcct taagtcacca gtcctgcatt     540
   tgggtgcaga gattccgttt taaaaattcg aagcatcgct ttggttcgct ctttgttcgc     600
   gggcgcgggt tctcgctcat ccatccatcc atccatccaa ccatccatcc atccattcat     660
   tctttcttga cgcactcgct cgctccccgg tcgctccggg cgcattaccg cttcttcttc     720
   tgcttgaggg acttcctgcg tttcaggatc atctcataga gcttgtccat gccctcggtg     780
   aggccctcgc cgatgatggc gcacgccggc tggacgtgat aggtggtggc cgggataagc     840
   tcgtgcagcg ccagctgctt ctcaatctct gccaccggca gcgacttggg caggtcctgc     900
   ttgttggcga tgaccagcag cggcgtgccc tggttctcgg cgaacttggt caccttgtgc     960
   agctccgtct tggcctcctc cagccggtcc acgtccaccg agtccaccac gtagatgatg    1020
   ccgtccgtgc agcggctgta ggacttccac agcggccgca gcttctcctg gccgcccacg    1080
   tcccagaagt ggcagctgat gcccttggcc gtgccgttgc tcagcttgat cttctcggtg    1140
   ttgaagccga tggtgggcac cgtgttcacg aactcgttga acttgagccg gtagagcacc    1200
   gtggtcttgc cggccgagtc caagcccaac atgacgatat gcagggactg gaaggccgag    1260
   atgttagagg agatgttgcc catggctcct ggctgcggcg ccagccactg cggctgcggc    1320
   gggagggcgc cgccccccga gcagtcacgg gcccgacgcg gccgggcgca cctgggcccc    1380
   gcccgccgcc gcccgcaccg gccccggggc tcggcgtccc cgcgggcgca cggcttggcg    1440
   cgctcagacg ccgcagtccc cgcgccctcg ccggccgggc tcgcgatcgg cgggcatcgc    1500
   gtctctacgc gcggctttgt ctcccgcgaa gccgcctcag atgttcggcg ccctccccag    1560
   gcctgggctc gctctgtggt ttcctcgggc cgcgcccccg cgctccggca gctgcccggg    1620
   ctctccgctc tcccggcgcc cggcgctcgc ctctggcctg ggacggcgct gctcctgctc    1680
   ctgctcccgc tcccgctccc                                                 1700
<210> 43
   <211> 1700
   <212> DNA
   <213> Homo Sapiens
<400> 43
   agcaaactga ttttgcctgt ttagtctcac tagccagggt ggtatggaag tgggtgggga      60
   gagggctggt gcgtggcaca gataactggg aagcgcgcgc accacacaca cacacacaca     120
   cacacacaca cacacacaca cacacggccc cagttaacat ttatgcagta cttttggggg     180
   gtctcttaca cacacctgaa tagcatactc tcaataaagg ataggcacac ccctgtttgt     240
   cctcccccaa tttccagact agcattttac cctgggtcta gaattggtgg tgataagccg     300
   acttagatgg agtatttgag aaaaggggag gtgacccttt gcctgaatgg gggagggggc     360
   gctctccact caccgcctga gtccctggaa cgaggagggc caggacatcc tggatttctt     420
   caggccgggc cggtggccgg tctccaccgc gtaggaggtg cggtccatgg cgcgacagta     480
   caggtagcgc tcggtgtccg agtagccgcg atgccggacg cggccggtgg ccccgctcga     540
   ggcgtagcgg ccgcgggcag ccccgggcgg cggcgggggc ggcggcaggg ggggcggcgg     600
   cggcggctgt agcggacact ggggctgggg ctggggcgag ggtggcggcg gcgggggcag     660
   gtggtgatgg ggatgcagga ggcggaactg gggctgccgg agcgggtact ggtggtgctg     720
   ctcgtgcctc cagagatgct tgggggcttt gagcgtggcc ccgccggcgc tgtcgctgcc     780
   ctctccgctg cccgcccggg ccggcgcgct gctgccctct gcctccatcc tggctcgcgg     840
   ctccgcgacc tgctgcccag cccgggttca ccgcgctggc ccgagcgcct tcctgatgct     900
   gctgctgctg ctgccgccgc cgccgcttct gcagcccagc agaggctggt gcttagcttc     960
   tcacttgaag gcgccagagc ctcagggcta ccgagagggg gccctgccag cgggaggggt    1020
   cacagaacgc ggcagggctc cttccaccga ggctatggca gagacacgct cccgggctcc    1080
   ccaagtcacc aagactaggg tgcgcggtgc ccggtttcgc cagaggttgg tccttcttcc    1140
   tccctttctt cttcccttcc tttgggcgcc ggcggcgaga gcagggattg tcagcaactc    1200
   gggctgcaga agcagcagca acggcggcgg cggcagtggt agccgcctcc agggcaccgg    1260
   aaccctgatc ccggagctgc tgaaaagttg gacagctcca ggctggcacg cccgagctgt    1320
   gactttcacc aaaaccactc cagccagtca ggagtcagac ctgtcctctt gatccttccc    1380
   ctcgccgtgc ccgcgcctcc ctcctccaca ctgctcccca ctgccccgga gccttcctct    1440
   tcacctccgc ttgcctcagt gctcttagag aaatggagtt tggcgattcc tggctggttc    1500
   tcagagcttg ttgacgatga gccccatgct ggtgcttcca aagataagga atgtgaccag    1560
   gcaaagttgc ctggaatatt gtctatgcgc gtcgctgaaa aattggatct gggtgaagat    1620
   ccaatagtaa gattaattca gtctctttcc ctgacatagg ataaaacatt gtgtggctct    1680
   ttcccacagc agggagaatg                                                1700
<210> 44
   <211> 1700
   <212> DNA
   <213> Homo Sapiens
<400> 44
   ggacagggaa gcttagctaa gaaccaggag tttgacatac ttaatctctc atttcatcct    60
   actgatgact ttgtgaggta tatcttttta atcctagatc tacaaaagag aaagctgagg   120
   cttggagagg ttcttcattt tgttcaaagt catacaactt ttacgtggtg tagccaggat   180
   gggaaccaga gcctggctct ctgcacttcc aacaccactg ctgaatccgg gtttgtctgt   240
   tttccctctc ttcttttaac tattgtcaca cagattccat catagaaccc atcacgtgga   300
   gggccactat ttctctttct tttgtatagt atgtttgctt cccctacccc acagagtaag   360
   gtgctcagca aatgtggctg tctggaggca acaccccctt ctcaccgcca cacttggggt   420
   tggggtgcga ggcaaggcag ggctggcgag gtggatgttg cagctgtcac cactgctagg   480
   tgttgggaaa cgactctcct gcctgtgcat tgacccgaag gcattttcca tttcctaggc   540
   ccctcccgct tcccgtccct cacctctagc ccctcggctt cagctcaggc ccctcgggga   600
   gcatcccttg ccgtgagact gacagccttt gggggcgcag ggtcctgttc tctgcgctct   660
   agcccatctg tgcgcagagc ctcgttccca ggcgcctgga acccggcggg cattgacgtc   720
   aagcgccggc ggagcgctgc ctacagacgg ttgacccggg ccctcctcca cacccccttc   780
   cttcttcgcc tcctccctct ttcctgcacg ggggctcggg ctcactataa aaggtgggag   840
   cgcgtggtgc cccagcaacg acgagtttca gaacgatgga gagctcccgc gtgaggctgc   900
   tgcccctcct gggcgccgcc ctgctgctga tgctacctct gttgggtacc cgtgcccagg   960
   aggacgccga gctccagccc cgagccctgg acatctactc tgccgtggat gatgcctccc  1020
   acgagaagga gctggtcggt attcccctcg ctctcgaccc ccttgagctg tcgccttgtc  1080
   tcttctcttg cacgcctccc tcctcccccc acccccactc ctattcccag agtcagggcg  1140
   cggggagctg agcgcaacgc ccaggcaccc actgccatcc gaagagcgtc tcgagctcac  1200
   gggctcctgg cagtctgttg agcgaatccc tcatcccggc ccctctgagc aacagggacc  1260
   ccagcggctc agagacccgc ggtcagtacc tgggacagcg tccgctaagt ttccacccct  1320
   cgaccattcc ctgtgtccgc ggagtcccac cgcagagtgc gtgtgggtcc ggggctcctt  1380
   ataactaggg ctggaagtgc gcacctgggc tgggctcgca gccaaggcgg caacttcagg  1440
   ctccgaagcg gtgtgttgca gatcgaagcg ctgcaagaag tcttgaagaa gctcaagagt  1500
   aaacgtgttc ccatctatga gaagaagtat ggccaagtcc ccatggtaag gtttgtggtc  1560
   actcccttcc cgtgtttttc caagagaaag tacaccgcct tgaatcgtac acacagtctt  1620
   ctccgtagga tgtggctaaa taacttaggt aatgggcttg caggattctg tgggctcctt  1680
   cttccttccc gggtgaggaa                                              1700
<210> 45
   <211> 1700
   <212> DNA
   <213> Homo Sapiens
<400> 45
   gtgtttgcac ttcatgttac tttttgagtt tataaacata aaaacagaat ttacttctgt    60
   tacagaccta gttactggga attcattact tgccatggac tacctttgct aagaaaagtc   120
   tgaatgagaa gatggcagga cgtctgaaaa aaaaagttat aattaataaa atctgcggag   180
   aattgtaaat tctgccttca ttgttgatcc aaacctttct tctaagacct cccctttcta   240
   gacatctatc tacctatttt cactttactt tccagcatca aggaggtctg agtggataag   300
   gcctaagcac tgatgtggct ttggtaatga acctgaagtt aaaaactggt tagcttatgc   360
   taagctgcca tttgaaaggt ctcctccagc atctgaatta agggaaacaa tgttttgaag   420
   cagtcacaaa acttttctga tgcattagat ctgaggtagg acctgataat ttgtacttct   480
   aaattatgca ggtgatgctg atgctgctgg tccggagacc actttggaga gttttacttt   540
   aaaggaaatg aaactgccct aaaatgctaa tactaaatag ccacacaaag ccggaaaaaa   600
   gcaaagaatt tcaaaccgaa gatgagaggt cctgaaaaat aacagcggtc atcctactcc   660
   cggtgcggct atgcggccat gaaaaggagt cttggggccg gtcgtgcccc tccacgcagg   720
   ctccctcata gggtcatccc atcaaccctc gccaaaaacg ccggccccct catagggccg   780
   tccggccacg cccctccgtg ccctaaccac agagccacac gttacgccgc cgccccgtta   840
   cgcccatccg gctcccgcat agggcgtcac gtacgtttaa cgtcggcccg gcgcgcgcgt   900
   ccgggaggtt gcccgtaggc cgctaactgg tggttcccag tccccgccga ctcggcctct   960
   ttcttctgcg gccaccagat cgcgggatat cggagacttc gaacaatccg ctggagtcct  1020
   ttacagttta aaaacagagg accggccggg cgcggtggct cacgcctgta atcccagcac  1080
   tttgggaggc cgaggcgggc ggatcacgag ttcaggagat agagaccatc ctggctaaca  1140
   ccgtgagacc ccgtccctat caaaagtaca aaaaaattag ccgggtgtgg tggcacgcgc  1200
   ctgtagtccc agctactcag gaggctgagg cagaagaatc gctagaacca gggaggcgga  1260
   ggttgcagtg accgagatcg cgccgctgca ctccagccta ggcgacagag cgagactgcg  1320
   tctcaaaaca aaaaaaacgg aggatcgaaa attttgaaga ctgagagcaa ggcaacaatc   1380
   ttcccttgac ctttttgctc ctctcacaag ggtttttcag acggctgaaa ccaacgaaaa   1440
   gagtgaggaa gcaggcagaa aatttcagct ttagtctctc agcatcctcc tgctttcttc   1500
   tcggggagct ggcacttccc ccttctctag tagctgtaaa tacgcctccc aactccaagg   1560
   agacctttct tgcatctccc ttactgttct cacgcctgta gggctcttcg tgtgaggatt   1620
   cagtgtatgt atgtatgtat atgaatgtgt gcgaataatt atttcctaat tgagtttttt   1680
   tttaaaaaaa tcatcttccg                                               1700
<210> 46
   <211> 1700
   <212> DNA
   <213> Homo Sapiens
<400> 46
   gtacacgtga aaacaatact attttatatc acgactcatg ccatccgtat gtgtaatcaa     60
   agccttctaa gtcttgatat gtccttacaa agacgaagga tagagtgaat gtcaaaattt    120
   gccaaacagg agttactgaa agtctgaagg ggaaagccta gttaggtttt atcttagaag    180
   tgatttttaa aacgaaacac gcagataagc tcgagctttc tggagccgat tcctaaggcc    240
   ttgcaggtgg taagggggtc gagaattggc tgaggttcct cggaagtccg cgccgattcg    300
   gagggccacc tctgactttc tgatctgggg tgcaaccccc agtccctgtc ccggtcgaca    360
   aaggtgtgga gtcaggttcc aggcaagctg ggggtggggg tgcgcacaag ggaggggcag    420
   gggactggga ccccctcttc ttcggggtgc ttcgggccgt ttctccagag gctggtggtg    480
   tccccgaccc aagcagctca gccccgctcg cgaggtccga agtggcggga acgggtcctc    540
   cgcggcggtc ccaaggcgag ggaggctgta aaacctggca gagaatccgg cgcgccgggt    600
   cggggatgtc tcgggtgccg ggggcagggg ccgcaggctc gcaggtagaa gcttccccac    660
   gcgagcgggg gcctcggccg cggaaggggc gcccccgggg ccgccggcag cggcgagcgg    720
   gcgggaggga gctttccggg cccctcccgc gcctcgggct ggcggggcga tggcgacgac    780
   ggtttcccgg ctcccccgcc ccggacgccg atggcggtgc gttccggccc tcccccaaca    840
   gcttctcccc cgcggtcgcc ggagccggtg aggccgagag ggcggggagc cgcgcaggga    900
   aggcgagagg gcggaggttg cgtggtgcag gcggcggcgg cgggaggagg agggccgggc    960
   gggcagggga ggaggaggag gcgggcgccg tgtcgcacgc agctccaggc ggggcagccc   1020
   cggtagctga gggacgcagc tagaccttgg cgggacgggg ctttcgccgg ggcccaggcc   1080
   cagggaccag gcggaggcgt cgcgggagcc tttggggcac cacagagatg cgggtgagtg   1140
   tcggcagccg ccggggccga gaggaggtgg cggcggcggc ggcggcgcag gcccggcccc   1200
   gagccgcggt gtcgcagtga ctcgggccgc gacggccgaa accaggcgcc tggcgaggcc   1260
   tgagccgccg aaacgcggcc gggcagaatg gcggccgggc cgggggactc gtgggcggag   1320
   gacgccgaag gtggcagagg agcgagcggg gccgggccct gagtcacccc ggacgggccg   1380
   gggcggccag gcctggacta cgcactggga aaaagcacga ttcatcgcgc ctggaggcgg   1440
   cggcctggtg gcgccggccc ctttccccgc cgcgaccggg ctgggttctc ggagggtggg   1500
   ccgacgtgtg gcccggggcg gctcgggcgt ccgcgcctcg cctggtgagg cgacctccgc   1560
   gggacgccgg gacccgtgcc cttgccccct cgcgcccacg cttcttcccg gcgctgcttt   1620
   ccggccgacg cgtgttcccc tgtgagctct gggctcaagc gcaggagcag ccgcggagcc   1680
   gggctctctc gctttccggt                                               1700
<210> 47
   <211> 1700
   <212> DNA
   <213> Homo Sapiens
<400> 47
   gctagagatt gaaatcctct ggaaccaacc actggacgct ctggattggg gcggaacgcg      60
   cttccactgt ctcgaaagca gcaccttctc ggatttgagc ctttgcggcg cgcctgggct     120
   gagatctgag aaatgcgccc tgggccacag gcagcagcgg aggcggggaa ggaacaactt     180
   tccctccgcc ctctaaccac aagggcgggg tggggcggcc tcgctggggt cgggctcctc     240
   cactttccca gctgggatcc cctgccggag ctggcgtggg ggccggggag ggggaccaga     300
   ggggcttcct tctctcccca agccccgcca gatgttggaa aggcaaacaa taggggaaac     360
   gcggagaaac aaaaggccat tcgccacgat ctcagtcggc tgcgtagaga gatctctgtg     420
   gggagggtta ggggagaggg tcaggaaaga gcgcgagggg ggggatcccg agagatttag     480
   agtctctggt acttgccagg caccaggggt cggcgcttta ccctcccagc aggggtctga     540
   aggtgggaag gggcaatcaa agaaaacttg aagggttttc accttcttcc ccagttcagg      600
   gaaagggagg ggctgggatt ccagatgttt gacagttgtt ggccccaaag ttaagcgcga     660
   tttgtacggc ctttacacgc tccccacctc cgtgaggcat cgtcagcccc gggggcgacc     720
   tggctgacgg tgcggacttc ccctacgctg ccccaagccg agactcctcc ctcaggatca     780
   ggacgcccaa gcgttgtccc ccagcccagg cgcacttttg taaaagtttg tcttcccgcc     840
   acccacagcc caacacgcac cctgcaggcg tccgcggctg gggatgggct cggagtcttg     900
   caccccgaaa gaaggacgat cttattgatt tatatctttt caaatcaagg acaaatcaag     960
   atcccggggt ggaaagtggg cgacccttgt tcctgccggg aagtgcacac gaggagccgg    1020
   ccagaaaccc aggcgccggc aggggtctga agaggactcc agccccggaa gcccgtggcc    1080
   ggcgggcaga agaccggtcc gccgcctccc gcaccttctt gcacccgcgc ccaggtgcgc    1140
   cccaaacctc gccgcccgcc cgggcgagat cgggagcgag cagccaccgg gctgcgccgc    1200
   ctcccctagc ttggagcggg ttcttactca ccgtggggtt ttacctccag cccagggccc    1260
   ggtcggctcc cttcgccggg tcagctgcag ccagcgcgaa agaaaatggt gagctcgggg    1320
   caggtggggc gtcgcttgcc aatccacgca agggactctg ccgccggtgc gcgaggtccc    1380
   ggcgagccaa ccggcggacg ggcgagcgcc tggctccagc tgcagccgcc gtcgctccgg    1440
   agcagccccc tcatgcaccc agcgcgccgc gcagccggcc ggggctcgac tgtctcgcgg    1500
   ggacgccgag cggggacccc tggcggccgc ctgcggtatg gcagcaccgt ggagagaaaa    1560
   gtcgtctttg ggatcatctt ccctccggaa agagatggga catgaccagt ctaagggggc     1620
   ttccagcctt gcagaaaaga cactgggcca gggcagtgaa gaaggtgtca gatcagaaag    1680
   gatgatctct gggtccacaa                                                1700
<210> 48
   <211> 1700
   <212> DNA
   <213> Homo Sapiens
<400> 48
   ctaggatctc taaccactca ccacttgtat tcctggggtg gacgtgagct cgcgctaaac      60
   taaggacacc agccgagcat tcagcgcggt tccctacgcc ctccaggaat tttggaaagc     120
   tatccaagct cggcgctatc tttaaaagga atgagttgtg gctcctaact agcttgccga     180
   ggagagcgta gagtgacacg ggacgggggc ccagggcact gaggccaaga gttcccgagt     240
   cacgccagtg ctcgcccctt tggcctttgc ctaaaatcct gcgatgcctc gctgacaaag     300
   cacccagtgc cagagcctgc ggcccagcga agctgctccc tggaaacaca cacacacaca     360
   cacacacaca cacacacaca cacacacaca cacacaaaag ggggtgggga gagggaaagg     420
   tttaggttta ggagaggaag ccagccagcc agccctgcag atgcaaccgt tgcaatttgg     480
   gtctcttctg tcttctgacg taatgccagt ttcctgcttt cgcgctgcag tgtgcagttc     540
   gctgctggga agtcagatca atcgattgaa cgccaagatc atcactgcca ctatcttgcg     600
   atttattttc cacgaataca ctttaattgg cccgagcccc gcccccaaat tccacgactt     660
   ttggcttttc aggaagcatt gttctcccct ccccccagga aggccgcctc tacgcacagc     720
   cagcatcagg aaacggcgcc gctcggggct ccgatggaga gccggggtgg ggtcggctca     780
   aaccttctgc cccccgctct tttgagattc ctgatccctg gctttgtctt aagcaaaagt     840
   actgtagaag ctgcagcgtt tcctccccct ttccagaaac cgagcgagga cgtgggggaa     900
   aagccaccct ggaacgtgta aggacgctcc cccacgccca acacgccaca gggccgggat     960
   gcgcggctgc cgccccgcca cctcacgcgc gcagcgctaa ctgcagctcc gcagccaccg    1020
   ccgtcgccgc cgccctgccg agcccgggga gactcccaag ggctctttaa atacgagcta    1080
   taaatactac aggcgctcgg cgcgtgcgtc acgacacatc cggctcccga ttgggcggcg    1140
   taacctcgct tatttgcata ggccgattgc acaaccgggc ggcgacctca gcccggtgct    1200
   cgtgccacgc cttccccact tctgcgggca cgcggggccg cgggcgggac agcggggtgg   1260
   cgattgcggc gtccagagtc ccttgaactc gggaaccttt caagtcctga gtcaggcggc    1320
   ggtcctccgg gacgccagtg cctctcagaa ggctcagttt tggccagcca agggcttacc    1380
   gtttaaactg ggtctttttt atttctacag aaaaggatag ttttcagagt gacaccccag    1440
   caaacgctct gggtggctcg cagaggctga gtttaaacca cgtgagacct ggccgcggcg    1500
   agagtcctgt ccaatgactt gcgacttggg gtgttcaaaa agggcttttc gcatgatctc    1560
   taataagacc tcgttatctt gaaaacgaaa cagggagggt tcgaaaacgc aagatgtgta    1620
   atgccttgtg gtttaaacct agtttttaaa aaatgcacca atgagccaat atgaaacagc    1680
   gacatggaga ctttccccca                                                1700
<210> 49
   <211> 1700
   <212> DNA
   <213> Homo Sapiens
<400> 49
   cattccaagt caccaagttc cagacaggca cagtagcaca cacgtgtgaa cagaatccat      60
   cagtacagtc tggggcccag gcctttgcac accaacaact taaggttctt ggtaatggcc     120
   cagcgtgcac ccatgaggcc actgctcttt tacacacgca ctcagaacac ccacagcttt     180
   aaaacggcaa tcttactaca aaaatgacac tttgcatttg cacagcactt tgttttttgt      240
   gtactttaca catttattac ctaatgactg tgttttttga aatctccaat aactctgggt      300
   ggtggtaaga ataggcatcc ttaagccacc ttcccagatg aggaaactta tctgtgttcc     360
   cctccttctt ctcaacagcc ggctgatgga acccaagccc tccacaccag cagtggggcc     420
   agctgcacag agcggccaga agggagaggc cccggggcca cgcccttctc tctactctca     480
   gttcctgact cattgtcaca tcatggcgag taacaggaca accctcccca cagaatcatg     540
   ggggacacta ccttcctgag gccaatgtgt tcaaccaagc gggaaactct ccgggtagag     600
   tgaaatccga agttgctatg ctacaagata acctagagca aggggaataa ctgacgtttt     660
   aatgtcttct tttatggggg gctgaggggt gcctactcac actcccccct ctcgtcttcc      720
   cccgctactt ccctcctgct ggtcggttca actccacaag cgcgcgtttg tttcgagtac     780
   agtaacttag agcagatggg gggcactttc tagagggcag tggaaagccc acttgctact     840
   gctgcgccgg ggacctgcgg ctccctgggc ggccacgccc tgcctcggcg gcgcctggcg     900
   cgtctggaat gcggggcctc cctccgcccc tctccccacg ccgcctcgcc ccgctcccgc     960
   tagggcttct tagagaagcc cgacccccca ccacctctgc cctgcaccct cccggcccga    1020
   gggccggtgg cccctcaccc ctgccccaaa caccttgtcc ctgagccccc tccccaaaga    1080
   ctctccagtg ccggccgtcc cttcagcccc ctgcccccga cggcctccgg ggccagttgc    1140
   cggggcctcc ctgccaggcc gtacccttct tcccagcgtc tccacacccc gctagctggc    1200
   ggcccatcgc gggcgggcag ggcgcgcccc gcttacctgg gccgtgcgcc gagagctgag    1260
   agcgtcccca aatgctgagc agagccggct ggcctgggtg gggcttttat acccgccaca    1320
   tcctccctag gcgaggcgag gcccgccccg ccccgccccg cctccccctt acacggcccg      1380
   ctcccacccc aagtcagggg tgactcaccc cggcccgccg gcccgcaggg accgcgccct    1440
   ggaacgctcc cgagccgcgc cacgcgggag cggccccagc gcccctggcc ctgccctccg    1500
   agaaagcccg gccgaggctg ccccgaggcc cacagggacc ccgtttcttc ctcctttcct    1560
   cattttactt cttaagtgcc acgtcgagct ctcagcctcg gcttgggtta ataaattaac    1620
   aagactttag tagtagactc tgggcttgat taagtgtttt tttttttttt tttttttttt    1680
   tggcgttaat gcaatggagg                                                1700
<210> 50
   <211> 1700
   <212> DNA
   <213> Homo Sapiens
<400> 50
   ggctccctgg tagctcaagt gtcagtccag acattgagac ctcggcaaag agcttccctt      60
   taccctctct tcccactggc ctttgtgggc ggtgtggaat gtgctgtagg ccttgcgaga     120
   gcggcatttc tttttttttt ttctctcttt ttttttaacc tattgtagcc tctgagaggg      180
   cagcattttt gggcgcacct gcacccaagc ctctgacagt gccctgggtt cctccagcaa     240
   cgcgcatcta gggatgctat gccctgtggg ccctatgccc ggggcacgga gctaggggag     300
   gcggtcaccc tacttctctc acgtccctgt ccaccttgcg cctcagcgga gccttaggta     360
   aacaagccag gctcccagtc cccaaggacg gccttcaccc cagaccccag gaggaggcgt     420
   cttgggccat ctcgggaagg ctagcttgct cgggaggggc agatcacgaa aacggttctt     480
   taggcagtgt cacttcctct ccactgccag ccacccaatt taggcggctc catccctccc     540
   gctttcatgg atatgcagca ggatgcagag agcacagagc tgtgattggc acgagctcac     600
   aacgggcttt cccgctcaat ctactcaact tccagcaagg gacaggccct gcccattggg     660
   gttcgctgag gcctcaaacg cgcatgctgt caagggagca gctgcggggg cccgctagtg     720
   gggacaaagg cgcagccacc ggggcgaaag ctccgccccg cagagggtgg gcctgcggcg     780
   accgcgagct cagagtccgg acttgtcgcg caatctgtct ccttcggttc caccccaacc     840
   tagctggact ctcggtccgg tccctgccct ctggagactc agccattaaa gttgaggtgg     900
   ggggatgagg atgtgggcgg cgtccaggta actctgaggg gccctgatgc cttccctaga     960
   gccagccatc aggagtaaca cgatgcgccc ccacatcacc gaacccagaa cctcagtgct    1020
   tcagcacccc ttcctcgaca tgagctccgc ccaatgagga gaagggtctt ctgcattggc    1080
   cggtggtggt cccctatcca attagagagg ctaacggcct tctctcgtct ggtttagaag    1140
   gaatcgccgg gggaatacag gccaaactac aattcccgag aggtagctgg gctctgcggg    1200
   gggggggggg ggggcgcctg cgtgttcacg tgacttcctt ccttctcgcg gagaccgagt    1260
   tacgcgatag gcctgtgagg gggcggggcc cggcgcctgt tcgccgtgtt agtggccaat    1320
   cggcagccag gcagggtggg cgcgcgtagg gggcggggcc gggcgcgcgg cagggcgcga    1380
   gagcgcaccc gcggcggcgg tggcggcgac tgtggggggg cggcggggaa cattggctaa    1440
   gccgacagtg gaggcttagg caccggtggc gggcggctgc ggttcctggt gctgctcggc    1500
   gcgcggccag ctttcggaac ggaacgctcg gcgtcgcggg ccccgcccgg aaagtttgcc    1560
   gtggagtcgc gacctcttgg cccgcgcggc ccggcatgaa gcggcgttga ggagctgctg    1620
   ccgccgcttg ccgctgccgc cgccgccgcc tgaggaggag ctgcagcacc ctgggccacg    1680
   ccgatgacta ctgcaaactg                                                1700
<210> 51
   <211> 1700
   <212> DNA
   <213> Homo Sapiens
<400> 51
   gccctggaca ttccctgtct tcctgaccca aggtctagtt tggctccctc tagggctccc 60
   tggtagctca agtgtcagtc cagacattga gacctcggca aagagcttcc ctttaccctc 120
   tcttcccact ggcctttgtg ggcggtgtgg aatgtgctgt aggccttgcg agagcggcat 180
   ttcttttttt tttttctctc ttttttttta acctattgta gcctctgaga gggcagcatt 240
   tttgggcgca cctgcaccca agcctctgac agtgccctgg gttcctccag caacgcgcat 300
   ctagggatgc tatgccctgt gggccctatg cccggggcac ggagctaggg gaggcggtca 360
   ccctacttct ctcacgtccc tgtccacctt gcgcctcagc ggagccttag gtaaacaagc 420
   caggctccca gtccccaagg acggccttca ccccagaccc caggaggagg cgtcttgggc 480
   catctcggga aggctagctt gctcgggagg ggcagatcac gaaaacggtt ctttaggcag 540
   tgtcacttcc tctccactgc cagccaccca atttaggcgg ctccatccct cccgctttca 600
   tggatatgca gcaggatgca gagagcacag agctgtgatt ggcacgagct cacaacgggc 660
   tttcccgctc aatctactca acttccagca agggacaggc cctgcccatt ggggttcgct 720
   gaggcctcaa acgcgcatgc tgtcaaggga gcagctgcgg gggcccgcta gtggggacaa 780
   aggcgcagcc accggggcga aagctccgcc ccgcagaggg tgggcctgcg gcgaccgcga 840
   gctcagagtc cggacttgtc gcgcaatctg tctccttcgg ttccacccca acctagctgg 900
   actctcggtc cggtccctgc cctctggaga ctcagccatt aaagttgagg tggggggatg 960
   aggatgtggg cggcgtccag gtaactctga ggggccctga tgccttccct agagccagcc 1020
   atcaggagta acacgatgcg cccccacatc accgaaccca gaacctcagt gcttcagcac 1080
   cccttcctcg acatgagctc cgcccaatga ggagaagggt cttctgcatt ggccggtggt 1140
   ggtcccctat ccaattagag aggctaacgg ccttctctcg tctggtttag aaggaatcgc 1200
   cgggggaata caggccaaac tacaattccc gagaggtagc tgggctctgc gggggggggg 1260
   gggggggcgc ctgcgtgttc acgtgacttc cttccttctc gcggagaccg agttacgcga 1320
   taggcctgtg agggggcggg gcccggcgcc tgttcgccgt gttagtggcc aatcggcagc 1380
   caggcagggt gggcgcgcgt agggggcggg gccgggcgcg cggcagggcg cgagagcgca 1440
   cccgcggcgg cggtggcggc gactgtgggg gggcggcggg gaacattggc taagccgaca 1500
   gtggaggctt aggcaccggt ggcgggcggc tgcggttcct ggtgctgctc ggcgcgcggc 1560
   cagctttcgg aacggaacgc tcggcgtcgc gggccccgcc cggaaagttt gccgtggagt 1620
   cgcgacctct tggcccgcgc ggcccggcat gaagcggcgt tgaggagctg ctgccgccgc 1680
   ttgccgctgc cgccgccgcc 1700
<210> 52
   <211> 1700
   <212> DNA
   <213> Homo Sapiens
<400> 52
   cgagggtctc ggctcccctc tctgtgtctg cctctcgccc agcgccggga gaagcagcaa 60
   caagttttgc atttcagcaa tcaatttcag ccattacatt tgcaccaatc agcgccgccc 120
   aagttccggg ctcggggcgg ggctcgctct taaggtggtc cggggtcctg gctgccgaag 180
   cccccgccac gaacccgcac taccccagct ctggggtccc ctgcgcccgc ctctcctcgg 240
   ggtccggcgt cagtgcgctg gggcgcgccc ccggcctccg ggcgcatcga gctggagagg     300
   gtgcaggcgg aggagaatgg cgggagcttg gctttgttca cccccttccc attcacactg     360
   ttcccaactc cccaccccca actggagcga gacccaccgg gtgggcaggg ggcggggagg 420
   tgggcggtga gcggtacggg gcgctcagac aatgaggtcg ccaggcaaag accctagact 480
   cgccacggaa agtcgctaag tgtctactgc acttcggctc cggggtgaac actccgttct 540
   ccgctagcgg cgcccaagtt agccggccct gggattttct tggagggtaa agcagacggg 600
   gaacctcgaa gttagggttc tcctcgagag acctgggcgc ttgctcactc ctccgtcact 660
   agggcgttca caattgttcc ccttccccac tgcctggggt tcccggttct caccggccgg 720
   cgtgggctcc aaggagtcac agtcactcag ccactttatg ggggagcaca tcggttgagc 780
   ccagtaaccg gagcgacttg cgaggcggct gggaaccccg agaagacacg cacgaagagg 840
   gcgcacccgc cgggccgcac accttgccct gggccacccg gtccaagacc cagaccctct 900
   gcgggtgcga agttggcctc cttaagagat caattaaact gaagaggccg aggaatggaa 960
   ttgggctgtg atactaaaat caataggagt aatttaatat ctgccctgct ctttatgaaa 1020
   tatttatccc aataatctta gttaaaatgt ttagatcgtc ctgatcccca cagaggctga 1080
   aatctatctt gcaattagaa gttggaagtt agaggtgatg gaggatagag gtgtttattc 1140
   tgtgcgcctg tgtgcgtgtg ctcgcgcctg cgtgcgtgtg tttgtgcatg cgtgtgtgtg 1200
   tgtgtgtgtg ttcctcccta aagtgtgtgt agggaaggag gggggagaag gcctttaatt 1260
   ttccttgcca taaaatagca cgaggcgatt ttgctagcag attcgcattc ttaggagaag 1320
   ggcgaaaaaa gaactttctt tgaagtgccc ctgatatcgc tattaaatct aaataaaatt 1380
   aagcgtggtg tatgaaaatg cctttccccc cccacaaaag aaagtgttta tcgcccgagt 1440
   ctgtctagcg tttgctaaat tacgcatgaa atctgaaatg aaataaacgt tataataaaa 1500
   ccaacccctg agcccttttt atttaaaaac cttagattaa gcactccacc gtcgcgggca 1560
   ggagttaaaa agttaccatg tcgtacgcgc agattcattg ctcccctgag ttgcggctag 1620
   gaaggcagtg ctaagtatgc cagaaccgac atgccggggg tggggggaaa tgggctcaac 1680
   tttggggtag cggcagaatt 1700
<210> 53
   <211> 1700
   <212> DNA
   <213> Homo Sapiens
<400> 53
   ctttacgggg atcttcgcca atgcctggct gagaggcggt ctcagggggt tgagaactct 60
   attcttagag gtctggtatc tgggagactg agaaagcctt tcttaagtct ccctgctggg 120
   ccacgctgta ttcacagatc atcttcaact ccttcttctt ccccacctac tccttcgccc 180
   tcacctcctt ggcttatctc ttgctagaaa atgcaccaag ctccaatctc cgttcctttt 240
   agcttcctcc ctgagaagac actgtgacct cgctgtcctt gttcgtgtta ccatggagag 300
   gtaggaggat aaatatcgct tccagttgtc agcttttttg ttttctcctt tccctcttag 360
   acagccccca gccccaatcc cagtacccgt ttcaccaccc tcccctggag aattagcata 420
   accggagtaa ataggtaatc aagcagtgtg cttgcaagcc ttaattttgg gacagataag 480
   gaagtgaaat tgattggaat tatacctgcc gctgctggag gctgagggag gcggccgccg 540
   gaggcgaggc cagctctgtc ggcccagggc cccgccccac cccctccttc ctccctcctc 600
   cttctcagcc ccacccttct ccacctcctc ccactttgca ctctcctcta gcggccgcct 660
   gtccttttta aggaactcca ccctaggtgg agtcctcacg atagtcacac ctctcctttg 720
   ggcctagcag ggccagagaa agacggaatc actctaccca ctcaggttgc ccagagagca 780
   ctttaggttc tgactcgcgt ggggcgcgag gtaccccgtg gggcagctag gtgcctccaa 840
   gaaaccccgc cccagaggac ccgcacgagt tgctgccatt tttcgctgaa gcccctgccc 900
   tggggctggt gttcttctct atgattctac caatcgtcgg catttttttc ctcccttctt 960
   tctttttagg aaggcgctgg ggtgtgcggc ggcaacggcg gcggtgaagg gatcctcttg 1020
   tggtatctcc tcgtgggttc ctgtcttgaa ggatttcttc tccgcctcaa ctttatgttc 1080
   aagtagcgct ttgggggttg ggttggtttt catagccccc acccccggcc ctctcctttt 1140
   aaagggtgtg tggagttcag aggatgggtg gagagcgggc ttggccggaa ctgaattggt 1200
   agaggtaaaa ggggaacgga ggaccttaag ctctggccac ttcggcccag cgaacccact 1260
   ttatttttgt ccaaggaaaa gctgcagggg ggaggattct ttttaggaag tctactctgc 1320
   cggcccctga tttttgggtt ggatgcttga gagctttgtt tcccttgttc cgagagtggc 1380
   ccagctgggt ctgaatcgac tccctcccct ttcgaccccc tcctcatttt ccagcccgga 1440
   gaaatagggg agtgggggcc caagaacgag aagacgagaa cgcgtcgccc tgcgctatgt 1500
   cagaatgggg cgggtgtgag gggaacagct ctcttgcgat cagctcagga gtatgagcct 1560
   cccggaggac ggcatgagtt ctggacactt caggagtcct cagctagtga catggtcggt 1620
   aagtgactct gggactaata cccacccact cccacctctt tcgtctagat tctatccccc 1680
   tcccagtccc cctgctcccc 1700
<210> 54
   <211> 1700
   <212> DNA
   <213> Homo Sapiens
<400> 54
<210> 55
   <211> 1700
   <212> DNA
   <213> Homo Sapiens
<400> 55
   ttcttcccct cctcctcctc ctcctccttc tccttctcct tcttcttctt ttgttcagcg     60
   aggagtccag atcagagagg tggcccagat agttcaccaa atggcaagac cagactagcc    120
   tctaaaccaa gacaatttac cctgtctttc tttctttttg aaatatacat cctttgctct    180
   tagagatctc aaaatcttct gatgacccat acatttattt gaatttttgt ttcctgataa    240
   aagtcatcat gcgcaccttg gaaaaagtga agaaaagtta aaagaaaata tccacccaga    300
   gacaacattt tagtggacct tcttgtcttt atttctgtat atatgagctc atagggtata    360
   tatgtttgta tatgtttaaa tttacatcat aagcatgccc agttcatctt tagaattttt    420
   taaacaaata taatttaaaa taggaatcta atatttcatg ttattgatag accgtagtag    480
   actgtgtttg tatttttaga taatatcttt ttttatcact gtaaataaga ctttattttg    540
   tgccttcaat tttcatcctt gtcaatattt tctgttattt tctcaggata catatgtaga    600
   agaaacatca atcagtcaaa tattttaatg ctcataagac atgccattcc agggcttcct    660
   agaaaaacag tattatcgta ttttgttccc ggaggtgcag gcgagtgcct ggtctacggg    720
   tgtttacaat catcaccggg cacccacaag agaccctgaa cttagaatat ttccgcggta    780
   ggagttccgc ctcgtccttc caggggatac tgacgactcg ctgggttctc gccttccaag    840
   tgctggaggg gggaagtagg gccacccggg gtagaaacct aacgccagaa accttccagc    900
   gccttatcag ttggtggaat tttcgtggca gacagtcgcg ctctacctaa gtcgtagaag    960
   aaaagacacc ttgtggggga cttggcgtcg gaagccttga gttcaaatcg tagccctagg   1020
   tccccagcct tgcccttcct ggcttgctgc gaggatgaaa tgagattttt gtacgtgaaa   1080
   atgctttcta acccctctcg aggggtggaa acgtctccat tcgaccagag tccgggtctg   1140
   cggagcaatt tacccgccgc tttccaagag caggctcccc agcgtagcga gtccttgtta   1200
   tggaaagggt cgtttcggct caggatgcgc gctcccggcg tagacctggg gataggggtc   1260
   cctgtcgcgc tcgccccacc cctgcaggga gcaaggccgt ccttctgcag caccgcgcca   1320
   cgcggcggga atttgcagtg tcctgctttc tggtttcagc ttcttcgctc gcttgggtct   1380
   ctctcggtca cctctcggag gggcctggag tccctcctcc ctcctcctac ttcagcgcca   1440
   gaagcattga aagccgcccg cccgccgaga gtggaaccgg tacctcgaat ctcgctcatt   1500
   ccgggtcaat tggaggcgca ttagcggctg ccttgcagtg caacagaggc tcgagtgaca   1560
   ggccacctgc ctaagcgctg gcgcccagga aagccgggga ggaaactagg aagggaggcg   1620
   acagcagaaa agccagagag tccagagact cctaggcagg cctccgtgca gccccaacag   1680
   cgtggtgccg ggaggtgtgt                                               1700
<210> 56
   <211> 1700
   <212> DNA
   <213> Homo Sapiens
<400> 56
   ttaccaaaaa agcggagggg gctttgtcta acacctgtat acacctccct ccgtagcatc 60
   ccgctcattc tagcttctta gggaaaccat agacaacgac gtttcagaga gtgccagagg 120
   tgaagccgac ccgaaaccac attacagaaa ggcttcctgt ttcaaacagg tccaataaag 180
   ccaccaaatt aagtctacga ttataaagaa ctgcgaatca gaaaataatt ccatataggt 240
   gcagtcttct attacttttt tccccacttt cggaaatttc aaagaaaaat tgctacattc 300
   ttaaatatga ggccattatt tcaaaggctg taacaacctg gaacgcaaat tcgttaaatt 360
   atgatacaat aaggccaacc tcgaggatcc tcggtggact actagcgcac ttccttacaa 420
   ttagaaagtg aaaaactggg tcagccctag agtcacgcaa taatgctgcg tcacgaaact 480
   gcgcattcca agagaaactt ttctcaggcc cagaattaag tcggagggat ccgccgcgca 540
   gctcaatccc taggaagcat gaaagaatgt attgtaaaga gtaaaatcac gcgcaatcca 600
   tcccaatata accgcagttg ttcgtgggcc ttcttgcaga gaaaggctgc ccaaccttga 660
   cggggccatg ggcgctgcgg gagggcaaaa gaccacagag atctgcgaag ccaaagtaaa 720
   caacggggtt ggggcggcga gaatgatcaa tagcgatgct ccgaaaggac tccgcgatag 780
   gaatcgagcg ggaaggattc cctccatcct aacagtccat gggttaagag gccgaagctt 840
   ccctaaatac cacactcccg cgagagaagg gactgagaac aacttctcaa actattctct 900
   ctcgaaatcg ctcccttcct cgaaaattcc atctctgaga ctcggatgag gtccccaccc 960
   cctccaccct tgtcccgtga ccgtcgcccg ctcagcctcc cagccgagtc cgcggggcct 1020
   ggggcgccca ccccgcccac ccaagggacc gcgcaggggt gaactccccc gcgccccacc 1080
   tgcgccttcc agacctcggg cgccccggcg ttgcctcgag agctccctgc gcggccgccg 1140
   cggcacggac cagctcccac tcccttacac tgggcgccgc tgcgctcgcc gggggccggt 1200
   cccgaggttc ccaaggcctc gcgcgcgcgc ttgccgtggc aaccaagacg ttccacgacg 1260
   cgcgctctcg aacgcttcgc gtcacgcggc cgcgcggccc cgcccgtcgg cctcgctccc 1320
   gccacagagc ccgcagcacg ccgccgccgc agcctaggtc acgtgagtac ccacgcgcgc 1380
   gtcttgccag cggattcatc accggcctgc tcagactagg ttctgcccac tctgaccttc 1440
   taaatggtac gtgggaggac gtccgtcccc ttcggaccca agagtcaccg taacactcta 1500
   gaaggggaga aaaggagcga gggcggcagg cgacagagaa cctcgcgagt cagcggcccc 1560
   gcgcagaccc ccccaggcac ggtcccctgc ggccacgtcg gctgctcggc gcctgcgcaa 1620
   tctctttctc tccagcgaaa ccgaggcctc cggagagcct agtagagagt gtgggcagtg 1680
   agcgcttgta gccgctagag 1700
<210> 57
   <211> 1700
   <212> DNA
   <213> Homo Sapiens
<400> 57
   aatctaggtc tggtaggtta cttgcacaca ctgggtggag gtggaagggg caaagtattc 60
   ctgaggtgat ggccctgatc ctacctcaca tctccttggg gcctggttgg acctggctga 120
   gcgtgtttga gcagtggctg attttggaaa aacaggagga ctcttctcca ggaggctgtg 180
   actaccaagg ttgcaaatgc tccacctgaa cttgggtgca tgagggatct ctctctctca 240
   cacacacaca cccccccacc gcgtaagata aatttctgtt tgtgagctgc ctacattaaa 300
   tgctttgctc ctgaccttct gtgattaaaa tctgttccgt ttgcagggtt cggtgcgctt 360
   ggatttagtg tattttgttg gtacagtaaa aaagagaact gcttctctat aggtgacccc 420
   cgccccaaag tcccccctcc cactaacttc tctttgagaa gagagtaaaa ccgaattcga 480
   gagaaaagag gcagtaacta ttcgggagga ggcagactgg agtgggcacc agtcccggta 540
   gtaagagtct taacgtcttc ctcgctttcc tctcgcagag tagtatggaa gcatcagctc 600
   ctccccctgg gcctccaaaa gttacctttc ctttgggtgt gattctcgaa cccgaagcgt 660
   ttgagtccta agaaactaga acaagttaga gccaaagagg cctggcgggc gggggtgcgg 720
   gtggagtccg ggccgggtgg cgtggaaggc cgagcacagc tgcgagcggc gagtcccggg 780
   agaggaggag cgggagaaag cggggcgggc ctgcggtcgg ggttcggccg ggggaggggg 840
   tccccgtttc cctctcctca tcctcgtctc ctctggctct ggtgacgttg cggtttcctc 900
   ccccctccag cggagcctca gcgcccgccg cgtccctccc tccgcccctt tcgcccacac 960
   ctacccgccc ccgccggccc ggctctcagt agcgtcgccc gaggctgcag cagcgcatcc 1020
   cggggcatgg cgcggcgggg gcgcggaggg ctcggttcgg agggggccgg gagcccgggc 1080
   gccctggagt gaggaggacc gggagctggc tctggaggct gcggaggcga cgccggagag 1140
   aacgaagcct cggctgggag cggtaagtgg aggggcgcgc aagggacgag ctggggcgcg 1200
   cggcgcaact ttcttcggga gtttcgggcg ggtagccttc ggggctgtcc cggcgcgggc 1260
   gggagacgga gagccacgtc cccggctgag agcggagcgc cgcgggttgc gcgcccgggc 1320
   ccggctcggc tggactccga gagagcgcct gacgcgtgaa gcctgagcca cgggcttccc 1380
   cggcggcgga gcagcctcga ggggcagctt ttcgatcctc cccaccccct tgaacgccga 1440
   gcaccgagcc gggaacacga tgagaacctg gagatcgcat tccgtgcgtc ggatctggcc 1500
   ggcccagccg cgatttcctc gacggtcctt ggggcctcgg cttcactctg cggggttggg 1560
   tggcacaccc tcgcgtcctc ctttccctcc gtaccccagc catttggggg caggggaggt 1620
   ggcggcggct tgtgtgcaga tcctggcgct ggcaacccta ctttgcggct gcagaagccg 1680
   aggaagagct ccccaagcct 1700
<210> 58
   <211> 1700
   <212> DNA
   <213> Homo Sapiens
<400> 58
   aacagaattc agaactctgg ggcagttctt tggagttgga gctcctaggg caaagataac 60
   gctaatgcgt tgtttaaaag ggttcaattt ttaaaatact tttattggta acacccattc 120
   tgttcatcag aaatgcagcc atgcattttt gtgcccggta aagattttct gttctgtgta 180
   aatctacaaa ctaaaataat tagtttagat tcttgacgtg accctgcagt ggaagtattt 240
   gcaccagtga atgctgaaaa gcaaataaaa atgaaagctt tgcggaggcc gtgggccgga 300
   cgggcgcggg gcgcctcctg ccatcttagc gtctgccggg atccttcccg cgccttcgct 360
   gcccgaacca gctcggtctt gggacgtttc ccccaaatcc tcacaagtca aatcgaactc 420
   aggtccgagt tcgtcactgc aaaggaaccc ggggtgcggg gtgggaggga taggaggaga 480
   gatgggctgg gtgccaaatg gtagaacaaa tggttgattt aattaaagtt gtcaatctaa 540
   tttcccatcc ctctcacacc agcccttttt tttttctttt ttcttttttc ctcttaaact 600
   tgctgaatcc cactatctcc gcgttggtaa gaaaacctga ataccttggg aattgtgctt 660
   aaattgcact tgcaactccc ctggaagttc ctgaaacttc ccgagaatga ggcggggcgg 720
   ctcaagggga aagggcaggg aggagagacc ctcgggaagc tgatgacgcg tccggggcaa 780
   gtcagcctgt ttccgagacg cgcgcctcac ggtcgtagca gcgctgcgcc caccctgccc 840
   ctttagtgac ccagccagcc tttgctgggg gtttctgata acgctcatcg ataagctctc 900
   cttgctccca aaaggcgtgc ttctctttct ctccaggtac aaagtagggg tgggaagggg 960
   agagctacac aatgttgtaa aaagaagcgt ggctttattt ggaagacggt tgaatttctt 1020
   ttgcttcctc gacccagtta gtaacgacaa gaaagatact gaatatcgcc ctccgaaaat 1080
   cgggcgcatt tgaactcggt gaaaagtgtc cctgtgagga agcctaatgt ttacttttgt 1140
   gcaaggataa ctggaaatta aagcgtctag taatgcagag atttcattaa gcgagcgctc 1200
   ccctcccccc cgcgccagga atgaaataag ccagttagac agaaaacact tttgggagaa 1260
   gactaattac ttctgggtag gccgaattga ctggaaatta taccaagcaa aggcagtaca 1320
   ttccattccg tcctggcgat gttgattcta tattattaga acattgttcc cccagtgtgg 1380
   gggtgctttg tacttcccaa agtcagggta gactggttac ctgggaaaat gcagtgttag 1440
   tgctttgctc ctactttctt cactgtttaa acatgttcca catttttcta atgccttctg 1500
   agggttctca acccagccac tgagttcctc acccttggcg actgtgatac caacaatgct 1560
   gtcaccctcc ttgggaaaca tggttgcaaa gatgctccgc aggaggcaga taaaaggctg 1620
   tgctttcaga tactgaaggt tgctttgtta cttagatgtt tccaattcct gcatagagaa 1680
   ggagggaaaa cacggggtgc 1700
<210> 59
   <211> 1700
   <212> DNA
   <213> Homo Sapiens
<400> 59
   aacagaattc agaactctgg ggcagttctt tggagttgga gctcctaggg caaagataac      60
   gctaatgcgt tgtttaaaag ggttcaattt ttaaaatact tttattggta acacccattc     120
   tgttcatcag aaatgcagcc atgcattttt gtgcccggta aagattttct gttctgtgta     180
   aatctacaaa ctaaaataat tagtttagat tcttgacgtg accctgcagt ggaagtattt     240
   gcaccagtga atgctgaaaa gcaaataaaa atgaaagctt tgcggaggcc gtgggccgga     300
   cgggcgcggg gcgcctcctg ccatcttagc gtctgccggg atccttcccg cgccttcgct     360
   gcccgaacca gctcggtctt gggacgtttc ccccaaatcc tcacaagtca aatcgaactc     420
   aggtccgagt tcgtcactgc aaaggaaccc ggggtgcggg gtgggaggga taggaggaga     480
   gatgggctgg gtgccaaatg gtagaacaaa tggttgattt aattaaagtt gtcaatctaa     540
   tttcccatcc ctctcacacc agcccttttt tttttctttt ttcttttttc ctcttaaact      600
   tgctgaatcc cactatctcc gcgttggtaa gaaaacctga ataccttggg aattgtgctt     660
   aaattgcact tgcaactccc ctggaagttc ctgaaacttc ccgagaatga ggcggggcgg     720
   ctcaagggga aagggcaggg aggagagacc ctcgggaagc tgatgacgcg tccggggcaa     780
   gtcagcctgt ttccgagacg cgcgcctcac ggtcgtagca gcgctgcgcc caccctgccc     840
   ctttagtgac ccagccagcc tttgctgggg gtttctgata acgctcatcg ataagctctc     900
   cttgctccca aaaggcgtgc ttctctttct ctccaggtac aaagtagggg tgggaagggg     960
   agagctacac aatgttgtaa aaagaagcgt ggctttattt ggaagacggt tgaatttctt    1020
   ttgcttcctc gacccagtta gtaacgacaa gaaagatact gaatatcgcc ctccgaaaat    1080
   cgggcgcatt tgaactcggt gaaaagtgtc cctgtgagga agcctaatgt ttacttttgt    1140
   gcaaggataa ctggaaatta aagcgtctag taatgcagag atttcattaa gcgagcgctc    1200
   ccctcccccc cgcgccagga atgaaataag ccagttagac agaaaacact tttgggagaa    1260
   gactaattac ttctgggtag gccgaattga ctggaaatta taccaagcaa aggcagtaca    1320
   ttccattccg tcctggcgat gttgattcta tattattaga acattgttcc cccagtgtgg    1380
   gggtgctttg tacttcccaa agtcagggta gactggttac ctgggaaaat gcagtgttag    1440
   tgctttgctc ctactttctt cactgtttaa acatgttcca catttttcta atgccttctg    1500
   agggttctca acccagccac tgagttcctc acccttggcg actgtgatac caacaatgct    1560
   gtcaccctcc ttgggaaaca tggttgcaaa gatgctccgc aggaggcaga taaaaggctg    1620
   tgctttcaga tactgaaggt tgctttgtta cttagatgtt tccaattcct gcatagagaa    1680
   ggagggaaaa cacggggtgc                                                1700
<210> 60
   <211> 1700
   <212> DNA
   <213> Homo Sapiens
<400> 60
   actggatgag tggggttctc tgccccagct ggacttccac aggaggatgg aggaaagcat     60
   cctcttgagc aagtctggag agtgaagggg tggggttgca aggttggaga aggaggtgtt    120
   tccctcataa aaatgactgg gcctttcttg caatggctgg cacaaagtcg gaagggtctg    180
   gggacgggag gggcctgaag tgatttctga ggagttgggt ttttcctgta aggccctggg    240
   agggaaggca aggtactttg cgcaatgaca catttcagtt gttggatgtg aagattctcc    300
   ttcgaagaca gaataaagaa aggccccttc attaatgcgg gtttgaaact ggtctgaaat    360
   ggtgtgcctg cagggattcg ggaggaaatg cgacgcccac ccccagactt gggaaaggcg    420
   ggggcaataa ttctctaaga gaactggagc ccgaaagagg aatgaaaagc ccccgcgccg    480
   cgcacataca ctctctctca ctggaaagtt aggagcgagg aaagaactct gaagtgattt    540
   gggaacgatc tgaaagcgcg caagttggaa gtcgggttga agccagcata gcggagcagg    600
   ggtcaggcct ctgcgtggcc ggagtgggag agcgcttctg agaacaagat ggggcaggac    660
   cgaaggggcg tgaggtcgac gctccctagg gatgattcgg agggtgaagg aggtttgggg    720
   gtggttccgg gctctcggag gtgactgagg gagtccctga gccaatgccc gggggtggga    780
   ggcgctaccg gtactcactg agctgatctc cagcgccagg gcgcactgca gcgccttcac    840
   cttcggcatc cgtgcgggga ggggcggcgg ggagtgaccc cggccacggg cccgagtgag    900
   gcggggagac ctggggctgg gcggggacgg ggaggagacg aggtggcggc ggcggtggca    960
   gcagtggccc ccaggccggg gcccgcggtc cagcctgggt tccgccggag aagcagctga   1020
   gcgcggggcg cagactcgtt gtcatggcag ccaggagggg cggggccgga agagaggccg   1080
   cgcggggcac tccagggcta cccgcgctgg gacgagcgct ggaggcggag cttgacgaac   1140
   gccgagcgag gcggagtcgg gcagggggcg gggcttccga aggggcgggg tgaggcaagg   1200
   cagctcttct gtgacgtcaa actgcgccca aagccggacg aaagggagct gctgtttgta   1260
   taatgggacg gtgctctgtt cagggagttg gagttcctgt aaggccgtcg gagggaaccc   1320
   ctgagttaca acgtggtgat ggcaccttgg gctagtggcg acccactgtg gcctcagttt   1380
   tcccaattgt aaaagaataa taattattgt tagtcctcct atttaaattt cacggcacgg   1440
   tttacaaagc cagcataagg atactgggaa ttagggaaga gacttgccca aatcaaagtc   1500
   ggaaccagga ctggaaccca ggagctctca agttaaaccc cagttgcctc cctgaggaga   1560
   ggggcttgca tcagatggtc catactccta gtctcctatt ctgtgattcc ctttgttagc   1620
   gcgttccctt ttcccttcaa attttcctgt ctaattcacc catgtatgcg tcttaatgca   1680
   tattgcttaa gtggaactgt                                               1700
<210> 61
   <211> 1700
   <212> DNA
   <213> Homo Sapiens
<400> 61
<210> 62
   <211> 1700
   <212> DNA
   <213> Homo Sapiens
<400> 62
   gctggcaagt gaaaagccca cagtccacag catcctcact gtccagcccc acttccactg     60
   ttgagctcag gaatttgttt ccctgcctcc ttttggaagc gatcctgtgt gtgcaggtag    120
   gggcagaggg agtaaagcca aacgtaacta tagcatctca gatttgcaga ctctcaaacc    180
   agcctcacac ctcagtgagg tgacaatggc taacattttc atctcagttt tacagaagcc    240
   cagccacagc aggtgacctg cgcaaagcta cacagcctct ggctccaagg cagggctctc    300
   tcccctgcct gtgccctctc gccaccgcag cccctgttct ccagctgggg gaccacccag    360
   aggaccacct ggaccctgct gcctccgttg ggccatttgc ttggcggagt agttgggggg    420
   tggggggcct gcaacctagg accccacctg gcgctgcccc agcccaccga gcagtgagcc     480
   agctctcccc tcatcggtgg cagggggagc tgtcgggatc tcagggctgc cctgaggggc   540
   cgcgcagatc tgggggatgt aaaggcccaa acggactcgg aatggccggt gcggccgggc    600
   tctgggaaat gaagggctcg cggtgtctcg cactttttgt gggggcggga gtaattacca    660
   ggtggccgcg gagccagccg agggggcggc tgctaacccg ggcggcgacc gcaggcggag    720
   acgccccgcc cgggacaggg accgggccgc gccggcgctc gcggacagac acccgcggtc    780
   ccggccgctg ccgcgtctcc cgcccccgcc acgccccggc ggcctcgctt ggggcgcgcc   840
   cccgcctcgc acgcgcacgc gcctgcaccc gcactggccc gcccggttcc cccgcgctcg    900
   cggggccgct ccgggggagg tgccgagacc cggccttggc ccgcgcgcgg tttccggggt    960
   ccggcgcgcc ggggacccac aggcgaggcg agagaaaggg gttggctcgc ggggacacct   1020
   acctgcgcgg cgcggggctg tggcgccggc caccattacc ccgcgcccct cctcttgcca   1080
   gcgcgcacgc agatggcggg gtggcctggg gaggtcttcg ggtcccttcc tgggaacgca   1140
   gggccaagtt gtgctccgat tccacgcccc ccccacccac gtcgggcaca cgcagccctg   1200
   agtgagggtc catgccctgc ctttcacgct gaccacagcc cctaggcttg gcgcctgccg   1260
   ggaagggccg caatggctca gggacccctg cccccactac catcctgccc tggggggaca  1320
   ggctctagaa cactgcgtgg ggctcagagt gggcctagct gtaaaaggac aggggtcctt    1380
   ctccagccac gcccactccc gggtgccctg gctgtcagca gcctctcacc acaaccgcag 1440
   ggtgagaagc ccagagaaca gtccaggagt agcccttctg tcctttacca cgtccgtgtg 1500
   cccagcacgg gcaaatcagt agccacgcaa taaatgaagt gcggtgttgc agctctgggc 1560
   ctctctgcac ctgccctgac ctcagcctgt ccagcccagc ctggggatct gcacttaggt 1620
   ttgcagcctc ctgggtccct gcccggtgct cctcagagcc ggagcctgtc tgccgcaggg 1680
   atggcttaga ggacctccta 1700
<210> 63
   <211> 1700
   <212> DNA
   <213> Homo Sapiens
<400> 63
   gcaagactcc atctcaaaaa aaaaaaaaaa aaaaagcgac ttgcccagta atagctctaa 60
   aatatggaag agctgggatt ggacttagac tatctggttc tggaactggg ctggcaagtg 120
   aaaagcccac agtccacagc atcctcactg tccagcccca cttccactgt tgagctcagg 180
   aatttgtttc cctgcctcct tttggaagcg atcctgtgtg tgcaggtagg ggcagaggga 240
   gtaaagccaa acgtaactat agcatctcag atttgcagac tctcaaacca gcctcacacc 300
   tcagtgaggt gacaatggct aacattttca tctcagtttt acagaagccc agccacagca 360
   ggtgacctgc gcaaagctac acagcctctg gctccaaggc agggctctct cccctgcctg 420
   tgccctctcg ccaccgcagc ccctgttctc cagctggggg accacccaga ggaccacctg 480
   gaccctgctg cctccgttgg gccatttgct tggcggagta gttggggggt ggggggcctg 540
   caacctagga ccccacctgg cgctgcccca gcccaccgag cagtgagcca gctctcccct 600
   catcggtggc agggggagct gtcgggatct cagggctgcc ctgaggggcc gcgcagatct 660
   gggggatgta aaggcccaaa cggactcgga atggccggtg cggccgggct ctgggaaatg 720
   aagggctcgc ggtgtctcgc actttttgtg ggggcgggag taattaccag gtggccgcgg 780
   agccagccga gggggcggct gctaacccgg gcggcgaccg caggcggaga cgccccgccc 840
   gggacaggga ccgggccgcg ccggcgctcg cggacagaca cccgcggtcc cggccgctgc 900
   cgcgtctccc gcccccgcca cgccccggcg gcctcgcttg gggcgcgccc ccgcctcgca 960
   cgcgcacgcg cctgcacccg cactggcccg cccggttccc ccgcgctcgc ggggccgctc 1020
   cgggggaggt gccgagaccc ggccttggcc cgcgcgcggt ttccggggtc cggcgcgccg 1080
   gggacccaca ggcgaggcga gagaaagggg ttggctcgcg gggacaccta cctgcgcggc 1140
   gcggggctgt ggcgccggcc accattaccc cgcgcccctc ctcttgccag cgcgcacgca 1200
   gatggcgggg tggcctgggg aggtcttcgg gtcccttcct gggaacgcag ggccaagttg 1260
   tgctccgatt ccacgccccc cccacccacg tcgggcacac gcagccctga gtgagggtcc 1320
   atgccctgcc tttcacgctg accacagccc ctaggcttgg cgcctgccgg gaagggccgc 1380
   aatggctcag ggacccctgc ccccactacc atcctgccct ggggggacag gctctagaac 1440
   actgcgtggg gctcagagtg ggcctagctg taaaaggaca ggggtccttc tccagccacg 1500
   cccactcccg ggtgccctgg ctgtcagcag cctctcacca caaccgcagg gtgagaagcc 1560
   cagagaacag tccaggagta gcccttctgt cctttaccac gtccgtgtgc ccagcacggg 1620
   caaatcagta gccacgcaat aaatgaagtg cggtgttgca gctctgggcc tctctgcacc 1680
   tgccctgacc tcagcctgtc 1700
<210> 64
   <211> 1700
   <212> DNA
   <213> Homo Sapiens
<400> 64
   aggtgggcag gtcttctgga gcagcaggcg gctctcccag ttcttatggc tggagctagg   60
   atcctcctct gcctataact ccctcaattc tcccaaccca acacccccta ccttccttct  120
   ttcctcctcc cttccttctc cccttcctcc tttccttccc ccctctttct ttccttcccc  180
   ctctttcttc cttccccctc ctgccttcct tccccctcca gtcttccctt cccaccctgt  240
   tgtgggcggg ctgcagtgct gatcccaccg ggacgtttgg acaggcattg tctgtgacaa  300
   cggtggcatt ggaaggccct ggcagctgaa cgcccagcct cccaccctcc atgtcctcac  360
   actccccatg cacaaactct gcaggaagca ggccagggaa cgcagttcac tgtcttcccc  420
   cgaccagcag ccccagcctg gccacatcgc agccgcggca gaggacacgg cttattccca  480
   cgagccaacc ggggcttatt cccaccctcg gcggtcccag cgggctcagc aagcgagagc  540
   aggcttccgg agttacaggg gcttccctgc ctccgcagcg agggaagcag gaggcggcag  600
   gccctgaggc ccgtgggctg aaggctcagg cctcacgtgg agccacccgc ggaggaccca  660
   ggctaggcca acctgcccgt ggggaacccg cccgaccctg gcgagttagg ggtctggaag  720
   gccgggtgga gcttggtcaa cagctcgggc tcctcaccct gggccccgct tttcccggag  780
   gcctcggggg cacccagcgg gtaggcctcc ctggcccggc cggtcctgga gcaggtgagg  840
   aaggccaggc aggccccgcg gaggcgggcc aggtcccggt gggtggtctc gctgacgaag  900
   cagtagagca cggggtcggc gacgcagttg aagctggtga gcaggaggga gaagtggtag  960
   gcgttgaaaa cgcccttggc gaagtcgcag ctggcctccc agacgctgcg caccagcagc 1020
   aacacgtggt agggcaggaa gcaggccagg aagatgacca cggtgctgag caccagccgc 1080
   tggatctggt ccttgcggct cttctgggtg ccgtggctcc ggcgcacggc gcgcaggatg 1140
   ccctggtagg acgccagcag caggcagatg gggaagagga agcccaccag gaagcggtag 1200
   tagttgatgg cgcgctgcca tgcctggatg gggtagtgct caaagcacac gcggtgctgg 1260
   ttctcgtcct cgatgacctc ctcgtgcatc aggaagtaga tgctggtcag cagctccttg 1320
   gcccagatga ccacgctgac gccgacggcc gccttcaggg tccggaactg gtggaagcgg 1380
   aagggatggg ccacagccag gtagcggtcc acggagatgc agcagaggaa gcccacgctg 1440
   atgtagatgt tctcgtacag gaggatgccg cacacctggc aggacaggtc gccgtgagac 1500
   cagttgtcgt gctgcagcac gtactgcagc cagaagggca gcgagcagat gtagaagagg 1560
   tcggccaccg tcaggttgca caggtacacg cccagctcgt tccgggcctt gatctgcagg 1620
   tagccgaagt agagggacag gcagttggcc gggaagccca ccaccagcac ggtaacatag 1680
   accaccgggg ccagcgtctg                                             1700
<210> 65
   <211> 1700
   <212> DNA
   <213> Homo Sapiens
<400> 65
   aggtgggcag gtcttctgga gcagcaggcg gctctcccag ttcttatggc tggagctagg 60
   atcctcctct gcctataact ccctcaattc tcccaaccca acacccccta ccttccttct 120
   ttcctcctcc cttccttctc cccttcctcc tttccttccc ccctctttct ttccttcccc 180
   ctctttcttc cttccccctc ctgccttcct tccccctcca gtcttccctt cccaccctgt 240
   tgtgggcggg ctgcagtgct gatcccaccg ggacgtttgg acaggcattg tctgtgacaa 300
   cggtggcatt ggaaggccct ggcagctgaa cgcccagcct cccaccctcc atgtcctcac 360
   actccccatg cacaaactct gcaggaagca ggccagggaa cgcagttcac tgtcttcccc 420
   cgaccagcag ccccagcctg gccacatcgc agccgcggca gaggacacgg cttattccca 480
   cgagccaacc ggggcttatt cccaccctcg gcggtcccag cgggctcagc aagcgagagc 540
   aggcttccgg agttacaggg gcttccctgc ctccgcagcg agggaagcag gaggcggcag 600
   gccctgaggc ccgtgggctg aaggctcagg cctcacgtgg agccacccgc ggaggaccca 660
   ggctaggcca acctgcccgt ggggaacccg cccgaccctg gcgagttagg ggtctggaag 720
   gccgggtgga gcttggtcaa cagctcgggc tcctcaccct gggccccgct tttcccggag 780
   gcctcggggg cacccagcgg gtaggcctcc ctggcccggc cggtcctgga gcaggtgagg 840
   aaggccaggc aggccccgcg gaggcgggcc aggtcccggt gggtggtctc gctgacgaag 900
   cagtagagca cggggtcggc gacgcagttg aagctggtga gcaggaggga gaagtggtag 960
   gcgttgaaaa cgcccttggc gaagtcgcag ctggcctccc agacgctgcg caccagcagc 1020
   aacacgtggt agggcaggaa gcaggccagg aagatgacca cggtgctgag caccagccgc 1080
   tggatctggt ccttgcggct cttctgggtg ccgtggctcc ggcgcacggc gcgcaggatg 1140
   ccctggtagg acgccagcag caggcagatg gggaagagga agcccaccag gaagcggtag 1200
   tagttgatgg cgcgctgcca tgcctggatg gggtagtgct caaagcacac gcggtgctgg 1260
   ttctcgtcct cgatgacctc ctcgtgcatc aggaagtaga tgctggtcag cagctccttg 1320
   gcccagatga ccacgctgac gccgacggcc gccttcaggg tccggaactg gtggaagcgg 1380
   aagggatggg ccacagccag gtagcggtcc acggagatgc agcagaggaa gcccacgctg 1440
   atgtagatgt tctcgtacag gaggatgccg cacacctggc aggacaggtc gccgtgagac 1500
   cagttgtcgt gctgcagcac gtactgcagc cagaagggca gcgagcagat gtagaagagg 1560
   tcggccaccg tcaggttgca caggtacacg cccagctcgt tccgggcctt gatctgcagg 1620
   tagccgaagt agagggacag gcagttggcc gggaagccca ccaccagcac ggtaacatag 1680
   accaccgggg ccagcgtctg 1700
<210> 66
   <211> 1700
   <212> DNA
   <213> Artificial Sequence
<220>
   <223> chemically treated genomic DNA (Homo sapiens)
<400> 66
   ttttttagga ttagagaatt tcgaagagtt cgtgtattgt ttttttgaaa gtttcgaagt 60
   taagtaattt taaaaatgtt tagtaaagtt tttttgggaa tggtgagatg gggggtgggg 120
   aggatgggat atttttattg ttatcgttcg tttttcgttt tttcggtttt tttatatgaa 180
   gtgattggat ttgggagatt tagcggaaat ttttaggtta tttagggaat tgtttttttt 240
   tttggttttg tagcggcgag gcggggtagg gggtgggggg ggtgggtcgt gagagcgtcg 300
   agacgcgcgg ggcgcggaga tgtgtaagtg gcgaagtttg atcgagagta ggttggagta 360
   gtcgtttaat ttttggcgcg ggatttgttg aggggttacg gtgagttttt gtgggtgcgg 420
   gtcggaggga tgtttaggtg tttcgtcgtt ttacggttcg ggcggaggcg ggttcggaat 480
   tttttttaag ttcggtttta gtttggagtt cgcgcgtttt gggcggatgt acggattgag 540
   aggcgtttgg ttgggttttt agttcgttat tgttattcgg tttcgtgcgt ttattggtcg 600
   ggatgtgtcg gtttagtagt ttttaatttt tttcgaattt tattggtgag ttttaggaga 660
   gcgagttgag ggagaaaggt ttaaagggcg cgagcgttta gggcgttggg agttcgtggg 720
   acgggtacgg ggagggtaga gttagtcgag gggagcggtt gtcgggaggt gcgttgagta 780
   gggttcgtag ttaaggttta gcgcgattcg agcgtttagg attttgcggt cgtggaggag 840
   gcgtgttttg ggaaggagtc gttattggga tttgaagagt gaggggaggg agaggggttt 900
   agagggtgat ttggaggagg gttttggaag ttacgttagg ttggtttttt aggtttattt 960
   ttatagtttt ttgcggtttt tgttttgggg agttatgttt tgttatcgag attcgcgtta 1020
   ttagattgta tcggggttga tttgggggtt gggaatttgt tattttgttg tatagatatt 1080
   gatttttttt tttttttttt aaaaagtaag gtttgttttt attttggttt ttgtcggatt 1140
   tttttatttg taatttattt ttttattttt atttttttta atcggaagat ttttttatta 1200
   ttttattttt ttattttgtt tttaatttga ttttttatta tataattaat gtttaaattt 1260
   tataagggtt ggtcggtgcg gggttggtgt gtttttttgt agatgaaagt ttaagatttt 1320
   ttaattggtt ttagtttatt tgtttggttt taaaataagg gtaattgtaa ttaaatattt 1380
   ttttttagtt tttcgattaa gtcgattttt tttttatata tgatttaaat ggtaatttta 1440
   ttaaattgaa aattagtatt tgatttgtag cgttagtatt tggaaagtgt ttgtcggtaa 1500
   atcgattgta tgaaggcgtg tagttttatt ttttgttatt tttgtgggtt ttagtatttt 1560
   aaaaaattgt ttggtaattt tttggattta atttgtgttt tttggttgta gagtagtatt 1620
   aattagggga gtaatatggt tggtatttcg tgtttagaat ttcgtatttt aggttgggaa 1680
   atatttaatt aatgtttggg 1700
<210> 67
   <211> 1700
   <212> DNA
   <213> Artificial Sequence
<220>
   <223> chemically treated genomic DNA (Homo sapiens)
<400> 67
   tttagatatt aattaggtgt tttttagttt gaaatacgga gttttggata cgaagtgtta      60
   attatattat ttttttggtt agtgttgttt tataattaaa gatatagggt taagtttaag     120
   aaattattag ataatttttt aaagtgttag aatttataaa aatggtaggg gatgaagtta     180
   tacgttttta tatagtcgat ttgtcgataa gtatttttta aatgttgacg ttgtaagtta     240
   ggtattagtt tttagtttaa taaaattgtt atttgaatta tatatggggg aggaatcgat     300
   ttgatcgaga agttaaagag ggatatttgg ttgtaattat ttttattttg aaattagata     360
   aatagattga aattagttaa gaggttttaa atttttattt ataaaaggat atattagttt     420
   cgtatcgatt aatttttgtg agatttaagt attaattata taataggaaa ttaggttaaa     480
   agtaagatga agaggtggga taatgaaaag atttttcggt taagaaaagt aaaaatagaa     540
   aagtgagtta taaatgagaa aattcgatag aaattaaaat gaaaataaat tttgtttttt     600
   aaaaagaaga aaaaaaaatt agtgtttgta tagtagaatg gtaaattttt agtttttaaa     660
   ttagtttcgg tgtaatttgg tagcgcggat ttcggtaata aaatataatt ttttaaggta     720
   gaggtcgtag ggaattatgg aaatgaattt gaagagttaa tttgacgtga tttttagagt     780
   ttttttttag gttatttttt gggttttttt tttttttttt attttttagg ttttagtagc     840
   ggtttttttt tagggtacgt tttttttacg gtcgtagggt tttgggcgtt cggatcgcgt     900
   tggattttag ttgcggattt tgtttagcgt atttttcggt agtcgttttt ttcggttggt     960
   tttgtttttt tcgtgttcgt tttacgggtt tttagcgttt tgggcgttcg cgttttttgg    1020
   attttttttt tttagttcgt ttttttggga tttattagtg ggattcggga gaagttggag    1080
   gttgttgagt cgatatattt cgattaatga gcgtacgggg tcgggtggta gtggcgggtt    1140
   gagagtttag ttaagcgttt tttagttcgt gtattcgttt aaggcgcgcg ggttttaggt    1200
   tgaggtcgga tttgggggag gtttcggatt cgttttcgtt cgggtcgtgg ggcgacgagg    1260
   tatttgggta ttttttcgat tcgtatttat agagatttat cgtgattttt tagtagattt    1320
   cgcgttagga gttgggcggt tgttttagtt tgttttcggt taagtttcgt tatttgtata    1380
   ttttcgcgtt tcgcgcgttt cggcgttttt acggtttatt ttttttattt tttatttcgt    1440
   ttcgtcgttg taggattaga gagaggagta gttttttggg tgatttgaag gttttcgtta    1500
   ggttttttaa atttagttat tttatgtgag ggggtcgggg agacgggggg cgggcggtgg    1560
   taatgaaaat gttttatttt ttttattttt tattttatta tttttaaaag ggttttgtta    1620
   aatattttta gaattgttta gtttcggggt ttttaagaaa ataatatacg ggtttttcgg    1680
   gattttttag ttttgagggg                                                  1700
<210> 68
   <211> 1700
   <212> DNA
   <213> Artificial Sequence
<220>
   <223> chemically treated genomic DNA (Homo sapiens)
<400> 68
   tagttggtaa gtggtagatt tggaattgga atttagttta gtttgatttt ttaatttatg      60
   tttttgatta ttatattgtt tttttaagtt tagatttgaa attttatttt ttgtgtggtt     120
   gtgtgtttgg gataggggta attaattttt gattatttta tatgttgtat agaatttgga     180
   gaggattttt taaagtaaat gaatttcgaa agttggattg tagagtaaac gagtgtagtt     240
   aatttagtta ggggtttgta agagggagaa agagaaaaag attgtggaat ggaaagtttt     300
   ttaatttaag ttttttttaa ggggtagtta ttttttgttt tatagtttag ggtttgtatg     360
   tgtttttttt ggagtggaaa aatatataag ttataaggaa tttaatagat agaaaggcgt     420
   atagaggaat ttaaagtgtg ggttgggggg cgaggcggtg ggcgggaggc gagcgggcgt     480
   aggcggaata tcgtttttta agttaagtcg tcgtaaataa aaaggcgtaa agggagagaa     540
   gttggtgttt aacgtgagtt aggagtagcg tttcggtttt ttttttgttt attttaaaag     600
   tattttttgt attgttttta aggtgagaaa taggaaagaa aacgtcggtt tgtgcgttcg     660
   ttgtttgttt ttttggttgt ttgtttttgt agggttgttg ggagttttta agttttgtga     720
   gaattttggg agttggtgat gttagattag ttgggttatt tgaaggttag tagttcgggt     780
   agggtttatc gaaagtttat tcgtatatat taggtaattt aattttttat tttgtgtgat     840
   agaagtagta ggaagtgagt tgtttagagg taggagggtt tattttttgt taaagggggg    900
   attagaattt ttttatgcga gttgtttgag gattgggatg tcgagaacgc gagcgattcg     960
   agtagggttt gtttgggtat cgtcggggta ggattcggaa cgtattcgga aggttttttg   1020
   taagtattta tttggaagga gaatttggga tttttttggg aatttttcgt ttcggttgga    1080
   ttggtcgagt aagtttggaa aatggtaaat gattatttgg attaattata ggtttttagt    1140
   tggtttgttt gttataattt atgattcggg gttgggaaaa agattaatag tttacgtgtt    1200
   aaaaaagggg tagagtttga tggagttggg tggatttttt tatgttattt gtttttatat    1260
   ttagaggata agtatttttg tagatattta gtgtaaggga gattatgttt gattgtatgg   1320
   atgttttgtt agtgagtttt gggtaaattt tggattttta tattgcgagt tcgttttttt    1380
   gtatgtttta ggagaaagtt tttaaagtat gttttagtgg attgatttaa atcgaatggt    1440
   agtatcggta tattgtttaa tgtaggttta tttttttttt tttttttatt aagaaaaaaa   1500
   aaattgtttt ttttgtatgt aataaagacg ttggaaataa attgtattgg tagtaagata    1560
   aaggatttaa tgatttaatg ttgaaggggt gtgatatgtt ggtatgtata ttgatttttt    1620
   ttgttgaaaa ttttttgtta gatgtttttt ttttaaataa tttttttcgt ttttatgttt   1680
   ttttttttat taattttata                                                1700
<210> 69
   <211> 1700
   <212> DNA
   <213> Artificial Sequence
<220>
   <223> chemically treated genomic DNA (Homo sapiens)
<400> 69
   tataaggttg gtaaggagag gagtatgggg gcgggagggg ttatttggga agaaaatatt      60
   taataggaaa tttttagtaa agggaattaa tatgtatgtt agtatattat atttttttag     120
   tattaaatta ttaaattttt tgttttgtta ttaatgtagt ttatttttaa cgtttttatt     180
   gtatgtaaga gagataattt tttttttttt ggtagaagaa gggaaaaaaa taaatttata     240
   ttgagtagtg tgtcgatgtt gttattcggt ttgggttaat ttattgaagt atgttttgag     300
   agtttttttt tggagtatgt aggaagacgg attcgtagtg tagaaattta ggatttgttt     360
   aggatttatt gatagaatat ttatatagtt aaatatgatt ttttttgtat tgaatgtttg      420
   taaaagtgtt tattttttag gtgtggaggt aaatggtata gaaaagttta tttaatttta     480
   ttaaattttg tttttttttt ggtacgtagg ttgttggttt tttttttagt ttcgaattat      540
   gaattatgat agataagtta gttaaaagtt tgtaattgat ttaaatgatt atttattatt     600
   ttttaggttt gttcggttaa tttagtcggg gcggggggtt tttagaaaga ttttaagttt     660
   tttttttaag taaatgtttg taaaaagttt ttcgaatgcg tttcggattt tatttcgacg      720
   gtgtttagat aaattttgtt cggatcgttc gcgttttcgg tattttagtt tttaaatagt     780
   tcgtatgggg gaattttggt tttttttttg gtaaagaata gatttttttg tttttgaata      840
   gtttattttt tattattttt gttatataga atgaaagatt gaattgttta atatatgcga     900
   gtgaattttc ggtgaatttt attcgggttg ttaattttta aatgatttaa ttagtttgat     960
   attattaatt tttaggattt ttatagagtt taaaaatttt tagtagtttt gtaaaagtag    1020
   atagttagag aggtaggtag cgagcgtata agtcggcgtt ttttttttta ttttttattt      1080
   taaaaataat ataagaagtg tttttaaaat gagtagggga ggagtcggga cgttgttttt    1140
   ggtttacgtt gagtattaat tttttttttt ttacgttttt ttatttgcgg cggtttagtt     1200
   tggaaaacgg tgtttcgttt gcgttcgttc gtttttcgtt tatcgtttcg ttttttagtt     1260
   tatattttaa atttttttgt gcgttttttt gtttgttaaa ttttttataa tttatgtatt      1320
   tttttatttt agaaaaagta tatgtaagtt ttaaattgta gaatagagaa tggttatttt    1380
   ttgggaaagg tttgggttgg gaaatttttt attttatagt tttttttttt tttttttttt       1440
   tgtaagtttt tggttgaatt gattgtattc gtttgttttg taatttagtt ttcgagattt    1500
   atttattttg aaaaattttt tttaggtttt atgtagtata tagagtagtt aggaattggt    1560
   tatttttgtt ttaaatatat agttatatag aaaatgagat tttagatttg gatttgaaaa    1620
   aatagtatag tggttaagag tatgggttgg ggaattaaat tgaattagat tttaatttta     1680
   ggtttgttat ttattagttg                                                1700
<210> 70
   <211> 1700
   <212> DNA
   <213> Artificial Sequence
<220>
   <223> chemically treated genomic DNA (Homo sapiens)
<400> 70
   ttttttttgg atatcggagg gaatttcgaa tatatcgttt tattgtttac gggattggga 60
   agtatacgga ggttttgttg gttaattttt gttttgttat tgttaataag ttattattag 120
   tttttataat tatcgggggg ttggcgtcga tcgcgggtgg cgcggggcgg gattcgtttt 180
   tgggacggtg gagggcggtg gcgttgtagt cgcgtcggtt tttgagtttt agggagcgga 240
   gggtacggag tcgggcgagt tttgcggtgt tagaattttg cgtcgttttg acgttggggt 300
   ttagggttta tttcgggggc gcgcgcgtgg ttgcggaggc gggaaggttt ttttgtttgt 360
   cgtcgcggag tttcgcgttt ttgtatttcg agttttgggg gttcgttttg ggttgttttt 420
   ttttttcggt ttcgtcgttt atttggttcg gtttttgtta gcgtttcggt ttttttcggc 480
   gcggcgcggg gttcggtgtt tagttggtcg ttttgcgttt cggcgcggtt gcgttttttt 540
   tttggttttg gttttggttt cggtttcggg tcgcgttgtt tttggttgtt cgtgcggtcg 600
   ttttgtcgtt gcgcgtcgtt tcgtaggatg ttttggatga ggaaggacgt gagcggtttg 660
   gacggggtcg gcggcgcggt ttttttcgtt ttcgtttttt cgggtcgcgg tttcggaatt 720
   ttgagtattt cggcggtttc gttttggttt gaatgtattc gttcggttcg tatcgttttt 780
   atttttcgtt tttcggttcg tttatagttt aggggagcgg gtcgcgtcgt ttttttattg 840
   gtcgcgggac gggtcgcgtc gcggattggt cgttttgagt tttaggtgat ttcgtcgatt 900
   tgcgagtttt gagtattgtt tcgtttcgtt tttttttagg ggattttttt tttagttttt 960
   tttttttttt taattttttt ttttttttta attttttttt tttttttagt tttttttttt 1020
   tcgttttgag tcgttttttt aggttaggag cgtattttaa agcggggttt ttttgtttta 1080
   atttattcgt aatagagaga agtttttggg gatgggacga ttaagataaa tagtttagtt 1140
   agttttttag gatcgttaga gttaattata ggggatgagt acgtagttat tgtagtcgcg 1200
   cggtatcgtg agagttttgg gtgttttttt tttttgggag agatggtgta aatttagatt 1260
   tgtaatattt acgattttaa agaggtatga taagtaggtg tagttagatt ttaggttgaa 1320
   agaaggaaaa tatttttgtt aaagtttttt ttcgttgtag aagtggtttt ttttggtggt 1380
   ttttttattc gtaataattt gttaattttt tttttttttt agatatatga tatattattt 1440
   ttttgaaaag tttttagacg gatggaaggc gttttaggtt aggataaatg taaaaattgt 1500
   atttaaaaaa gtagtttaaa agaatattag tatatttttt ttgtagatta ttaattgtta 1560
   atatgtaatt tattggttaa ataatgggtt agttagattg gtttaatcgg tattgtgatt 1620
   ggattggacg gtatcgtgat aatagacgaa agtggaatag agatgatgga agtatttttt 1680
   ttttttgttt tttttttttt 1700
<210> 71
   <211> 1700
   <212> DNA
   <213> Artificial Sequence
<220>
   <223> chemically treated genomic DNA (Homo sapiens)
<400> 71
   agaagagaaa agataaggaa agaaaatatt tttattattt ttgttttatt ttcgtttgtt      60
   gttacggtat cgtttagttt agttatagta tcggttggat taatttggtt aatttattgt     120
   ttagttaatg ggttatatgt taatagttgg taatttgtaa aaagagtatg ttgatgtttt     180
   tttgaattat ttttttaaat gtagtttttg tatttgtttt ggtttaaaac gttttttatt        240
   cgtttggaaa ttttttaaaa ggatggtatg ttatgtgttt ggggaggaag gaaagttaat       300
   aggttattgc ggataaagga attattaaag aaaattattt ttgtaacggg aaaaggtttt     360
   ggtaaaggtg tttttttttt tttagtttgg ggtttggttg tatttatttg ttatgttttt     420
   ttgaggtcgt agatattgta gatttgagtt tgtattattt tttttagaga gagagagtat     480
   ttagaatttt tacggtatcg cgcggttgta gtgattgcgt gtttattttt tgtaattggt     540
   tttgacggtt ttgaagagtt aattggattg tttgttttga tcgttttatt tttaggagtt     600
   tttttttgtt gcgggtgggt tggggtagag gagtttcgtt ttggggtgcg tttttggttt     660
   gggaaaacgg tttagggcgg agggaggaga gttggagaag gagaggaaat tggggaagga     720
   gagggaattg gggaaggaga gggaattggg gaaggaattt tttagggagg agcggagcgg     780
   ggtagtgttt agggttcgta gatcggcggg gttatttggg gtttagggcg gttaattcgc     840
   ggcgcggttc gtttcgcggt taatgggagg gcggcgcggt tcgttttttt gggttataag     900
   cgagtcggga ggcggaaagt gaaagcggtg cgggtcgggc gggtgtattt aggttaaggc     960
   ggggtcgtcg ggatgtttag ggtttcggag tcgcggttcg gggaggcgaa agcggagggg    1020
   gtcgcgtcgt cgatttcgtt taagtcgttt acgttttttt ttatttagga tattttgcgg    1080
   gacggcgcgt agcggtaagg cggtcgtacg agtagttaga gatagcgcga ttcggagtcg    1140
   gagttagagt tagagttaga gggaggacgt agtcgcgtcg gggcgtagaa cgattagttg    1200
   agtatcgggt ttcgcgtcgc gtcggaggag gtcgagacgt tggtagagat cgagttaggt    1260
   aagcggcgag gtcggggaag gggggtagtt taaggcggat ttttagagtt cggggtgtag    1320
   ggacgcgggg tttcgcggcg ataggtagag ggattttttc gttttcgtag ttacgcgcgc    1380
   gttttcggaa tgaattttga gttttagcgt tagggcggcg taggattttg atatcgtagg    1440
   attcgttcgg tttcgtgttt ttcgtttttt ggggtttaga agtcggcgcg attgtagcgt    1500
   tatcgttttt tatcgtttta ggagcggatt tcgtttcgcg ttattcgcga tcggcgttag    1560
   tttttcggta gttatgagaa ttaataataa tttattaata gtgataaagt aggggttgat    1620
   tagtaaagtt ttcgtgtgtt ttttaatttc gtgggtagta aagcggtata ttcggggttt    1680
   ttttcggtgt ttaggagaga                                                1700
<210> 72
   <211> 1700
   <212> DNA
   <213> Artificial Sequence
<220>
   <223> chemically treated genomic DNA (Homo sapiens)
<400> 72
   ttggtttttt aaagttttga gattataggt gtgagttatc gcgtcggttt ttaaatatgt      60
   tatttataaa ttgtttttgg ttcgtttttt agatcgtagg tttaattacg tttttagaaa     120
   aattttttat tagttttgtt ggttttcggg tagtttttat aatgtattaa ttcgtttttt      180
   ttttatgttt ttaaggagtt ttttttgttt tacgatttga gattagaatt gttttttttt    240
   tattgttgat gtttaggttt aaatgatatt tttttatacg taaaacgggt tttaagtttt     300
   atttatggtt gtgacgattt gaaagtaaat gcgtgtatat gataacgtgt gagcgatgag     360
   aagtttgtta tttgtaaaat tttatgcgta tgttaattat tatggtcgtg ataattatgt     420
   atattataga aatattaatt aagcgttggg ttcgttttag gatttttagt attgttggga     480
   ggtttggtat tcgataggtg ttttaggagg gtaggaaagg agagatatgt ttggttttaa     540
   gtttcgcgag ggttttgggg agcgttgggt ttttaaggac gttttggatt tgaatatttg     600
   gtgagagcgt cgggtagggg aatagtagga gtaaagttcg gaaggtggtt atgaggataa    660
   gaagggagga tatttggtag gttgttaaaa ttaggtttat ttgtcgggta ggtggaggcg    720
   agtaattatt taagtttttt gggatagcgg gagagggggt aggggagtcg tcgtattatt    780
   ttttttgtgg tcgttagggg gttttcgttt taggtgagcg ttaagtttta gttttaggtc    840
   gtttttttat gttttaagta agttttgtag cgtagttaat tgttaggggt tgtttcgttt    900
   atttttcgtt ttcgtagtta tttgtttgcg cgaaatgttt gttaattttt gattggtagg    960
   tagtttggta aattaaatcg cgatttttga aagtaaaata ttgtagtatt ttggtagttt   1020
   tgaattggga agggatgaag gaggttgtgt tttcgggttg tacgaagagt tcgagttatt   1080
   ttttagaagg ttttgatagg tgggttttag aggagacgtc gtcggtgagt agtgattaat   1140
   atcgggagga aggggaattg aatttaattt tcgttttttt ttggaaaaag cgaagttatt   1200
   taacgttttt tagtgtatat attttttttt tttattgtta ttagtttcgt taatttgggt   1260
   ttcgttgttt tggaatgttt tttttagttt tgtatcgagg ttaagaggag cgggggtatg   1320
   ggttatttta ttaaaggtga tgttaggttt tattaaatta ggaagtgata tggagttaat   1380
   tttgttagaa tttttttttt tcgtgtcgag cggttcgggt tttttggcgg tagtagatgg   1440
   tggagttagt aggtgggatg aggggaggcg tttttggttt aagttcgttt ttggaataga   1500
   ggtgttgttt tttcgagttg taagttttta gtttagtggg acgggacgga agaatgtaat   1560
   tttttcgtga gttaaagtcg aggaacggga agtttggtag ggaattggcg tttattttta   1620
   gaagttagat cgtcgggtgg tgggaaagag cgtgttttat tgatttgttt agaaagaggt   1680
   aaattcgaat atagacgtta                                               1700
<210> 73
   <211> 1700
   <212> DNA
   <213> Artificial Sequence
<220>
   <223> chemically treated genomic DNA (Homo sapiens)
<400> 73
   tagcgtttgt attcgaattt gttttttttt gaataaatta ataaaatacg ttttttttta      60
   ttattcgacg atttggtttt tggaggtgag cgttagtttt ttgttaagtt tttcgttttt     120
   cggttttggt ttacgagaag gttatatttt ttcgtttcgt tttattgagt tggaaattta     180
   taattcgagg agatagtatt tttgttttag aagcgggttt agattaagaa cgtttttttt     240
   tattttattt gttaatttta ttatttgttg tcgttaggaa gttcgagtcg ttcggtacga     300
   agaggagaaa ttttggtaaa gttaatttta tgttattttt tggtttggta gagtttggta     360
   ttatttttag taaggtagtt tatgttttcg ttttttttgg tttcgatgta aaattggaaa     420
   gaatatttta aggtaacggg atttaggttg gcgaggttgg tgatagtaag aaggaagggt     480
   atgtatatta ggggacgtta ggtgatttcg ttttttttag aaaaaaacga aggttaaatt     540
   taattttttt ttttttcggt gttaattatt atttatcggc ggcgtttttt ttaaggttta     600
   tttattaaaa ttttttgaga aatgattcgg atttttcgtg taattcggag gtatagtttt     660
   ttttattttt ttttaattta gagttgttaa gatgttgtag tgttttgttt ttaaaggtcg     720
   cgatttgatt tgttagattg tttgttagtt agaagttggt aggtatttcg cgtaggtaag     780
   tggttgcgag gacggggagt aggcgggata gtttttggta attggttgcg ttgtaaagtt     840
   tgtttgaagt ataagggagc gatttggggt tagagtttgg cgtttatttg gagcggggat     900
   tttttagcgg ttataggaag aatggtgcgg cgattttttt gttttttttt tcgttgtttt     960
   agagagttta ggtggttgtt cgtttttatt tgttcggtag gtgggtttgg ttttagtagt    1020
   ttgttaggtg tttttttttt ttatttttat agttattttt cgggttttgt ttttgttgtt    1080
   tttttattcg acgtttttat taagtattta aatttaaaac gtttttgaag atttagcgtt    1140
   ttttagagtt ttcgcgagat ttgggattaa atatattttt ttttttttgt ttttttggaa    1200
   tatttgtcgg atattaagtt ttttagtaat attgaaggtt ttgaaacgag tttagcgttt    1260
   ggttaatgtt tttataatgt atataattat tacggttatg ataattaata tgcgtatgga    1320
   gttttataga tgataagttt tttatcgttt atacgttgtt atatatacgt atttgttttt    1380
   agatcgttat agttatgagt gagatttgaa gttcgtttta cgtatgaaga aatgttattt    1440
   gggtttagat attaataatg agaaagaggt agttttagtt ttagatcgtg gggtagagaa     1500
   agttttttgg gaatatggga gaggaacgag ttggtgtatt gtagaaattg ttcgaaggtt    1560
   agtagggttg gtgagagatt tttttggggg cgtggttggg tttgcgattt gagggacggg    1620
   ttagggatag tttataaata atatatttgg gggtcggcgc ggtggtttat atttgtaatt    1680
   ttaggatttt gggaggttag                                                1700
<210> 74
   <211> 1700
   <212> DNA
   <213> Artificial Sequence
<220>
   <223> chemically treated genomic DNA (Homo sapiens)
<400> 74
   tatgaattag ttatattata tagttgtttt attaaaaatt aaaattttgt agagtatatt      60
   aatagagatt gtatttaaga gaaaggtagt tattttttag ttatttatat aattatttat     120
   taagtattta tgtgttagtt gatatttttg ggtgttggaa taaaataagg aaaaaattaa     180
   gatttttatt tttacgaagt ttattaaagt gtggtaaatg atagagtaat tatttttaat     240
   agtttttagt ttgtattttg taattaattg aatatagtaa atattaaaag ttatttgagg     300
   tagggattta ttttattttt atttgttgga ttttatttcg tgtttagtat attagtttgt     360
   aagtaatcga tatggattaa ggggtaaaaa taggttttta aatagttaaa attttgataa     420
   gtaattattt gtttgattat taaatatgtc gttcgaaatt tttatttttt tttttaaagc     480
   gtagtaaata agtatttgcg tatttattac gtgtaaaata gtttttaata taatgttttg     540
   tgtgtaaaag gaacggcggg aatggattta agttgcgtat cgtgataaat ggcgtgaata     600
   ttttgagcgg gtggatagtt tttttttcgt gagttttttt tttttttttt ttagttatta     660
   agatagtacg cgtgtgtaga agttgtagcg gtgggacgta gtgttaagtt tgggggcgta     720
   tatattagtt cgcgcgtcgc gagttcgttt gggtagttcg gcgggcgcgg cgaaatagtt     780
   tttacgttcg ttttttatcg atttttcggt attagagcgg acgaggcgtt tttttagagg     840
   aaagattggg cgtcgtagac gttttttgga gaagtttttg gttattcgtt ttatttcggg     900
   gttttttttt ttttgggttt tttttgattt tttgggtggg cgttttagat aggttcgtcg     960
   gacgcgattt ggaaacgggt cgtgttcggt ttagttcgta tttattcgtt cggttatagt    1020
   ttagttatag tttagggatt ttgaggcgtt cgcgtttttt tgtcgcggta tttttagttt    1080
   aagtaggtcg gacgtcgagg tttcgcggta tcgagttttc gttcgtttcg ttttttcgtt    1140
   ttttatttcg tttcgttttc gtttttttat ttttttttcg ttttcgtttc gttttttcga    1200
   taattttttc gcgttttcgg ttcggtacgc gcgttgcgtt cggtcgtcgt tgtcgtcgtc    1260
   gtcgtcgtcg tcgttgtcgt cgagcgtttt ttcgttgcgt ttggttttta gtttcgggtc    1320
   ggaatcggaa gtttgggggg cggagttcgg cggaggttag gagatcgaaa acgcggtcga    1380
   gttcggagtt cggagttgga gttagagttt ggattagaat ttggtcgtcg tttgtatcgt    1440
   cgtcgtcgtt gtcgttcgtc gttttttttt cgcgtcgtag tcgtttcgtc gttatcgtcg    1500
   cgagttcggt cgttagtggt tttcggattt taggtgtttg ggttgaaggt cggttcggat    1560
   atcggattta gttagttttt agattgttcg ggcggtagcg gacgtcgttg tcgtcgtcgt    1620
   tttttcgtcg ttttagtttg gcgttttgtt tcgagagacg ggagaggcga gcggagttga    1680
   tagtgatttt gatagtgatt                                                1700
<210> 75
   <211> 1700
   <212> DNA
   <213> Artificial Sequence
<220>
   <223> chemically treated genomic DNA (Homo sapiens)
<400> 75
   aattattgtt aaaattattg ttagtttcgt tcgttttttt cgtttttcgg aataaaacgt 60
   taggttgagg cgacgaagag gcggcggcgg tagcggcgtt cgttgtcgtt cggatagttt 120
   gggaattgat tgggttcgat gttcggatcg atttttaatt tagatattta aagttcgagg 180
   attattggcg gtcgagttcg cggcggtggc ggcgaggcgg ttgcggcgcg gggaaggggc 240
   ggcgggcggt agcggcggcg gcggtgtagg cggcggttaa gttttggttt aggttttggt 300
   tttagtttcg ggtttcgggt tcggtcgcgt tttcggtttt ttggttttcg tcgggtttcg 360
   ttttttaaat tttcggtttc ggttcgaagt tggaagttag gcgtagcgga ggaacgttcg 420
   gcggtagcgg cggcggcggc ggcggcggta gcggcggtcg ggcgtagcgc gcgtgtcggg 480
   tcgggagcgc gaggagattg tcggggaagc gaggcgggag cgggggaggg gtgagagagc 540
   gagggcgggg cggagtggag ggcgaggagg cggggcggac ggaggttcga tatcgcgagg 600
   tttcggcgtt cggtttgttt gggttagaga tatcgcgata aaggggcgcg ggcgttttaa 660
   agtttttaag ttgtggttgg gttgtggtcg ggcgagtaga tgcgggttag gtcgggtacg 720
   attcgttttt aaatcgcgtt cgacgggttt gtttggagcg tttatttaaa gggttaggag 780
   aagtttaaga ggaagaggat ttcgaggtgg ggcgagtggt tagggatttt tttaaggggc 840
   gtttgcggcg tttagttttt tttttggggg gacgtttcgt tcgttttgat atcgaggagt 900
   cggtggaagg cggacgtggg agttgtttcg tcgcgttcgt cgagttgttt aggcgagttc 960
   gcgacgcgcg agttggtgtg tgcgttttta gatttagtat tgcgttttat cgttgtagtt 1020
   tttgtatacg cgtgttgttt tggtgattag aaaagaaaaa gaaaaaattt acggaggaag 1080
   ggttgtttat tcgtttagga tatttacgtt atttattacg atgcgtaatt tgaatttatt 1140
   ttcgtcgttt tttttatata taaagtattg tgttgaaaat tgttttgtac gtaataggtg 1200
   cgtaaatatt tgtttattgc gttttgaaga aaaaaatgag gatttcgaac gatatatttg 1260
   atgattagat aagtgattat ttgttaaaat tttagttatt taagggtttg tttttatttt 1320
   ttaatttatg tcgattgttt ataagttggt gtgttaggta cggaatggga tttaataggt 1380
   aaaaatagga tggattttta ttttaagtag tttttagtat ttgttgtatt tagttggttg 1440
   tagaatataa attagagatt attgaaggta gttgttttgt tatttattat attttagtga 1500
   gtttcgtgaa ggtagagatt ttgatttttt ttttatttta ttttagtatt tagaagtatt 1560
   aattagtata taagtgttta ataaataatt gtataaatga ttaagggatg attgtttttt 1620
   ttttaaatat agtttttgtt aatgtatttt ataaagtttt ggtttttaat aaaataattg 1680
   tataatatgg ttaatttatg 1700
<210> 76
   <211> 1700
   <212> DNA
   <213> Artificial Sequence
<220>
   <223> chemically treated genomic DNA (Homo sapiens)
<400> 76
   attttcgcgg ttatttttag ttaggttagt atagcgtttc gtgttcgttg aatcgtttta     60
   ggtagttttt tgatgattta aattattatt atttttgggt ttagagtagg ataaatgaaa    120
   ttttaatagg ggtttgtttt gtgatcgaaa attatttagt tttatagttt ttgaatgtaa    180
   ggagaggtgg tttttttaga attaatatgt tttttaaggt gggtacgttt acggttttgg    240
   gaaatgtata ttttaggtaa tttataggtg cgatttttta gtggaggcgg ggtagtttga    300
   atttttggtt ttgtaggagt gggaagaaat cgtttttgtt ttttcgataa ttgagttttt    360
   tatttggggt tgggagggcg gatagtagga tcggtttttt tattttttat cgcggttttt     420
   cgatttggtt agtagttttg gcgtttagat aaaaggtttt ttgagttggg ggtggggggt      480
   aggtcgagag taggtcggta aattagattt cgtagttttt taagttatta gttttgtatt    540
   tgggtgtaga gatttcgttt taaaaattcg aagtatcgtt ttggttcgtt ttttgttcgc    600
   gggcgcgggt tttcgtttat ttatttattt atttatttaa ttatttattt atttatttat    660
   ttttttttga cgtattcgtt cgtttttcgg tcgtttcggg cgtattatcg tttttttttt    720
   tgtttgaggg attttttgcg ttttaggatt attttataga gtttgtttat gttttcggtg     780
   aggttttcgt cgatgatggc gtacgtcggt tggacgtgat aggtggtggt cgggataagt    840
   tcgtgtagcg ttagttgttt tttaattttt gttatcggta gcgatttggg taggttttgt    900
   ttgttggcga tgattagtag cggcgtgttt tggttttcgg cgaatttggt tattttgtgt    960
   agtttcgttt tggttttttt tagtcggttt acgtttatcg agtttattac gtagatgatg    1020
   tcgttcgtgt agcggttgta ggatttttat agcggtcgta gttttttttg gtcgtttacg     1080
   ttttagaagt ggtagttgat gtttttggtc gtgtcgttgt ttagtttgat tttttcggtg     1140
   ttgaagtcga tggtgggtat cgtgtttacg aattcgttga atttgagtcg gtagagtatc   1200
   gtggttttgt cggtcgagtt taagtttaat atgacgatat gtagggattg gaaggtcgag   1260
   atgttagagg agatgttgtt tatggttttt ggttgcggcg ttagttattg cggttgcggc   1320
   gggagggcgt cgtttttcga gtagttacgg gttcgacgcg gtcgggcgta tttgggtttc   1380
   gttcgtcgtc gttcgtatcg gtttcggggt tcggcgtttt cgcgggcgta cggtttggcg   1440
   cgtttagacg tcgtagtttt cgcgttttcg tcggtcgggt tcgcgatcgg cgggtatcgc   1500
   gtttttacgc gcggttttgt ttttcgcgaa gtcgttttag atgttcggcg ttttttttag     1560
   gtttgggttc gttttgtggt tttttcgggt cgcgttttcg cgtttcggta gttgttcggg   1620
   tttttcgttt tttcggcgtt cggcgttcgt ttttggtttg ggacggcgtt gtttttgttt   1680
   ttgttttcgt tttcgttttt                                               1700
<210> 77
   <211> 1700
   <212> DNA
   <213> Artificial Sequence
<220>
   <223> chemically treated genomic DNA (Homo sapiens)
<400> 77
   gggagcggga gcgggagtag gagtaggagt agcgtcgttt taggttagag gcgagcgtcg      60
   ggcgtcggga gagcggagag ttcgggtagt tgtcggagcg cgggggcgcg gttcgaggaa     120
   attatagagc gagtttaggt ttggggaggg cgtcgaatat ttgaggcggt ttcgcgggag     180
   ataaagtcgc gcgtagagac gcgatgttcg tcgatcgcga gttcggtcgg cgagggcgcg     240
   gggattgcgg cgtttgagcg cgttaagtcg tgcgttcgcg gggacgtcga gtttcggggt     300
   cggtgcgggc ggcggcgggc ggggtttagg tgcgttcggt cgcgtcgggt tcgtgattgt     360
   tcggggggcg gcgttttttc gtcgtagtcg tagtggttgg cgtcgtagtt aggagttatg     420
   ggtaatattt tttttaatat ttcggttttt tagtttttgt atatcgttat gttgggtttg     480
   gattcggtcg gtaagattac ggtgttttat cggtttaagt ttaacgagtt cgtgaatacg     540
   gtgtttatta tcggttttaa tatcgagaag attaagttga gtaacggtac ggttaagggt     600
   attagttgtt atttttggga cgtgggcggt taggagaagt tgcggtcgtt gtggaagttt     660
   tatagtcgtt gtacggacgg tattatttac gtggtggatt cggtggacgt ggatcggttg     720
   gaggaggtta agacggagtt gtataaggtg attaagttcg tcgagaatta gggtacgtcg     780
   ttgttggtta tcgttaataa gtaggatttg tttaagtcgt tgtcggtggt agagattgag     840
   aagtagttgg cgttgtacga gtttatttcg gttattattt attacgttta gtcggcgtgc     900
   gttattatcg gcgagggttt tatcgagggt atggataagt tttatgagat gattttgaaa     960
   cgtaggaagt tttttaagta gaagaagaag cggtaatgcg ttcggagcga tcggggagcg    1020
   agcgagtgcg ttaagaaaga atgaatggat ggatggatgg ttggatggat ggatggatgg    1080
   atgagcgaga attcgcgttc gcgaataaag agcgaattaa agcgatgttt cgaattttta    1140
   aaacggaatt tttgtattta aatgtaggat tggtgattta aggagttgcg aagtttgatt    1200
   tatcggttta ttttcgattt gttttttatt tttagtttag gggatttttt gtttgaacgt    1260
   tagagttatt gattaggtcg gggggtcgcg gtggggagtg gaagagtcgg ttttgttgtt    1320
   cgttttttta gttttaggtg gaaggtttag ttgtcggaaa gataaaagcg attttttttt    1380
   atttttgtag ggttagaagt ttaggttgtt tcgtttttat tgggggatcg tatttgtgaa    1440
   ttatttgagg tatgtatttt ttagaatcgt gggcgtattt attttggggg gtatgttggt    1500
   tttgggggga ttattttttt ttgtatttag gggttgtgaa gttgagtaat tttcggttat    1560
   agggtaggtt tttgttgaaa ttttatttgt tttgttttgg gtttaaaggt ggtggtggtt    1620
   tgggttatta gaggattgtt tgggacggtt tagcgggtac ggagcgttgt gttggtttgg    1680
   ttggggatgg tcgcggaggt                                                1700
<210> 78
   <211> 1700
   <212> DNA
   <213> Artificial Sequence
<220>
   <223> chemically treated genomic DNA (Homo sapiens)
<400> 78
   agtaaattga ttttgtttgt ttagttttat tagttagggt ggtatggaag tgggtgggga 60
   gagggttggt gcgtggtata gataattggg aagcgcgcgt attatatata tatatatata 120
   tatatatata tatatatata tatacggttt tagttaatat ttatgtagta tttttggggg 180
   gttttttata tatatttgaa tagtatattt ttaataaagg ataggtatat ttttgtttgt 240
   ttttttttaa tttttagatt agtattttat tttgggttta gaattggtgg tgataagtcg 300
   atttagatgg agtatttgag aaaaggggag gtgatttttt gtttgaatgg gggagggggc 360
   gttttttatt tatcgtttga gtttttggaa cgaggagggt taggatattt tggatttttt 420
   taggtcgggt cggtggtcgg tttttatcgc gtaggaggtg cggtttatgg cgcgatagta 480
   taggtagcgt tcggtgttcg agtagtcgcg atgtcggacg cggtcggtgg tttcgttcga 540
   ggcgtagcgg tcgcgggtag tttcgggcgg cggcgggggc ggcggtaggg ggggcggcgg 600
   cggcggttgt agcggatatt ggggttgggg ttggggcgag ggtggcggcg gcgggggtag 660
   gtggtgatgg ggatgtagga ggcggaattg gggttgtcgg agcgggtatt ggtggtgttg 720
   ttcgtgtttt tagagatgtt tgggggtttt gagcgtggtt tcgtcggcgt tgtcgttgtt 780
   tttttcgttg ttcgttcggg tcggcgcgtt gttgtttttt gtttttattt tggttcgcgg 840
   tttcgcgatt tgttgtttag ttcgggttta tcgcgttggt tcgagcgttt ttttgatgtt 900
   gttgttgttg ttgtcgtcgt cgtcgttttt gtagtttagt agaggttggt gtttagtttt 960
   ttatttgaag gcgttagagt tttagggtta tcgagagggg gttttgttag cgggaggggt 1020
   tatagaacgc ggtagggttt tttttatcga ggttatggta gagatacgtt ttcgggtttt 1080
   ttaagttatt aagattaggg tgcgcggtgt tcggtttcgt tagaggttgg tttttttttt 1140
   tttttttttt tttttttttt tttgggcgtc ggcggcgaga gtagggattg ttagtaattc 1200
   gggttgtaga agtagtagta acggcggcgg cggtagtggt agtcgttttt agggtatcgg 1260
   aattttgatt tcggagttgt tgaaaagttg gatagtttta ggttggtacg ttcgagttgt 1320
   gatttttatt aaaattattt tagttagtta ggagttagat ttgttttttt gatttttttt 1380
   ttcgtcgtgt tcgcgttttt tttttttata ttgtttttta ttgtttcgga gttttttttt 1440
   ttattttcgt ttgttttagt gtttttagag aaatggagtt tggcgatttt tggttggttt 1500
   ttagagtttg ttgacgatga gttttatgtt ggtgttttta aagataagga atgtgattag 1560
   gtaaagttgt ttggaatatt gtttatgcgc gtcgttgaaa aattggattt gggtgaagat 1620
   ttaatagtaa gattaattta gttttttttt ttgatatagg ataaaatatt gtgtggtttt 1680
   tttttatagt agggagaatg 1700
<210> 79
   <211> 1700
   <212> DNA
   <213> Artificial Sequence
<220>
   <223> chemically treated genomic DNA (Homo sapiens)
<400> 79
   tatttttttt gttgtgggaa agagttatat aatgttttat tttatgttag ggaaagagat      60
   tgaattaatt ttattattgg atttttattt agatttaatt ttttagcgac gcgtatagat     120
   aatattttag gtaattttgt ttggttatat tttttatttt tggaagtatt agtatggggt     180
   ttatcgttaa taagttttga gaattagtta ggaatcgtta aattttattt ttttaagagt     240
   attgaggtaa gcggaggtga agaggaaggt ttcggggtag tggggagtag tgtggaggag     300
   ggaggcgcgg gtacggcgag gggaaggatt aagaggatag gtttgatttt tgattggttg     360
   gagtggtttt ggtgaaagtt atagttcggg cgtgttagtt tggagttgtt taatttttta     420
   gtagtttcgg gattagggtt tcggtgtttt ggaggcggtt attattgtcg tcgtcgtcgt     480
   tgttgttgtt tttgtagttc gagttgttga taatttttgt tttcgtcgtc ggcgtttaaa     540
   ggaagggaag aagaaaggga ggaagaagga ttaatttttg gcgaaatcgg gtatcgcgta     600
   ttttagtttt ggtgatttgg ggagttcggg agcgtgtttt tgttatagtt tcggtggaag     660
   gagttttgtc gcgttttgtg atttttttcg ttggtagggt tttttttcgg tagttttgag     720
   gttttggcgt ttttaagtga gaagttaagt attagttttt gttgggttgt agaagcggcg     780
   gcggcggtag tagtagtagt agtattagga aggcgttcgg gttagcgcgg tgaattcggg     840
   ttgggtagta ggtcgcggag tcgcgagtta ggatggaggt agagggtagt agcgcgtcgg     900
   ttcgggcggg tagcggagag ggtagcgata gcgtcggcgg ggttacgttt aaagttttta     960
   agtatttttg gaggtacgag tagtattatt agtattcgtt tcggtagttt tagtttcgtt    1020
   ttttgtattt ttattattat ttgttttcgt cgtcgttatt ttcgttttag ttttagtttt    1080
   agtgttcgtt atagtcgtcg tcgtcgtttt ttttgtcgtc gttttcgtcg tcgttcgggg    1140
   ttgttcgcgg tcgttacgtt tcgagcgggg ttatcggtcg cgttcggtat cgcggttatt    1200
   cggatatcga gcgttatttg tattgtcgcg ttatggatcg tattttttac gcggtggaga    1260
   tcggttatcg gttcggtttg aagaaattta ggatgttttg gtttttttcg ttttagggat    1320
   ttaggcggtg agtggagagc gttttttttt ttatttaggt aaagggttat tttttttttt    1380
   tttaaatatt ttatttaagt cggtttatta ttattaattt tagatttagg gtaaaatgtt    1440
   agtttggaaa ttgggggagg ataaataggg gtgtgtttat tttttattga gagtatgtta    1500
   tttaggtgtg tgtaagagat tttttaaaag tattgtataa atgttaattg gggtcgtgtg     1560
   tgtgtgtgtg tgtgtgtgtg tgtgtgtgtg tgtgtgtggt gcgcgcgttt tttagttatt    1620
   tgtgttacgt attagttttt tttttattta tttttatatt attttggtta gtgagattaa    1680
   ataggtaaaa ttagtttgtt                                                1700
<210> 80
   <211> 1700
   <212> DNA
   <213> Artificial Sequence
<220>
   <223> chemically treated genomic DNA (Homo sapiens)
<400> 80
   ggatagggaa gtttagttaa gaattaggag tttgatatat ttaatttttt attttatttt      60
   attgatgatt ttgtgaggta tattttttta attttagatt tataaaagag aaagttgagg     120
   tttggagagg ttttttattt tgtttaaagt tatataattt ttacgtggtg tagttaggat     180
   gggaattaga gtttggtttt ttgtattttt aatattattg ttgaattcgg gtttgtttgt     240
   tttttttttt tttttttaat tattgttata tagattttat tatagaattt attacgtgga      300
   gggttattat tttttttttt tttgtatagt atgtttgttt tttttatttt atagagtaag 360
   gtgtttagta aatgtggttg tttggaggta atattttttt tttatcgtta tatttggggt 420
   tggggtgcga ggtaaggtag ggttggcgag gtggatgttg tagttgttat tattgttagg 480
   tgttgggaaa cgattttttt gtttgtgtat tgattcgaag gtatttttta ttttttaggt 540
   ttttttcgtt tttcgttttt tatttttagt ttttcggttt tagtttaggt ttttcgggga 600
   gtattttttg tcgtgagatt gatagttttt gggggcgtag ggttttgttt tttgcgtttt 660
   agtttatttg tgcgtagagt ttcgttttta ggcgtttgga attcggcggg tattgacgtt 720
   aagcgtcggc ggagcgttgt ttatagacgg ttgattcggg ttttttttta tatttttttt 780
   ttttttcgtt tttttttttt tttttgtacg ggggttcggg tttattataa aaggtgggag 840
   cgcgtggtgt tttagtaacg acgagtttta gaacgatgga gagttttcgc gtgaggttgt 900
   tgtttttttt gggcgtcgtt ttgttgttga tgttattttt gttgggtatt cgtgtttagg 960
   aggacgtcga gttttagttt cgagttttgg atatttattt tgtcgtggat gatgtttttt 1020
   acgagaagga gttggtcggt atttttttcg ttttcgattt ttttgagttg tcgttttgtt 1080
   tttttttttg tacgtttttt tttttttttt atttttattt ttatttttag agttagggcg 1140
   cggggagttg agcgtaacgt ttaggtattt attgttattc gaagagcgtt tcgagtttac 1200
   gggttttggg tagtttgttg agcgaatttt ttatttcggt ttttttgagt aatagggatt 1260
   ttagcggttt agagattcgc ggttagtatt tgggatagcg ttcgttaagt ttttattttt 1320
   cgattatttt ttgtgttcgc ggagttttat cgtagagtgc gtgtgggttc ggggtttttt 1380
   ataattaggg ttggaagtgc gtatttgggt tgggttcgta gttaaggcgg taattttagg 1440
   tttcgaagcg gtgtgttgta gatcgaagcg ttgtaagaag ttttgaagaa gtttaagagt 1500
   aaacgtgttt ttatttatga gaagaagtat ggttaagttt ttatggtaag gtttgtggtt 1560
   attttttttt cgtgtttttt taagagaaag tatatcgttt tgaatcgtat atatagtttt 1620
   tttcgtagga tgtggttaaa taatttaggt aatgggtttg taggatttgg tgggtttttt 1680
   tttttttttc gggtgaggaa 1700
<210> 81
   <211> 1700
   <212> DNA
   <213> Artificial Sequence
<220>
   <223> chemically treated genomic DNA (Homo sapiens)
<400> 81 ttagttaagt ttttttgttt 1700
<210> 82
   <211> 1700
   <212> DNA
   <213> Artificial Sequence
<220>
   <223> chemically treated genomic DNA (Homo sapiens)
<400> 82
   gtgtttgtat tttatgttat tttttgagtt tataaatata aaaatagaat ttatttttgt      60
   tatagattta gttattggga atttattatt tgttatggat tatttttgtt aagaaaagtt     120
   tgaatgagaa gatggtagga cgtttgaaaa aaaaagttat aattaataaa atttgcggag     180
   aattgtaaat tttgttttta ttgttgattt aaattttttt tttaagattt ttttttttta     240
   gatatttatt tatttatttt tattttattt tttagtatta aggaggtttg agtggataag     300
   gtttaagtat tgatgtggtt ttggtaatga atttgaagtt aaaaattggt tagtttatgt     360
   taagttgtta tttgaaaggt tttttttagt atttgaatta agggaaataa tgttttgaag      420
   tagttataaa atttttttga tgtattagat ttgaggtagg atttgataat ttgtattttt     480
   aaattatgta ggtgatgttg atgttgttgg ttcggagatt attttggaga gttttatttt     540
   aaaggaaatg aaattgtttt aaaatgttaa tattaaatag ttatataaag tcggaaaaaa     600
   gtaaagaatt ttaaatcgaa gatgagaggt tttgaaaaat aatagcggtt attttatttt     660
   cggtgcggtt atgcggttat gaaaaggagt tttggggtcg gtcgtgtttt tttacgtagg     720
   tttttttata gggttatttt attaattttc gttaaaaacg tcggtttttt tatagggtcg      780
   ttcggttacg ttttttcgtg ttttaattat agagttatac gttacgtcgt cgtttcgtta     840
   cgtttattcg gttttcgtat agggcgttac gtacgtttaa cgtcggttcg gcgcgcgcgt     900
   tcgggaggtt gttcgtagct cgttaattgg tggtttttag ttttcgtcga ttcggttttt     960
   tttttttgcg gttattagat cgcgggatat cggagatttc gaataattcg ttggagtttt     1020
   ttatagttta aaaatagagg atcggtcggg cgcggtggtt tacgtttgta attttagtat    1080
   tttgggaggt cgaggcgggc ggattacgag tttaggagat agagattatt ttggttaata    1140
   tcgtgagatt tcgtttttat taaaagtata aaaaaattag tcgggtgtgg tggtacgcgt    1200
   ttgtagtttt agttatttag gaggttgagg tagaagaatc gttagaatta gggaggcgga    1260
   ggttgtagtg atcgagatcg cgtcgttgta ttttagttta ggcgatagag cgagattgcg    1320
   ttttaaaata aaaaaaacgg aggatcgaaa attttgaaga ttgagagtaa ggtaataatt     1380
   tttttttgat ttttttgttt tttttataag ggttttttag acggttgaaa ttaacgaaaa      1440
   gagtgaggaa gtaggtagaa aattttagtt ttagtttttt agtatttttt tgtttttttt    1500
   tcggggagtt ggtatttttt ttttttttag tagttgtaaa tacgtttttt aattttaagg    1560
   agattttttt tgtatttttt ttattgtttt tacgtttgta gggtttttcg tgtgaggatt    1620
   tagtgtatgt atgtatgtat atgaatgtgt gcgaataatt attttttaat tgagtttttt    1680
   tttaaaaaaa ttatttttcg                                               1700
<210> 83
   <211> 1700
   <212> DNA
   <213> Artificial Sequence
<220>
   <223> chemically treated genomic DNA (Homo sapiens)
<400> 83
   cggaagatga tttttttaaa aaaaaattta attaggaaat aattattcgt atatatttat      60
   atatatatat atatatattg aatttttata cgaagagttt tataggcgtg agaatagtaa     120
   gggagatgta agaaaggttt ttttggagtt gggaggcgta tttatagtta ttagagaagg     180
   gggaagtgtt agtttttcga gaagaaagta ggaggatgtt gagagattaa agttgaaatt     240
   ttttgtttgt ttttttattt ttttcgttgg ttttagtcgt ttgaaaaatt tttgtgagag     300
   gagtaaaaag gttaagggaa gattgttgtt ttgtttttag tttttaaaat tttcgatttt     360
   tcgttttttt tgttttgaga cgtagtttcg ttttgtcgtt taggttggag tgtagcggcg     420
   cgatttcggt tattgtaatt ttcgtttttt tggttttagc gatttttttg ttttagtttt     480
   ttgagtagtt gggattatag gcgcgtgtta ttatattcgg ttaatttttt tgtatttttg     540
   atagggacgg ggttttacgg tgttagttag gatggttttt attttttgaa ttcgtgattc      600
   gttcgtttcg gttttttaaa gtgttgggat tataggcgtg agttatcgcg ttcggtcggt     660
   tttttgtttt taaattgtaa aggattttag cggattgttc gaagttttcg atatttcgcg     720
   atttggtggt cgtagaagaa agaggtcgag tcggcgggga ttgggaatta ttagttagcg     780
   gtttacgggt aatttttcgg acgcgcgcgt cgggtcgacg ttaaacgtac gtgacgtttt     840
   atgcgggagt cggatgggcg taacggggcg gcggcgtaac gtgtggtttt gtggttaggg     900
   tacggagggg cgtggtcgga cggttttatg agggggtcgg cgtttttggc gagggttgat     960
   gggatgattt tatgagggag tttgcgtgga ggggtacgat cggttttaag attttttttt    1020
   atggtcgtat agtcgtatcg ggagtaggat gatcgttgtt attttttagg attttttatt    1080
   ttcggtttga aattttttgt tttttttcgg ttttgtgtgg ttatttagta ttagtatttt    1140
   agggtagttt tatttttttt aaagtaaaat tttttaaagt ggttttcgga ttagtagtat    1200
   tagtattatt tgtataattt agaagtataa attattaggt tttattttag atttaatgta    1260
   ttagaaaagt tttgtgattg ttttaaaata ttgttttttt taatttagat gttggaggag    1320
   attttttaaa tggtagttta gtataagtta attagttttt aattttaggt ttattattaa    1380
   agttatatta gtgtttaggt tttatttatt tagatttttt tgatgttgga aagtaaagtg    1440
   aaaataggta gatagatgtt tagaaagggg aggttttaga agaaaggttt ggattaataa    1500
   tgaaggtaga atttataatt tttcgtagat tttattaatt ataatttttt tttttagacg    1560
   ttttgttatt tttttattta gatttttttt agtaaaggta gtttatggta agtaatgaat    1620
   ttttagtaat taggtttgta atagaagtaa attttgtttt tatgtttata aatttaaaaa    1680
   gtaatatgaa gtgtaaatat                                                1700
<210> 84
   <211> 1700
   <212> DNA
   <213> Artificial Sequence
<220>
   <223> chemically treated genomic DNA (Homo sapiens)
<400> 84
   gtatacgtga aaataatatt attttatatt acgatttatg ttattcgtat gtgtaattaa      60
   agttttttaa gttttgatat gtttttataa agacgaagga tagagtgaat gttaaaattt     120
   gttaaatagg agttattgaa agtttgaagg ggaaagttta gttaggtttt attttagaag     180
   tgatttttaa aacgaaatac gtagataagt tcgagttttt tggagtcgat ttttaaggtt     240
   ttgtaggtgg taagggggtc gagaattggt tgaggttttt cggaagttcg cgtcgattcg     300
   gagggttatt tttgattttt tgatttgggg tgtaattttt agtttttgtt tcggtcgata     360
   aaggtgtgga gttaggtttt aggtaagttg ggggtggggg tgcgtataag ggaggggtag     420
   gggattggga tttttttttt ttcggggtgt ttcgggtcgt ttttttagag gttggtggtg     480
   ttttcgattt aagtagttta gtttcgttcg cgaggttcga agtggcggga acgggttttt     540
   cgcggcggtt ttaaggcgag ggaggttgta aaatttggta gagaattcgg cgcgtcgggt     600
   cggggatgtt tcgggtgtcg gggtaggggg tcgtaggttc gtaggtagaa gtttttttac     660
   gcgagcgggg gtttcggtcg cggaaggggc gttttcgggg tcgtcggtag cggcgagcgg     720
   gcgggaggga gtttttcggg tttttttcgc gtttcgggtt ggcggggcga tggcgacgac     780
   ggtttttcgg ttttttcgtt tcggacgtcg atggcggtgc gtttcggttt ttttttaata      840
   gttttttttt cgcggtcgtc ggagtcggtg aggtcgagag ggcggggagt cgcgtaggga     900
   aggcgagagg gcggaggttg cgtggtgtag gcggcggcgg cgggaggagg agggtcgggc     960
   gggtagggga ggaggaggag gcgggcgtcg tgtcgtacgt agttttaggc ggggtagttt    1020
   cggtagttga gggacgtagt tagattttgg cgggacgggg ttttcgtcgg ggtttaggtt    1080
   tagggattag gcggaggcgt cgcgggagtt tttggggtat tatagagatg cgggtgagtg     1140
   tcggtagtcg tcggggtcga gaggaggtgg cggcggcggc ggcggcgtag gttcggtttc    1200
   gagtcgcggt gtcgtagtga ttcgggtcgc gacggtcgaa attaggcgtt tggcgaggtt    1260
   tgagtcgtcg aaacgcggtc gggtagaatg gcggtcgggt cgggggattc gtgggcggag    1320
   gacgtcgaag gtggtagagg agcgagcggg gtcgggtttt gagttatttc ggacgggtcg    1380
   gggcggttag gtttggatta cgtattggga aaaagtacga tttatcgcgt ttggaggcgg    1440
   cggtttggtg gcgtcggttt tttttttcgt cgcgatcggg ttgggttttc ggagggtggg     1500
   tcgacgtgtg gttcggggcg gttcgggcgt tcgcgtttcg tttggtgagg cgattttcgc    1560
   gggacgtcgg gattcgtgtt tttgtttttt cgcgtttacg ttttttttcg gcgttgtttt    1620
   tcggtcgacg cgtgtttttt tgtgagtttt gggtttaagc gtaggagtag tcgcggagtc    1680
   gggttttttc gtttttcggt                                                 1700
<210> 85
   <211> 1700
   <212> DNA
   <213> Artificial Sequence
<220>
   <223> chemically treated genomic DNA (Homo sapiens)
<400> 85
   atcggaaagc gagagagttc ggtttcgcgg ttgtttttgc gtttgagttt agagtttata     60
   ggggaatacg cgtcggtcgg aaagtagcgt cgggaagaag cgtgggcgcg agggggtaag    120
   ggtacgggtt tcggcgtttc gcggaggtcg ttttattagg cgaggcgcgg acgttcgagt    180
   cgtttcgggt tatacgtcgg tttatttttc gagaatttag ttcggtcgcg gcggggaaag    240
   gggtcggcgt tattaggtcg tcgtttttag gcgcgatgaa tcgtgttttt ttttagtgcg    300
   tagtttaggt ttggtcgttt cggttcgttc ggggtgattt agggttcggt ttcgttcgtt    360
   tttttgttat tttcggcgtt tttcgtttac gagtttttcg gttcggtcgt tattttgttc    420
   ggtcgcgttt cggcggttta ggtttcgtta ggcgtttggt ttcggtcgtc gcggttcgag    480
   ttattgcgat atcgcggttc ggggtcgggt ttgcgtcgtc gtcgtcgtcg ttattttttt    540
   tcggtttcgg cggttgtcga tatttattcg tatttttgtg gtgttttaaa ggttttcgcg    600
   acgttttcgt ttggtttttg ggtttgggtt tcggcgaaag tttcgtttcg ttaaggttta    660
   gttgcgtttt ttagttatcg gggttgtttc gtttggagtt gcgtgcgata cggcgttcgt    720
   tttttttttt ttttttgttc gttcggtttt ttttttttcg tcgtcgtcgt ttgtattacg    780
   taattttcgt tttttcgttt tttttgcgcg gtttttcgtt ttttcggttt tatcggtttc    840
   ggcgatcgcg ggggagaagt tgttggggga gggtcggaac gtatcgttat cggcgttcgg    900
   ggcgggggag tcgggaaatc gtcgtcgtta tcgtttcgtt agttcgaggc gcgggagggg    960
   ttcggaaagt tttttttcgt tcgttcgtcg ttgtcggcgg tttcgggggc gtttttttcg   1020
   cggtcgaggt tttcgttcgc gtggggaagt ttttatttgc gagtttgcgg tttttgtttt   1080
   cggtattcga gatattttcg attcggcgcg tcggattttt tgttaggttt tatagttttt   1140
   ttcgttttgg gatcgtcgcg gaggattcgt tttcgttatt tcggatttcg cgagcggggt   1200
   tgagttgttt gggtcgggga tattattagt ttttggagaa acggttcgaa gtatttcgaa   1260
   gaagaggggg ttttagtttt ttgttttttt tttgtgcgta tttttatttt tagtttgttt   1320
   ggaatttgat tttatatttt tgtcgatcgg gatagggatt gggggttgta ttttagatta   1380
   gaaagttaga ggtggttttt cgaatcggcg cggattttcg aggaatttta gttaattttc   1440
   gattttttta ttatttgtaa ggttttagga atcggtttta gaaagttcga gtttatttgc   1500
   gtgtttcgtt ttaaaaatta tttttaagat aaaatttaat taggtttttt tttttagatt   1560
   tttagtaatt tttgtttggt aaattttgat atttatttta tttttcgttt ttgtaaggat   1620
   atattaagat ttagaaggtt ttgattatat atacggatgg tatgagtcgt gatataaaat   1680
   agtattgttt ttacgtgtat                                               1700
<210> 86
   <211> 1700
   <212> DNA
   <213> Artificial Sequence
<220>
   <223> chemically treated genomic DNA (Homo sapiens)
<400> 86
   gttagagatt gaaatttttt ggaattaatt attggacgtt ttggattggg gcggaacgcg    60
   tttttattgt ttcgaaagta gtattttttc ggatttgagt ttttgcggcg cgtttgggtt    120
   gagatttgag aaatgcgttt tgggttatag gtagtagcgg aggcggggaa ggaataattt    180
   ttttttcgtt ttttaattat aagggcgggg tggggcggtt tcgttggggt cgggtttttt    240
   tattttttta gttgggattt tttgtcggag ttggcgtggg ggtcggggag ggggattaga    300
   ggggtttttt ttttttttta agtttcgtta gatgttggaa aggtaaataa taggggaaac    360
   gcggagaaat aaaaggttat tcgttacgat tttagtcggt tgcgtagaga gatttttgtg    420
   gggagggtta ggggagaggg ttaggaaaga gcgcgagggg ggggatttcg agagatttag    480
   agtttttggt atttgttagg tattaggggt cggcgtttta tttttttagt aggggtttga    540
   aggtgggaag gggtaattaa agaaaatttg aagggttttt attttttttt ttagtttagg    600
   gaaagggagg ggttgggatt ttagatgttt gatagttgtt ggttttaaag ttaagcgcga    660
   tttgtacggt ttttatacgt tttttatttt cgtgaggtat cgttagtttc gggggcgatt    720
   tggttgacgg tgcggatttt ttttacgttg ttttaagtcg agattttttt tttaggatta    780
   ggacgtttaa gcgttgtttt ttagtttagg cgtatttttg taaaagtttg ttttttcgtt    840
   atttatagtt taatacgtat tttgtaggcg ttcgcggttg gggatgggtt cggagttttg    900
   tatttcgaaa gaaggacgat tttattgatt tatatttttt taaattaagg ataaattaag    960
   atttcggggt ggaaagtggg cgatttttgt ttttgtcggg aagtgtatac gaggagtcgg    1020
   ttagaaattt aggcgtcggt aggggtttga agaggatttt agtttcggaa gttcgtggtc    1080
   ggcgggtaga agatcggttc gtcgtttttc gtattttttt gtattcgcgt ttaggtgcgt    1140
   tttaaatttc gtcgttcgtt cgggcgagat cgggagcgag tagttatcgg gttgcgtcgt    1200
   tttttttagt ttggagcggg tttttattta tcgtggggtt ttatttttag tttagggttc    1260
   ggtcggtttt tttcgtcggg ttagttgtag ttagcgcgaa agaaaatggt gagttcgggg    1320
   taggtggggc gtcgtttgtt aatttacgta agggattttg tcgtcggtgc gcgaggtttc    1380
   ggcgagttaa tcggcggacg ggcgagcgtt tggttttagt tgtagtcgtc gtcgtttcgg    1440
   agtagttttt ttatgtattt agcgcgtcgc gtagtcggtc ggggttcgat tgtttcgcgg    1500
   ggacgtcgag cggggatttt tggcggtcgt ttgcggtatg gtagtatcgt ggagagaaaa    1560
   gtcgtttttg ggattatttt tttttcggaa agagatggga tatgattagt ttaagggggt    1620
   ttttagtttt gtagaaaaga tattgggtta gggtagtgaa gaaggtgtta gattagaaag    1680
   gatgattttt gggtttataa                                                1700
<210> 87
   <211> 1700
   <212> DNA
   <213> Artificial Sequence
<220>
   <223> chemically treated genomic DNA (Homo sapiens)
<400> 87
   ttgtggattt agagattatt ttttttgatt tgatattttt tttattgttt tggtttagtg      60
   ttttttttgt aaggttggaa gtttttttag attggttatg ttttattttt tttcggaggg      120
   aagatgattt taaagacgat tttttttttt acggtgttgt tatatcgtag gcggtcgtta      180
   ggggttttcg ttcggcgttt tcgcgagata gtcgagtttc ggtcggttgc gcggcgcgtt      240
   gggtgtatga gggggttgtt tcggagcgac ggcggttgta gttggagtta ggcgttcgtt      300
   cgttcgtcgg ttggttcgtc gggatttcgc gtatcggcgg tagagttttt tgcgtggatt      360
   ggtaagcgac gttttatttg tttcgagttt attatttttt ttcgcgttgg ttgtagttga      420
   ttcggcgaag ggagtcgatc gggttttggg ttggaggtaa aattttacgg tgagtaagaa      480
   ttcgttttaa gttaggggag gcggcgtagt tcggtggttg ttcgttttcg atttcgttcg      540
   ggcgggcggc gaggtttggg gcgtatttgg gcgcgggtgt aagaaggtgc gggaggcggc      600
   ggatcggttt tttgttcgtc ggttacgggt tttcggggtt ggagtttttt ttagattttt      660
   gtcggcgttt gggtttttgg tcggtttttc gtgtgtattt ttcggtagga ataagggtcg      720
   tttatttttt atttcgggat tttgatttgt ttttgatttg aaaagatata aattaataag      780
   atcgtttttt tttcggggtg taagatttcg agtttatttt tagtcgcgga cgtttgtagg      840
   gtgcgtgttg ggttgtgggt ggcgggaaga taaattttta taaaagtgcg tttgggttgg      900
   gggataacgt ttgggcgttt tgattttgag ggaggagttt cggtttgggg tagcgtaggg      960
   gaagttcgta tcgttagtta ggtcgttttc ggggttgacg atgttttacg gaggtgggga      1020
   gcgtgtaaag gtcgtataaa tcgcgtttaa ttttggggtt aataattgtt aaatatttgg      1080
   aattttagtt tttttttttt tttgaattgg ggaagaaggt gaaaattttt taagtttttt      1140
   ttgattgttt tttttatttt ttagattttt gttgggaggg taaagcgtcg atttttggtg      1200
   tttggtaagt attagagatt ttaaattttt cgggattttt tttttcgcgt ttttttttga      1260
   tttttttttt taattttttt tatagagatt tttttacgta gtcgattgag atcgtggcga      1320
   atggtttttt gttttttcgc gtttttttta ttgtttgttt ttttaatatt tggcggggtt      1380
   tggggagaga aggaagtttt tttggttttt tttttcggtt tttacgttag tttcggtagg      1440
   ggattttagt tgggaaagtg gaggagttcg attttagcga ggtcgtttta tttcgttttt      1500
   gtggttagag ggcggaggga aagttgtttt tttttcgttt tcgttgttgt ttgtggttta      1560
   gggcgtattt tttagatttt agtttaggcg cgtcgtaaag gtttaaattc gagaaggtgt      1620
   tgttttcgag atagtggaag cgcgtttcgt tttaatttag agcgtttagt ggttggtttt      1680
   agaggatttt aatttttagt                                                  1700
<210> 88
   <211> 1700
   <212> DNA
   <213> Artificial Sequence
<220>
   <223> chemically treated genomic DNA (Homo sapiens)
<400> 88
   ttaggatttt taattattta ttatttgtat ttttggggtg gacgtgagtt cgcgttaaat 60
   taaggatatt agtcgagtat ttagcgcggt tttttacgtt ttttaggaat tttggaaagt 120
   tatttaagtt cggcgttatt tttaaaagga atgagttgtg gtttttaatt agtttgtcga 180
   ggagagcgta gagtgatacg ggacgggggt ttagggtatt gaggttaaga gttttcgagt 240
   tacgttagtg ttcgtttttt tggtttttgt ttaaaatttt gcgatgtttc gttgataaag 300
   tatttagtgt tagagtttgc ggtttagcga agttgttttt tggaaatata tatatatata 360
   tatatatata tatatatata tatatatata tatataaaag ggggtgggga gagggaaagg 420
   tttaggttta ggagaggaag ttagttagtt agttttgtag atgtaatcgt tgtaatttgg 480
   gttttttttg ttttttgacg taatgttagt tttttgtttt cgcgttgtag tgtgtagttc 540
   gttgttggga agttagatta atcgattgaa cgttaagatt attattgtta ttattttgcg 600
   atttattttt tacgaatata ttttaattgg ttcgagtttc gtttttaaat tttacgattt 660
   ttggtttttt aggaagtatt gttttttttt ttttttagga aggtcgtttt tacgtatagt 720
   tagtattagg aaacggcgtc gttcggggtt tcgatggaga gtcggggtgg ggtcggttta 780
   aattttttgt ttttcgtttt tttgagattt ttgatttttg gttttgtttt aagtaaaagt 840
   attgtagaag ttgtagcgtt tttttttttt ttttagaaat cgagcgagga cgtgggggaa 900
   aagttatttt ggaacgtgta aggacgtttt tttacgttta atacgttata gggtcgggat 960
   gcgcggttgt cgtttcgtta ttttacgcgc gtagcgttaa ttgtagtttc gtagttatcg 1020
   tcgtcgtcgt cgttttgtcg agttcgggga gatttttaag ggttttttaa atacgagtta 1080
   taaatattat aggcgttcgg cgcgtgcgtt acgatatatt cggttttcga ttgggcggcg 1140
   taatttcgtt tatttgtata ggtcgattgt ataatcgggc ggcgatttta gttcggtgtt 1200
   cgtgttacgt tttttttatt tttgcgggta cgcggggtcg cgggcgggat agcggggtgg 1260
   cgattgcggc gtttagagtt ttttgaattc gggaattttt taagttttga gttaggcggc 1320
   ggtttttcgg gacgttagtg ttttttagaa ggtttagttt tggttagtta agggtttatc 1380
   gtttaaattg ggtttttttt atttttatag aaaaggatag tttttagagt gatattttag 1440
   taaacgtttt gggtggttcg tagaggttga gtttaaatta cgtgagattt ggtcgcggcg 1500
   agagttttgt ttaatgattt gcgatttggg gtgtttaaaa agggtttttc gtatgatttt 1560
   taataagatt tcgttatttt gaaaacgaaa tagggagggt tcgaaaacgt aagatgtgta 1620
   atgttttgtg gtttaaattt agtttttaaa aaatgtatta atgagttaat atgaaatagc 1680
   gatatggaga ttttttttta 1700
<210> 89
   <211> 1700
   <212> DNA
   <213> Artificial Sequence
<220>
   <223> chemically treated genomic DNA (Homo sapiens)
<400> 89
   tgggggaaag tttttatgtc gttgttttat attggtttat tggtgtattt tttaaaaatt      60
   aggtttaaat tataaggtat tatatatttt gcgttttcga attttttttg tttcgttttt     120
   aagataacga ggttttatta gagattatgc gaaaagtttt ttttgaatat tttaagtcgt     180
   aagttattgg ataggatttt cgtcgcggtt aggttttacg tggtttaaat ttagtttttg     240
   cgagttattt agagcgtttg ttggggtgtt attttgaaaa ttattttttt ttgtagaaat     300
   aaaaaagatt tagtttaaac ggtaagtttt tggttggtta aaattgagtt ttttgagagg     360
   tattggcgtt tcggaggatc gtcgtttgat ttaggatttg aaaggttttc gagtttaagg     420
   gattttggac gtcgtaatcg ttatttcgtt gtttcgttcg cggtttcgcg tgttcgtaga     480
   agtggggaag gcgtggtacg agtatcgggt tgaggtcgtc gttcggttgt gtaatcggtt     540
   tatgtaaata agcgaggtta cgtcgtttaa tcgggagtcg gatgtgtcgt gacgtacgcg     600
   tcgagcgttt gtagtattta tagttcgtat ttaaagagtt tttgggagtt ttttcgggtt     660
   cggtagggcg gcggcgacgg cggtggttgc ggagttgtag ttagcgttgc gcgcgtgagg     720
   tggcggggcg gtagtcgcgt atttcggttt tgtggcgtgt tgggcgtggg ggagcgtttt     780
   tatacgtttt agggtggttt tttttttacg ttttcgttcg gtttttggaa agggggagga     840
   aacgttgtag tttttatagt atttttgttt aagataaagt tagggattag gaattttaaa     900
   agagcggggg gtagaaggtt tgagtcgatt ttatttcggt tttttatcgg agtttcgagc     960
   ggcgtcgttt tttgatgttg gttgtgcgta gaggcggttt ttttgggggg aggggagaat    1020
   aatgtttttt gaaaagttaa aagtcgtgga atttgggggc ggggttcggg ttaattaaag    1080
   tgtattcgtg gaaaataaat cgtaagatag tggtagtgat gattttggcg tttaatcgat    1140
   tgatttgatt ttttagtagc gaattgtata ttgtagcgcg aaagtaggaa attggtatta    1200
   cgttagaaga tagaagagat ttaaattgta acggttgtat ttgtagggtt ggttggttgg    1260
   tttttttttt taaatttaaa tttttttttt tttttatttt tttttgtgtg tgtgtgtgtg    1320
   tgtgtgtgtg tgtgtgtgtg tgtgtgtgtg tgtgttttta gggagtagtt tcgttgggtc    1380
   gtaggttttg gtattgggtg ttttgttagc gaggtatcgt aggattttag gtaaaggtta    1440
   aaggggcgag tattggcgtg attcgggaat ttttggtttt agtgttttgg gttttcgttt    1500
   cgtgttattt tacgtttttt tcggtaagtt agttaggagt tataatttat tttttttaaa    1560
   gatagcgtcg agtttggata gttttttaaa atttttggag ggcgtaggga atcgcgttga    1620
   atgttcggtt ggtgttttta gtttagcgcg agtttacgtt tattttagga atataagtgg    1680
   tgagtggtta gagattttag                                                 1700
<210> 90
   <211> 1700
   <212> DNA
   <213> Artificial Sequence
<220>
   <223> chemically treated genomic DNA (Homo sapiens)
<400> 90
   tattttaagt tattaagttt tagataggta tagtagtata tacgtgtgaa tagaatttat      60
   tagtatagtt tggggtttag gtttttgtat attaataatt taaggttttt ggtaatggtt     120
   tagcgtgtat ttatgaggtt attgtttttt tatatacgta tttagaatat ttatagtttt     180
   aaaacggtaa ttttattata aaaatgatat tttgtatttg tatagtattt tgttttttgt    240
   gtattttata tatttattat ttaatgattg tgttttttga aatttttaat aattttgggt  300
   ggtggtaaga ataggtattt ttaagttatt tttttagatg aggaaattta tttgtgtttt     360
   tttttttttt tttaatagtc ggttgatgga atttaagttt tttatattag tagtggggtt     420
   agttgtatag agcggttaga agggagaggt ttcggggtta cgtttttttt tttattttta     480
   gtttttgatt tattgttata ttatggcgag taataggata atttttttta tagaattatg    540
   ggggatatta tttttttgag gttaatgtgt ttaattaagc gggaaatttt tcgggtagag    600
   tgaaattcga agttgttatg ttataagata atttagagta aggggaataa ttgacgtttt     660
   aatgtttttt tttatggggg gttgaggggt gtttatttat attttttttt ttcgtttttt     720
   ttcgttattt tttttttgtt ggtcggttta attttataag cgcgcgtttg tttcgagtat    780
   agtaatttag agtagatggg gggtattttt tagagggtag tggaaagttt atttgttatt     840
   gttgcgtcgg ggatttgcgg ttttttgggc ggttacgttt tgtttcggcg gcgtttggcg    900
   cgtttggaat gcggggtttt ttttcgtttt tttttttacg tcgtttcgtt tcgttttcgt    960
   tagggttttt tagagaagtt cgatttttta ttatttttgt tttgtatttt ttcggttcga   1020
   gggtcggtgg ttttttattt ttgttttaaa tattttgttt ttgagttttt tttttaaaga   1080
   ttttttagtg tcggtcgttt ttttagtttt ttgttttcga cggttttcgg ggttagttgt    1140
   cggggttttt ttgttaggtc gtattttttt ttttagcgtt tttatatttc gttagttggc    1200
   ggtttatcgc gggcgggtag ggcgcgtttc gtttatttgg gtcgtgcgtc gagagttgag    1260
   agcgttttta aatgttgagt agagtcggtt ggtttgggtg gggtttttat attcgttata    1320
   ttttttttag gcgaggcgag gttcgtttcg tttcgtttcg tttttttttt atacggttcg    1380
   tttttatttt aagttagggg tgatttattt cggttcgtcg gttcgtaggg atcgcgtttt    1440
   ggaacgtttt cgagtcgcgt tacgcgggag cggttttagc gtttttggtt ttgtttttcg   1500
   agaaagttcg gtcgaggttg tttcgaggtt tatagggatt tcgttttttt tttttttttt   1560
   tattttattt tttaagtgtt acgtcgagtt tttagtttcg gtttgggtta ataaattaat    1620
   aagattttag tagtagattt tgggtttgat taagtgtttt tttttttttt tttttttttt    1680
   tggcgttaat gtaatggagg                                                1700
<210> 91
   <211> 1700
   <212> DNA
   <213> Artificial Sequence
<220>
   <223> chemically treated genomic DNA (Homo sapiens)
<400> 91
   tttttattgt attaacgtta aaaaaaaaaa aaaaaaaaaa aaaatattta attaagttta      60
   gagtttatta ttaaagtttt gttaatttat taatttaagt cgaggttgag agttcgacgt     120
   ggtatttaag aagtaaaatg aggaaaggag gaagaaacgg ggtttttgtg ggtttcgggg     180
   tagtttcggt cgggtttttt cggagggtag ggttaggggc gttggggtcg ttttcgcgtg     240
   gcgcggttcg ggagcgtttt agggcgcggt ttttgcgggt cggcgggtcg gggtgagtta     300
   tttttgattt ggggtgggag cgggtcgtgt aagggggagg cggggcgggg cggggcgggt     360
   ttcgtttcgt ttagggagga tgtggcgggt ataaaagttt tatttaggtt agtcggtttt 420
   gtttagtatt tggggacgtt tttagttttc ggcgtacggt ttaggtaagc ggggcgcgtt 480
   ttgttcgttc gcgatgggtc gttagttagc ggggtgtgga gacgttggga agaagggtac 540
   ggtttggtag ggaggtttcg gtaattggtt tcggaggtcg tcgggggtag ggggttgaag 600
   ggacggtcgg tattggagag tttttgggga gggggtttag ggataaggtg tttggggtag 660
   gggtgagggg ttatcggttt tcgggtcggg agggtgtagg gtagaggtgg tggggggtcg 720
   ggttttttta agaagtttta gcgggagcgg ggcgaggcgg cgtggggaga ggggcggagg 780
   gaggtttcgt attttagacg cgttaggcgt cgtcgaggta gggcgtggtc gtttagggag 840
   tcgtaggttt tcggcgtagt agtagtaagt gggtttttta ttgtttttta gaaagtgttt 900
   tttatttgtt ttaagttatt gtattcgaaa taaacgcgcg tttgtggagt tgaatcgatt 960
   agtaggaggg aagtagcggg ggaagacgag aggggggagt gtgagtaggt attttttagt 1020
   tttttataaa agaagatatt aaaacgttag ttattttttt tgttttaggt tattttgtag 1080
   tatagtaatt tcggatttta ttttattcgg agagtttttc gtttggttga atatattggt 1140
   tttaggaagg tagtgttttt tatgattttg tggggagggt tgttttgtta ttcgttatga 1200
   tgtgataatg agttaggaat tgagagtaga gagaagggcg tggtttcggg gttttttttt 1260
   tttggtcgtt ttgtgtagtt ggttttattg ttggtgtgga gggtttgggt tttattagtc 1320
   ggttgttgag aagaaggagg ggaatataga taagtttttt tatttgggaa ggtggtttaa 1380
   ggatgtttat ttttattatt atttagagtt attggagatt ttaaaaaata tagttattag 1440
   gtaataaatg tgtaaagtat ataaaaaata aagtgttgtg taaatgtaaa gtgttatttt 1500
   tgtagtaaga ttgtcgtttt aaagttgtgg gtgttttgag tgcgtgtgta aaagagtagt 1560
   ggttttatgg gtgtacgttg ggttattatt aagaatttta agttgttggt gtgtaaaggt 1620
   ttgggtttta gattgtattg atggattttg tttatacgtg tgtgttattg tgtttgtttg 1680
   gaatttggtg atttggaatg 1700
<210> 92
   <211> 1700
   <212> DNA
   <213> Artificial Sequence
<220>
   <223> chemically treated genomic DNA (Homo sapiens)
<400> 92
   ggttttttgg tagtttaagt gttagtttag atattgagat ttcggtaaag agtttttttt      60
   tatttttttt ttttattggt ttttgtgggc ggtgtggaat gtgttgtagg ttttgcgaga    120
   gcggtatttt tttttttttt tttttttttt ttttttaatt tattgtagtt tttgagaggg    180
   tagtattttt gggcgtattt gtatttaagt ttttgatagt gttttgggtt tttttagtaa    240
   cgcgtattta gggatgttat gttttgtggg ttttatgttc ggggtacgga gttaggggag    300
   gcggttattt tatttttttt acgtttttgt ttattttgcg ttttagcgga gttttaggta    360
   aataagttag gtttttagtt tttaaggacg gtttttattt tagattttag gaggaggcgt    420
   tttgggttat ttcgggaagg ttagtttgtt cgggaggggt agattacgaa aacggttttt    480
   taggtagtgt tatttttttt ttattgttag ttatttaatt taggcggttt tatttttttc     540
   gtttttatgg atatgtagta ggatgtagag agtatagagt tgtgattggt acgagtttat    600
   aacgggtttt ttcgtttaat ttatttaatt tttagtaagg gataggtttt gtttattggg    660
   gttcgttgag gttttaaacg cgtatgttgt taagggagta gttgcggggg ttcgttagtg    720
   gggataaagg cgtagttatc ggggcgaaag tttcgtttcg tagagggtgg gtttgcggcg    780
   atcgcgagtt tagagttcgg atttgtcgcg taatttgttt ttttcggttt tattttaatt    840
   tagttggatt ttcggttcgg tttttgtttt ttggagattt agttattaaa gttgaggtgg    900
   ggggatgagg atgtgggcgg cgtttaggta attttgaggg gttttgatgt tttttttaga   960
   gttagttatt aggagtaata cgatgcgttt ttatattatc gaatttagaa ttttagtgtt   1020
   ttagtatttt tttttcgata tgagtttcgt ttaatgagga gaagggtttt ttgtattggt   1080
   cggtggtggt tttttattta attagagagg ttaacggttt tttttcgttt ggtttagaag   1140
   gaatcgtcgg gggaatatag gttaaattat aattttcgag aggtagttgg gttttgcggg   1200
   gggggggggg ggggcgtttg cgtgtttacg tgattttttt ttttttcgcg gagatcgagt    1260
   tacgcgatag gtttgtgagg gggcggggtt cggcgtttgt tcgtcgtgtt agtggttaat   1320
   cggtagttag gtagggtggg cgcgcgtagg gggcggggtc gggcgcgcgg tagggcgcga   1380
   gagcgtattc gcggcggcgg tggcggcgat tgtggggggg cggcggggaa tattggttaa   1440
   gtcgatagtg gaggtttagg tatcggtggc gggcggttgc ggtttttggt gttgttcggc   1500
   gcgcggttag ttttcggaac ggaacgttcg gcgtcgcggg tttcgttcgg aaagtttgtc   1560
   gtggagtcgc gattttttgg ttcgcgcggt tcggtatgaa gcggcgttga ggagttgttg   1620
   tcgtcgtttg tcgttgtcgt cgtcgtcgtt tgaggaggag ttgtagtatt ttgggttacg   1680
   tcgatgatta ttgtaaattg                                               1700
<210> 93
   <211> 1700
   <212> DNA
   <213> Artificial Sequence
<220>
   <223> chemically treated genomic DNA (Homo sapiens)
<400> 93
   tagtttgtag tagttatcgg cgtggtttag ggtgttgtag ttttttttta ggcggcggcg      60
   gcggtagcgg taagcggcgg tagtagtttt ttaacgtcgt tttatgtcgg gtcgcgcggg     120
   ttaagaggtc gcgattttac ggtaaatttt tcgggcgggg ttcgcgacgt cgagcgtttc     180
   gtttcgaaag ttggtcgcgc gtcgagtagt attaggaatc gtagtcgttc gttatcggtg     240
   tttaagtttt tattgtcggt ttagttaatg tttttcgtcg tttttttata gtcgtcgtta    300
   tcgtcgtcgc gggtgcgttt tcgcgttttg tcgcgcgttc ggtttcgttt tttacgcgcg     360
   tttattttgt ttggttgtcg attggttatt aatacggcga ataggcgtcg ggtttcgttt     420
   ttttataggt ttatcgcgta attcggtttt cgcgagaagg aaggaagtta cgtgaatacg     480
   taggcgtttt tttttttttt ttcgtagagt ttagttattt ttcgggaatt gtagtttggt     540
   ttgtattttt tcggcgattt tttttaaatt agacgagaga aggtcgttag tttttttaat    600
   tggatagggg attattatcg gttaatgtag aagatttttt tttttattgg gcggagttta     660
   tgtcgaggaa ggggtgttga agtattgagg ttttgggttc ggtgatgtgg gggcgtatcg     720
   tgttattttt gatggttggt tttagggaag gtattagggt tttttagagt tatttggacg     780
   tcgtttatat ttttattttt ttattttaat tttaatggtt gagtttttag agggtaggga     840
   tcggatcgag agtttagtta ggttggggtg gaatcgaagg agatagattg cgcgataagt     900
   tcggattttg agttcgcggt cgtcgtaggt ttattttttg cggggcggag ttttcgtttc     960
   ggtggttgcg tttttgtttt tattagcggg ttttcgtagt tgtttttttg atagtatgcg   1020
   cgtttgaggt tttagcgaat tttaatgggt agggtttgtt ttttgttgga agttgagtag    1080
   attgagcggg aaagttcgtt gtgagttcgt gttaattata gttttgtgtt ttttgtattt    1140
   tgttgtatat ttatgaaagc gggagggatg gagtcgttta aattgggtgg ttggtagtgg    1200
   agaggaagtg atattgttta aagaatcgtt ttcgtgattt gtttttttcg agtaagttag   1260
   ttttttcgag atggtttaag acgttttttt ttggggtttg gggtgaaggt cgtttttggg   1320
   gattgggagt ttggtttgtt tatttaaggt ttcgttgagg cgtaaggtgg atagggacgt    1380
   gagagaagta gggtgatcgt tttttttagt ttcgtgtttc gggtataggg tttatagggt   1440
   atagtatttt tagatgcgcg ttgttggagg aatttagggt attgttagag gtttgggtgt    1500
   aggtgcgttt aaaaatgttg tttttttaga ggttataata ggttaaaaaa aaagagagaa   1560
   aaaaaaaaaa gaaatgtcgt tttcgtaagg tttatagtat attttatatc gtttataaag    1620
   gttagtggga agagagggta aagggaagtt ttttgtcgag gttttaatgt ttggattgat    1680
   atttgagtta ttagggagtt                                                1700
<210> 94
   <211> 1700
   <212> DNA
   <213> Artificial Sequence
<220>
   <223> chemically treated genomic DNA (Homo sapiens)
<400> 94
   gttttggata ttttttgttt ttttgattta aggtttagtt tggttttttt tagggttttt    60
   tggtagttta agtgttagtt tagatattga gatttcggta aagagttttt ttttattttt   120
   tttttttatt ggtttttgtg ggcggtgtgg aatgtgttgt aggttttgcg agagcggtat   180
   tttttttttt tttttttttt ttttttttta atttattgta gtttttgaga gggtagtatt   240
   tttgggcgta tttgtattta agtttttgat agtgttttgg gtttttttag taacgcgtat   300
   ttagggatgt tatgttttgt gggttttatg ttcggggtac ggagttaggg gaggcggtta   360
   ttttattttt tttacgtttt tgtttatttt gcgttttagc ggagttttag gtaaataagt    420
   taggttttta gtttttaagg acggttttta ttttagattt taggaggagg cgttttgggt   480
   tatttcggga aggttagttt gttcgggagg ggtagattac gaaaacggtt ttttaggtag   540
   tgttattttt tttttattgt tagttattta atttaggcgg ttttattttt ttcgttttta   600
   tggatatgta gtaggatgta gagagtatag agttgtgatt ggtacgagtt tataacgggt   660
   tttttcgttt aatttattta atttttagta agggataggt tttgtttatt ggggttcgtt   720
   gaggttttaa acgcgtatgt tgttaaggga gtagttgcgg gggttcgtta gtggggataa 780
   aggcgtagtt atcggggcga aagtttcgtt tcgtagaggg tgggtttgcg gcgatcgcga 840
   gtttagagtt cggatttgtc gcgtaatttg tttttttcgg ttttatttta atttagttgg 900
   attttcggtt cggtttttgt tttttggaga tttagttatt aaagttgagg tggggggatg 960
   aggatgtggg cggcgtttag gtaattttga ggggttttga tgtttttttt agagttagtt 1020
   attaggagta atacgatgcg tttttatatt atcgaattta gaattttagt gttttagtat 1080
   ttttttttcg atatgagttt cgtttaatga ggagaagggt tttttgtatt ggtcggtggt 1140
   ggttttttat ttaattagag aggttaacgg ttttttttcg tttggtttag aaggaatcgt 1200
   cgggggaata taggttaaat tataattttc gagaggtagt tgggttttgc gggggggggg 1260
   gggggggcgt ttgcgtgttt acgtgatttt tttttttttc gcggagatcg agttacgcga 1320
   taggtttgtg agggggcggg gttcggcgtt tgttcgtcgt gttagtggtt aatcggtagt 1380
   taggtagggt gggcgcgcgt agggggcggg gtcgggcgcg cggtagggcg cgagagcgta 1440
   ttcgcggcgg cggtggcggc gattgtgggg gggcggcggg gaatattggt taagtcgata 1500
   gtggaggttt aggtatcggt ggcgggcggt tgcggttttt ggtgttgttc ggcgcgcggt 1560
   tagttttcgg aacggaacgt tcggcgtcgc gggtttcgtt cggaaagttt gtcgtggagt 1620
   cgcgattttt tggttcgcgc ggttcggtat gaagcggcgt tgaggagttg ttgtcgtcgt 1680
   ttgtcgttgt cgtcgtcgtt 1700
<210> 95
   <211> 1700
   <212> DNA
   <213> Artificial Sequence
<220>
   <223> chemically treated genomic DNA (Homo sapiens)
<400> 95
   ggcggcggcg gtagcggtaa gcggcggtag tagtttttta acgtcgtttt atgtcgggtc 60
   gcgcgggtta agaggtcgcg attttacggt aaatttttcg ggcggggttc gcgacgtcga 120
   gcgtttcgtt tcgaaagttg gtcgcgcgtc gagtagtatt aggaatcgta gtcgttcgtt 180
   atcggtgttt aagtttttat tgtcggttta gttaatgttt ttcgtcgttt ttttatagtc 240
   gtcgttatcg tcgtcgcggg tgcgttttcg cgttttgtcg cgcgttcggt ttcgtttttt 300
   acgcgcgttt attttgtttg gttgtcgatt ggttattaat acggcgaata ggcgtcgggt 360
   ttcgtttttt tataggttta tcgcgtaatt cggttttcgc gagaaggaag gaagttacgt 420
   gaatacgtag gcgttttttt tttttttttc gtagagttta gttatttttc gggaattgta 480
   gtttggtttg tattttttcg gcgatttttt ttaaattaga cgagagaagg tcgttagttt 540
   ttttaattgg ataggggatt attatcggtt aatgtagaag attttttttt ttattgggcg 600
   gagtttatgt cgaggaaggg gtgttgaagt attgaggttt tgggttcggt gatgtggggg 660
   cgtatcgtgt tatttttgat ggttggtttt agggaaggta ttagggtttt ttagagttat 720
   ttggacgtcg tttatatttt tattttttta ttttaatttt aatggttgag tttttagagg 780
   gtagggatcg gatcgagagt ttagttaggt tggggtggaa tcgaaggaga tagattgcgc 840
   gataagttcg gattttgagt tcgcggtcgt cgtaggttta ttttttgcgg ggcggagttt 900
   tcgtttcggt ggttgcgttt ttgtttttat tagcgggttt tcgtagttgt ttttttgata 960
   gtatgcgcgt ttgaggtttt agcgaatttt aatgggtagg gtttgttttt tgttggaagt 1020
   tgagtagatt gagcgggaaa gttcgttgtg agttcgtgtt aattatagtt ttgtgttttt 1080
   tgtattttgt tgtatattta tgaaagcggg agggatggag tcgtttaaat tgggtggttg 1140
   gtagtggaga ggaagtgata ttgtttaaag aatcgttttc gtgatttgtt tttttcgagt 1200
   aagttagttt tttcgagatg gtttaagacg tttttttttg gggtttgggg tgaaggtcgt 1260
   ttttggggat tgggagtttg gtttgtttat ttaaggtttc gttgaggcgt aaggtggata 1320
   gggacgtgag agaagtaggg tgatcgtttt ttttagtttc gtgtttcggg tatagggttt 1380
   atagggtata gtatttttag atgcgcgttg ttggaggaat ttagggtatt gttagaggtt 1440
   tgggtgtagg tgcgtttaaa aatgttgttt ttttagaggt tataataggt taaaaaaaaa 1500
   gagagaaaaa aaaaaaagaa atgtcgtttt cgtaaggttt atagtatatt ttatatcgtt 1560
   tataaaggtt agtgggaaga gagggtaaag ggaagttttt tgtcgaggtt ttaatgtttg 1620
   gattgatatt tgagttatta gggagtttta gagggagtta aattagattt tgggttagga 1680
   agatagggaa tgtttagggt 1700
<210> 96
   <211> 1700
   <212> DNA
   <213> Artificial Sequence
<220>
   <223> chemically treated genomic DNA (Homo sapiens)
<400> 96
   cgagggtttc ggtttttttt tttgtgtttg tttttcgttt agcgtcggga gaagtagtaa      60
   taagttttgt attttagtaa ttaattttag ttattatatt tgtattaatt agcgtcgttt     120
   aagtttcggg ttcggggcgg ggttcgtttt taaggtggtt cggggttttg gttgtcgaag     180
   ttttcgttac gaattcgtat tattttagtt ttggggtttt ttgcgttcgt tttttttcgg     240
   ggttcggcgt tagtgcgttg ggggcgcgtt tcggttttcg ggcgtatcga gttggagagg     300
   gtgtaggcgg aggagaatgg cgggagtttg gttttgttta tttttttttt atttatattg     360
   tttttaattt tttattttta attggagcga gatttatcgg gtgggtaggg ggcggggagg     420
   tgggcggtga gcggtacggg gcgtttagat aatgaggtcg ttaggtaaag attttagatt     480
   cgttacggaa agtcgttaag tgtttattgt atttcggttt cggggtgaat atttcgtttt     540
   tcgttagcgg cgtttaagtt agtcggtttt gggatttttt tggagggtaa agtagacggg     600
   gaatttcgaa gttagggttt ttttcgagag atttgggcgt ttgtttattt tttcgttatt     660
   agggcgttta taattgtttt ttttttttat tgtttggggt tttcggtttt tatcggtcgg     720
   cgtgggtttt aaggagttat agttatttag ttattttatg ggggagtata tcggttgagt     780
   ttagtaatcg gagcgatttg cgaggcggtt gggaatttcg agaagatacg tacgaagagg     840
   gcgtattcgt cgggtcgtat attttgtttt gggttattcg gtttaagatt tagatttttt     900
   gcgggtgcga agttggtttt tttaagagat taattaaatt gaagaggtcg aggaatggaa     960
   ttgggttgtg atattaaaat taataggagt aatttaatat ttgttttgtt ttttatgaaa    1020
   tatttatttt aataatttta gttaaaatgt ttagatcgtt ttgattttta tagaggttga    1080
   aatttatttt gtaattagaa gttggaagtt agaggtgatg gaggatagag gtgtttattt    1140
   tgtgcgtttg tgtgcgtgtg ttcgcgtttg cgtgcgtgtg tttgtgtatg cgtgtgtgtg    1200
   tgtgtgtgtg ttttttttta aagtgtgtgt agggaaggag gggggagaag gtttttaatt    1260
   ttttttgtta taaaatagta cgaggcgatt ttgttagtag attcgtattt ttaggagaag    1320
   ggcgaaaaaa gaattttttt tgaagtgttt ttgatatcgt tattaaattt aaataaaatt    1380
   aagcgtggtg tatgaaaatg tttttttttt tttataaaag aaagtgttta tcgttcgagt    1440
   ttgtttagcg tttgttaaat tacgtatgaa atttgaaatg aaataaacgt tataataaaa    1500
   ttaatttttg agtttttttt atttaaaaat tttagattaa gtattttatc gtcgcgggta    1560
   ggagttaaaa agttattatg tcgtacgcgt agatttattg tttttttgag ttgcggttag    1620
   gaaggtagtg ttaagtatgt tagaatcgat atgtcggggg tggggggaaa tgggtttaat    1680
   tttggggtag cggtagaatt                                                1700
<210> 97
   <211> 1700
   <212> DNA
   <213> Artificial Sequence
<220>
   <223> chemically treated genomic DNA (Homo sapiens)
<400> 97
   aattttgtcg ttattttaaa gttgagttta ttttttttta ttttcggtat gtcggttttg      60
   gtatatttag tattgttttt ttagtcgtaa tttaggggag taatgaattt gcgcgtacga     120
   tatggtaatt ttttaatttt tgttcgcgac ggtggagtgt ttaatttaag gtttttaaat     180
   aaaaagggtt taggggttgg ttttattata acgtttattt tattttagat tttatgcgta     240
   atttagtaaa cgttagatag attcgggcga taaatatttt tttttgtggg gggggaaagg     300
   tatttttata tattacgttt aattttattt agatttaata gcgatattag gggtatttta     360
   aagaaagttt ttttttcgtt ttttttttaa gaatgcgaat ttgttagtaa aatcgtttcg       420
   tgttatttta tggtaaggaa aattaaaggt tttttttttt tttttttttt atatatattt        480
   tagggaggaa tatatatata tatatatacg tatgtataaa tatacgtacg taggcgcgag     540
   tatacgtata taggcgtata gaataaatat ttttattttt tattattttt aatttttaat     600
   ttttaattgt aagatagatt ttagtttttg tggggattag gacgatttaa atattttaat      660
   taagattatt gggataaata ttttataaag agtagggtag atattaaatt atttttattg     720
   attttagtat tatagtttaa ttttattttt cggttttttt agtttaattg attttttaag     780
   gaggttaatt tcgtattcgt agagggtttg ggttttggat cgggtggttt agggtaaggt     840
   gtgcggttcg gcgggtgcgt ttttttcgtg cgtgtttttt cggggttttt agtcgtttcg      900
   taagtcgttt cggttattgg gtttaatcga tgtgtttttt tataaagtgg ttgagtgatt     960
   gtgatttttt ggagtttacg tcggtcggtg agaatcggga attttaggta gtggggaagg     1020
   ggaataattg tgaacgtttt agtgacggag gagtgagtaa gcgtttaggt ttttcgagga    1080
   gaattttaat ttcgaggttt ttcgtttgtt ttatttttta agaaaatttt agggtcggtt    1140
   aatttgggcg tcgttagcgg agaacggagt gtttatttcg gagtcgaagt gtagtagata    1200
   tttagcgatt tttcgtggcg agtttagggt ttttgtttgg cgattttatt gtttgagcgt    1260
   ttcgtatcgt ttatcgttta ttttttcgtt ttttgtttat tcggtgggtt tcgttttagt     1320
   tgggggtggg gagttgggaa tagtgtgaat gggaaggggg tgaataaagt taagttttcg    1380
   ttattttttt tcgtttgtat tttttttagt tcgatgcgtt cggaggtcgg ggcgcgtttt     1440
   tagcgtattg acgtcggatt tcgaggagag gcgggcgtag gggattttag agttggggta    1500
   gtgcgggttc gtggcggggg tttcggtagt taggatttcg gattatttta agagcgagtt    1560
   tcgtttcgag ttcggaattt gggcggcgtt gattggtgta aatgtaatgg ttgaaattga    1620
   ttgttgaaat gtaaaatttg ttgttgtttt tttcggcgtt gggcgagagg tagatataga    1680
   gaggggagtc gagattttcg                                                1700
<210> 98
   <211> 1700
   <212> DNA
   <213> Artificial Sequence
<220>
   <223> chemically treated genomic DNA (Homo sapiens)
<400> 98
   ttttacgggg attttcgtta atgtttggtt gagaggcggt tttagggggt tgagaatttt 60
   atttttagag gtttggtatt tgggagattg agaaagtttt ttttaagttt ttttgttggg 120
   ttacgttgta tttatagatt atttttaatt tttttttttt ttttatttat tttttcgttt 180
   ttattttttt ggtttatttt ttgttagaaa atgtattaag ttttaatttt cgtttttttt 240
   agtttttttt ttgagaagat attgtgattt cgttgttttt gttcgtgtta ttatggagag 300
   gtaggaggat aaatatcgtt tttagttgtt agtttttttg tttttttttt ttttttttag 360
   atagttttta gttttaattt tagtattcgt tttattattt ttttttggag aattagtata 420
   atcggagtaa ataggtaatt aagtagtgtg tttgtaagtt ttaattttgg gatagataag 480
   gaagtgaaat tgattggaat tatatttgtc gttgttggag gttgagggag gcggtcgtcg 540
   gaggcgaggt tagttttgtc ggtttagggt ttcgttttat tttttttttt tttttttttt 600
   ttttttagtt ttattttttt ttattttttt ttattttgta ttttttttta gcggtcgttt 660
   gtttttttta aggaatttta ttttaggtgg agtttttacg atagttatat tttttttttg 720
   ggtttagtag ggttagagaa agacggaatt attttattta tttaggttgt ttagagagta 780
   ttttaggttt tgattcgcgt ggggcgcgag gtatttcgtg gggtagttag gtgtttttaa 840
   gaaatttcgt tttagaggat tcgtacgagt tgttgttatt tttcgttgaa gtttttgttt 900
   tggggttggt gttttttttt atgattttat taatcgtcgg tatttttttt tttttttttt 960
   tttttttagg aaggcgttgg ggtgtgcggc ggtaacggcg gcggtgaagg gatttttttg 1020
   tggtattttt tcgtgggttt ttgttttgaa ggattttttt ttcgttttaa ttttatgttt 1080
   aagtagcgtt ttgggggttg ggttggtttt tatagttttt attttcggtt tttttttttt 1140
   aaagggtgtg tggagtttag aggatgggtg gagagcgggt ttggtcggaa ttgaattggt 1200
   agaggtaaaa ggggaacgga ggattttaag ttttggttat ttcggtttag cgaatttatt 1260
   ttatttttgt ttaaggaaaa gttgtagggg ggaggatttt ttttaggaag tttattttgt 1320
   cggtttttga tttttgggtt ggatgtttga gagttttgtt ttttttgttt cgagagtggt 1380
   ttagttgggt ttgaatcgat tttttttttt ttcgattttt tttttatttt ttagttcgga 1440
   gaaatagggg agtgggggtt taagaacgag aagacgagaa cgcgtcgttt tgcgttatgt 1500
   tagaatgggg cgggtgtgag gggaatagtt tttttgcgat tagtttagga gtatgagttt 1560
   ttcggaggac ggtatgagtt ttggatattt taggagtttt tagttagtga tatggtcggt 1620
   aagtgatttt gggattaata tttatttatt tttatttttt tcgtttagat tttatttttt 1680
   ttttagtttt tttgtttttt 1700
<210> 99
   <211> 1700
   <212> DNA
   <213> Artificial Sequence
<220>
   <223> chemically treated genomic DNA (Homo sapiens)
<400> 99
   ggggagtagg gggattggga gggggataga atttagacga aagaggtggg agtgggtggg 60
   tattagtttt agagttattt atcgattatg ttattagttg aggatttttg aagtgtttag 120
   aatttatgtc gtttttcggg aggtttatat ttttgagttg atcgtaagag agttgttttt 180
   tttatattcg ttttattttg atatagcgta gggcgacgcg ttttcgtttt ttcgtttttg 240
   ggtttttatt tttttatttt ttcgggttgg aaaatgagga gggggtcgaa aggggaggga 300
   gtcgatttag atttagttgg gttattttcg gaataaggga aataaagttt ttaagtattt 360
   aatttaaaaa ttaggggtcg gtagagtaga ttttttaaaa agaatttttt tttttgtagt 420
   ttttttttgg ataaaaataa agtgggttcg ttgggtcgaa gtggttagag tttaaggttt 480
   ttcgtttttt ttttattttt attaatttag tttcggttaa gttcgttttt tatttatttt 540
   ttgaatttta tatatttttt aaaaggagag ggtcgggggt gggggttatg aaaattaatt 600
   taatttttaa agcgttattt gaatataaag ttgaggcgga gaagaaattt tttaagatag 660
   gaatttacga ggagatatta taagaggatt tttttatcgt cgtcgttgtc gtcgtatatt 720
   ttagcgtttt tttaaaaaga aagaagggag gaaaaaaatg tcgacgattg gtagaattat 780
   agagaagaat attagtttta gggtaggggt tttagcgaaa aatggtagta attcgtgcgg 840
   gttttttggg gcggggtttt ttggaggtat ttagttgttt tacggggtat ttcgcgtttt 900
   acgcgagtta gaatttaaag tgttttttgg gtaatttgag tgggtagagt gatttcgttt 960
   ttttttggtt ttgttaggtt taaaggagag gtgtgattat cgtgaggatt ttatttaggg 1020
   tggagttttt taaaaaggat aggcggtcgt tagaggagag tgtaaagtgg gaggaggtgg 1080
   agaagggtgg ggttgagaag gaggagggag gaaggagggg gtggggcggg gttttgggtc 1140
   gatagagttg gtttcgtttt cggcggtcgt tttttttagt ttttagtagc ggtaggtata 1200
   attttaatta attttatttt tttatttgtt ttaaaattaa ggtttgtaag tatattgttt 1260
   gattatttat ttatttcggt tatgttaatt ttttagggga gggtggtgaa acgggtattg 1320
   ggattggggt tgggggttgt ttaagaggga aaggagaaaa taaaaaagtt gataattgga 1380
   agcgatattt atttttttat ttttttatgg taatacgaat aaggatagcg aggttatagt 1440
   gtttttttag ggaggaagtt aaaaggaacg gagattggag tttggtgtat tttttagtaa 1500
   gagataagtt aaggaggtga gggcgaagga gtaggtgggg aagaagaagg agttgaagat 1560
   gatttgtgaa tatagcgtgg tttagtaggg agatttaaga aaggtttttt tagtttttta 1620
   gatattagat ttttaagaat agagttttta attttttgag atcgtttttt agttaggtat 1680
   tggcgaagat tttcgtaaag 1700
<210> 100
   <211> 1700
   <212> DNA
   <213> Artificial Sequence
<220>
   <223> chemically treated genomic DNA (Homo sapiens)
<400> 100
   ttagatagta gaagtttata ttttttggag gtggaagttg gaggggaagg gtttgaggta 60
   gagataaaag taagagaggg attttttagg tttttttggg gacgggagga tggtgtttgt 120
   ttagttttgc gtttgtaatt tttacgttat atatattata gttatgtttg ggttacgttt 180
   gtttagagtt tatcgggtat gtatatatgt gatatgttcg aagttgaggg taagttattt 240
   agggtagagt atgtttttag gttttgcgtt tgttttaggg tagttgtaat gtagtaagcg 300
   tgttattgtt acggaggtaa gaaagttagc gtttttttac gtacgtatat aggttaggtg 360
   gttttttttg tataagatgg gttattaata aatttattta tgttatatat ttgtttttta 420
   tcgtttttat tttattcgta aagtatgggg aaaagaaagt aaaagttttt ttcgatttgg 480
   tttttttttg cgtttttttt tggagtcgaa gagtttgatt cgggaaaaga tatttaattt 540
   gtttttgtcg tagttggttt tgtttttgtt ggggttgttg atatatttgt aggcgttgga 600
   tttttcgcgt tcgcgggttt gttttttttg acggatgagc gagttggcga tagtcgtcgt 660
   tatggttacg acgtttatta ttatcgtttt ttttgttgtt tttttttggg ttcgtttttt 720
   tttttttttt tttcgttgtt tgttcgttgt ttcgcgattt cgtcgttttg gtttttttag 780
   tttgcgtttg ttcgggtttt ttcgtattcg ggttttagtt aaggaggggg tttagtatgt 840
   cgttcgagtt ttttcggcgg cggttcggtt ttcgcgcggg ttgttggttg tttgggtcgg 900
   agtcgacgtt atagtttcgg gttcgggatc gcgggcgaga ttggtacgcg gatgttgaat 960
   tgcgcgattg cgtgtggtcg gtttttgcgt tcggcggata tcgtgtatgt ttagttgttt 1020
   cggttcgaat ttcgtcggat tttttcgttt tgttgttttt ttgatggggg ttagaggagg 1080
   gaggcgggcg gagggagtga gcgcgggcgg gagagaggtc gggagttcgg gcggtcggac 1140
   ggaggaggga cgaggtagcg cgcgggggag cgcgttttcg ttttggtttt ttcgagtttt 1200
   cgattagttt ttgtaatttt ttggcgtgat aatcgggaaa agttggttcg tgttaggttt 1260
   tcgataggga ttaaataggt aatggtttcg tattttcgtt gttgttgttg ttttgggcgc 1320
   ggtatagtcg tagtatagga atttagtcgt cgtaggtatt tttcggaata gcgcgatagt 1380
   ttttttgtag ttttttttcg cgacggtttt tttttttcgg ggttgagttc gtaggttcgg 1440
   agtcgtaggt cgtttttttt atttttattt ttatttttat ttttttaagt tttcggtcgt 1500
   tcgtttcggt taatatttgt aggggttttc gtattgtttt agtcgtttaa attttttttt 1560
   tttttttttt tcgaaaggag tttcgggagg aaatagtgat ggaggaggaa ttgagatgag 1620
   gtagttgggt tgacggagcg ggggtttgtt cggtattggg ggatggagtg gtgggggtgt 1680
   ttttttagtg tttgagttga 1700
<210> 101
   <211> 1700
   <212> DNA
   <213> Artificial Sequence
<220>
   <223> chemically treated genomic DNA (Homo sapiens)
<400> 101
   ttagtttaaa tattgaggaa atatttttat tattttattt tttaatgtcg agtaaatttt      60
   cgtttcgtta gtttaattat tttattttag tttttttttt attattgttt tttttcgaag     120
   tttttttcgg gaaaaggagg ggagaaggat ttggacgatt ggggtagtgc ggaagttttt     180
   ataggtgtta gtcggaacgg gcggtcgggg atttgggggg gtgggggtgg gggtgggggt     240
   gggaggggcg gtttgcggtt tcgagtttac gggtttagtt tcgggaggga ggggtcgtcg     300
   cgggaggggg ttgtaaggag gttgtcgcgt tgtttcggag ggtgtttgcg gcggttgaat     360
   ttttgtgttg cgattgtgtc gcgtttagag tagtagtaat agcgaagatg cgaggttatt     420
   atttgtttga tttttgtcgg aaatttggta cgggttaatt tttttcgatt attacgttaa     480
   gaagttgtaa ggattagtcg aagattcgga ggggttaggg cgagggcgcg ttttttcgcg    540
   cgttgtttcg tttttttttc gttcggtcgt tcgagttttc ggtttttttt tcgttcgcgt     600
   ttattttttt cgttcgtttt ttttttttgg tttttattag aagggtaata gggcgagggg     660
   gttcggcgaa attcggatcg gagtagttgg atatgtacgg tgttcgtcgg gcgtaggggt     720
   cgattatacg tagtcgcgta gtttagtatt cgcgtgttag tttcgttcgc gatttcgggt     780
   tcggggttgt ggcgtcgatt tcgatttagg tagttagtag ttcgcgcggg agtcggatcg     840
   tcgtcggagg agttcggacg gtatgttgag tttttttttt ggttgaagtt cgagtgcgga     900
   gaagttcggg taaacgtagg ttaaggagat taaagcggcg aagtcgcgag atagcggata     960
   agtagcggag gagaaggagg aggaggcgaa tttagagagg ggtagtaaaa gaagcggtgg    1020
   tggtgggcgt cgtggttatg gcggcggtta tcgttagttc gtttattcgt tagaagaggt    1080
   aagttcgcga gcgcgagaaa tttaacgttt gtaagtgtgt tagtagtttt agtaaaggta    1140
   agattagttg cgataaaaat aagttaaatg ttttttttcg ggttaaattt ttcggtttta    1200
   agaagaggcg tagaagaaga ttaggtcgga aagggttttt gttttttttt ttttatattt    1260
   tacgagtgag gtgggggcgg tggggagtag gtatgtagta taggtgagtt tgttggtggt    1320
   ttattttgtg tagaagaggt tatttagttt gtgtgcgtgc gtgggaaggc gttggttttt    1380
   ttgttttcgt aatagtaata cgtttgttgt attgtaattg ttttgggata ggcgtagagt    1440
   ttgggggtat attttgtttt gggtggtttg tttttagttt cggatatgtt atatatgtgt    1500
   atgttcggtg ggttttgggt aggcgtgatt tagatatggt tgtgatatgt atggcgtgga    1560
   aattgtaggc gtagagttgg gtagatatta ttttttcgtt tttaggagag tttggggaat    1620
   tttttttttg tttttatttt tgttttagat tttttttttt tagtttttat ttttaaggga    1680
   tgtgggtttt tgttgtttag                                                1700
<210> 102
   <211> 1700
   <212> DNA
   <213> Artificial Sequence
<220>
   <223> chemically treated genomic DNA (Homo sapiens)
<400> 102
   tttttttttt tttttttttt tttttttttt tttttttttt tttttttttt ttgtttagcg      60
   aggagtttag attagagagg tggtttagat agtttattaa atggtaagat tagattagtt     120
   tttaaattaa gataatttat tttgtttttt tttttttttg aaatatatat tttttgtttt     180
   tagagatttt aaaatttttt gatgatttat atatttattt gaatttttgt tttttgataa     240
   aagttattat gcgtattttg gaaaaagtga agaaaagtta aaagaaaata tttatttaga     300
   gataatattt tagtggattt ttttgttttt atttttgtat atatgagttt atagggtata     360
   tatgtttgta tatgtttaaa tttatattat aagtatgttt agtttatttt tagaattttt     420
   taaataaata taatttaaaa taggaattta atattttatg ttattgatag atcgtagtag 480
   attgtgtttg tatttttaga taatattttt ttttattatt gtaaataaga ttttattttg 540
   tgtttttaat ttttattttt gttaatattt tttgttattt ttttaggata tatatgtaga 600
   agaaatatta attagttaaa tattttaatg tttataagat atgttatttt agggtttttt 660
   agaaaaatag tattatcgta ttttgttttc ggaggtgtag gcgagtgttt ggtttacggg 720
   tgtttataat tattatcggg tatttataag agattttgaa tttagaatat tttcgcggta 780
   ggagtttcgt ttcgtttttt taggggatat tgacgattcg ttgggttttc gttttttaag 840
   tgttggaggg gggaagtagg gttattcggg gtagaaattt aacgttagaa attttttagc 900
   gttttattag ttggtggaat tttcgtggta gatagtcgcg ttttatttaa gtcgtagaag 960
   aaaagatatt ttgtggggga tttggcgtcg gaagttttga gtttaaatcg tagttttagg 1020
   tttttagttt tgtttttttt ggtttgttgc gaggatgaaa tgagattttt gtacgtgaaa 1080
   atgtttttta atttttttcg aggggtggaa acgtttttat tcgattagag ttcgggtttg 1140
   cggagtaatt tattcgtcgt tttttaagag taggtttttt agcgtagcga gtttttgtta 1200
   tggaaagggt cgtttcggtt taggatgcgc gttttcggcg tagatttggg gataggggtt 1260
   tttgtcgcgt tcgttttatt tttgtaggga gtaaggtcgt ttttttgtag tatcgcgtta 1320
   cgcggcggga atttgtagtg ttttgttttt tggttttagt tttttcgttc gtttgggttt 1380
   ttttcggtta tttttcggag gggtttggag tttttttttt ttttttttat tttagcgtta 1440
   gaagtattga aagtcgttcg ttcgtcgaga gtggaatcgg tatttcgaat ttcgtttatt 1500
   tcgggttaat tggaggcgta ttagcggttg ttttgtagtg taatagaggt tcgagtgata 1560
   ggttatttgt ttaagcgttg gcgtttagga aagtcgggga ggaaattagg aagggaggcg 1620
   atagtagaaa agttagagag tttagagatt tttaggtagg ttttcgtgta gttttaatag 1680
   cgtggtgtcg ggaggtgtgt 1700
<210> 103
   <211> 1700
   <212> DNA
   <213> Artificial Sequence
<220>
   <223> chemically treated genomic DNA (Homo sapiens)
<400> 103
   atatattttt cggtattacg ttgttggggt tgtacggagg tttgtttagg agtttttgga 60
   ttttttggtt tttttgttgt cgtttttttt tttagttttt ttttcggttt ttttgggcgt 120
   tagcgtttag gtaggtggtt tgttattcga gtttttgttg tattgtaagg tagtcgttaa 180
   tgcgttttta attgattcgg aatgagcgag attcgaggta tcggttttat tttcggcggg 240
   cgggcggttt ttaatgtttt tggcgttgaa gtaggaggag ggaggaggga ttttaggttt 300
   tttcgagagg tgatcgagag agatttaagc gagcgaagaa gttgaaatta gaaagtagga 360
   tattgtaaat tttcgtcgcg tggcgcggtg ttgtagaagg acggttttgt tttttgtagg 420
   ggtggggcga gcgcgatagg gatttttatt tttaggttta cgtcgggagc gcgtattttg 480
   agtcgaaacg atttttttta taataaggat tcgttacgtt ggggagtttg tttttggaaa 540
   gcggcgggta aattgtttcg tagattcgga ttttggtcga atggagacgt ttttattttt 600
   cgagaggggt tagaaagtat ttttacgtat aaaaatttta ttttattttc gtagtaagtt 660
   aggaagggta aggttgggga tttagggtta cgatttgaat ttaaggtttt cgacgttaag 720
   ttttttataa ggtgtttttt tttttacgat ttaggtagag cgcgattgtt tgttacgaaa 780
   attttattaa ttgataaggc gttggaaggt ttttggcgtt aggtttttat ttcgggtggt 840
   tttatttttt ttttttagta tttggaaggc gagaatttag cgagtcgtta gtattttttg 900
   gaaggacgag gcggaatttt tatcgcggaa atattttaag tttagggttt tttgtgggtg 960
   ttcggtgatg attgtaaata ttcgtagatt aggtattcgt ttgtattttc gggaataaaa 1020
   tacgataata ttgttttttt aggaagtttt ggaatggtat gttttatgag tattaaaata 1080
   tttgattgat tgatgttttt tttatatatg tattttgaga aaataataga aaatattgat 1140
   aaggatgaaa attgaaggta taaaataaag ttttatttat agtgataaaa aaagatatta 1200
   tttaaaaata taaatatagt ttattacggt ttattaataa tatgaaatat tagattttta 1260
   ttttaaatta tatttgttta aaaaatttta aagatgaatt gggtatgttt atgatgtaaa 1320
   tttaaatata tataaatata tatattttat gagtttatat atatagaaat aaagataaga 1380
   aggtttatta aaatgttgtt tttgggtgga tatttttttt taattttttt ttattttttt 1440
   taaggtgcgt atgatgattt ttattaggaa ataaaaattt aaataaatgt atgggttatt 1500
   agaagatttt gagattttta agagtaaagg atgtatattt taaaaagaaa gaaagatagg 1560
   gtaaattgtt ttggtttaga ggttagtttg gttttgttat ttggtgaatt atttgggtta 1620
   tttttttgat ttggattttt cgttgaataa aagaagaaga aggagaagga gaaggaggag 1680
   gaggaggagg aggggaagaa 1700
<210> 104
   <211> 1700
   <212> DNA
   <213> Artificial Sequence
<220>
   <223> chemically treated genomic DNA (Homo sapiens)
<400> 104
   ttattaaaaa agcggagggg gttttgttta atatttgtat atattttttt tcgtagtatt 60
   tcgtttattt tagtttttta gggaaattat agataacgac gttttagaga gtgttagagg 120
   tgaagtcgat tcgaaattat attatagaaa ggttttttgt tttaaatagg tttaataaag 180
   ttattaaatt aagtttacga ttataaagaa ttgcgaatta gaaaataatt ttatataggt 240
   gtagtttttt attatttttt tttttatttt cggaaatttt aaagaaaaat tgttatattt 300
   ttaaatatga ggttattatt ttaaaggttg taataatttg gaacgtaaat tcgttaaatt 360
   atgatataat aaggttaatt tcgaggattt tcggtggatt attagcgtat ttttttataa 420
   ttagaaagtg aaaaattggg ttagttttag agttacgtaa taatgttgcg ttacgaaatt 480
   gcgtatttta agagaaattt tttttaggtt tagaattaag tcggagggat tcgtcgcgta 540
   gtttaatttt taggaagtat gaaagaatgt attgtaaaga gtaaaattac gcgtaattta 600
   ttttaatata atcgtagttg ttcgtgggtt tttttgtaga gaaaggttgt ttaattttga 660
   cggggttatg ggcgttgcgg gagggtaaaa gattatagag atttgcgaag ttaaagtaaa 720
   taacggggtt ggggcggcga gaatgattaa tagcgatgtt tcgaaaggat ttcgcgatag 780
   gaatcgagcg ggaaggattt tttttatttt aatagtttat gggttaagag gtcgaagttt 840
   ttttaaatat tatattttcg cgagagaagg gattgagaat aattttttaa attatttttt 900
   ttcgaaatcg tttttttttt cgaaaatttt atttttgaga ttcggatgag gtttttattt 960
   tttttatttt tgtttcgtga tcgtcgttcg tttagttttt tagtcgagtt cgcggggttt 1020
   ggggcgttta tttcgtttat ttaagggatc gcgtaggggt gaattttttc gcgttttatt 1080
   tgcgtttttt agatttcggg cgtttcggcg ttgtttcgag agttttttgc gcggtcgtcg 1140
   cggtacggat tagtttttat ttttttatat tgggcgtcgt tgcgttcgtc gggggtcggt 1200
   ttcgaggttt ttaaggtttc gcgcgcgcgt ttgtcgtggt aattaagacg ttttacgacg 1260
   cgcgttttcg aacgtttcgc gttacgcggt cgcgcggttt cgttcgtcgg tttcgttttc 1320
   gttatagagt tcgtagtacg tcgtcgtcgt agtttaggtt acgtgagtat ttacgcgcgc 1380
   gttttgttag cggatttatt atcggtttgt ttagattagg ttttgtttat tttgattttt 1440
   taaatggtac gtgggaggac gttcgttttt ttcggattta agagttatcg taatatttta 1500
   gaaggggaga aaaggagcga gggcggtagg cgatagagaa tttcgcgagt tagcggtttc 1560
   gcgtagattt ttttaggtac ggttttttgc ggttacgtcg gttgttcggc gtttgcgtaa 1620
   tttttttttt tttagcgaaa tcgaggtttt cggagagttt agtagagagt gtgggtagtg 1680
   agcgtttgta gtcgttagag 1700
<210> 105
   <211> 1700
   <212> DNA
   <213> Artificial Sequence
<220>
   <223> chemically treated genomic DNA (Homo sapiens)
<400> 105
   ttttagcggt tataagcgtt tattgtttat attttttatt aggtttttcg gaggtttcgg 60
   tttcgttgga gagaaagaga ttgcgtaggc gtcgagtagt cgacgtggtc gtaggggatc 120
   gtgtttgggg gggtttgcgc ggggtcgttg attcgcgagg ttttttgtcg tttgtcgttt 180
   tcgttttttt tttttttttt tagagtgtta cggtgatttt tgggttcgaa ggggacggac 240
   gtttttttac gtattattta gaaggttaga gtgggtagaa tttagtttga gtaggtcggt 300
   gatgaattcg ttggtaagac gcgcgcgtgg gtatttacgt gatttaggtt gcggcggcgg 360
   cgtgttgcgg gttttgtggc gggagcgagg tcgacgggcg gggtcgcgcg gtcgcgtgac 420
   gcgaagcgtt cgagagcgcg cgtcgtggaa cgttttggtt gttacggtaa gcgcgcgcgc 480
   gaggttttgg gaatttcggg atcggttttc ggcgagcgta gcggcgttta gtgtaaggga 540
   gtgggagttg gttcgtgtcg cggcggtcgc gtagggagtt ttcgaggtaa cgtcggggcg 600
   ttcgaggttt ggaaggcgta ggtggggcgc gggggagttt atttttgcgc ggttttttgg 660
   gtgggcgggg tgggcgtttt aggtttcgcg gattcggttg ggaggttgag cgggcgacgg 720
   ttacgggata agggtggagg gggtggggat tttattcgag ttttagagat ggaattttcg 780
   aggaagggag cgatttcgag agagaatagt ttgagaagtt gtttttagtt ttttttttcg      840
   cgggagtgtg gtatttaggg aagtttcggt tttttaattt atggattgtt aggatggagg      900
   gaattttttt cgttcgattt ttatcgcgga gttttttcgg agtatcgtta ttgattattt        960
   tcgtcgtttt aatttcgttg tttattttgg tttcgtagat ttttgtggtt ttttgttttt     1020
   tcgtagcgtt tatggtttcg ttaaggttgg gtagtttttt tttgtaagaa ggtttacgaa     1080
   taattgcggt tatattggga tggattgcgc gtgattttat tttttataat atattttttt     1140
   atgtttttta gggattgagt tgcgcggcgg attttttcga tttaattttg ggtttgagaa      1200
   aagttttttt tggaatgcgt agtttcgtga cgtagtatta ttgcgtgatt ttagggttga      1260
   tttagttttt tattttttaa ttgtaaggaa gtgcgttagt agtttatcga ggattttcga     1320
   ggttggtttt attgtattat aatttaacga atttgcgttt taggttgtta tagtttttga     1380
   aataatggtt ttatatttaa gaatgtagta attttttttt gaaattttcg aaagtgggga       1440
   aaaaagtaat agaagattgt atttatatgg aattattttt tgattcgtag ttttttataa      1500
   tcgtagattt aatttggtgg ttttattgga tttgtttgaa ataggaagtt tttttgtaat     1560
   gtggtttcgg gtcggtttta tttttggtat tttttgaaac gtcgttgttt atggtttttt     1620
   taagaagtta gaatgagcgg gatgttacgg agggaggtgt atataggtgt tagataaagt     1680
   ttttttcgtt tttttggtaa                                                   1700
<210> 106
   <211> 1700
   <212> DNA
   <213> Artificial Sequence
<220>
   <223> chemically treated genomic DNA (Homo sapiens)
<400> 106
   aatttaggtt tggtaggtta tttgtatata ttgggtggag gtggaagggg taaagtattt       60
   ttgaggtgat ggttttgatt ttattttata tttttttggg gtttggttgg atttggttga      120
   gcgtgtttga gtagtggttg atttttggaa aataggagga ttttttttta ggaggttgtg      180
   attattaagg ttgtaaatgt tttatttgaa tttgggtgta tgagggattt ttttttttta      240
   tatatatata tttttttatc gcgtaagata aatttttgtt tgtgagttgt ttatattaaa      300
   tgttttgttt ttgatttttt gtgattaaaa tttgtttcgt ttgtagggtt cggtgcgttt      360
   ggatttagtg tattttgttg gtatagtaaa aaagagaatt gtttttttat aggtgatttt       420
   cgttttaaag tttttttttt tattaatttt tttttgagaa gagagtaaaa tcgaattcga       480
   gagaaaagag gtagtaatta ttcgggagga ggtagattgg agtgggtatt agtttcggta      540
   gtaagagttt taacgttttt ttcgtttttt tttcgtagag tagtatggaa gtattagttt      600
   ttttttttgg gtttttaaaa gttatttttt ttttgggtgt gattttcgaa ttcgaagcgt      660
   ttgagtttta agaaattaga ataagttaga gttaaagagg tttggcgggc gggggtgcgg      720
   gtggagttcg ggtcgggtgg cgtggaaggt cgagtatagt tgcgagcggc gagtttcggg      780
   agaggaggag cgggagaaag cggggcgggt ttgcggtcgg ggttcggtcg ggggaggggg      840
   ttttcgtttt ttttttttta ttttcgtttt ttttggtttt ggtgacgttg cggttttttt      900
   ttttttttag cggagtttta gcgttcgtcg cgtttttttt ttcgtttttt tcgtttatat      960
   ttattcgttt tcgtcggttc ggtttttagt agcgtcgttc gaggttgtag tagcgtattt     1020
   cggggtatgg cgcggcgggg gcgcggaggg ttcggttcgg agggggtcgg gagttcgggc     1080
   gttttggagt gaggaggatc gggagttggt tttggaggtt gcggaggcga cgtcggagag     1140
   aacgaagttt cggttgggag cggtaagtgg aggggcgcgt aagggacgag ttggggcgcg     1200
   cggcgtaatt tttttcggga gtttcgggcg ggtagttttc ggggttgttt cggcgcgggc     1260
   gggagacgga gagttacgtt ttcggttgag agcggagcgt cgcgggttgc gcgttcgggt     1320
   tcggttcggt tggatttcga gagagcgttt gacgcgtgaa gtttgagtta cgggtttttt     1380
   cggcggcgga gtagtttcga ggggtagttt ttcgattttt tttatttttt tgaacgtcga     1440
   gtatcgagtc gggaatacga tgagaatttg gagatcgtat ttcgtgcgtc ggatttggtc     1500
   ggtttagtcg cgattttttc gacggttttt ggggtttcgg ttttattttg cggggttggg      1560
   tggtatattt tcgcgttttt tttttttttc gtattttagt tatttggggg taggggaggt      1620
   ggcggcggtt tgtgtgtaga ttttggcgtt ggtaatttta ttttgcggtt gtagaagtcg     1680
   aggaagagtt ttttaagttt                                                  1700
<210> 107
   <211> 1700
   <212> DNA
   <213> Artificial Sequence
<220>
   <223> chemically treated genomic DNA (Homo sapiens)
<400> 107
   aggtttgggg agtttttttt cggtttttgt agtcgtaaag tagggttgtt agcgttagga 60
   tttgtatata agtcgtcgtt attttttttg tttttaaatg gttggggtac ggagggaaag 120
   gaggacgcga gggtgtgtta tttaatttcg tagagtgaag tcgaggtttt aaggatcgtc 180
   gaggaaatcg cggttgggtc ggttagattc gacgtacgga atgcgatttt taggttttta 240
   tcgtgttttc ggttcggtgt tcggcgttta agggggtggg gaggatcgaa aagttgtttt 300
   tcgaggttgt ttcgtcgtcg gggaagttcg tggtttaggt tttacgcgtt aggcgttttt 360
   tcggagttta gtcgagtcgg gttcgggcgc gtaattcgcg gcgtttcgtt tttagtcggg 420
   gacgtggttt ttcgtttttc gttcgcgtcg ggatagtttc gaaggttatt cgttcgaaat 480
   tttcgaagaa agttgcgtcg cgcgttttag ttcgtttttt gcgcgttttt ttatttatcg 540
   tttttagtcg aggtttcgtt tttttcggcg tcgttttcgt agtttttaga gttagttttc 600
   ggtttttttt attttagggc gttcgggttt tcggtttttt tcgaatcgag tttttcgcgt 660
   tttcgtcgcg ttatgtttcg ggatgcgttg ttgtagtttc gggcgacgtt attgagagtc 720
   gggtcggcgg gggcgggtag gtgtgggcga aaggggcgga gggagggacg cggcgggcgt 780
   tgaggtttcg ttggaggggg gaggaaatcg taacgttatt agagttagag gagacgagga 840
   tgaggagagg gaaacgggga tttttttttt cggtcgaatt tcgatcgtag gttcgtttcg 900
   ttttttttcg tttttttttt ttcgggattc gtcgttcgta gttgtgttcg gttttttacg 960
   ttattcggtt cggattttat tcgtattttc gttcgttagg tttttttggt tttaatttgt 1020
   tttagttttt taggatttaa acgtttcggg ttcgagaatt atatttaaag gaaaggtaat 1080
   ttttggaggt ttagggggag gagttgatgt ttttatatta ttttgcgaga ggaaagcgag 1140
   gaagacgtta agatttttat tatcgggatt ggtgtttatt ttagtttgtt ttttttcgaa 1200
   tagttattgt tttttttttt tcgaattcgg ttttattttt tttttaaaga gaagttagtg 1260
   ggagggggga ttttggggcg ggggttattt atagagaagt agtttttttt tttattgtat 1320
   taataaaata tattaaattt aagcgtatcg aattttgtaa acggaataga ttttaattat 1380
   agaaggttag gagtaaagta tttaatgtag gtagtttata aatagaaatt tattttacgc 1440
   ggtggggggg tgtgtgtgtg tgagagagag agatttttta tgtatttaag tttaggtgga 1500
   gtatttgtaa ttttggtagt tatagttttt tggagaagag tttttttgtt ttttaaaaat 1560
   tagttattgt ttaaatacgt ttagttaggt ttaattaggt tttaaggaga tgtgaggtag 1620
   gattagggtt attattttag gaatattttg ttttttttat ttttatttag tgtgtgtaag 1680
   taatttatta gatttagatt 1700
<210> 108
   <211> 1700
   <212> DNA
   <213> Artificial Sequence
<220>
   <223> chemically treated genomic DNA (Homo sapiens)
<400> 108
   aatagaattt agaattttgg ggtagttttt tggagttgga gtttttaggg taaagataac 60
   gttaatgcgt tgtttaaaag ggtttaattt ttaaaatatt tttattggta atatttattt 120
   tgtttattag aaatgtagtt atgtattttt gtgttcggta aagatttttt gttttgtgta 180
   aatttataaa ttaaaataat tagtttagat ttttgacgtg attttgtagt ggaagtattt 240
   gtattagtga atgttgaaaa gtaaataaaa atgaaagttt tgcggaggtc gtgggtcgga 300
   cgggcgcggg gcgttttttg ttattttagc gtttgtcggg atttttttcg cgttttcgtt 360
   gttcgaatta gttcggtttt gggacgtttt ttttaaattt ttataagtta aatcgaattt 420
   aggttcgagt tcgttattgt aaaggaattc ggggtgcggg gtgggaggga taggaggaga 480
   gatgggttgg gtgttaaatg gtagaataaa tggttgattt aattaaagtt gttaatttaa 540
   ttttttattt tttttatatt agtttttttt tttttttttt tttttttttt tttttaaatt 600
   tgttgaattt tattattttc gcgttggtaa gaaaatttga atattttggg aattgtgttt 660
   aaattgtatt tgtaattttt ttggaagttt ttgaaatttt tcgagaatga ggcggggcgg 720
   tttaagggga aagggtaggg aggagagatt ttcgggaagt tgatgacgcg ttcggggtaa 780
   gttagtttgt tttcgagacg cgcgttttac ggtcgtagta gcgttgcgtt tattttgttt 840
   ttttagtgat ttagttagtt tttgttgggg gtttttgata acgtttatcg ataagttttt 900
   tttgttttta aaaggcgtgt tttttttttt ttttaggtat aaagtagggg tgggaagggg 960
   agagttatat aatgttgtaa aaagaagcgt ggttttattt ggaagacggt tgaatttttt 1020
   ttgttttttc gatttagtta gtaacgataa gaaagatatt gaatatcgtt tttcgaaaat 1080
   cgggcgtatt tgaattcggt gaaaagtgtt tttgtgagga agtttaatgt ttatttttgt 1140
   gtaaggataa ttggaaatta aagcgtttag taatgtagag attttattaa gcgagcgttt 1200
   tttttttttt cgcgttagga atgaaataag ttagttagat agaaaatatt tttgggagaa 1260
   gattaattat ttttgggtag gtcgaattga ttggaaatta tattaagtaa aggtagtata 1320
   ttttatttcg ttttggcgat gttgatttta tattattaga atattgtttt tttagtgtgg 1380
   gggtgttttg tattttttaa agttagggta gattggttat ttgggaaaat gtagtgttag 1440
   tgttttgttt ttattttttt tattgtttaa atatgtttta tattttttta atgttttttg 1500
   agggttttta atttagttat tgagtttttt atttttggcg attgtgatat taataatgtt 1560
   gttatttttt ttgggaaata tggttgtaaa gatgtttcgt aggaggtaga taaaaggttg 1620
   tgtttttaga tattgaaggt tgttttgtta tttagatgtt tttaattttt gtatagagaa 1680
   ggagggaaaa tacggggtgt 1700
<210> 109
   <211> 1700
   <212> DNA
   <213> Artificial Sequence
<220>
   <223> chemically treated genomic DNA (Homo sapiens)
<400> 109
   gtatttcgtg tttttttttt ttttttatgt aggaattgga aatatttaag taataaagta 60
   atttttagta tttgaaagta tagtttttta tttgtttttt gcggagtatt tttgtaatta 120
   tgttttttaa ggagggtgat agtattgttg gtattatagt cgttaagggt gaggaattta 180
   gtggttgggt tgagaatttt tagaaggtat tagaaaaatg tggaatatgt ttaaatagtg 240
   aagaaagtag gagtaaagta ttaatattgt atttttttag gtaattagtt tattttgatt 300
   ttgggaagta taaagtattt ttatattggg ggaataatgt tttaataata tagaattaat 360
   atcgttagga cggaatggaa tgtattgttt ttgtttggta taatttttag ttaattcggt 420
   ttatttagaa gtaattagtt tttttttaaa agtgtttttt gtttaattgg tttattttat 480
   ttttggcgcg ggggggaggg gagcgttcgt ttaatgaaat ttttgtatta ttagacgttt 540
   taatttttag ttatttttgt ataaaagtaa atattaggtt tttttatagg gatatttttt 600
   atcgagttta aatgcgttcg attttcggag ggcgatattt agtatttttt ttgtcgttat 660
   taattgggtc gaggaagtaa aagaaattta atcgtttttt aaataaagtt acgttttttt 720
   ttataatatt gtgtagtttt ttttttttta tttttatttt gtatttggag agaaagagaa 780
   gtacgttttt tgggagtaag gagagtttat cgatgagcgt tattagaaat ttttagtaaa 840
   ggttggttgg gttattaaag gggtagggtg ggcgtagcgt tgttacgatc gtgaggcgcg 900
   cgtttcggaa ataggttgat ttgtttcgga cgcgttatta gtttttcgag ggtttttttt 960
   ttttgttttt tttttttgag tcgtttcgtt ttattttcgg gaagttttag gaatttttag 1020
   gggagttgta agtgtaattt aagtataatt tttaaggtat ttaggttttt ttattaacgc 1080
   ggagatagtg ggatttagta agtttaagag gaaaaaagaa aaagaaaaaa aaaaagggtt 1140
   ggtgtgagag ggatgggaaa ttagattgat aattttaatt aaattaatta tttgttttat 1200
   tatttggtat ttagtttatt ttttttttta ttttttttat ttcgtatttc gggttttttt 1260
   gtagtgacga attcggattt gagttcgatt tgatttgtga ggatttgggg gaaacgtttt 1320
   aagatcgagt tggttcgggt agcgaaggcg cgggaaggat ttcggtagac gttaagatgg 1380
   taggaggcgt ttcgcgttcg ttcggtttac ggttttcgta aagtttttat ttttatttgt 1440
   tttttagtat ttattggtgt aaatattttt attgtagggt tacgttaaga atttaaatta 1500
   attattttag tttgtagatt tatatagaat agaaaatttt tatcgggtat aaaaatgtat 1560
   ggttgtattt ttgatgaata gaatgggtgt tattaataaa agtattttaa aaattgaatt 1620
   tttttaaata acgtattagc gttatttttg ttttaggagt tttaatttta aagaattgtt 1680
   ttagagtttt gaattttgtt 1700
<210> 110
   <211> 1700
   <212> DNA
   <213> Artificial Sequence
<220>
   <223> chemically treated genomic DNA (Homo sapiens)
<400> 110
   aatagaattt agaattttgg ggtagttttt tggagttgga gtttttaggg taaagataac 60
   gttaatgcgt tgtttaaaag ggtttaattt ttaaaatatt tttattggta atatttattt 120
   tgtttattag aaatgtagtt atgtattttt gtgttcggta aagatttttt gttttgtgta      180
   aatttataaa ttaaaataat tagtttagat ttttgacgtg attttgtagt ggaagtattt      240
   gtattagtga atgttgaaaa gtaaataaaa atgaaagttt tgcggaggtc gtgggtcgga      300
   cgggcgcggg gcgttttttg ttattttagc gtttgtcggg atttttttcg cgttttcgtt      360
   gttcgaatta gttcggtttt gggacgtttt ttttaaattt ttataagtta aatcgaattt      420
   aggttcgagt tcgttattgt aaaggaattc ggggtgcggg gtgggaggga taggaggaga      480
   gatgggttgg gtgttaaatg gtagaataaa tggttgattt aattaaagtt gttaatttaa      540
   tttttatttt tttttatatt agtttttttt tttttttttt tttttttttt tttttaaatt      600
   tgttgaattt tattattttc gcgttggtaa gaaaatttga atattttggg aattgtgttt      660
   aaattgtatt tgtaattttt ttggaagttt ttgaaatttt tcgagaatga ggcggggcgg      720
   tttaagggga aagggtaggg aggagagatt ttcgggaagt tgatgacgcg ttcggggtaa      780
   gttagtttgt tttcgagacg cgcgttttac ggtcgtagta gcgttgcgtt tattttgttt      840
   ttttagtgat ttagttagtt tttgttgggg gtttttgata acgtttatcg ataagttttt      900
   tttgttttta aaaggcgtgt tttttttttt ttttaggtat aaagtagggg tgggaagggg      960
   agagttatat aatgttgtaa aaagaagcgt ggttttattt ggaagacggt tgaatttttt     1020
   ttgttttttc gatttagtta gtaacgataa gaaagatatt gaatatcgtt tttcgaaaat     1080
   cgggcgtatt tgaattcggt gaaaagtgtt tttgtgagga agtttaatgt ttatttttgt    1140
   gtaaggataa ttggaaatta aagcgtttag taatgtagag attttattaa gcgagcgttt     1200
   tttttttttt cgcgttagga atgaaataag ttagttagat agaaaatatt tttgggagaa     1260
   gattaattat ttttgggtag gtcgaattga ttggaaatta tattaagtaa aggtagtata     1320
   ttttatttcg ttttggcgat gttgatttta tattattaga atattgtttt tttagtgtgg     1380
   gggtgttttg tattttttaa agttagggta gattggttat ttgggaaaat gtagtgttag    1440
   tgttttgttt ttattttttt tattgtttaa atatgtttta tattttttta atgttttttg   1500
   agggttttta atttagttat tgagtttttt atttttggcg attgtgatat taataatgtt     1560
   gttatttttt ttgggaaata tggttgtaaa gatgtttcgt aggaggtaga taaaaggttg     1620
   tgtttttaga tattgaaggt tgttttgtta tttagatgtt tttaattttt gtatagagaa     1680
   ggagggaaaa tacggggtgt                                                 1700
<210> 111
   <211> 1700
   <212> DNA
   <213> Artificial Sequence
<220>
   <223> chemically treated genomic DNA (Homo sapiens)
<400> 111
   gtatttcgtg tttttttttt ttttttatgt aggaattgga aatatttaag taataaagta 60
   atttttagta tttgaaagta tagtttttta tttgtttttt gcggagtatt tttgtaatta 120
   tgttttttaa ggagggtgat agtattgttg gtattatagt cgttaagggt gaggaattta 180
   gtggttgggt tgagaatttt tagaaggtat tagaaaaatg tggaatatgt ttaaatagtg 240
   aagaaagtag gagtaaagta ttaatattgt atttttttag gtaattagtt tattttgatt 300
   ttgggaagta taaagtattt ttatattggg ggaataatgt tttaataata tagaattaat 360
   atcgttagga cggaatggaa tgtattgttt ttgtttggta taatttttag ttaattcggt 420
   ttatttagaa gtaattagtt tttttttaaa agtgtttttt gtttaattgg tttattttat 480
   ttttggcgcg ggggggaggg gagcgttcgt ttaatgaaat ttttgtatta ttagacgttt 540
   taatttttag ttatttttgt ataaaagtaa atattaggtt tttttatagg gatatttttt 600
   atcgagttta aatgcgttcg attttcggag ggcgatattt agtatttttt ttgtcgttat 660
   taattgggtc gaggaagtaa aagaaattta atcgtttttt aaataaagtt acgttttttt 720
   ttataatatt gtgtagtttt ttttttttta tttttatttt gtatttggag agaaagagaa 780
   gtacgttttt tgggagtaag gagagtttat cgatgagcgt tattagaaat ttttagtaaa 840
   ggttggttgg gttattaaag gggtagggtg ggcgtagcgt tgttacgatc gtgaggcgcg 900
   cgtttcggaa ataggttgat ttgtttcgga cgcgttatta gtttttcgag ggtttttttt 960
   ttttgttttt tttttttgag tcgtttcgtt ttattttcgg gaagttttag gaatttttag 1020
   gggagttgta agtgtaattt aagtataatt tttaaggtat ttaggttttt ttattaacgc 1080
   ggagatagtg ggatttagta agtttaagag gaaaaaagaa aaagaaaaaa aaaaagggtt 1140
   ggtgtgagag ggatgggaaa ttagattgat aattttaatt aaattaatta tttgttttat 1200
   tatttggtat ttagtttatt ttttttttta ttttttttat ttcgtatttc gggttttttt 1260
   gtagtgacga attcggattt gagttcgatt tgatttgtga ggatttgggg gaaacgtttt 1320
   aagatcgagt tggttcgggt agcgaaggcg cgggaaggat ttcggtagac gttaagatgg 1380
   taggaggcgt ttcgcgttcg ttcggtttac ggttttcgta aagtttttat ttttatttgt 1440
   tttttagtat ttattggtgt aaatattttt attgtagggt tacgttaaga atttaaatta 1500
   attattttag tttgtagatt tatatagaat agaaaatttt tatcgggtat aaaaatgtat 1560
   ggttgtattt ttgatgaata gaatgggtgt tattaataaa agtattttaa aaattgaatt 1620
   tttttaaata acgtattagc gttatttttg ttttaggagt tttaatttta aagaattgtt 1680
   ttagagtttt gaattttgtt 1700
<210> 112
   <211> 1700
   <212> DNA
   <213> Artificial Sequence
<220>
   <223> chemically treated genomic DNA (Homo sapiens)
<400> 112
   attggatgag tggggttttt tgttttagtt ggatttttat aggaggatgg aggaaagtat   60
   ttttttgagt aagtttggag agtgaagggg tggggttgta aggttggaga aggaggtgtt  120
   ttttttataa aaatgattgg gttttttttg taatggttgg tataaagtcg gaagggtttg  180
   gggacgggag gggtttgaag tgatttttga ggagttgggt tttttttgta aggttttggg  240
   agggaaggta aggtattttg cgtaatgata tattttagtt gttggatgtg aagatttttt  300
   ttcgaagata gaataaagaa aggttttttt attaatgcgg gtttgaaatt ggtttgaaat  360
   ggtgtgtttg tagggattcg ggaggaaatg cgacgtttat ttttagattt gggaaaggcg  420
   ggggtaataa ttttttaaga gaattggagt tcgaaagagg aatgaaaagt tttcgcgtcg  480
   cgtatatata ttttttttta ttggaaagtt aggagcgagg aaagaatttt gaagtgattt  540
   gggaacgatt tgaaagcgcg taagttggaa gtcgggttga agttagtata gcggagtagg  600
   ggttaggttt ttgcgtggtc ggagtgggag agcgtttttg agaataagat ggggtaggat  660
   cgaaggggcg tgaggtcgac gttttttagg gatgattcgg agggtgaagg aggtttgggg  720
   gtggtttcgg gttttcggag gtgattgagg gagtttttga gttaatgttc gggggtggga  780
   ggcgttatcg gtatttattg agttgatttt tagcgttagg gcgtattgta gcgtttttat  840
   tttcggtatt cgtgcgggga ggggcggcgg ggagtgattt cggttacggg ttcgagtgag  900
   gcggggagat ttggggttgg gcggggacgg ggaggagacg aggtggcggc ggcggtggta  960
   gtagtggttt ttaggtcggg gttcgcggtt tagtttgggt ttcgtcggag aagtagttga  1020
   gcgcggggcg tagattcgtt gttatggtag ttaggagggg cggggtcgga agagaggtcg  1080
   cgcggggtat tttagggtta ttcgcgttgg gacgagcgtt ggaggcggag tttgacgaac  1140
   gtcgagcgag gcggagtcgg gtagggggcg gggttttcga aggggcgggg tgaggtaagg  1200
   tagttttttt gtgacgttaa attgcgttta aagtcggacg aaagggagtt gttgtttgta  1260
   taatgggacg gtgttttgtt tagggagttg gagtttttgt aaggtcgtcg gagggaattt  1320
   ttgagttata acgtggtgat ggtattttgg gttagtggcg atttattgtg gttttagttt  1380
   ttttaattgt aaaagaataa taattattgt tagttttttt atttaaattt tacggtacgg  1440
   tttataaagt tagtataagg atattgggaa ttagggaaga gatttgttta aattaaagtc  1500
   ggaattagga ttggaattta ggagttttta agttaaattt tagttgtttt tttgaggaga  1560
   ggggtttgta ttagatggtt tatattttta gttttttatt ttgtgatttt ttttgttagc  1620
   gcgttttttt ttttttttaa atttttttgt ttaatttatt tatgtatgcg ttttaatgta  1680
   tattgtttaa gtggaattgt                                              1700
<210> 113
   <211> 1700
   <212> DNA
   <213> Artificial Sequence
<220>
   <223> chemically treated genomic DNA (Homo sapiens)
<400> 113
   atagttttat ttaagtaata tgtattaaga cgtatatatg ggtgaattag ataggaaaat   60
   ttgaagggaa aagggaacgc gttaataaag ggaattatag aataggagat taggagtatg  120
   gattatttga tgtaagtttt tttttttagg gaggtaattg gggtttaatt tgagagtttt  180
   tgggttttag ttttggtttc gattttgatt tgggtaagtt ttttttttaa tttttagtat  240
   ttttatgttg gttttgtaaa tcgtgtcgtg aaatttaaat aggaggatta ataataatta  300
   ttattttttt ataattggga aaattgaggt tatagtgggt cgttattagt ttaaggtgtt  360
   attattacgt tgtaatttag gggttttttt cgacggtttt ataggaattt taattttttg  420
   aatagagtat cgttttatta tataaatagt agtttttttt cgttcggttt tgggcgtagt  480
   ttgacgttat agaagagttg ttttgtttta tttcgttttt tcggaagttt cgttttttgt      540
   tcgatttcgt ttcgttcggc gttcgttaag tttcgttttt agcgttcgtt ttagcgcggg     600
   tagttttgga gtgtttcgcg cggttttttt ttcggtttcg ttttttttgg ttgttatgat       660
   aacgagtttg cgtttcgcgt ttagttgttt tttcggcgga atttaggttg gatcgcgggt     720
   ttcggtttgg gggttattgt tgttatcgtc gtcgttattt cgtttttttt tcgttttcgt     780
   ttagttttag gttttttcgt tttattcggg ttcgtggtcg gggttatttt tcgtcgtttt      840
   ttttcgtacg gatgtcgaag gtgaaggcgt tgtagtgcgt tttggcgttg gagattagtt     900
   tagtgagtat cggtagcgtt ttttattttc gggtattggt ttagggattt ttttagttat     960
   tttcgagagt tcggaattat ttttaaattt tttttatttt tcgaattatt tttagggagc    1020
   gtcgatttta cgttttttcg gttttgtttt attttgtttt tagaagcgtt tttttatttc     1080
   ggttacgtag aggtttgatt tttgtttcgt tatgttggtt ttaattcgat ttttaatttg    1140
   cgcgttttta gatcgttttt aaattatttt agagtttttt tttcgttttt aattttttag     1200
   tgagagagag tgtatgtgcg cggcgcgggg gttttttatt tttttttcgg gttttagttt     1260
   ttttagagaa ttattgtttt cgtttttttt aagtttgggg gtgggcgtcg tatttttttt    1320
   cgaatttttg taggtatatt attttagatt agttttaaat tcgtattaat gaaggggttt     1380
   ttttttattt tgttttcgaa ggagaatttt tatatttaat aattgaaatg tgttattgcg    1440
   taaagtattt tgtttttttt tttagggttt tataggaaaa atttaatttt ttagaaatta    1500
   ttttaggttt ttttcgtttt tagatttttt cgattttgtg ttagttattg taagaaaggt    1560
   ttagttattt ttatgaggga aatatttttt tttttaattt tgtaatttta tttttttatt     1620
   ttttagattt gtttaagagg atgttttttt ttattttttt gtggaagttt agttggggta     1680
   gagaatttta tttatttagt                                                1700
<210> 114
   <211> 1700
   <212> DNA
   <213> Artificial Sequence
<220>
   <223> chemically treated genomic DNA (Homo sapiens)
<400> 114
   ttaggagttt agattcgggt aggaaataaa gttttaaagc ggttttgaag tgtttttttt 60
   ttagattttt ttttagcgcg ggttatttat tttagtttag ggaagagatt attttgtttg 120
   aacgtagtta aacgtatcgt acgtgggagt taagtgggag atgaaataat ttaatgcgtt 180
   ttttttatta ttattatttt tattaaatta gttatcgtcg gtttaaattt atcgggaatg 240
   ttcgtgtatt ttatcgatta gggaaataaa agggtgtata taaattagtg gaagcgggtg 300
   ggaaggaata agggatgtag attgtcgtga gtaaaacgtt tcgcggtttg gtgagaggtt 360
   gttttttttt taagttattt attttagatt gaaatattaa gttagtttag gttatttttt 420
   gagtagtatt ataagttaaa ataagattta gaaaatttat taatttttaa ttgcgttgtt 480
   tttagattgg gagtataggg ggatagggtg tagggttgtg gggaggtcga ggggaggggg 540
   atttgtggaa aagtaaacgt tcgagaaatt ttagttttcg attttagttg ttttttattt 600
   tatttagttc ggtcgaggag tcgtttttat attttatggt aatttcggaa tttatgatgt 660
   attttttaga gttgggtttt tagtttttta ggtttcggag tttaagagtt ggattttata 720
   ggtttaatat ttattgtttg gttttttttg ttatatttta gtttagatag ttatttttat 780
   ttaggatagt tgcgaagcgt aaacggagga tgtaggtatt tttaaaaaga gtagtcgtgg 840
   ttcgcgtttt acggacgagt tattgttgta ggaggttcgg tggcgcggcg cgttcgtagt 900
   tgcggtagcg gcggagttcg cgcggggatt ttttcgcgcg tatttagcgt ttcgtttcgg 960
   ggtagtgtta ttttgggcgt cgtttcgtta tttttacggt gtttcgtcgt tttttattat 1020
   taggatggag ggtatttttt agtacgagtc gatattttag agggtatcgg cgagttttcg 1080
   aaaaaattag ggcggtgggg cgaggaagat tcgcgtttcg gattttagag aaggaaagaa 1140
   agttagaggg agtcggagcg gagttgtatt ttacgtcgaa gttttttttt cgaatcgttt 1200
   ggagttgtaa gtggtttttg attgaatata gttgtttagt tgttggttac ggggtgcgcg 1260
   gaggaggcgg ggttagaggg agaaaagggt gggggcgagt agtttggagg tcgagtagag 1320
   gagatcgtgt tgggagagta gagtaggtat ttttttagtt ttttgtaata gtaattaagg 1380
   attcgagttt aggaggggga gtagaaatag gaaaagttat ttgaataatc gtgtaggaaa 1440
   ttaaaaaaaa aaaaagaaaa gaaaaagaaa aaaaattttt tttataggag tcgtttttta 1500
   tgatttaata aggttaagat aaatttaaat attgtatatt agatagtacg gtgtattttt 1560
   tacggaatat taagatttta taagggtgta gggtagttat tttttttaat tagtttaaag 1620
   ttaacgttac gggtgtaaat ttaatgttaa attttttttg tttgttattg gggtagagta 1680
   tattttttgt atgtttataa 1700
<210> 115
   <211> 1700
   <212> DNA
   <213> Artificial Sequence
<220>
   <223> chemically treated genomic DNA (Homo sapiens)
<400> 115
   ttatggatat gtagaaaatg tattttgttt tagtgataga tagagggagt ttgatattga      60
   atttatattc gtagcgttga ttttagattg attagggaaa atgattgttt tgtatttttg     120
   taaaatttta atgtttcgtg agaagtgtat cgtgttgttt gatgtatagt gtttaggttt     180
   gttttgattt tattaaatta tggaaagcgg tttttatgag ggaagttttt tttttttttt     240
   tttttttttt ttttttttaa ttttttgtac gattatttag gtggtttttt ttgtttttat     300
   tttttttttt ggattcgggt ttttaattat tattgtagag aattggaagg atgtttgttt     360
   tgttttttta gtacggtttt ttttgttcgg tttttaggtt attcgttttt attttttttt     420
   ttttttagtt tcgttttttt cgcgtatttc gtggttagta gttagatagt tgtgtttaat     480
   tagaaattat ttataatttt agacgattcg aaggggaaat ttcggcgtga agtgtagttt     540
   cgtttcggtt ttttttagtt tttttttttt ttttggaatt cgaggcgcgg atttttttcg     600
   ttttatcgtt ttagtttttt cgggagttcg tcggtgtttt ttagggtgtc ggttcgtgtt     660
   gggaagtgtt ttttattttg gtaatggggg gcggcgaggt atcgtaggag tggcgaggcg     720
   gcgtttaggg tggtattgtt tcggaacggg gcgttgggtg cgcgcgggag ggttttcgcg     780
   cgggtttcgt cgttgtcgta gttgcgagcg cgtcgcgtta tcgagttttt tgtagtaatg     840
   gttcgttcgt gaaacgcgag ttacggttgt tttttttaag agtgtttgta tttttcgttt     900
   gcgtttcgta attgttttgg gtgaaaatgg ttgtttagat taaaatgtgg tagaagggat     960
   taagtagtgg atattgagtt tgtgaagttt aatttttaag tttcgagatt tgggggattg    1020
   agagtttagt tttgaaaagt gtattatgaa tttcggagtt gttatgaaat atggaaacga    1080
   tttttcggtc gagttgagtg aggtaagaaa tagttgaggt cgggagttag ggtttttcgg    1140
   gcgtttgttt ttttataaat tttttttttt tcgatttttt tatagtttta tattttattt    1200
   ttttgtgttt ttagtttgga ggtagcgtaa ttgaagatta gtagattttt tggattttgt    1260
   tttaatttgt agtgttattt aggaaatgat ttgaattggt ttgatatttt agtttgaaat    1320
   gagtggttta aaaaagaaat aattttttat taagtcgcga ggcgttttat ttacgatagt    1380
   ttgtattttt tgtttttttt tattcgtttt tattaattta tatatatttt tttatttttt    1440
   tgatcggtaa aatatacgga tattttcgat gagtttaagt cgacggtaat taatttggtg    1500
   ggggtggtgg tggtgagggg gacgtattgg attattttat tttttatttg gtttttacgt    1560
   gcgatgcgtt tggttacgtt taggtaaggt ggtttttttt ttaggttgag atgaatgatt    1620
   cgcgttgggg aggggtttgg ggagaaagta ttttaaagtc gttttagaat tttatttttt    1680
   gttcggattt ggatttttga                                                1700
<210> 116
   <211> 1700
   <212> DNA
   <213> Artificial Sequence
<220>
   <223> chemically treated genomic DNA (Homo sapiens)
<400> 116
   gttggtaagt gaaaagttta tagtttatag tatttttatt gtttagtttt atttttattg      60
   ttgagtttag gaatttgttt ttttgttttt ttttggaagc gattttgtgt gtgtaggtag     120
   gggtagaggg agtaaagtta aacgtaatta tagtatttta gatttgtaga tttttaaatt     180
   agttttatat tttagtgagg tgataatggt taatattttt attttagttt tatagaagtt     240
   tagttatagt aggtgatttg cgtaaagtta tatagttttt ggttttaagg tagggttttt     300
   ttttttgttt gtgttttttc gttatcgtag tttttgtttt ttagttgggg gattatttag     360
   aggattattt ggattttgtt gttttcgttg ggttatttgt ttggcggagt agttgggggg     420
   tggggggttt gtaatttagg attttatttg gcgttgtttt agtttatcga gtagtgagtt     480
   agtttttttt ttatcggtgg tagggggagt tgtcgggatt ttagggttgt tttgaggggt     540
   cgcgtagatt tgggggatgt aaaggtttaa acggattcgg aatggtcggt gcggtcgggt     600
   tttgggaaat gaagggttcg cggtgtttcg tattttttgt gggggcggga gtaattatta     660
   ggtggtcgcg gagttagtcg agggggcggt tgttaattcg ggcggcgatc gtaggcggag     720
   acgtttcgtt cgggataggg atcgggtcgc gtcggcgttc gcggatagat attcgcggtt     780
   tcggtcgttg tcgcgttttt cgttttcgtt acgtttcggc ggtttcgttt ggggcgcgtt     840
   ttcgtttcgt acgcgtacgc gtttgtattc gtattggttc gttcggtttt ttcgcgttcg     900
   cggggtcgtt tcgggggagg tgtcgagatt cggttttggt tcgcgcgcgg ttttcggggt     960
   tcggcgcgtc ggggatttat aggcgaggcg agagaaaggg gttggttcgc ggggatattt    1020
   atttgcgcgg cgcggggttg tggcgtcggt tattattatt tcgcgttttt ttttttgtta    1080
   gcgcgtacgt agatggcggg gtggtttggg gaggttttcg ggtttttttt tgggaacgta    1140
   gggttaagtt gtgtttcgat tttacgtttt ttttatttac gtcgggtata cgtagttttg    1200
   agtgagggtt tatgttttgt tttttacgtt gattatagtt tttaggtttg gcgtttgtcg    1260
   ggaagggtcg taatggttta gggatttttg tttttattat tattttgttt tggggggata    1320
   ggttttagaa tattgcgtgg ggtttagagt gggtttagtt gtaaaaggat aggggttttt    1380
   ttttagttac gtttattttc gggtgttttg gttgttagta gttttttatt ataatcgtag    1440
   ggtgagaagt ttagagaata gtttaggagt agtttttttg ttttttatta cgttcgtgtg    1500
   tttagtacgg gtaaattagt agttacgtaa taaatgaagt gcggtgttgt agttttgggt    1560
   ttttttgtat ttgttttgat tttagtttgt ttagtttagt ttggggattt gtatttaggt    1620
   ttgtagtttt ttgggttttt gttcggtgtt ttttagagtc ggagtttgtt tgtcgtaggg    1680
   atggtttaga ggatttttta                                               1700
<210> 117
   <211> 1700
   <212> DNA
   <213> Artificial Sequence
<220>
   <223> chemically treated genomic DNA (Homo sapiens)
<400> 117
   taggaggttt tttaagttat ttttgcggta gataggtttc ggttttgagg agtatcgggt      60
   agggatttag gaggttgtaa atttaagtgt agatttttag gttgggttgg ataggttgag     120
   gttagggtag gtgtagagag gtttagagtt gtaatatcgt attttattta ttgcgtggtt     180
   attgatttgt tcgtgttggg tatacggacg tggtaaagga tagaagggtt atttttggat     240
   tgttttttgg gttttttatt ttgcggttgt ggtgagaggt tgttgatagt tagggtattc     300
   gggagtgggc gtggttggag aaggattttt gtttttttat agttaggttt attttgagtt     360
   ttacgtagtg ttttagagtt tgttttttta gggtaggatg gtagtggggg taggggtttt     420
   tgagttattg cggttttttt cggtaggcgt taagtttagg ggttgtggtt agcgtgaaag     480
   gtagggtatg gatttttatt tagggttgcg tgtgttcgac gtgggtgggg ggggcgtgga     540
   atcggagtat aatttggttt tgcgttttta ggaagggatt cgaagatttt tttaggttat     600
   ttcgttattt gcgtgcgcgt tggtaagagg aggggcgcgg ggtaatggtg gtcggcgtta     660
   tagtttcgcg tcgcgtaggt aggtgttttc gcgagttaat tttttttttt cgtttcgttt     720
   gtgggttttc ggcgcgtcgg atttcggaaa tcgcgcgcgg gttaaggtcg ggtttcggta     780
   tttttttcgg agcggtttcg cgagcgcggg ggaatcgggc gggttagtgc gggtgtaggc     840
   gcgtgcgcgt gcgaggcggg ggcgcgtttt aagcgaggtc gtcggggcgt ggcgggggcg     900
   ggagacgcgg tagcggtcgg gatcgcgggt gtttgttcgc gagcgtcggc gcggttcggt     960
   ttttgtttcg ggcggggcgt tttcgtttgc ggtcgtcgtt cgggttagta gtcgtttttt    1020
   cggttggttt cgcggttatt tggtaattat tttcgttttt ataaaaagtg cgagatatcg    1080
   cgagtttttt attttttaga gttcggtcgt atcggttatt tcgagttcgt ttgggttttt    1140
   atatttttta gatttgcgcg gttttttagg gtagttttga gatttcgata gttttttttg    1200
   ttatcgatga ggggagagtt ggtttattgt tcggtgggtt ggggtagcgt taggtggggt    1260
   tttaggttgt aggtttttta ttttttaatt atttcgttaa gtaaatggtt taacggaggt    1320
   agtagggttt aggtggtttt ttgggtggtt ttttagttgg agaatagggg ttgcggtggc    1380
   gagagggtat aggtagggga gagagttttg ttttggagtt agaggttgtg tagttttgcg    1440
   taggttattt gttgtggttg ggtttttgta aaattgagat gaaaatgtta gttattgtta    1500
   ttttattgag gtgtgaggtt ggtttgagag tttgtaaatt tgagatgtta tagttacgtt    1560
   tggttttatt ttttttgttt ttatttgtat atataggatc gtttttaaaa ggaggtaggg    1620
   aaataaattt ttgagtttaa tagtggaagt ggggttggat agtgaggatg ttgtggattg    1680
   tgggtttttt atttgttagt                                                1700
<210> 118
   <211> 1700
   <212> DNA
   <213> Artificial Sequence
<220>
   <223> chemically treated genomic DNA (Homo sapiens)
<400> 118
   gtaagatttt attttaaaaa aaaaaaaaaa aaaaagcgat ttgtttagta atagttttaa      60
   aatatggaag agttgggatt ggatttagat tatttggttt tggaattggg ttggtaagtg     120
   aaaagtttat agtttatagt atttttattg tttagtttta tttttattgt tgagtttagg     180
   aatttgtttt tttgtttttt tttggaagcg attttgtgtg tgtaggtagg ggtagaggga     240
   gtaaagttaa acgtaattat agtattttag atttgtagat ttttaaatta gttttatatt     300
   ttagtgaggt gataatggtt aatattttta ttttagtttt atagaagttt agttatagta     360
   ggtgatttgc gtaaagttat atagtttttg gttttaaggt agggtttttt tttttgtttg     420
   tgttttttcg ttatcgtagt ttttgttttt tagttggggg attatttaga ggattatttg     480
   gattttgttg ttttcgttgg gttatttgtt tggcggagta gttggggggt ggggggtttg     540
   taatttagga ttttatttgg cgttgtttta gtttatcgag tagtgagtta gttttttttt     600
   tatcggtggt agggggagtt gtcgggattt tagggttgtt ttgaggggtc gcgtagattt     660
   gggggatgta aaggtttaaa cggattcgga atggtcggtg cggtcgggtt ttgggaaatg     720
   aagggttcgc ggtgtttcgt attttttgtg ggggcgggag taattattag gtggtcgcgg     780
   agttagtcga gggggcggtt gttaattcgg gcggcgatcg taggcggaga cgtttcgttc     840
   gggataggga tcgggtcgcg tcggcgttcg cggatagata ttcgcggttt cggtcgttgt     900
   cgcgtttttc gttttcgtta cgtttcggcg gtttcgtttg gggcgcgttt tcgtttcgta     960
   cgcgtacgcg tttgtattcg tattggttcg ttcggttttt tcgcgttcgc ggggtcgttt    1020
   cgggggaggt gtcgagattc ggttttggtt cgcgcgcggt tttcggggtt cggcgcgtcg    1080
   gggatttata ggcgaggcga gagaaagggg ttggttcgcg gggatattta tttgcgcggc    1140
   gcggggttgt ggcgtcggtt attattattt cgcgtttttt tttttgttag cgcgtacgta    1200
   gatggcgggg tggtttgggg aggttttcgg gttttttttt gggaacgtag ggttaagttg    1260
   tgtttcgatt ttacgttttt tttatttacg tcgggtatac gtagttttga gtgagggttt    1320
   atgttttgtt ttttacgttg attatagttt ttaggtttgg cgtttgtcgg gaagggtcgt    1380
   aatggtttag ggatttttgt ttttattatt attttgtttt ggggggatag gttttagaat     1440
   attgcgtggg gtttagagtg ggtttagttg taaaaggata ggggtttttt tttagttacg    1500
   tttattttcg ggtgttttgg ttgttagtag ttttttatta taatcgtagg gtgagaagtt   1560
   tagagaatag tttaggagta gtttttttgt tttttattac gttcgtgtgt ttagtacggg    1620
   taaattagta gttacgtaat aaatgaagtg cggtgttgta gttttgggtt tttttgtatt    1680
   tgttttgatt ttagtttgtt                                                1700
<210> 119
   <211> 1700
   <212> DNA
   <213> Artificial Sequence
<220>
   <223> chemically treated genomic DNA (Homo sapiens)
<400> 119
   gataggttga ggttagggta ggtgtagaga ggtttagagt tgtaatatcg tattttattt      60
   attgcgtggt tattgatttg ttcgtgttgg gtatacggac gtggtaaagg atagaagggt     120
   tatttttgga ttgttttttg ggttttttat tttgcggttg tggtgagagg ttgttgatag       180
   ttagggtatt cgggagtggg cgtggttgga gaaggatttt tgttttttta tagttaggtt      240
   tattttgagt tttacgtagt gttttagagt ttgttttttt agggtaggat ggtagtgggg      300
   gtaggggttt ttgagttatt gcggtttttt tcggtaggcg ttaagtttag gggttgtggt     360
   tagcgtgaaa ggtagggtat ggatttttat ttagggttgc gtgtgttcga cgtgggtggg     420
   gggggcgtgg aatcggagta taatttggtt ttgcgttttt aggaagggat tcgaagattt     480
   ttttaggtta tttcgttatt tgcgtgcgcg ttggtaagag gaggggcgcg gggtaatggt     540
   ggtcggcgtt atagtttcgc gtcgcgtagg taggtgtttt cgcgagttaa tttttttttt      600
   tcgtttcgtt tgtgggtttt cggcgcgtcg gatttcggaa atcgcgcgcg ggttaaggtc     660
   gggtttcggt atttttttcg gagcggtttc gcgagcgcgg gggaatcggg cgggttagtg      720
   cgggtgtagg cgcgtgcgcg tgcgaggcgg gggcgcgttt taagcgaggt cgtcggggcg     780
   tggcgggggc gggagacgcg gtagcggtcg ggatcgcggg tgtttgttcg cgagcgtcgg     840
   cgcggttcgg tttttgtttc gggcggggcg ttttcgtttg cggtcgtcgt tcgggttagt     900
   agtcgttttt tcggttggtt tcgcggttat ttggtaatta ttttcgtttt tataaaaagt     960
   gcgagatatc gcgagttttt tattttttag agttcggtcg tatcggttat ttcgagttcg    1020
   tttgggtttt tatatttttt agatttgcgc ggttttttag ggtagttttg agatttcgat       1080
   agtttttttt gttatcgatg aggggagagt tggtttattg ttcggtgggt tggggtagcg     1140
   ttaggtgggg ttttaggttg taggtttttt attttttaat tatttcgtta agtaaatggt    1200
   ttaacggagg tagtagggtt taggtggttt tttgggtggt tttttagttg gagaataggg    1260
   gttgcggtgg cgagagggta taggtagggg agagagtttt gttttggagt tagaggttgt    1320
   gtagttttgc gtaggttatt tgttgtggtt gggtttttgt aaaattgaga tgaaaatgtt    1380
   agttattgtt attttattga ggtgtgaggt tggtttgaga gtttgtaaat ttgagatgtt    1440
   atagttacgt ttggttttat tttttttgtt tttatttgta tatataggat cgtttttaaa      1500
   aggaggtagg gaaataaatt tttgagttta atagtggaag tggggttgga tagtgaggat    1560
   gttgtggatt gtgggttttt tatttgttag tttagtttta gaattagata gtttaagttt    1620
   aattttagtt tttttatatt ttagagttat tattgggtaa gtcgtttttt tttttttttt       1680
   tttttgagat ggagttttgt                                                1700
<210> 120
   <211> 1700
   <212> DNA
   <213> Artificial Sequence
<220>
   <223> chemically treated genomic DNA (Homo sapiens)
<400> 120
   aggtgggtag gttttttgga gtagtaggcg gtttttttag tttttatggt tggagttagg      60
   attttttttt gtttataatt tttttaattt ttttaattta atatttttta tttttttttt   120
   tttttttttt tttttttttt tttttttttt tttttttttt tttttttttt tttttttttt          180
   tttttttttt tttttttttt ttgttttttt ttttttttta gttttttttt tttattttgt         240
   tgtgggcggg ttgtagtgtt gattttatcg ggacgtttgg ataggtattg tttgtgataa    300
   cggtggtatt ggaaggtttt ggtagttgaa cgtttagttt tttatttttt atgtttttat      360
   attttttatg tataaatttt gtaggaagta ggttagggaa cgtagtttat tgtttttttt       420
   cgattagtag ttttagtttg gttatatcgt agtcgcggta gaggatacgg tttattttta     480
   cgagttaatc ggggtttatt tttattttcg gcggttttag cgggtttagt aagcgagagt     540
   aggttttcgg agttataggg gtttttttgt tttcgtagcg agggaagtag gaggcggtag     600
   gttttgaggt tcgtgggttg aaggtttagg ttttacgtgg agttattcgc ggaggattta     660
   ggttaggtta atttgttcgt ggggaattcg ttcgattttg gcgagttagg ggtttggaag     720
   gtcgggtgga gtttggttaa tagttcgggt tttttatttt gggtttcgtt tttttcggag     780
   gtttcggggg tatttagcgg gtaggttttt ttggttcggt cggttttgga gtaggtgagg     840
   aaggttaggt aggtttcgcg gaggcgggtt aggtttcggt gggtggtttc gttgacgaag     900
   tagtagagta cggggtcggc gacgtagttg aagttggtga gtaggaggga gaagtggtag     960
   gcgttgaaaa cgtttttggc gaagtcgtag ttggtttttt agacgttgcg tattagtagt    1020
   aatacgtggt agggtaggaa gtaggttagg aagatgatta cggtgttgag tattagtcgt    1080
   tggatttggt ttttgcggtt tttttgggtg tcgtggtttc ggcgtacggc gcgtaggatg    1140
   ttttggtagg acgttagtag taggtagatg gggaagagga agtttattag gaagcggtag    1200
   tagttgatgg cgcgttgtta tgtttggatg gggtagtgtt taaagtatac gcggtgttgg    1260
   ttttcgtttt cgatgatttt ttcgtgtatt aggaagtaga tgttggttag tagttttttg   1320
   gtttagatga ttacgttgac gtcgacggtc gtttttaggg ttcggaattg gtggaagcgg    1380
   aagggatggg ttatagttag gtagcggttt acggagatgt agtagaggaa gtttacgttg    1440
   atgtagatgt tttcgtatag gaggatgtcg tatatttggt aggataggtc gtcgtgagat    1500
   tagttgtcgt gttgtagtac gtattgtagt tagaagggta gcgagtagat gtagaagagg    1560
   tcggttatcg ttaggttgta taggtatacg tttagttcgt ttcgggtttt gatttgtagg    1620
   tagtcgaagt agagggatag gtagttggtc gggaagttta ttattagtac ggtaatatag    1680
   attatcgggg ttagcgtttg                                                1700
<210> 121
   <211> 1700
   <212> DNA
   <213> Artificial Sequence
<220>
   <223> chemically treated genomic DNA (Homo sapiens)
<400> 121
   tagacgttgg tttcggtggt ttatgttatc gtgttggtgg tgggtttttc ggttaattgt     60
   ttgttttttt atttcggtta tttgtagatt aaggttcgga acgagttggg cgtgtatttg   120
   tgtaatttga cggtggtcga ttttttttat atttgttcgt tgtttttttg gttgtagtac      180
   gtgttgtagt acgataattg gttttacggc gatttgtttt gttaggtgtg cggtattttt      240
   ttgtacgaga atatttatat tagcgtgggt tttttttgtt gtattttcgt ggatcgttat      300
   ttggttgtgg tttatttttt tcgtttttat tagtttcgga ttttgaaggc ggtcgtcggc      360
   gttagcgtgg ttatttgggt taaggagttg ttgattagta tttatttttt gatgtacgag      420
   gaggttatcg aggacgagaa ttagtatcgc gtgtgttttg agtattattt tatttaggta      480
   tggtagcgcg ttattaatta ttatcgtttt ttggtgggtt tttttttttt tatttgtttg      540
   ttgttggcgt tttattaggg tattttgcgc gtcgtgcgtc ggagttacgg tatttagaag      600
   agtcgtaagg attagattta gcggttggtg tttagtatcg tggttatttt tttggtttgt      660
   tttttgtttt attacgtgtt gttgttggtg cgtagcgttt gggaggttag ttgcgatttc      720
   gttaagggcg tttttaacgt ttattatttt ttttttttgt ttattagttt taattgcgtc      780
   gtcgatttcg tgttttattg tttcgttagc gagattattt atcgggattt ggttcgtttt      840
   cgcggggttt gtttggtttt ttttatttgt tttaggatcg gtcgggttag ggaggtttat      900
   tcgttgggtg ttttcgaggt tttcgggaaa agcggggttt agggtgagga gttcgagttg      960
   ttgattaagt tttattcggt tttttagatt tttaattcgt tagggtcggg cgggtttttt     1020
   acgggtaggt tggtttagtt tgggtttttc gcgggtggtt ttacgtgagg tttgagtttt      1080
   tagtttacgg gttttagggt ttgtcgtttt ttgttttttt cgttgcggag gtagggaagt     1140
   ttttgtaatt tcggaagttt gttttcgttt gttgagttcg ttgggatcgt cgagggtggg     1200
   aataagtttc ggttggttcg tgggaataag tcgtgttttt tgtcgcggtt gcgatgtggt     1260
   taggttgggg ttgttggtcg ggggaagata gtgaattgcg ttttttggtt tgttttttgt     1320
   agagtttgtg tatggggagt gtgaggatat ggagggtggg aggttgggcg tttagttgtt     1380
   agggtttttt aatgttatcg ttgttataga taatgtttgt ttaaacgttt cggtgggatt     1440
   agtattgtag ttcgtttata atagggtggg aagggaagat tggaggggga aggaaggtag     1500
   gagggggaag gaagaaagag ggggaaggaa agaaagaggg gggaaggaaa ggaggaaggg     1560
   gagaaggaag ggaggaggaa agaaggaagg tagggggtgt tgggttggga gaattgaggg     1620
   agttataggt agaggaggat tttagtttta gttataagaa ttgggagagt cgtttgttgt     1680
   tttagaagat ttgtttattt                                                 1700
<210> 122
   <211> 1700
   <212> DNA
   <213> Artificial Sequence
<220>
   <223> chemically treated genomic DNA (Homo sapiens)
<400> 122
   aggtgggtag gttttttgga gtagtaggcg gtttttttag tttttatggt tggagttagg 60
   attttttttt gtttataatt tttttaattt ttttaattta atatttttta tttttttttt 120
   tttttttttt tttttttttt tttttttttt tttttttttt tttttttttt tttttttttt 180
   tttttttttt tttttttttt ttgttttttt ttttttttta gttttttttt tttattttgt 240
   tgtgggcggg ttgtagtgtt gattttatcg ggacgtttgg ataggtattg tttgtgataa 300
   cggtggtatt ggaaggtttt ggtagttgaa cgtttagttt tttatttttt atgtttttat 360
   attttttatg tataaatttt gtaggaagta ggttagggaa cgtagtttat tgtttttttt 420
   cgattagtag ttttagtttg gttatatcgt agtcgcggta gaggatacgg tttattttta 480
   cgagttaatc ggggtttatt tttattttcg gcggttttag cgggtttagt aagcgagagt 540
   aggttttcgg agttataggg gtttttttgt tttcgtagcg agggaagtag gaggcggtag 600
   gttttgaggt tcgtgggttg aaggtttagg ttttacgtgg agttattcgc ggaggattta 660
   ggttaggtta atttgttcgt ggggaattcg ttcgattttg gcgagttagg ggtttggaag 720
   gtcgggtgga gtttggttaa tagttcgggt tttttatttt gggtttcgtt tttttcggag 780
   gtttcggggg tatttagcgg gtaggttttt ttggttcggt cggttttgga gtaggtgagg 840
   aaggttaggt aggtttcgcg gaggcgggtt aggtttcggt gggtggtttc gttgacgaag 900
   tagtagagta cggggtcggc gacgtagttg aagttggtga gtaggaggga gaagtggtag 960
   gcgttgaaaa cgtttttggc gaagtcgtag ttggtttttt agacgttgcg tattagtagt 1020
   aatacgtggt agggtaggaa gtaggttagg aagatgatta cggtgttgag tattagtcgt 1080
   tggatttggt ttttgcggtt tttttgggtg tcgtggtttc ggcgtacggc gcgtaggatg 1140
   ttttggtagg acgttagtag taggtagatg gggaagagga agtttattag gaagcggtag 1200
   tagttgatgg cgcgttgtta tgtttggatg gggtagtgtt taaagtatac gcggtgttgg 1260
   ttttcgtttt cgatgatttt ttcgtgtatt aggaagtaga tgttggttag tagttttttg 1320
   gtttagatga ttacgttgac gtcgacggtc gtttttaggg ttcggaattg gtggaagcgg 1380
   aagggatggg ttatagttag gtagcggttt acggagatgt agtagaggaa gtttacgttg 1440
   atgtagatgt tttcgtatag gaggatgtcg tatatttggt aggataggtc gtcgtgagat 1500
   tagttgtcgt gttgtagtac gtattgtagt tagaagggta gcgagtagat gtagaagagg 1560
   tcggttatcg ttaggttgta taggtatacg tttagttcgt ttcgggtttt gatttgtagg 1620
   tagtcgaagt agagggatag gtagttggtc gggaagttta ttattagtac ggtaatatag 1680
   attatcgggg ttagcgtttg 1700
<210> 123
   <211> 1700
   <212> DNA
   <213> Artificial Sequence
<220>
   <223> chemically treated genomic DNA (Homo sapiens)
<400> 123
   tagacgttgg tttcggtggt ttatgttatc gtgttggtgg tgggtttttc ggttaattgt 60
   ttgttttttt atttcggtta tttgtagatt aaggttcgga acgagttggg cgtgtatttg 120
   tgtaatttga cggtggtcga ttttttttat atttgttcgt tgtttttttg gttgtagtac 180
   gtgttgtagt acgataattg gttttacggc gatttgtttt gttaggtgtg cggtattttt 240
   ttgtacgaga atatttatat tagcgtgggt tttttttgtt gtattttcgt ggatcgttat 300
   ttggttgtgg tttatttttt tcgtttttat tagtttcgga ttttgaaggc ggtcgtcggc 360
   gttagcgtgg ttatttgggt taaggagttg ttgattagta tttatttttt gatgtacgag 420
   gaggttatcg aggacgagaa ttagtatcgc gtgtgttttg agtattattt tatttaggta 480
   tggtagcgcg ttattaatta ttatcgtttt ttggtgggtt tttttttttt tatttgtttg 540
   ttgttggcgt tttattaggg tattttgcgc gtcgtgcgtc ggagttacgg tatttagaag 600
   agtcgtaagg attagattta gcggttggtg tttagtatcg tggttatttt tttggtttgt 660
   tttttgtttt attacgtgtt gttgttggtg cgtagcgttt gggaggttag ttgcgatttc 720
   gttaagggcg tttttaacgt ttattatttt ttttttttgt ttattagttt taattgcgtc 780
   gtcgatttcg tgttttattg tttcgttagc gagattattt atcgggattt ggttcgtttt 840
   cgcggggttt gtttggtttt ttttatttgt tttaggatcg gtcgggttag ggaggtttat 900
   tcgttgggtg ttttcgaggt tttcgggaaa agcggggttt agggtgagga gttcgagttg 960
   ttgattaagt tttattcggt tttttagatt tttaattcgt tagggtcggg cgggtttttt 1020
   acgggtaggt tggtttagtt tgggtttttc gcgggtggtt ttacgtgagg tttgagtttt 1080
   tagtttacgg gttttagggt ttgtcgtttt ttgttttttt cgttgcggag gtagggaagt 1140
   ttttgtaatt tcggaagttt gttttcgttt gttgagttcg ttgggatcgt cgagggtggg 1200
   aataagtttc ggttggttcg tgggaataag tcgtgttttt tgtcgcggtt gcgatgtggt 1260
   taggttgggg ttgttggtcg ggggaagata gtgaattgcg ttttttggtt tgttttttgt 1320
   agagtttgtg tatggggagt gtgaggatat ggagggtggg aggttgggcg tttagttgtt 1380
   agggtttttt aatgttatcg ttgttataga taatgtttgt ttaaacgttt cggtgggatt 1440
   agtattgtag ttcgtttata atagggtggg aagggaagat tggaggggga aggaaggtag 1500
   gagggggaag gaagaaagag ggggaaggaa agaaagaggg gggaaggaaa ggaggaaggg 1560
   gagaaggaag ggaggaggaa agaaggaagg tagggggtgt tgggttggga gaattgaggg 1620
   agttataggt agaggaggat tttagtttta gttataagaa ttgggagagt cgtttgttgt 1680
   tttagaagat ttgtttattt 1700
<210> 124
   <211> 1700
   <212> DNA
   <213> Artificial Sequence
<220>
   <223> chemically treated genomic DNA (Homo sapiens)
<400> 124
   ttttttagga ttagagaatt ttgaagagtt tgtgtattgt ttttttgaaa gttttgaagt 60
   taagtaattt taaaaatgtt tagtaaagtt tttttgggaa tggtgagatg gggggtgggg 120
   aggatgggat atttttattg ttattgtttg ttttttgttt ttttggtttt tttatatgaa 180
   gtgattggat ttgggagatt tagtggaaat ttttaggtta tttagggaat tgtttttttt 240
   tttggttttg tagtggtgag gtggggtagg gggtgggggg ggtgggttgt gagagtgttg 300
   agatgtgtgg ggtgtggaga tgtgtaagtg gtgaagtttg attgagagta ggttggagta 360
   gttgtttaat ttttggtgtg ggatttgttg aggggttatg gtgagttttt gtgggtgtgg 420
   gttggaggga tgtttaggtg ttttgttgtt ttatggtttg ggtggaggtg ggtttggaat 480
   tttttttaag tttggtttta gtttggagtt tgtgtgtttt gggtggatgt atggattgag 540
   aggtgtttgg ttgggttttt agtttgttat tgttatttgg ttttgtgtgt ttattggttg      600
   ggatgtgttg gtttagtagt ttttaatttt ttttgaattt tattggtgag ttttaggaga      660
   gtgagttgag ggagaaaggt ttaaagggtg tgagtgttta gggtgttggg agtttgtggg      720
   atgggtatgg ggagggtaga gttagttgag gggagtggtt gttgggaggt gtgttgagta      780
   gggtttgtag ttaaggttta gtgtgatttg agtgtttagg attttgtggt tgtggaggag      840
   gtgtgttttg ggaaggagtt gttattggga tttgaagagt gaggggaggg agaggggttt      900
   agagggtgat ttggaggagg gttttggaag ttatgttagg ttggtttttt aggtttattt      960
   ttatagtttt ttgtggtttt tgttttgggg agttatgttt tgttattgag atttgtgtta     1020
   ttagattgta ttggggttga tttgggggtt gggaatttgt tattttgttg tatagatatt     1080
   gatttttttt tttttttttt aaaaagtaag gtttgttttt attttggttt ttgttggatt     1140
   tttttatttg taatttattt ttttattttt atttttttta attggaagat ttttttatta    1200
   ttttattttt ttattttgtt tttaatttga ttttttatta tataattaat gtttaaattt    1260
   tataagggtt ggttggtgtg gggttggtgt gtttttttgt agatgaaagt ttaagatttt    1320
   ttaattggtt ttagtttatt tgtttggttt taaaataagg gtaattgtaa ttaaatattt     1380
   ttttttagtt ttttgattaa gttgattttt tttttatata tgatttaaat ggtaatttta    1440
   ttaaattgaa aattagtatt tgatttgtag tgttagtatt tggaaagtgt ttgttggtaa     1500
   attgattgta tgaaggtgtg tagttttatt ttttgttatt tttgtgggtt ttagtatttt     1560
   aaaaaattgt ttggtaattt tttggattta attttgtgtt tttggttgta gagtagtatt     1620
   aattagggga gtaatatggt tggtattttg tgtttagaat tttgtatttt aggttgggaa    1680
   atatttaatt aatgtttggg                                                 1700
<210> 125
   <211> 1700
   <212> DNA
   <213> Artificial Sequence
<220>
   <223> chemically treated genomic DNA (Homo sapiens)
<400> 125
   tttagatatt aattaggtgt tttttagttt gaaatatgga gttttggata tgaagtgtta      60
   attatattat ttttttggtt agtgttgttt tataattaaa gatatagggt taagtttaag     120
   aaattattag ataatttttt aaagtgttag aatttataaa aatggtaggg gatgaagtta     180
   tatgttttta tatagttgat ttgttgataa gtatttttta aatgttgatg ttgtaagtta     240
   ggtattagtt tttagtttaa taaaattgtt atttgaatta tatatggggg aggaattgat     300
   ttgattgaga agttaaagag ggatatttgg ttgtaattat ttttattttg aaattagata     360
   aatagattga aattagttaa gaggttttaa atttttattt ataaaaggat atattagttt     420
   tgtattgatt aatttttgtg agatttaagt attaattata taataggaaa ttaggttaaa     480
   agtaagatga agaggtggga taatgaaaag attttttggt taagaaaagt aaaaatagaa     540
   aagtgagtta taaatgagaa aatttgatag aaattaaaat gaaaataaat tttgtttttt     600
   aaaaagaaga aaaaaaaatt agtgtttgta tagtagaatg gtaaattttt agtttttaaa     660
   ttagttttgg tgtaatttgg tagtgtggat tttggtaata aaatataatt ttttaaggta     720
   gaggttgtag ggaattatgg aaatgaattt gaagagttaa tttgatgtga tttttagagt     780
   ttttttttag gttatttttt gggttttttt tttttttttt attttttagg ttttagtagt     840
   ggtttttttt tagggtatgt tttttttatg gttgtagggt tttgggtgtt tggattgtgt     900
   tggattttag ttgtggattt tgtttagtgt attttttggt agttgttttt tttggttggt     960
   tttgtttttt ttgtgtttgt tttatgggtt tttagtgttt tgggtgtttg tgttttttgg    1020
   attttttttt tttagtttgt ttttttggga tttattagtg ggatttggga gaagttggag    1080
   gttgttgagt tgatatattt tgattaatga gtgtatgggg ttgggtggta gtggtgggtt    1140
   gagagtttag ttaagtgttt tttagtttgt gtatttgttt aaggtgtgtg ggttttaggt    1200
   tgaggttgga tttgggggag gttttggatt tgtttttgtt tgggttgtgg ggtgatgagg    1260
   tatttgggta tttttttgat ttgtatttat agagatttat tgtgattttt tagtagattt    1320
   tgtgttagga gttgggtggt tgttttagtt tgtttttggt taagttttgt tatttgtata    1380
   tttttgtgtt ttgtgtgttt tggtgttttt atggtttatt ttttttattt tttattttgt    1440
   tttgttgttg taggattaga gagaggagta gttttttggg tgatttgaag gtttttgtta    1500
   ggttttttaa atttagttat tttatgtgag ggggttgggg agatgggggg tgggtggtgg    1560
   taatgaaaat gttttatttt ttttattttt tattttatta tttttaaaag ggttttgtta    1620
   aatattttta gaattgttta gttttggggt ttttaagaaa ataatatatg ggttttttgg    1680
   gattttttag ttttgagggg                                              1700
<210> 126
   <211> 1700
   <212> DNA
   <213> Artificial Sequence
<220>
   <223> chemically treated genomic DNA (Homo sapiens)
<400> 126
   tagttggtaa gtggtagatt tggaattgga atttagttta gtttgatttt ttaatttatg      60
   tttttgatta ttatattgtt tttttaagtt tagatttgaa attttatttt ttgtgtggtt     120
   gtgtgtttgg gataggggta attaattttt gattatttta tatgttgtat agaatttgga     180
   gaggattttt taaagtaaat gaattttgaa agttggattg tagagtaaat gagtgtagtt     240
   aatttagtta ggggtttgta agagggagaa agagaaaaag attgtggaat ggaaagtttt     300
   ttaatttaag ttttttttaa ggggtagtta ttttttgttt tatagtttag ggtttgtatg     360
   tgtttttttt ggagtggaaa aatatataag ttataaggaa tttaatagat agaaaggtgt     420
   atagaggaat ttaaagtgtg ggttgggggg tgaggtggtg ggtgggaggt gagtgggtgt     480
   aggtggaata ttgtttttta agttaagttg ttgtaaataa aaaggtgtaa agggagagaa     540
   gttggtgttt aatgtgagtt aggagtagtg ttttggtttt ttttttgttt attttaaaag     600
   tattttttgt attgttttta aggtgagaaa taggaaagaa aatgttggtt tgtgtgtttg     660
   ttgtttgttt ttttggttgt ttgtttttgt agggttgttg ggagttttta agttttgtga     720
   gaattttggg agttggtgat gttagattag ttgggttatt tgaaggttag tagtttgggt     780
   agggtttatt gaaagtttat ttgtatatat taggtaattt aattttttat tttgtgtgat     840
   agaagtagta ggaagtgagt tgtttagagg taggagggtt tattttttgt taaagggggg     900
   attagaattt ttttatgtga gttgtttgag gattgggatg ttgagaatgt gagtgatttg     960
   agtagggttt gtttgggtat tgttggggta ggatttggaa tgtatttgga aggttttttg   1020
   taagtattta tttggaagga gaatttggga tttttttggg aattttttgt tttggttgga   1080
   ttggttgagt aagtttggaa aatggtaaat gattatttgg attaattata ggtttttagt    1140
   tggtttgttt gttataattt atgatttggg gttgggaaaa agattaatag tttatgtgtt    1200
   aaaaaagggg tagagtttga tggagttggg tggatttttt tatgttattt gtttttatat    1260
   ttagaggata agtatttttg tagatattta gtgtaaggga gattatgttt gattgtatgg    1320
   atgttttgtt agtgagtttt gggtaaattt tggattttta tattgtgagt ttgttttttt   1380
   gtatgtttta ggagaaagtt tttaaagtat gttttagtgg attgatttaa attgaatggt    1440
   agtattggta tattgtttaa tgtaggttta tttttttttt tttttttatt aagaaaaaaa    1500
   aaattgtttt ttttgtatgt aataaagatg ttggaaataa attgtattgg tagtaagata    1560
   aaggatttaa tgatttaatg ttgaaggggt gtgatatgtt ggtatgtata ttgatttttt    1620
   ttgttgaaaa ttttttgtta gatgtttttt ttttaaataa ttttttttgt ttttatgttt   1680
   ttttttttat taattttata                                                1700
<210> 127
   <211> 1700
   <212> DNA
   <213> Artificial Sequence
<220>
   <223> chemically treated genomic DNA (Homo sapiens)
<400> 127
   tataaggttg gtaaggagag gagtatgggg gtgggagggg ttatttggga agaaaatatt      60
   taataggaaa tttttagtaa agggaattaa tatgtatgtt agtatattat atttttttag    120
   tattaaatta ttaaattttt tgttttgtta ttaatgtagt ttatttttaa tgtttttatt     180
   gtatgtaaga gagataattt tttttttttt ggtagaagaa gggaaaaaaa taaatttata     240
   ttgagtagtg tgttgatgtt gttatttggt ttgggttaat ttattgaagt atgttttgag     300
   agtttttttt tggagtatgt aggaagatgg atttgtagtg tagaaattta ggatttgttt    360
   aggatttatt gatagaatat ttatatagtt aaatatgatt ttttttgtat tgaatgtttg    420
   taaaagtgtt tattttttag gtgtggaggt aaatggtata gaaaagttta tttaatttta     480
   ttaaattttg tttttttttt ggtatgtagg ttgttggttt tttttttagt tttgaattat    540
   gaattatgat agataagtta gttaaaagtt tgtaattgat ttaaatgatt atttattatt     600
   ttttaggttt gtttggttaa tttagttggg gtggggggtt tttagaaaga ttttaagttt     660
   tttttttaag taaatgtttg taaaaagttt tttgaatgtg ttttggattt tattttgatg   720
   gtgtttagat aaattttgtt tggattgttt gtgtttttgg tattttagtt tttaaatagt     780
   ttgtatgggg gaattttggt tttttttttg gtaaagaata gatttttttg tttttgaata   840
   gtttattttt tattattttt gttatataga atgaaagatt gaattgttta atatatgtga     900
   gtgaattttt ggtgaatttt atttgggttg ttaattttta aatgatttaa ttagtttgat      960
   attattaatt tttaggattt ttatagagtt taaaaatttt tagtagtttt gtaaaagtag   1020
   atagttagag aggtaggtag tgagtgtata agttggtgtt ttttttttta ttttttattt   1080
   taaaaataat ataagaagtg tttttaaaat gagtagggga ggagttggga tgttgttttt   1140
   ggtttatgtt gagtattaat tttttttttt ttatgttttt ttatttgtgg tggtttagtt   1200
   tggaaaatgg tgttttgttt gtgtttgttt gttttttgtt tattgttttg ttttttagtt   1260
   tatattttaa atttttttgt gtgttttttt gtttgttaaa ttttttataa tttatgtatt   1320
   tttttatttt agaaaaagta tatgtaagtt ttaaattgta gaatagagaa tggttatttt   1380
   ttgggaaagg tttgggttgg gaaatttttt attttatagt tttttttttt tttttttttt   1440
   tgtaagtttt tggttgaatt gattgtattt gtttgttttg taatttagtt tttgagattt   1500
   atttattttg aaaaattttt tttaggtttt atgtagtata tagagtagtt aggaattggt   1560
   tatttttgtt ttaaatatat agttatatag aaaatgagat tttagatttg gatttgaaaa   1620
   aatagtatag tggttaagag tatgggttgg ggaattaaat tgaattagat tttaatttta   1680
   ggtttgttat ttattagttg                                                1700
<210> 128
   <211> 1700
   <212> DNA
   <213> Artificial Sequence
<220>
   <223> chemically treated genomic DNA (Homo sapiens)
<400> 128
   ttttttttgg atattggagg gaattttgaa tatattgttt tattgtttat gggattggga     60
   agtatatgga ggttttgttg gttaattttt gttttgttat tgttaataag ttattattag    120
   tttttataat tattgggggg ttggtgttga ttgtgggtgg tgtggggtgg gatttgtttt    180
   tgggatggtg gagggtggtg gtgttgtagt tgtgttggtt tttgagtttt agggagtgga    240
   gggtatggag ttgggtgagt tttgtggtgt tagaattttg tgttgttttg atgttggggt    300
   ttagggttta ttttgggggt gtgtgtgtgg ttgtggaggt gggaaggttt ttttgtttgt    360
   tgttgtggag ttttgtgttt ttgtattttg agttttgggg gtttgttttg ggttgttttt    420
   tttttttggt tttgttgttt atttggtttg gtttttgtta gtgttttggt tttttttggt     480
   gtggtgtggg gtttggtgtt tagttggttg ttttgtgttt tggtgtggtt gtgttttttt   540
   tttggttttg gttttggttt tggttttggg ttgtgttgtt tttggttgtt tgtgtggttg    600
   ttttgttgtt gtgtgttgtt ttgtaggatg ttttggatga ggaaggatgt gagtggtttg    660
   gatggggttg gtggtgtggt tttttttgtt tttgtttttt tgggttgtgg ttttggaatt   720
   ttgagtattt tggtggtttt gttttggttt gaatgtattt gtttggtttg tattgttttt    780
   attttttgtt ttttggtttg tttatagttt aggggagtgg gttgtgttgt ttttttattg  840
   gttgtgggat gggttgtgtt gtggattggt tgttttgagt tttaggtgat tttgttgatt    900
   tgtgagtttt gagtattgtt ttgttttgtt tttttttagg ggattttttt tttagttttt   960
   tttttttttt taattttttt ttttttttta attttttttt tttttttagt tttttttttt 1020
   ttgttttgag ttgttttttt aggttaggag tgtattttaa agtggggttt ttttgtttta   1080
   atttatttgt aatagagaga agtttttggg gatgggatga ttaagataaa tagtttagtt   1140
   agttttttag gattgttaga gttaattata ggggatgagt atgtagttat tgtagttgtg   1200
   tggtattgtg agagttttgg gtgttttttt tttttgggag agatggtgta aatttagatt   1260
   tgtaatattt atgattttaa agaggtatga taagtaggtg tagttagatt ttaggttgaa   1320
   agaaggaaaa tatttttgtt aaagtttttt tttgttgtag aagtggtttt ttttggtggt   1380
   ttttttattt gtaataattt gttaattttt tttttttttt agatatatga tatattattt   1440
   ttttgaaaag tttttagatg gatggaaggt gttttaggtt aggataaatg taaaaattgt   1500
   atttaaaaaa gtagtttaaa agaatattag tatatttttt ttgtagatta ttaattgtta   1560
   atatgtaatt tattggttaa ataatgggtt agttagattg gtttaattgg tattgtgatt   1620
   ggattggatg gtattgtgat aatagatgaa agtggaatag agatgatgga agtatttttt   1680
   ttttttgttt tttttttttt                                                1700
<210> 129
   <211> 1700
   <212> DNA
   <213> Artificial Sequence
<220>
   <223> chemically treated genomic DNA (Homo sapiens)
<400> 129
   agaagagaaa agataaggaa agaaaatatt tttattattt ttgttttatt tttgtttgtt      60
   gttatggtat tgtttagttt agttatagta ttggttggat taatttggtt aatttattgt     120
   ttagttaatg ggttatatgt taatagttgg taatttgtaa aaagagtatg ttgatgtttt     180
   tttgaattat ttttttaaat gtagtttttg tatttgtttt ggtttaaaat gttttttatt        240
   tgtttggaaa ttttttaaaa ggatggtatg ttatgtgttt ggggaggaag gaaagttaat     300
   aggttattgt ggataaagga attattaaag aaaattattt ttgtaatggg aaaaggtttt     360
   ggtaaaggtg tttttttttt tttagtttgg ggtttggttg tatttatttg ttatgttttt     420
   ttgaggttgt agatattgta gatttgagtt tgtattattt tttttagaga gagagagtat     480
   ttagaatttt tatggtattg tgtggttgta gtgattgtgt gtttattttt tgtaattggt     540
   tttgatggtt ttgaagagtt aattggattg tttgttttga ttgttttatt tttaggagtt     600
   tttttttgtt gtgggtgggt tggggtagag gagttttgtt ttggggtgtg tttttggttt     660
   gggaaaatgg tttagggtgg agggaggaga gttggagaag gagaggaaat tggggaagga     720
   gagggaattg gggaaggaga gggaattggg gaaggaattt tttagggagg agtggagtgg     780
   ggtagtgttt agggtttgta gattggtggg gttatttggg gtttagggtg gttaatttgt     840
   ggtgtggttt gttttgtggt taatgggagg gtggtgtggt ttgttttttt gggttataag     900
   tgagttggga ggtggaaagt gaaagtggtg tgggttgggt gggtgtattt aggttaaggt     960
   ggggttgttg ggatgtttag ggttttggag ttgtggtttg gggaggtgaa agtggagggg    1020
   gttgtgttgt tgattttgtt taagttgttt atgttttttt ttatttagga tattttgtgg     1080
   gatggtgtgt agtggtaagg tggttgtatg agtagttaga gatagtgtga tttggagttg    1140
   gagttagagt tagagttaga gggaggatgt agttgtgttg gggtgtagaa tgattagttg    1200
   agtattgggt tttgtgttgt gttggaggag gttgagatgt tggtagagat tgagttaggt    1260
   aagtggtgag gttggggaag gggggtagtt taaggtggat ttttagagtt tggggtgtag    1320
   ggatgtgggg ttttgtggtg ataggtagag ggattttttt gtttttgtag ttatgtgtgt    1380
   gtttttggaa tgaattttga gttttagtgt tagggtggtg taggattttg atattgtagg     1440
   atttgtttgg ttttgtgttt tttgtttttt ggggtttaga agttggtgtg attgtagtgt    1500
   tattgttttt tattgtttta ggagtggatt ttgttttgtg ttatttgtga ttggtgttag    1560
   ttttttggta gttatgagaa ttaataataa tttattaata gtgataaagt aggggttgat    1620
   tagtaaagtt tttgtgtgtt ttttaatttt gtgggtagta aagtggtata tttggggttt    1680
   tttttggtgt ttaggagaga                                                1700
<210> 130
   <211> 1700
   <212> DNA
   <213> Artificial Sequence
<220>
   <223> chemically treated genomic DNA (Homo sapiens)
<400> 130
   ttggtttttt aaagttttga gattataggt gtgagttatt gtgttggttt ttaaatatgt      60
   tatttataaa ttgtttttgg tttgtttttt agattgtagg tttaattatg tttttagaaa     120
   aattttttat tagttttgtt ggtttttggg tagtttttat aatgtattaa tttgtttttt      180
   ttttatgttt ttaaggagtt tttttgtttt tatgatttga gattagaatt gttttttttt     240
   tattgttgat gtttaggttt aaatgatatt tttttatatg taaaatgggt tttaagtttt     300
   atttatggtt gtgatgattt gaaagtaaat gtgtgtatat gataatgtgt gagtgatgag     360
   aagtttgtta tttgtaaaat tttatgtgta tgttaattat tatggttgtg ataattatgt     420
   atattataga aatattaatt aagtgttggg tttgttttag gatttttagt attgttggga     480
   ggtttggtat ttgataggtg ttttaggagg gtaggaaagg agagatatgt ttggttttaa     540
   gttttgtgag ggttttgggg agtgttgggt ttttaaggat gttttggatt tgaatatttg     600
   gtgagagtgt tgggtagggg aatagtagga gtaaagtttg gaaggtggtt atgaggataa     660
   gaagggagga tatttggtag gttgttaaaa ttaggtttat ttgttgggta ggtggaggtg     720
   agtaattatt taagtttttt gggatagtgg gagagggggt aggggagttg ttgtattatt     780
   ttttttgtgg ttgttagggg gtttttgttt taggtgagtg ttaagtttta gttttaggtt      840
   gtttttttat gttttaagta agttttgtag tgtagttaat tgttaggggt tgttttgttt     900
   attttttgtt tttgtagtta tttgtttgtg tgaaatgttt gttaattttt gattggtagg      960
   tagtttggta aattaaattg tgatttttga aagtaaaata ttgtagtatt ttggtagttt    1020
   tgaattggga agggatgaag gaggttgtgt ttttgggttg tatgaagagt ttgagttatt    1080
   ttttagaagg ttttgatagg tgggttttag aggagatgtt gttggtgagt agtgattaat    1140
   attgggagga aggggaattg aatttaattt ttgttttttt ttggaaaaag tgaagttatt    1200
   taatgttttt tagtgtatat attttttttt tttattgtta ttagttttgt taatttgggt    1260
   tttgttgttt tggaatgttt tttttagttt tgtattgagg ttaagaggag tgggggtatg    1320
   ggttatttta ttaaaggtga tgttaggttt tattaaatta ggaagtgata tggagttaat    1380
   tttgttagaa tttttttttt ttgtgttgag tggtttgggt tttttggtgg tagtagatgg     1440
   tggagttagt aggtgggatg aggggaggtg tttttggttt aagtttgttt ttggaataga    1500
   ggtgttgttt ttttgagttg taagttttta gtttagtggg atgggatgga agaatgtaat    1560
   ttttttgtga gttaaagttg aggaatggga agtttggtag ggaattggtg tttattttta      1620
   gaagttagat tgttgggtgg tgggaaagag tgtgttttat tgatttgttt agaaagaggt    1680
   aaatttgaat atagatgtta                                                1700
<210> 131
   <211> 1700
   <212> DNA
   <213> Artificial Sequence
<220>
   <223> chemically treated genomic DNA (Homo sapiens)
<400> 131
   tagtgtttgt atttgaattt gttttttttt gaataaatta ataaaatatg ttttttttta      60
   ttatttgatg atttggtttt tggaggtgag tgttagtttt ttgttaagtt ttttgttttt     120
   tggttttggt ttatgagaag gttatatttt tttgttttgt tttattgagt tggaaattta     180
   taatttgagg agatagtatt tttgttttag aagtgggttt agattaagaa tgtttttttt     240
   tattttattt gttaatttta ttatttgttg ttgttaggaa gtttgagttg tttggtatga     300
   agaggagaaa ttttggtaaa gttaatttta tgttattttt tggtttggta gagtttggta     360
   ttatttttag taaggtagtt tatgtttttg ttttttttgg ttttgatgta aaattggaaa     420
   gaatatttta aggtaatggg atttaggttg gtgaggttgg tgatagtaag aaggaagggt     480
   atgtatatta ggggatgtta ggtgattttg ttttttttag aaaaaaatga aggttaaatt     540
   taattttttt tttttttggt gttaattatt atttattggt ggtgtttttt ttaaggttta     600
   tttattaaaa ttttttgaga aatgatttgg attttttgtg taatttggag gtatagtttt     660
   ttttattttt ttttaattta gagttgttaa gatgttgtag tgttttgttt ttaaaggttg     720
   tgatttgatt tgttagattg tttgttagtt agaagttggt aggtattttg tgtaggtaag     780
   tggttgtgag gatggggagt aggtgggata gtttttggta attggttgtg ttgtaaagtt     840
   tgtttgaagt ataagggagt gatttggggt tagagtttgg tgtttatttg gagtggggat     900
   tttttagtgg ttataggaag aatggtgtgg tgattttttt gttttttttt ttgttgtttt     960
   agagagttta ggtggttgtt tgtttttatt tgtttggtag gtgggtttgg ttttagtagt    1020
   ttgttaggtg tttttttttt ttatttttat agttattttt tgggttttgt ttttgttgtt    1080
   tttttatttg atgtttttat taagtattta aatttaaaat gtttttgaag atttagtgtt    1140
   ttttagagtt tttgtgagat ttgggattaa atatattttt ttttttttgt ttttttggaa    1200
   tatttgttgg atattaagtt ttttagtaat attgaaggtt ttgaaatgag tttagtgttt    1260
   ggttaatgtt tttataatgt atataattat tatggttatg ataattaata tgtgtatgga    1320
   gttttataga tgataagttt tttattgttt atatgttgtt atatatatgt atttgttttt    1380
   agattgttat agttatgagt gagatttgaa gtttgtttta tgtatgaaga aatgttattt    1440
   gggtttagat attaataatg agaaagaggt agttttagtt ttagattgtg gggtagagaa    1500
   agttttttgg gaatatggga gaggaatgag ttggtgtatt gtagaaattg tttgaaggtt    1560
   agtagggttg gtgagagatt tttttggggg tgtggttggg tttgtgattt gagggatggg    1620
   ttagggatag tttataaata atatatttgg gggttggtgt ggtggtttat atttgtaatt    1680
   ttaggatttt gggaggttag                                                1700
<210> 132
   <211> 1700
   <212> DNA
   <213> Artificial Sequence
<220>
   <223> chemically treated genomic DNA (Homo sapiens)
<400> 132
   tatgaattag ttatattata tagttgtttt attaaaaatt aaaattttgt agagtatatt      60
   aatagagatt gtatttaaga gaaaggtagt tattttttag ttatttatat aattatttat     120
   taagtattta tgtgttagtt gatatttttg ggtgttggaa taaaataagg aaaaaattaa     180
   gatttttatt tttatgaagt ttattaaagt gtggtaaatg atagagtaat tatttttaat     240
   agtttttagt ttgtattttg taattaattg aatatagtaa atattaaaag ttatttgagg     300
   tagggattta ttttattttt atttgttgga ttttattttg tgtttagtat attagtttgt     360
   aagtaattga tatggattaa ggggtaaaaa taggttttta aatagttaaa attttgataa     420
   gtaattattt gtttgattat taaatatgtt gtttgaaatt tttatttttt tttttaaagt     480
   gtagtaaata agtatttgtg tatttattat gtgtaaaata gtttttaata taatgttttg     540
   tgtgtaaaag gaatggtggg aatggattta agttgtgtat tgtgataaat ggtgtgaata     600
   ttttgagtgg gtggatagtt ttttttttgt gagttttttt tttttttttt ttagttatta       660
   agatagtatg tgtgtgtaga agttgtagtg gtgggatgta gtgttaagtt tgggggtgta     720
   tatattagtt tgtgtgttgt gagtttgttt gggtagtttg gtgggtgtgg tgaaatagtt     780
   tttatgtttg ttttttattg attttttggt attagagtgg atgaggtgtt tttttagagg      840
   aaagattggg tgttgtagat gttttttgga gaagtttttg gttatttgtt ttattttggg       900
   gttttttttt ttttgggttt tttttgattt tttgggtggg tgttttagat aggtttgttg     960
   gatgtgattt ggaaatgggt tgtgtttggt ttagtttgta tttatttgtt tggttatagt    1020
   ttagttatag tttagggatt ttgaggtgtt tgtgtttttt tgttgtggta tttttagttt    1080
   aagtaggttg gatgttgagg ttttgtggta ttgagttttt gtttgttttg tttttttgtt     1140
   ttttattttg ttttgttttt gtttttttat tttttttttg tttttgtttt gtttttttga        1200
   taattttttt gtgtttttgg tttggtatgt gtgttgtgtt tggttgttgt tgttgttgtt    1260
   gttgttgttg ttgttgttgt tgagtgtttt tttgttgtgt ttggttttta gttttgggtt    1320
   ggaattggaa gtttgggggg tggagtttgg tggaggttag gagattgaaa atgtggttga    1380
   gtttggagtt tggagttgga gttagagttt ggattagaat ttggttgttg tttgtattgt    1440
   tgttgttgtt gttgtttgtt gttttttttt tgtgttgtag ttgttttgtt gttattgttg    1500
   tgagtttggt tgttagtggt ttttggattt taggtgtttg ggttgaaggt tggtttggat    1560
   attggattta gttagttttt agattgtttg ggtggtagtg gatgttgttg ttgttgttgt    1620
   ttttttgttg ttttagtttg gtgttttgtt ttgagagatg ggagaggtga gtggagttga      1680
   tagtgatttt gatagtgatt                                                1700
<210> 133
   <211> 1700
   <212> DNA
   <213> Artificial Sequence
<220>
   <223> chemically treated genomic DNA (Homo sapiens)
<400> 133
   aattattgtt aaaattattg ttagttttgt ttgttttttt tgttttttgg aataaaatgt      60
   taggttgagg tgatgaagag gtggtggtgg tagtggtgtt tgttgttgtt tggatagttt    120
   gggaattgat tgggtttgat gtttggattg atttttaatt tagatattta aagtttgagg    180
   attattggtg gttgagtttg tggtggtggt ggtgaggtgg ttgtggtgtg gggaaggggt    240
   ggtgggtggt agtggtggtg gtggtgtagg tggtggttaa gttttggttt aggttttggt    300
   tttagttttg ggttttgggt ttggttgtgt ttttggtttt ttggtttttg ttgggttttg     360
   ttttttaaat ttttggtttt ggtttgaagt tggaagttag gtgtagtgga ggaatgtttg    420
   gtggtagtgg tggtggtggt ggtggtggta gtggtggttg ggtgtagtgt gtgtgttggg    480
   ttgggagtgt gaggagattg ttggggaagt gaggtgggag tgggggaggg gtgagagagt    540
   gagggtgggg tggagtggag ggtgaggagg tggggtggat ggaggtttga tattgtgagg    600
   ttttggtgtt tggtttgttt gggttagaga tattgtgata aaggggtgtg ggtgttttaa    660
   agtttttaag ttgtggttgg gttgtggttg ggtgagtaga tgtgggttag gttgggtatg    720
   atttgttttt aaattgtgtt tgatgggttt gtttggagtg tttatttaaa gggttaggag    780
   aagtttaaga ggaagaggat tttgaggtgg ggtgagtggt tagggatttt tttaaggggt    840
   gtttgtggtg tttagttttt tttttggggg gatgttttgt ttgttttgat attgaggagt    900
   tggtggaagg tggatgtggg agttgttttg ttgtgtttgt tgagttgttt aggtgagttt    960
   gtgatgtgtg agttggtgtg tgtgttttta gatttagtat tgtgttttat tgttgtagtt   1020
   tttgtatatg tgtgttgttt tggtgattag aaaagaaaaa gaaaaaattt atggaggaag   1080
   ggttgtttat ttgtttagga tatttatgtt atttattatg atgtgtaatt tgaatttatt   1140
   tttgttgttt tttttatata taaagtattg tgttgaaaat tgttttgtat gtaataggtg   1200
   tgtaaatatt tgtttattgt gttttgaaga aaaaaatgag gattttgaat gatatatttg   1260
   atgattagat aagtgattat ttgttaaaat tttagttatt taagggtttg tttttatttt   1320
   ttaatttatg ttgattgttt ataagttggt gtgttaggta tggaatggga tttaataggt   1380
   aaaaatagga tggattttta ttttaagtag tttttagtat ttgttgtatt tagttggttg   1440
   tagaatataa attagagatt attgaaggta gttgttttgt tatttattat attttagtga    1500
   gttttgtgaa ggtagagatt ttgatttttt ttttatttta ttttagtatt tagaagtatt   1560
   aattagtata taagtgttta ataaataatt gtataaatga ttaagggatg attgtttttt   1620
   ttttaaatat agtttttgtt aatgtatttt ataaagtttt ggtttttaat aaaataattg   1680
   tataatatgg ttaatttatg                                                1700
<210> 134
   <211> 1700
   <212> DNA
   <213> Artificial Sequence
<220>
   <223> chemically treated genomic DNA (Homo sapiens)
<400> 134
   atttttgtgg ttatttttag ttaggttagt atagtgtttt gtgtttgttg aattgtttta 60
   ggtagttttt tgatgattta aattattatt atttttgggt ttagagtagg ataaatgaaa 120
   ttttaatagg ggtttgtttt gtgattgaaa attatttagt tttatagttt ttgaatgtaa 180
   ggagaggtgg tttttttaga attaatatgt tttttaaggt gggtatgttt atggttttgg 240
   gaaatgtata ttttaggtaa tttataggtg tgatttttta gtggaggtgg ggtagtttga 300
   atttttggtt ttgtaggagt gggaagaaat tgtttttgtt tttttgataa ttgagttttt 360
   tatttggggt tgggagggtg gatagtagga ttggtttttt tattttttat tgtggttttt 420
   tgatttggtt agtagttttg gtgtttagat aaaaggtttt ttgagttggg ggtggggggt 480
   aggttgagag taggttggta aattagattt tgtagttttt taagttatta gttttgtatt 540
   tgggtgtaga gattttgttt taaaaatttg aagtattgtt ttggtttgtt ttttgtttgt 600
   gggtgtgggt ttttgtttat ttatttattt atttatttaa ttatttattt atttatttat 660
   ttttttttga tgtatttgtt tgttttttgg ttgttttggg tgtattattg tttttttttt 720
   tgtttgaggg attttttgtg ttttaggatt attttataga gtttgtttat gtttttggtg 780
   aggtttttgt tgatgatggt gtatgttggt tggatgtgat aggtggtggt tgggataagt 840
   ttgtgtagtg ttagttgttt tttaattttt gttattggta gtgatttggg taggttttgt 900
   ttgttggtga tgattagtag tggtgtgttt tggtttttgg tgaatttggt tattttgtgt 960
   agttttgttt tggttttttt tagttggttt atgtttattg agtttattat gtagatgatg 1020
   ttgtttgtgt agtggttgta ggatttttat agtggttgta gttttttttg gttgtttatg 1080
   ttttagaagt ggtagttgat gtttttggtt gtgttgttgt ttagtttgat ttttttggtg 1140
   ttgaagttga tggtgggtat tgtgtttatg aatttgttga atttgagttg gtagagtatt 1200
   gtggttttgt tggttgagtt taagtttaat atgatgatat gtagggattg gaaggttgag 1260
   atgttagagg agatgttgtt tatggttttt ggttgtggtg ttagttattg tggttgtggt 1320
   gggagggtgt tgttttttga gtagttatgg gtttgatgtg gttgggtgta tttgggtttt 1380
   gtttgttgtt gtttgtattg gttttggggt ttggtgtttt tgtgggtgta tggtttggtg 1440
   tgtttagatg ttgtagtttt tgtgtttttg ttggttgggt ttgtgattgg tgggtattgt 1500
   gtttttatgt gtggttttgt tttttgtgaa gttgttttag atgtttggtg ttttttttag 1560
   gtttgggttt gttttgtggt ttttttgggt tgtgtttttg tgttttggta gttgtttggg 1620
   ttttttgttt ttttggtgtt tggtgtttgt ttttggtttg ggatggtgtt gtttttgttt 1680
   ttgtttttgt ttttgttttt 1700
<210> 135
   <211> 1700
   <212> DNA
   <213> Artificial Sequence
<220>
   <223> chemically treated genomic DNA (Homo sapiens)
<400> 135
   gggagtggga gtgggagtag gagtaggagt agtgttgttt taggttagag gtgagtgttg 60
   ggtgttggga gagtggagag tttgggtagt tgttggagtg tgggggtgtg gtttgaggaa 120
   attatagagt gagtttaggt ttggggaggg tgttgaatat ttgaggtggt tttgtgggag 180
   ataaagttgt gtgtagagat gtgatgtttg ttgattgtga gtttggttgg tgagggtgtg 240
   gggattgtgg tgtttgagtg tgttaagttg tgtgtttgtg gggatgttga gttttggggt 300
   tggtgtgggt ggtggtgggt ggggtttagg tgtgtttggt tgtgttgggt ttgtgattgt 360
   ttggggggtg gtgttttttt gttgtagttg tagtggttgg tgttgtagtt aggagttatg 420
   ggtaatattt tttttaatat tttggttttt tagtttttgt atattgttat gttgggtttg 480
   gatttggttg gtaagattat ggtgttttat tggtttaagt ttaatgagtt tgtgaatatg 540
   gtgtttatta ttggttttaa tattgagaag attaagttga gtaatggtat ggttaagggt 600
   attagttgtt atttttggga tgtgggtggt taggagaagt tgtggttgtt gtggaagttt 660
   tatagttgtt gtatggatgg tattatttat gtggtggatt tggtggatgt ggattggttg 720
   gaggaggtta agatggagtt gtataaggtg attaagtttg ttgagaatta gggtatgttg 780
   ttgttggtta ttgttaataa gtaggatttg tttaagttgt tgttggtggt agagattgag 840
   aagtagttgg tgttgtatga gtttattttg gttattattt attatgttta gttggtgtgt 900
   gttattattg gtgagggttt tattgagggt atggataagt tttatgagat gattttgaaa 960
   tgtaggaagt tttttaagta gaagaagaag tggtaatgtg tttggagtga ttggggagtg 1020
   agtgagtgtg ttaagaaaga atgaatggat ggatggatgg ttggatggat ggatggatgg 1080
   atgagtgaga atttgtgttt gtgaataaag agtgaattaa agtgatgttt tgaattttta 1140
   aaatggaatt tttgtattta aatgtaggat tggtgattta aggagttgtg aagtttgatt 1200
   tattggttta tttttgattt gttttttatt tttagtttag gggatttttt gtttgaatgt 1260
   tagagttatt gattaggttg gggggttgtg gtggggagtg gaagagttgg ttttgttgtt 1320
   tgttttttta gttttaggtg gaaggtttag ttgttggaaa gataaaagtg attttttttt 1380
   atttttgtag ggttagaagt ttaggttgtt ttgtttttat tgggggattg tatttgtgaa 1440
   ttatttgagg tatgtatttt ttagaattgt gggtgtattt attttggggg gtatgttggt 1500
   tttgggggga ttattttttt ttgtatttag gggttgtgaa gttgagtaat ttttggttat 1560
   agggtaggtt tttgttgaaa ttttatttgt tttgttttgg gtttaaaggt ggtggtggtt 1620
   tgggttatta gaggattgtt tgggatggtt tagtgggtat ggagtgttgt gttggtttgg 1680
   ttggggatgg ttgtggaggt 1700
<210> 136
   <211> 1700
   <212> DNA
   <213> Artificial Sequence
<220>
   <223> chemically treated genomic DNA (Homo sapiens)
<400> 136
   agtaaattga ttttgtttgt ttagttttat tagttagggt ggtatggaag tgggtgggga 60
   gagggttggt gtgtggtata gataattggg aagtgtgtgt attatatata tatatatata 120
   tatatatata tatatatata tatatggttt tagttaatat ttatgtagta tttttggggg 180
   gttttttata tatatttgaa tagtatattt ttaataaagg ataggtatat ttttgtttgt 240
   ttttttttaa tttttagatt agtattttat tttgggttta gaattggtgg tgataagttg 300
   atttagatgg agtatttgag aaaaggggag gtgatttttt gtttgaatgg gggagggggt 360
   gttttttatt tattgtttga gtttttggaa tgaggagggt taggatattt tggatttttt 420
   taggttgggt tggtggttgg tttttattgt gtaggaggtg tggtttatgg tgtgatagta 480
   taggtagtgt ttggtgtttg agtagttgtg atgttggatg tggttggtgg ttttgtttga 540
   ggtgtagtgg ttgtgggtag ttttgggtgg tggtgggggt ggtggtaggg ggggtggtgg 600
   tggtggttgt agtggatatt ggggttgggg ttggggtgag ggtggtggtg gtgggggtag 660
   gtggtgatgg ggatgtagga ggtggaattg gggttgttgg agtgggtatt ggtggtgttg 720
   tttgtgtttt tagagatgtt tgggggtttt gagtgtggtt ttgttggtgt tgttgttgtt 780
   ttttttgttg tttgtttggg ttggtgtgtt gttgtttttt gtttttattt tggtttgtgg 840
   ttttgtgatt tgttgtttag tttgggttta ttgtgttggt ttgagtgttt ttttgatgtt 900
   gttgttgttg ttgttgttgt tgttgttttt gtagtttagt agaggttggt gtttagtttt 960
   ttatttgaag gtgttagagt tttagggtta ttgagagggg gttttgttag tgggaggggt 1020
   tatagaatgt ggtagggttt tttttattga ggttatggta gagatatgtt tttgggtttt 1080
   ttaagttatt aagattaggg tgtgtggtgt ttggttttgt tagaggttgg tttttttttt 1140
   tttttttttt tttttttttt tttgggtgtt ggtggtgaga gtagggattg ttagtaattt 1200
   gggttgtaga agtagtagta atggtggtgg tggtagtggt agttgttttt agggtattgg 1260
   aattttgatt ttggagttgt tgaaaagttg gatagtttta ggttggtatg tttgagttgt 1320
   gatttttatt aaaattattt tagttagtta ggagttagat ttgttttttt gatttttttt 1380
   tttgttgtgt ttgtgttttt tttttttata ttgtttttta ttgttttgga gttttttttt 1440
   ttatttttgt ttgttttagt gtttttagag aaatggagtt tggtgatttt tggttggttt 1500
   ttagagtttg ttgatgatga gttttatgtt ggtgttttta aagataagga atgtgattag 1560
   gtaaagttgt ttggaatatt gtttatgtgt gttgttgaaa aattggattt gggtgaagat 1620
   ttaatagtaa gattaattta gttttttttt ttgatatagg ataaaatatt gtgtggtttt 1680
   tttttatagt agggagaatg 1700
<210> 137
   <211> 1700
   <212> DNA
   <213> Artificial Sequence
<220>
   <223> chemically treated genomic DNA (Homo sapiens)
<400> 137
   tatttttttt gttgtgggaa agagttatat aatgttttat tttatgttag ggaaagagat      60
   tgaattaatt ttattattgg atttttattt agatttaatt ttttagtgat gtgtatagat    120
   aatattttag gtaattttgt ttggttatat tttttatttt tggaagtatt agtatggggt    180
   ttattgttaa taagttttga gaattagtta ggaattgtta aattttattt ttttaagagt    240
   attgaggtaa gtggaggtga agaggaaggt tttggggtag tggggagtag tgtggaggag    300
   ggaggtgtgg gtatggtgag gggaaggatt aagaggatag gtttgatttt tgattggttg    360
   gagtggtttt ggtgaaagtt atagtttggg tgtgttagtt tggagttgtt taatttttta    420
   gtagttttgg gattagggtt ttggtgtttt ggaggtggtt attattgttg ttgttgttgt    480
   tgttgttgtt tttgtagttt gagttgttga taatttttgt ttttgttgtt ggtgtttaaa    540
   ggaagggaag aagaaaggga ggaagaagga ttaatttttg gtgaaattgg gtattgtgta    600
   ttttagtttt ggtgatttgg ggagtttggg agtgtgtttt tgttatagtt ttggtggaag    660
   gagttttgtt gtgttttgtg attttttttg ttggtagggt ttttttttgg tagttttgag    720
   gttttggtgt ttttaagtga gaagttaagt attagttttt gttgggttgt agaagtggtg    780
   gtggtggtag tagtagtagt agtattagga aggtgtttgg gttagtgtgg tgaatttggg    840
   ttgggtagta ggttgtggag ttgtgagtta ggatggaggt agagggtagt agtgtgttgg    900
   tttgggtggg tagtggagag ggtagtgata gtgttggtgg ggttatgttt aaagttttta    960
   agtatttttg gaggtatgag tagtattatt agtatttgtt ttggtagttt tagttttgtt   1020
   ttttgtattt ttattattat ttgtttttgt tgttgttatt tttgttttag ttttagtttt   1080
   agtgtttgtt atagttgttg ttgttgtttt ttttgttgtt gtttttgttg ttgtttgggg   1140
   ttgtttgtgg ttgttatgtt ttgagtgggg ttattggttg tgtttggtat tgtggttatt   1200
   tggatattga gtgttatttg tattgttgtg ttatggattg tattttttat gtggtggaga   1260
   ttggttattg gtttggtttg aagaaattta ggatgttttg gttttttttg ttttagggat   1320
   ttaggtggtg agtggagagt gttttttttt ttatttaggt aaagggttat tttttttttt   1380
   tttaaatatt ttatttaagt tggtttatta ttattaattt tagatttagg gtaaaatgtt   1440
   agtttggaaa ttgggggagg ataaataggg gtgtgtttat tttttattga gagtatgtta   1500
   tttaggtgtg tgtaagagat tttttaaaag tattgtataa atgttaattg gggttgtgtg   1560
   tgtgtgtgtg tgtgtgtgtg tgtgtgtgtg tgtgtgtggt gtgtgtgttt tttagttatt   1620
   tgtgttatgt attagttttt tttttattta tttttatatt attttggtta gtgagattaa   1680
   ataggtaaaa ttagtttgtt                                                1700
<210> 138
   <211> 1700
   <212> DNA
   <213> Artificial Sequence
<220>
   <223> chemically treated genomic DNA (Homo sapiens)
<400> 138
   ggatagggaa gtttagttaa gaattaggag tttgatatat ttaatttttt attttatttt      60
   attgatgatt ttgtgaggta tattttttta attttagatt tataaaagag aaagttgagg      120
   tttggagagg ttttttattt tgtttaaagt tatataattt ttatgtggtg tagttaggat      180
   gggaattaga gtttggtttt ttgtattttt aatattattg ttgaatttgg gtttgtttgt      240
   tttttttttt tttttttaat tattgttata tagattttat tatagaattt attatgtgga      300
   gggttattat tttttttttt tttgtatagt atgtttgttt tttttatttt atagagtaag      360
   gtgtttagta aatgtggttg tttggaggta atattttttt tttattgtta tatttggggt      420
   tggggtgtga ggtaaggtag ggttggtgag gtggatgttg tagttgttat tattgttagg      480
   tgttgggaaa tgattttttt gtttgtgtat tgatttgaag gtatttttta ttttttaggt      540
   tttttttgtt ttttgttttt tatttttagt tttttggttt tagtttaggt tttttgggga      600
   gtattttttg ttgtgagatt gatagttttt gggggtgtag ggttttgttt tttgtgtttt      660
   agtttatttg tgtgtagagt tttgttttta ggtgtttgga atttggtggg tattgatgtt      720
   aagtgttggt ggagtgttgt ttatagatgg ttgatttggg ttttttttta tatttttttt      780
   tttttttgtt tttttttttt tttttgtatg ggggtttggg tttattataa aaggtgggag      840
   tgtgtggtgt tttagtaatg atgagtttta gaatgatgga gagtttttgt gtgaggttgt      900
   tgtttttttt gggtgttgtt ttgttgttga tgttattttt gttgggtatt tgtgtttagg      960
   aggatgttga gttttagttt tgagttttgg atatttattt tgttgtggat gatgtttttt     1020
   atgagaagga gttggttggt attttttttg tttttgattt ttttgagttg ttgttttgtt     1080
   tttttttttg tatgtttttt tttttttttt atttttattt ttatttttag agttagggtg     1140
   tggggagttg agtgtaatgt ttaggtattt attgttattt gaagagtgtt ttgagtttat     1200
   gggtttttgg tagtttgttg agtgaatttt ttattttggt ttttttgagt aatagggatt     1260
   ttagtggttt agagatttgt ggttagtatt tgggatagtg tttgttaagt ttttattttt     1320
   tgattatttt ttgtgtttgt ggagttttat tgtagagtgt gtgtgggttt ggggtttttt     1380
   ataattaggg ttggaagtgt gtatttgggt tgggtttgta gttaaggtgg taattttagg     1440
   ttttgaagtg gtgtgttgta gattgaagtg ttgtaagaag ttttgaagaa gtttaagagt     1500
   aaatgtgttt ttatttatga gaagaagtat ggttaagttt ttatggtaag gtttgtggtt     1560
   attttttttt tgtgtttttt taagagaaag tatattgttt tgaattgtat atatagtttt     1620
   ttttgtagga tgtggttaaa taatttaggt aatgggtttg taggattttg tgggtttttt     1680
   tttttttttt gggtgaggaa                                                 1700
<210> 139
   <211> 1700
   <212> DNA
   <213> Artificial Sequence
<220>
   <223> chemically treated genomic DNA (Homo sapiens)
<400> 139
   ttttttattt gggaaggaag aaggagttta tagaattttg taagtttatt atttaagtta      60
   tttagttata ttttatggag aagattgtgt gtatgattta aggtggtgta tttttttttg    120
   gaaaaatatg ggaagggagt gattataaat tttattatgg ggatttggtt atattttttt    180
   ttatagatgg gaatatgttt atttttgagt ttttttaaga ttttttgtag tgttttgatt      240
   tgtaatatat tgttttggag tttgaagttg ttgttttggt tgtgagttta gtttaggtgt    300
   gtatttttag ttttagttat aaggagtttt ggatttatat gtattttgtg gtgggatttt    360
   gtggatatag ggaatggttg aggggtggaa atttagtgga tgttgtttta ggtattgatt    420
   gtgggttttt gagttgttgg ggtttttgtt gtttagaggg gttgggatga gggatttgtt    480
   taatagattg ttaggagttt gtgagtttga gatgtttttt ggatggtagt gggtgtttgg    540
   gtgttgtgtt tagttttttg tgttttgatt ttgggaatag gagtgggggt ggggggagga  600
   gggaggtgtg taagagaaga gataaggtga tagtttaagg gggttgagag tgaggggaat    660
   attgattagt ttttttttgt gggaggtatt atttatggta gagtagatgt ttagggtttg    720
   gggttggagt ttggtgtttt tttgggtatg ggtatttaat agaggtagta ttagtagtag    780
   ggtggtgttt aggaggggta gtagttttat gtgggagttt tttattgttt tgaaatttgt    840
   tgttgttggg gtattatgtg tttttatttt ttatagtgag tttgagtttt tgtgtaggaa    900
   agagggagga ggtgaagaag gaagggggtg tggaggaggg tttgggttaa ttgtttgtag    960
   gtagtgtttt gttggtgttt gatgttaatg tttgttgggt tttaggtgtt tgggaatgag   1020
   gttttgtgta tagatgggtt agagtgtaga gaataggatt ttgtgttttt aaaggttgtt   1080
   agttttatgg taagggatgt tttttgaggg gtttgagttg aagttgaggg gttagaggtg   1140
   agggatggga agtgggaggg gtttaggaaa tggaaaatgt ttttgggtta atgtataggt   1200
   aggagagttg ttttttaata tttagtagtg gtgatagttg taatatttat tttgttagtt  1260
   ttgttttgtt ttgtatttta attttaagtg tggtggtgag aagggggtgt tgtttttaga   1320
   tagttatatt tgttgagtat tttattttgt ggggtagggg aagtaaatat attatataaa   1380
   agaaagagaa atagtggttt tttatgtgat gggttttatg atggaatttg tgtgataata   1440
   gttaaaagaa gagagggaaa atagataaat ttggatttag tagtggtgtt ggaagtgtag   1500
   agagttaggt tttggttttt attttggtta tattatgtaa aagttgtatg attttgaata   1560
   aaatgaagaa tttttttaag ttttagtttt tttttttgta gatttaggat taaaaagata    1620
   tattttataa agttattagt aggatgaaat gagagattaa gtatgttaaa tttttggttt   1680
   ttagttaagt ttttttgttt                                                1700
<210> 140
   <211> 1700
   <212> DNA
   <213> Artificial Sequence
<220>
   <223> chemically treated genomic DNA (Homo sapiens)
<400> 140
   gtgtttgtat tttatgttat tttttgagtt tataaatata aaaatagaat ttatttttgt 60
   tatagattta gttattggga atttattatt tgttatggat tatttttgtt aagaaaagtt 120
   tgaatgagaa gatggtagga tgtttgaaaa aaaaagttat aattaataaa atttgtggag 180
   aattgtaaat tttgttttta ttgttgattt aaattttttt tttaagattt ttttttttta 240
   gatatttatt tatttatttt tattttattt tttagtatta aggaggtttg agtggataag 300
   gtttaagtat tgatgtggtt ttggtaatga atttgaagtt aaaaattggt tagtttatgt 360
   taagttgtta tttgaaaggt tttttttagt atttgaatta agggaaataa tgttttgaag 420
   tagttataaa atttttttga tgtattagat ttgaggtagg atttgataat ttgtattttt 480
   aaattatgta ggtgatgttg atgttgttgg tttggagatt attttggaga gttttatttt 540
   aaaggaaatg aaattgtttt aaaatgttaa tattaaatag ttatataaag ttggaaaaaa 600
   gtaaagaatt ttaaattgaa gatgagaggt tttgaaaaat aatagtggtt attttatttt 660
   tggtgtggtt atgtggttat gaaaaggagt tttggggttg gttgtgtttt tttatgtagg 720
   tttttttata gggttatttt attaattttt gttaaaaatg ttggtttttt tatagggttg 780
   tttggttatg tttttttgtg ttttaattat agagttatat gttatgttgt tgttttgtta 840
   tgtttatttg gtttttgtat agggtgttat gtatgtttaa tgttggtttg gtgtgtgtgt 900
   ttgggaggtt gtttgtaggt tgttaattgg tggtttttag tttttgttga tttggttttt 960
   tttttttgtg gttattagat tgtgggatat tggagatttt gaataatttg ttggagtttt 1020
   ttatagttta aaaatagagg attggttggg tgtggtggtt tatgtttgta attttagtat 1080
   tttgggaggt tgaggtgggt ggattatgag tttaggagat agagattatt ttggttaata 1140
   ttgtgagatt ttgtttttat taaaagtata aaaaaattag ttgggtgtgg tggtatgtgt 1200
   ttgtagtttt agttatttag gaggttgagg tagaagaatt gttagaatta gggaggtgga 1260
   ggttgtagtg attgagattg tgttgttgta ttttagttta ggtgatagag tgagattgtg 1320
   ttttaaaata aaaaaaatgg aggattgaaa attttgaaga ttgagagtaa ggtaataatt 1380
   tttttttgat ttttttgttt tttttataag ggttttttag atggttgaaa ttaatgaaaa 1440
   gagtgaggaa gtaggtagaa aattttagtt ttagtttttt agtatttttt tgtttttttt 1500
   ttggggagtt ggtatttttt ttttttttag tagttgtaaa tatgtttttt aattttaagg 1560
   agattttttt tgtatttttt ttattgtttt tatgtttgta gggttttttg tgtgaggatt 1620
   tagtgtatgt atgtatgtat atgaatgtgt gtgaataatt attttttaat tgagtttttt 1680
   tttaaaaaaa ttattttttg 1700
<210> 141
   <211> 1700
   <212> DNA
   <213> Artificial Sequence
<220>
   <223> chemically treated genomic DNA (Homo sapiens)
<400> 141
   tggaagatga tttttttaaa aaaaaattta attaggaaat aattatttgt atatatttat 60
   atatatatat atatatattg aatttttata tgaagagttt tataggtgtg agaatagtaa 120
   gggagatgta agaaaggttt ttttggagtt gggaggtgta tttatagtta ttagagaagg 180
   gggaagtgtt agttttttga gaagaaagta ggaggatgtt gagagattaa agttgaaatt 240
   ttttgtttgt ttttttattt tttttgttgg ttttagttgt ttgaaaaatt tttgtgagag 300
   gagtaaaaag gttaagggaa gattgttgtt ttgtttttag tttttaaaat ttttgatttt 360
   ttgttttttt tgttttgaga tgtagttttg ttttgttgtt taggttggag tgtagtggtg 420
   tgattttggt tattgtaatt tttgtttttt tggttttagt gatttttttg ttttagtttt 480
   ttgagtagtt gggattatag gtgtgtgtta ttatatttgg ttaatttttt tgtatttttg 540
   atagggatgg ggttttatgg tgttagttag gatggttttt attttttgaa tttgtgattt 600
   gtttgttttg gttttttaaa gtgttgggat tataggtgtg agttattgtg tttggttggt 660
   tttttgtttt taaattgtaa aggattttag tggattgttt gaagtttttg atattttgtg 720
   atttggtggt tgtagaagaa agaggttgag ttggtgggga ttgggaatta ttagttagtg 780
   gtttatgggt aattttttgg atgtgtgtgt tgggttgatg ttaaatgtat gtgatgtttt 840
   atgtgggagt tggatgggtg taatggggtg gtggtgtaat gtgtggtttt gtggttaggg 900
   tatggagggg tgtggttgga tggttttatg agggggttgg tgtttttggt gagggttgat 960
   gggatgattt tatgagggag tttgtgtgga ggggtatgat tggttttaag attttttttt 1020
   atggttgtat agttgtattg ggagtaggat gattgttgtt attttttagg attttttatt 1080
   tttggtttga aattttttgt ttttttttgg ttttgtgtgg ttatttagta ttagtatttt 1140
   agggtagttt tatttttttt aaagtaaaat tttttaaagt ggtttttgga ttagtagtat 1200
   tagtattatt tgtataattt agaagtataa attattaggt tttattttag atttaatgta 1260
   ttagaaaagt tttgtgattg ttttaaaata ttgttttttt taatttagat gttggaggag 1320
   attttttaaa tggtagttta gtataagtta attagttttt aattttaggt ttattattaa 1380
   agttatatta gtgtttaggt tttatttatt tagatttttt tgatgttgga aagtaaagtg 1440
   aaaataggta gatagatgtt tagaaagggg aggttttaga agaaaggttt ggattaataa 1500
   tgaaggtaga atttataatt ttttgtagat tttattaatt ataatttttt tttttagatg 1560
   ttttgttatt tttttattta gatttttttt agtaaaggta gtttatggta agtaatgaat 1620
   ttttagtaat taggtttgta atagaagtaa attttgtttt tatgtttata aatttaaaaa 1680
   gtaatatgaa gtgtaaatat 1700
<210> 142
   <211> 1700
   <212> DNA
   <213> Artificial Sequence
<220>
   <223> chemically treated genomic DNA (Homo sapiens)
<400> 142
   gtatatgtga aaataatatt attttatatt atgatttatg ttatttgtat gtgtaattaa      60
   agttttttaa gttttgatat gtttttataa agatgaagga tagagtgaat gttaaaattt     120
   gttaaatagg agttattgaa agtttgaagg ggaaagttta gttaggtttt attttagaag     180
   tgatttttaa aatgaaatat gtagataagt ttgagttttt tggagttgat ttttaaggtt     240
   ttgtaggtgg taagggggtt gagaattggt tgaggttttt tggaagtttg tgttgatttg     300
   gagggttatt tttgattttt tgatttgggg tgtaattttt agtttttgtt ttggttgata     360
   aaggtgtgga gttaggtttt aggtaagttg ggggtggggg tgtgtataag ggaggggtag     420
   gggattggga tttttttttt tttggggtgt tttgggttgt ttttttagag gttggtggtg      480
   tttttgattt aagtagttta gttttgtttg tgaggtttga agtggtggga atgggttttt     540
   tgtggtggtt ttaaggtgag ggaggttgta aaatttggta gagaatttgg tgtgttgggt     600
   tggggatgtt ttgggtgttg ggggtagggg ttgtaggttt gtaggtagaa gtttttttat     660
   gtgagtgggg gttttggttg tggaaggggt gtttttgggg ttgttggtag tggtgagtgg     720
   gtgggaggga gttttttggg ttttttttgt gttttgggtt ggtggggtga tggtgatgat   780
   ggttttttgg tttttttgtt ttggatgttg atggtggtgt gttttggttt ttttttaata    840
   gttttttttt tgtggttgtt ggagttggtg aggttgagag ggtggggagt tgtgtaggga     900
   aggtgagagg gtggaggttg tgtggtgtag gtggtggtgg tgggaggagg agggttgggt     960
   gggtagggga ggaggaggag gtgggtgttg tgttgtatgt agttttaggt ggggtagttt    1020
   tggtagttga gggatgtagt tagattttgg tgggatgggg tttttgttgg ggtttaggtt    1080
   tagggattag gtggaggtgt tgtgggagtt tttggggtat tatagagatg tgggtgagtg    1140
   ttggtagttg ttggggttga gaggaggtgg tggtggtggt ggtggtgtag gtttggtttt    1200
   gagttgtggt gttgtagtga tttgggttgt gatggttgaa attaggtgtt tggtgaggtt    1260
   tgagttgttg aaatgtggtt gggtagaatg gtggttgggt tgggggattt gtgggtggag    1320
   gatgttgaag gtggtagagg agtgagtggg gttgggtttt gagttatttt ggatgggttg    1380
   gggtggttag gtttggatta tgtattggga aaaagtatga tttattgtgt ttggaggtgg    1440
   tggtttggtg gtgttggttt ttttttttgt tgtgattggg ttgggttttt ggagggtggg   1500
   ttgatgtgtg gtttggggtg gtttgggtgt ttgtgttttg tttggtgagg tgatttttgt    1560
   gggatgttgg gatttgtgtt tttgtttttt tgtgtttatg tttttttttg gtgttgtttt    1620
   ttggttgatg tgtgtttttt tgtgagtttt gggtttaagt gtaggagtag ttgtggagtt    1680
   gggttttttt gttttttggt                                               1700
<210> 143
   <211> 1700
   <212> DNA
   <213> Artificial Sequence
<220>
   <223> chemically treated genomic DNA (Homo sapiens)
<400> 143
   attggaaagt gagagagttt ggttttgtgg ttgtttttgt gtttgagttt agagtttata      60
   ggggaatatg tgttggttgg aaagtagtgt tgggaagaag tgtgggtgtg agggggtaag     120
   ggtatgggtt ttggtgtttt gtggaggttg ttttattagg tgaggtgtgg atgtttgagt     180
   tgttttgggt tatatgttgg tttatttttt gagaatttag tttggttgtg gtggggaaag     240
   gggttggtgt tattaggttg ttgtttttag gtgtgatgaa ttgtgttttt ttttagtgtg     300
   tagtttaggt ttggttgttt tggtttgttt ggggtgattt agggtttggt tttgtttgtt     360
   tttttgttat ttttggtgtt ttttgtttat gagttttttg gtttggttgt tattttgttt      420
   ggttgtgttt tggtggttta ggttttgtta ggtgtttggt tttggttgtt gtggtttgag     480
   ttattgtgat attgtggttt ggggttgggt ttgtgttgtt gttgttgttg ttattttttt     540
   ttggttttgg tggttgttga tatttatttg tatttttgtg gtgttttaaa ggtttttgtg     600
   atgtttttgt ttggtttttg ggtttgggtt ttggtgaaag ttttgttttg ttaaggttta      660
   gttgtgtttt ttagttattg gggttgtttt gtttggagtt gtgtgtgata tggtgtttgt     720
   tttttttttt ttttttgttt gtttggtttt tttttttttg ttgttgttgt ttgtattatg     780
   taatttttgt ttttttgttt tttttgtgtg gttttttgtt tttttggttt tattggtttt     840
   ggtgattgtg ggggagaagt tgttggggga gggttggaat gtattgttat tggtgtttgg      900
   ggtgggggag ttgggaaatt gttgttgtta ttgttttgtt agtttgaggt gtgggagggg     960
   tttggaaagt ttttttttgt ttgtttgttg ttgttggtgg ttttgggggt gttttttttg     1020
   tggttgaggt ttttgtttgt gtggggaagt ttttatttgt gagtttgtgg tttttgtttt    1080
   tggtatttga gatatttttg atttggtgtg ttggattttt tgttaggttt tatagttttt      1140
   tttgttttgg gattgttgtg gaggatttgt ttttgttatt ttggattttg tgagtggggt    1200
   tgagttgttt gggttgggga tattattagt ttttggagaa atggtttgaa gtattttgaa    1260
   gaagaggggg ttttagtttt ttgttttttt tttgtgtgta tttttatttt tagtttgttt    1320
   ggaatttgat tttatatttt tgttgattgg gatagggatt gggggttgta ttttagatta    1380
   gaaagttaga ggtggttttt tgaattggtg tggatttttg aggaatttta gttaattttt     1440
   gattttttta ttatttgtaa ggttttagga attggtttta gaaagtttga gtttatttgt     1500
   gtgttttgtt ttaaaaatta tttttaagat aaaatttaat taggtttttt tttttagatt    1560
   tttagtaatt tttgtttggt aaattttgat atttatttta ttttttgttt ttgtaaggat      1620
   atattaagat ttagaaggtt ttgattatat atatggatgg tatgagttgt gatataaaat    1680
   agtattgttt ttatgtgtat                                                1700
<210> 144
   <211> 1700
   <212> DNA
   <213> Artificial Sequence
<220>
   <223> chemically treated genomic DNA (Homo sapiens)
<400> 144 gatgattttt gggtttataa 1700
<210> 145
   <211> 1700
   <212> DNA
   <213> Artificial Sequence
<220>
   <223> chemically treated genomic DNA (Homo sapiens)
<400> 145
   ttgtggattt agagattatt ttttttgatt tgatattttt tttattgttt tggtttagtg        60
   ttttttttgt aaggttggaa gtttttttag attggttatg ttttattttt ttttggaggg     120
   aagatgattt taaagatgat tttttttttt atggtgttgt tatattgtag gtggttgtta       180
   ggggtttttg tttggtgttt ttgtgagata gttgagtttt ggttggttgt gtggtgtgtt        240
   gggtgtatga gggggttgtt ttggagtgat ggtggttgta gttggagtta ggtgtttgtt       300
   tgtttgttgg ttggtttgtt gggattttgt gtattggtgg tagagttttt tgtgtggatt       360
   ggtaagtgat gttttatttg ttttgagttt attatttttt tttgtgttgg ttgtagttga         420
   tttggtgaag ggagttgatt gggttttggg ttggaggtaa aattttatgg tgagtaagaa       480
   tttgttttaa gttaggggag gtggtgtagt ttggtggttg tttgtttttg attttgtttg      540
   ggtgggtggt gaggtttggg gtgtatttgg gtgtgggtgt aagaaggtgt gggaggtggt       600
   ggattggttt tttgtttgtt ggttatgggt ttttggggtt ggagtttttt ttagattttt       660
   gttggtgttt gggtttttgg ttggtttttt gtgtgtattt tttggtagga ataagggttg        720
   tttatttttt attttgggat tttgatttgt ttttgatttg aaaagatata aattaataag         780
   attgtttttt ttttggggtg taagattttg agtttatttt tagttgtgga tgtttgtagg       840
   gtgtgtgttg ggttgtgggt ggtgggaaga taaattttta taaaagtgtg tttgggttgg       900
   gggataatgt ttgggtgttt tgattttgag ggaggagttt tggtttgggg tagtgtaggg       960
   gaagtttgta ttgttagtta ggttgttttt ggggttgatg atgttttatg gaggtgggga      1020
   gtgtgtaaag gttgtataaa ttgtgtttaa ttttggggtt aataattgtt aaatatttgg      1080
   aattttagtt tttttttttt tttgaattgg ggaagaaggt gaaaattttt taagtttttt      1140
   ttgattgttt ttttttattt ttagattttt gttgggaggg taaagtgttg atttttggtg      1200
   tttggtaagt attagagatt ttaaattttt tgggattttt ttttttgtgt ttttttttga     1260
   tttttttttt taattttttt tatagagatt tttttatgta gttgattgag attgtggtga      1320
   atggtttttt gtttttttgt gtttttttta ttgtttgttt ttttaatatt tggtggggtt     1380
   tggggagaga aggaagtttt tttggttttt ttttttggtt tttatgttag ttttggtagg      1440
   ggattttagt tgggaaagtg gaggagtttg attttagtga ggttgtttta ttttgttttt      1500
   gtggttagag ggtggaggga aagttgtttt ttttttgttt ttgttgttgt ttgtggttta      1560
   gggtgtattt tttagatttt agtttaggtg tgttgtaaag gtttaaattt gagaaggtgt      1620
   tgtttttgag atagtggaag tgtgttttgt tttaatttag agtgtttagt ggttggtttt      1680
   agaggatttt aatttttagt                                                  1700
<210> 146
   <211> 1700
   <212> DNA
   <213> Artificial Sequence
<220>
   <223> chemically treated genomic DNA (Homo sapiens)
<400> 146
   ttaggatttt taattattta ttatttgtat ttttggggtg gatgtgagtt tgtgttaaat        60
   taaggatatt agttgagtat ttagtgtggt tttttatgtt ttttaggaat tttggaaagt       120
   tatttaagtt tggtgttatt tttaaaagga atgagttgtg gtttttaatt agtttgttga       180
   ggagagtgta gagtgatatg ggatgggggt ttagggtatt gaggttaaga gtttttgagt       240
   tatgttagtg tttgtttttt tggtttttgt ttaaaatttt gtgatgtttt gttgataaag       300
   tatttagtgt tagagtttgt ggtttagtga agttgttttt tggaaatata tatatatata       360
   tatatatata tatatatata tatatatata tatataaaag ggggtgggga gagggaaagg       420
   tttaggttta ggagaggaag ttagttagtt agttttgtag atgtaattgt tgtaatttgg        480
   gttttttttg ttttttgatg taatgttagt tttttgtttt tgtgttgtag tgtgtagttt       540
   gttgttggga agttagatta attgattgaa tgttaagatt attattgtta ttattttgtg       600
   atttattttt tatgaatata ttttaattgg tttgagtttt gtttttaaat tttatgattt       660
   ttggtttttt aggaagtatt gttttttttt ttttttagga aggttgtttt tatgtatagt 720
   tagtattagg aaatggtgtt gtttggggtt ttgatggaga gttggggtgg ggttggttta 780
   aattttttgt tttttgtttt tttgagattt ttgatttttg gttttgtttt aagtaaaagt 840
   attgtagaag ttgtagtgtt tttttttttt ttttagaaat tgagtgagga tgtgggggaa 900
   aagttatttt ggaatgtgta aggatgtttt tttatgttta atatgttata gggttgggat 960
   gtgtggttgt tgttttgtta ttttatgtgt gtagtgttaa ttgtagtttt gtagttattg 1020
   ttgttgttgt tgttttgttg agtttgggga gatttttaag ggttttttaa atatgagtta 1080
   taaatattat aggtgtttgg tgtgtgtgtt atgatatatt tggtttttga ttgggtggtg 1140
   taattttgtt tatttgtata ggttgattgt ataattgggt ggtgatttta gtttggtgtt 1200
   tgtgttatgt tttttttatt tttgtgggta tgtggggttg tgggtgggat agtggggtgg 1260
   tgattgtggt gtttagagtt ttttgaattt gggaattttt taagttttga gttaggtggt 1320
   ggttttttgg gatgttagtg ttttttagaa ggtttagttt tggttagtta agggtttatt 1380
   gtttaaattg ggtttttttt atttttatag aaaaggatag tttttagagt gatattttag 1440
   taaatgtttt gggtggtttg tagaggttga gtttaaatta tgtgagattt ggttgtggtg 1500
   agagttttgt ttaatgattt gtgatttggg gtgtttaaaa agggtttttt gtatgatttt 1560
   taataagatt ttgttatttt gaaaatgaaa tagggagggt ttgaaaatgt aagatgtgta 1620
   atgttttgtg gtttaaattt agtttttaaa aaatgtatta atgagttaat atgaaatagt 1680
   gatatggaga ttttttttta 1700
<210> 147
   <211> 1700
   <212> DNA
   <213> Artificial Sequence
<220>
   <223> chemically treated genomic DNA (Homo sapiens)
<400> 147
<210> 148
   <211> 1700
   <212> DNA
   <213> Artificial Sequence
<220>
   <223> chemically treated genomic DNA (Homo sapiens)
<400> 148
   tattttaagt tattaagttt tagataggta tagtagtata tatgtgtgaa tagaatttat      60
   tagtatagtt tggggtttag gtttttgtat attaataatt taaggttttt ggtaatggtt     120
   tagtgtgtat ttatgaggtt attgtttttt tatatatgta tttagaatat ttatagtttt     180
   aaaatggtaa ttttattata aaaatgatat tttgtatttg tatagtattt tgttttttgt      240
   gtattttata tatttattat ttaatgattg tgttttttga aatttttaat aattttgggt      300
   ggtggtaaga ataggtattt ttaagttatt tttttagatg aggaaattta tttgtgtttt     360
   tttttttttt tttaatagtt ggttgatgga atttaagttt tttatattag tagtggggtt     420
   agttgtatag agtggttaga agggagaggt tttggggtta tgtttttttt tttattttta     480
   gtttttgatt tattgttata ttatggtgag taataggata atttttttta tagaattatg     540
   ggggatatta tttttttgag gttaatgtgt ttaattaagt gggaaatttt ttgggtagag      600
   tgaaatttga agttgttatg ttataagata atttagagta aggggaataa ttgatgtttt     660
   aatgtttttt tttatggggg gttgaggggt gtttatttat attttttttt tttgtttttt     720
   tttgttattt tttttttgtt ggttggttta attttataag tgtgtgtttg ttttgagtat      780
   agtaatttag agtagatggg gggtattttt tagagggtag tggaaagttt atttgttatt     840
   gttgtgttgg ggatttgtgg ttttttgggt ggttatgttt tgttttggtg gtgtttggtg      900
   tgtttggaat gtggggtttt tttttgtttt tttttttatg ttgttttgtt ttgtttttgt     960
   tagggttttt tagagaagtt tgatttttta ttatttttgt tttgtatttt tttggtttga    1020
   gggttggtgg ttttttattt ttgttttaaa tattttgttt ttgagttttt tttttaaaga   1080
   ttttttagtg ttggttgttt ttttagtttt ttgtttttga tggtttttgg ggttagttgt      1140
   tggggttttt ttgttaggtt gtattttttt ttttagtgtt tttatatttt gttagttggt     1200
   ggtttattgt gggtgggtag ggtgtgtttt gtttatttgg gttgtgtgtt gagagttgag    1260
   agtgttttta aatgttgagt agagttggtt ggtttgggtg gggtttttat atttgttata    1320
   ttttttttag gtgaggtgag gtttgttttg ttttgttttg tttttttttt atatggtttg    1380
   tttttatttt aagttagggg tgatttattt tggtttgttg gtttgtaggg attgtgtttt    1440
   ggaatgtttt tgagttgtgt tatgtgggag tggttttagt gtttttggtt ttgttttttg     1500
   agaaagtttg gttgaggttg ttttgaggtt tatagggatt ttgttttttt tttttttttt    1560
   tattttattt tttaagtgtt atgttgagtt tttagttttg gtttgggtta ataaattaat    1620
   aagattttag tagtagattt tgggtttgat taagtgtttt tttttttttt tttttttttt    1680
   tggtgttaat gtaatggagg                                                1700
<210> 149
   <211> 1700
   <212> DNA
   <213> Artificial Sequence
<220>
   <223> chemically treated genomic DNA (Homo sapiens).
<400> 149
   tttttattgt attaatgtta aaaaaaaaaa aaaaaaaaaa aaaatattta attaagttta     60
   gagtttatta ttaaagtttt gttaatttat taatttaagt tgaggttgag agtttgatgt    120
   ggtatttaag aagtaaaatg aggaaaggag gaagaaatgg ggtttttgtg ggttttgggg    180
   tagttttggt tgggtttttt tggagggtag ggttaggggt gttggggttg tttttgtgtg     240
   gtgtggtttg ggagtgtttt agggtgtggt ttttgtgggt tggtgggttg gggtgagtta    300
   tttttgattt ggggtgggag tgggttgtgt aagggggagg tggggtgggg tggggtgggt    360
   tttgttttgt ttagggagga tgtggtgggt ataaaagttt tatttaggtt agttggtttt    420
   gtttagtatt tggggatgtt tttagttttt ggtgtatggt ttaggtaagt ggggtgtgtt    480
   ttgtttgttt gtgatgggtt gttagttagt ggggtgtgga gatgttggga agaagggtat    540
   ggtttggtag ggaggttttg gtaattggtt ttggaggttg ttgggggtag ggggttgaag    600
   ggatggttgg tattggagag tttttgggga gggggtttag ggataaggtg tttggggtag    660
   gggtgagggg ttattggttt ttgggttggg agggtgtagg gtagaggtgg tggggggttg   720
   ggttttttta agaagtttta gtgggagtgg ggtgaggtgg tgtggggaga ggggtggagg    780
   gaggttttgt attttagatg tgttaggtgt tgttgaggta gggtgtggtt gtttagggag     840
   ttgtaggttt ttggtgtagt agtagtaagt gggtttttta ttgtttttta gaaagtgttt    900
   tttatttgtt ttaagttatt gtatttgaaa taaatgtgtg tttgtggagt tgaattgatt    960
   agtaggaggg aagtagtggg ggaagatgag aggggggagt gtgagtaggt attttttagt  1020
   tttttataaa agaagatatt aaaatgttag ttattttttt tgttttaggt tattttgtag   1080
   tatagtaatt ttggatttta ttttatttgg agagtttttt gtttggttga atatattggt   1140
   tttaggaagg tagtgttttt tatgattttg tggggagggt tgttttgtta tttgttatga   1200
   tgtgataatg agttaggaat tgagagtaga gagaagggtg tggttttggg gttttttttt   1260
   tttggttgtt ttgtgtagtt ggttttattg ttggtgtgga gggtttgggt tttattagtt   1320
   ggttgttgag aagaaggagg ggaatataga taagtttttt tatttgggaa ggtggtttaa   1380
   ggatgtttat ttttattatt atttagagtt attggagatt ttaaaaaata tagttattag   1440
   gtaataaatg tgtaaagtat ataaaaaata aagtgttgtg taaatgtaaa gtgttatttt   1500
   tgtagtaaga ttgttgtttt aaagttgtgg gtgttttgag tgtgtgtgta aaagagtagt   1560
   ggttttatgg gtgtatgttg ggttattatt aagaatttta agttgttggt gtgtaaaggt   1620
   ttgggtttta gattgtattg atggattttg tttatatgtg tgtgttattg tgtttgtttg    1680
   gaatttggtg atttggaatg                                                1700
<210> 150
   <211> 1700
   <212> DNA
   <213> Artificial Sequence
<220>
   <223> chemically treated genomic DNA (Homo sapiens)
<400> 150
   ggttttttgg tagtttaagt gttagtttag atattgagat tttggtaaag agtttttttt        60
   tatttttttt ttttattggt ttttgtgggt ggtgtggaat gtgttgtagg ttttgtgaga      120
   gtggtatttt tttttttttt tttttttttt ttttttaatt tattgtagtt tttgagaggg      180
   tagtattttt gggtgtattt gtatttaagt ttttgatagt gttttgggtt tttttagtaa      240
   tgtgtattta gggatgttat gttttgtggg ttttatgttt ggggtatgga gttaggggag      300
   gtggttattt tatttttttt atgtttttgt ttattttgtg ttttagtgga gttttaggta      360
   aataagttag gtttttagtt tttaaggatg gtttttattt tagattttag gaggaggtgt       420
   tttgggttat tttgggaagg ttagtttgtt tgggaggggt agattatgaa aatggttttt      480
   taggtagtgt tatttttttt ttattgttag ttatttaatt taggtggttt tatttttttt      540
   gtttttatgg atatgtagta ggatgtagag agtatagagt tgtgattggt atgagtttat      600
   aatgggtttt tttgtttaat ttatttaatt tttagtaagg gataggtttt gtttattggg      660
   gtttgttgag gttttaaatg tgtatgttgt taagggagta gttgtggggg tttgttagtg      720
   gggataaagg tgtagttatt ggggtgaaag ttttgttttg tagagggtgg gtttgtggtg      780
   attgtgagtt tagagtttgg atttgttgtg taatttgttt tttttggttt tattttaatt      840
   tagttggatt tttggtttgg tttttgtttt ttggagattt agttattaaa gttgaggtgg      900
   ggggatgagg atgtgggtgg tgtttaggta attttgaggg gttttgatgt tttttttaga      960
   gttagttatt aggagtaata tgatgtgttt ttatattatt gaatttagaa ttttagtgtt     1020
   ttagtatttt ttttttgata tgagttttgt ttaatgagga gaagggtttt ttgtattggt     1080
   tggtggtggt tttttattta attagagagg ttaatggttt ttttttgttt ggtttagaag    1140
   gaattgttgg gggaatatag gttaaattat aatttttgag aggtagttgg gttttgtggg     1200
   gggggggggg ggggtgtttg tgtgtttatg tgattttttt tttttttgtg gagattgagt     1260
   tatgtgatag gtttgtgagg gggtggggtt tggtgtttgt ttgttgtgtt agtggttaat     1320
   tggtagttag gtagggtggg tgtgtgtagg gggtggggtt gggtgtgtgg tagggtgtga     1380
   gagtgtattt gtggtggtgg tggtggtgat tgtggggggg tggtggggaa tattggttaa     1440
   gttgatagtg gaggtttagg tattggtggt gggtggttgt ggtttttggt gttgtttggt     1500
   gtgtggttag tttttggaat ggaatgtttg gtgttgtggg ttttgtttgg aaagtttgtt     1560
   gtggagttgt gattttttgg tttgtgtggt ttggtatgaa gtggtgttga ggagttgttg    1620
   ttgttgtttg ttgttgttgt tgttgttgtt tgaggaggag ttgtagtatt ttgggttatg     1680
   ttgatgatta ttgtaaattg                                                 1700
<210> 151
   <211> 1700
   <212> DNA
   <213> Artificial Sequence
<220>
   <223> chemically treated genomic DNA (Homo sapiens)
<400> 151
   tagtttgtag tagttattgg tgtggtttag ggtgttgtag ttttttttta ggtggtggtg      60
   gtggtagtgg taagtggtgg tagtagtttt ttaatgttgt tttatgttgg gttgtgtggg     120
   ttaagaggtt gtgattttat ggtaaatttt ttgggtgggg tttgtgatgt tgagtgtttt     180
   gttttgaaag ttggttgtgt gttgagtagt attaggaatt gtagttgttt gttattggtg     240
   tttaagtttt tattgttggt ttagttaatg ttttttgttg tttttttata gttgttgtta     300
   ttgttgttgt gggtgtgttt ttgtgttttg ttgtgtgttt ggttttgttt tttatgtgtg     360
   tttattttgt ttggttgttg attggttatt aatatggtga ataggtgttg ggttttgttt     420
   ttttataggt ttattgtgta atttggtttt tgtgagaagg aaggaagtta tgtgaatatg     480
   taggtgtttt tttttttttt tttgtagagt ttagttattt tttgggaatt gtagtttggt     540
   ttgtattttt ttggtgattt tttttaaatt agatgagaga aggttgttag tttttttaat     600
   tggatagggg attattattg gttaatgtag aagatttttt tttttattgg gtggagttta     660
   tgttgaggaa ggggtgttga agtattgagg ttttgggttt ggtgatgtgg gggtgtattg     720
   tgttattttt gatggttggt tttagggaag gtattagggt tttttagagt tatttggatg     780
   ttgtttatat ttttattttt ttattttaat tttaatggtt gagtttttag agggtaggga     840
   ttggattgag agtttagtta ggttggggtg gaattgaagg agatagattg tgtgataagt     900
   ttggattttg agtttgtggt tgttgtaggt ttattttttg tggggtggag tttttgtttt     960
   ggtggttgtg tttttgtttt tattagtggg tttttgtagt tgtttttttg atagtatgtg    1020
   tgtttgaggt tttagtgaat tttaatgggt agggtttgtt ttttgttgga agttgagtag    1080
   attgagtggg aaagtttgtt gtgagtttgt gttaattata gttttgtgtt ttttgtattt    1140
   tgttgtatat ttatgaaagt gggagggatg gagttgttta aattgggtgg ttggtagtgg    1200
   agaggaagtg atattgttta aagaattgtt tttgtgattt gttttttttg agtaagttag    1260
   tttttttgag atggtttaag atgttttttt ttggggtttg gggtgaaggt tgtttttggg    1320
   gattgggagt ttggtttgtt tatttaaggt tttgttgagg tgtaaggtgg atagggatgt    1380
   gagagaagta gggtgattgt tttttttagt tttgtgtttt gggtataggg tttatagggt    1440
   atagtatttt tagatgtgtg ttgttggagg aatttagggt attgttagag gtttgggtgt    1500
   aggtgtgttt aaaaatgttg tttttttaga ggttataata ggttaaaaaa aaagagagaa    1560
   aaaaaaaaaa gaaatgttgt ttttgtaagg tttatagtat attttatatt gtttataaag    1620
   gttagtggga agagagggta aagggaagtt ttttgttgag gttttaatgt ttggattgat    1680
   atttgagtta ttagggagtt                                                 1700
<210> 152
   <211> 1700
   <212> DNA
   <213> Artificial Sequence
<220>
   <223> chemically treated genomic DNA (Homo sapiens)
<400> 152
   gttttggata ttttttgttt ttttgattta aggtttagtt tggttttttt tagggttttt      60
   tggtagttta agtgttagtt tagatattga gattttggta aagagttttt ttttattttt     120
   tttttttatt ggtttttgtg ggtggtgtgg aatgtgttgt aggttttgtg agagtggtat    180
   tttttttttt tttttttttt ttttttttta atttattgta gtttttgaga gggtagtatt      240
   tttgggtgta tttgtattta agtttttgat agtgttttgg gtttttttag taatgtgtat   300
   ttagggatgt tatgttttgt gggttttatg tttggggtat ggagttaggg gaggtggtta    360
   ttttattttt tttatgtttt tgtttatttt gtgttttagt ggagttttag gtaaataagt    420
   taggttttta gtttttaagg atggttttta ttttagattt taggaggagg tgttttgggt    480
   tattttggga aggttagttt gtttgggagg ggtagattat gaaaatggtt ttttaggtag    540
   tgttattttt tttttattgt tagttattta atttaggtgg ttttattttt tttgttttta    600
   tggatatgta gtaggatgta gagagtatag agttgtgatt ggtatgagtt tataatgggt    660
   ttttttgttt aatttattta atttttagta agggataggt tttgtttatt ggggtttgtt   720
   gaggttttaa atgtgtatgt tgttaaggga gtagttgtgg gggtttgtta gtggggataa    780
   aggtgtagtt attggggtga aagttttgtt ttgtagaggg tgggtttgtg gtgattgtga    840
   gtttagagtt tggatttgtt gtgtaatttg ttttttttgg ttttatttta atttagttgg    900
   atttttggtt tggtttttgt tttttggaga tttagttatt aaagttgagg tggggggatg    960
   aggatgtggg tggtgtttag gtaattttga ggggttttga tgtttttttt agagttagtt     1020
   attaggagta atatgatgtg tttttatatt attgaattta gaattttagt gttttagtat   1080
   tttttttttg atatgagttt tgtttaatga ggagaagggt tttttgtatt ggttggtggt  1140
   ggttttttat ttaattagag aggttaatgg tttttttttg tttggtttag aaggaattgt  1200
   tgggggaata taggttaaat tataattttt gagaggtagt tgggttttgt gggggggggg   1260
   gggggggtgt ttgtgtgttt atgtgatttt tttttttttt gtggagattg agttatgtga    1320
   taggtttgtg agggggtggg gtttggtgtt tgtttgttgt gttagtggtt aattggtagt   1380
   taggtagggt gggtgtgtgt agggggtggg gttgggtgtg tggtagggtg tgagagtgta   1440
   tttgtggtgg tggtggtggt gattgtgggg gggtggtggg gaatattggt taagttgata   1500
   gtggaggttt aggtattggt ggtgggtggt tgtggttttt ggtgttgttt ggtgtgtggt   1560
   tagtttttgg aatggaatgt ttggtgttgt gggttttgtt tggaaagttt gttgtggagt   1620
   tgtgattttt tggtttgtgt ggtttggtat gaagtggtgt tgaggagttg ttgttgttgt   1680
   ttgttgttgt tgttgttgtt                                               1700
<210> 153
   <211> 1700
   <212> DNA
   <213> Artificial Sequence
<220>
   <223> chemically treated genomic DNA (Homo sapiens)
<400> 153
   ggtggtggtg gtagtggtaa gtggtggtag tagtttttta atgttgtttt atgttgggtt      60
   gtgtgggtta agaggttgtg attttatggt aaattttttg ggtggggttt gtgatgttga     120
   gtgttttgtt ttgaaagttg gttgtgtgtt gagtagtatt aggaattgta gttgtttgtt     180
   attggtgttt aagtttttat tgttggttta gttaatgttt tttgttgttt ttttatagtt     240
   gttgttattg ttgttgtggg tgtgtttttg tgttttgttg tgtgtttggt tttgtttttt     300
   atgtgtgttt attttgtttg gttgttgatt ggttattaat atggtgaata ggtgttgggt     360
   tttgtttttt tataggttta ttgtgtaatt tggtttttgt gagaaggaag gaagttatgt     420
   gaatatgtag gtgttttttt tttttttttt gtagagttta gttatttttt gggaattgta     480
   gtttggtttg tatttttttg gtgatttttt ttaaattaga tgagagaagg ttgttagttt     540
   ttttaattgg ataggggatt attattggtt aatgtagaag attttttttt ttattgggtg     600
   gagtttatgt tgaggaaggg gtgttgaagt attgaggttt tgggtttggt gatgtggggg     660
   tgtattgtgt tatttttgat ggttggtttt agggaaggta ttagggtttt ttagagttat     720
   ttggatgttg tttatatttt tattttttta ttttaatttt aatggttgag tttttagagg     780
   gtagggattg gattgagagt ttagttaggt tggggtggaa ttgaaggaga tagattgtgt     840
   gataagtttg gattttgagt ttgtggttgt tgtaggttta ttttttgtgg ggtggagttt     900
   ttgttttggt ggttgtgttt ttgtttttat tagtgggttt ttgtagttgt ttttttgata     960
   gtatgtgtgt ttgaggtttt agtgaatttt aatgggtagg gtttgttttt tgttggaagt    1020
   tgagtagatt gagtgggaaa gtttgttgtg agtttgtgtt aattatagtt ttgtgttttt    1080
   tgtattttgt tgtatattta tgaaagtggg agggatggag ttgtttaaat tgggtggttg    1140
   gtagtggaga ggaagtgata ttgtttaaag aattgttttt gtgatttgtt ttttttgagt    1200
   aagttagttt ttttgagatg gtttaagatg tttttttttg gggtttgggg tgaaggttgt    1260
   ttttggggat tgggagtttg gtttgtttat ttaaggtttt gttgaggtgt aaggtggata    1320
   gggatgtgag agaagtaggg tgattgtttt ttttagtttt gtgttttggg tatagggttt    1380
   atagggtata gtatttttag atgtgtgttg ttggaggaat ttagggtatt gttagaggtt    1440
   tgggtgtagg tgtgtttaaa aatgttgttt ttttagaggt tataataggt taaaaaaaaa    1500
   gagagaaaaa aaaaaaagaa atgttgtttt tgtaaggttt atagtatatt ttatattgtt    1560
   tataaaggtt agtgggaaga gagggtaaag ggaagttttt tgttgaggtt ttaatgtttg    1620
   gattgatatt tgagttatta gggagtttta gagggagtta aattagattt tgggttagga    1680
   agatagggaa tgtttagggt                                                1700
<210> 154
   <211> 1700
   <212> DNA
   <213> Artificial Sequence
<220>
   <223> chemically treated genomic DNA (Homo sapiens)
<400> 154
   tgagggtttt ggtttttttt tttgtgtttg ttttttgttt agtgttggga gaagtagtaa      60
   taagttttgt attttagtaa ttaattttag ttattatatt tgtattaatt agtgttgttt     120
   aagttttggg tttggggtgg ggtttgtttt taaggtggtt tggggttttg gttgttgaag     180
   tttttgttat gaatttgtat tattttagtt ttggggtttt ttgtgtttgt ttttttttgg     240
   ggtttggtgt tagtgtgttg ggggtgtgtt ttggtttttg ggtgtattga gttggagagg     300
   gtgtaggtgg aggagaatgg tgggagtttg gtttgtttta tttttttttt atttatattg     360
   tttttaattt tttattttta attggagtga gatttattgg gtgggtaggg ggtggggagg      420
   tgggtggtga gtggtatggg gtgtttagat aatgaggttg ttaggtaaag attttagatt      480
   tgttatggaa agttgttaag tgtttattgt attttggttt tggggtgaat attttgtttt      540
   ttgttagtgg tgtttaagtt agttggtttt gggatttttt tggagggtaa agtagatggg      600
   gaattttgaa gttagggttt tttttgagag atttgggtgt ttgtttattt ttttgttatt      660
   agggtgttta taattgtttt ttttttttat tgtttggggt ttttggtttt tattggttgg       720
   tgtgggtttt aaggagttat agttatttag ttattttatg ggggagtata ttggttgagt      780
   ttagtaattg gagtgatttg tgaggtggtt gggaattttg agaagatatg tatgaagagg      840
   gtgtatttgt tgggttgtat attttgtttt gggttatttg gtttaagatt tagatttttt      900
   gtgggtgtga agttggtttt tttaagagat taattaaatt gaagaggttg aggaatggaa      960
   ttgggttgtg atattaaaat taataggagt aatttaatat ttgttttgtt ttttatgaaa     1020
   tatttatttt aataatttta gttaaaatgt ttagattgtt ttgattttta tagaggttga     1080
   aatttatttt gtaattagaa gttggaagtt agaggtgatg gaggatagag gtgtttattt     1140
   tgtgtgtttg tgtgtgtgtg tttgtgtttg tgtgtgtgtg tttgtgtatg tgtgtgtgtg     1200
   tgtgtgtgtg ttttttttta aagtgtgtgt agggaaggag gggggagaag gtttttaatt     1260
   ttttttgtta taaaatagta tgaggtgatt ttgttagtag atttgtattt ttaggagaag     1320
   ggtgaaaaaa gaattttttt tgaagtgttt ttgatattgt tattaaattt aaataaaatt     1380
   aagtgtggtg tatgaaaatg tttttttttt tttataaaag aaagtgttta ttgtttgagt     1440
   ttgtttagtg tttgttaaat tatgtatgaa atttgaaatg aaataaatgt tataataaaa     1500
   ttaatttttg agtttttttt atttaaaaat tttagattaa gtattttatt gttgtgggta      1560
   ggagttaaaa agttattatg ttgtatgtgt agatttattg tttttttgag ttgtggttag     1620
   gaaggtagtg ttaagtatgt tagaattgat atgttggggg tggggggaaa tgggtttaat    1680
   tttggggtag tggtagaatt                                                 1700
<210> 155
   <211> 1700
   <212> DNA
   <213> Artificial Sequence
<220>
   <223> chemically treated genomic DNA (Homo sapiens)
<400> 155
   aattttgttg ttattttaaa gttgagttta ttttttttta tttttggtat gttggttttg     60
   gtatatttag tattgttttt ttagttgtaa tttaggggag taatgaattt gtgtgtatga    120
   tatggtaatt ttttaatttt tgtttgtgat ggtggagtgt ttaatttaag gtttttaaat    180
   aaaaagggtt taggggttgg ttttattata atgtttattt tattttagat tttatgtgta    240
   atttagtaaa tgttagatag atttgggtga taaatatttt tttttgtggg gggggaaagg    300
   tatttttata tattatgttt aattttattt agatttaata gtgatattag gggtatttta    360
   aagaaagttt tttttttgtt ttttttttaa gaatgtgaat ttgttagtaa aattgttttg    420
   tgttatttta tggtaaggaa aattaaaggt tttttttttt tttttttttt atatatattt    480
   tagggaggaa tatatatata tatatatatg tatgtataaa tatatgtatg taggtgtgag    540
   tatatgtata taggtgtata gaataaatat ttttattttt tattattttt aatttttaat    600
   ttttaattgt aagatagatt ttagtttttg tggggattag gatgatttaa atattttaat    660
   taagattatt gggataaata ttttataaag agtagggtag atattaaatt atttttattg    720
   attttagtat tatagtttaa ttttattttt tggttttttt agtttaattg attttttaag    780
   gaggttaatt ttgtatttgt agagggtttg ggttttggat tgggtggttt agggtaaggt    840
   gtgtggtttg gtgggtgtgt tttttttgtg tgtgtttttt tggggttttt agttgttttg    900
   taagttgttt tggttattgg gtttaattga tgtgtttttt tataaagtgg ttgagtgatt    960
   gtgatttttt ggagtttatg ttggttggtg agaattggga attttaggta gtggggaagg   1020
   ggaataattg tgaatgtttt agtgatggag gagtgagtaa gtgtttaggt tttttgagga   1080
   gaattttaat tttgaggttt tttgtttgtt ttatttttta agaaaatttt agggttggtt   1140
   aatttgggtg ttgttagtgg agaatggagt gtttattttg gagttgaagt gtagtagata   1200
   tttagtgatt ttttgtggtg agtttagggt ttttgtttgg tgattttatt gtttgagtgt   1260
   tttgtattgt ttattgttta tttttttgtt ttttgtttat ttggtgggtt ttgttttagt   1320
   tgggggtggg gagttgggaa tagtgtgaat gggaaggggg tgaataaagt taagtttttg    1380
   ttattttttt ttgtttgtat tttttttagt ttgatgtgtt tggaggttgg ggtgtgtttt   1440
   tagtgtattg atgttggatt ttgaggagag gtgggtgtag gggattttag agttggggta   1500
   gtgtgggttt gtggtggggg ttttggtagt taggattttg gattatttta agagtgagtt   1560
   ttgttttgag tttggaattt gggtggtgtt gattggtgta aatgtaatgg ttgaaattga   1620
   ttgttgaaat gtaaaatttg ttgttgtttt ttttggtgtt gggtgagagg tagatataga   1680
   gaggggagtt gagatttttg                                              1700
<210> 156
   <211> 1700
   <212> DNA
   <213> Artificial Sequence
<220>
   <223> chemically treated genomic DNA (Homo sapiens)
<400> 156
   ttttatgggg atttttgtta atgtttggtt gagaggtggt tttagggggt tgagaatttt      60
   atttttagag gtttggtatt tgggagattg agaaagtttt ttttaagttt ttttgttggg     120
   ttatgttgta tttatagatt atttttaatt tttttttttt ttttatttat tttttgtttt     180
   ttattttttt ggtttatttt ttgttagaaa atgtattaag ttttaatttt tgtttttttt     240
   agtttttttt ttgagaagat attgtgattt tgttgttttt gtttgtgtta ttatggagag     300
   gtaggaggat aaatattgtt tttagttgtt agtttttttg tttttttttt ttttttttag     360
   atagttttta gttttaattt tagtatttgt tttattattt ttttttggag aattagtata     420
   attggagtaa ataggtaatt aagtagtgtg tttgtaagtt ttaattttgg gatagataag     480
   gaagtgaaat tgattggaat tatatttgtt gttgttggag gttgagggag gtggttgttg     540
   gaggtgaggt tagttttgtt ggtttagggt tttgttttat tttttttttt tttttttttt     600
   ttttttagtt ttattttttt ttattttttt ttattttgta ttttttttta gtggttgttt     660
   gtttttttta aggaatttta ttttaggtgg agtttttatg atagttatat tttttttttg     720
   ggtttagtag ggttagagaa agatggaatt attttattta tttaggttgt ttagagagta     780
   ttttaggttt tgatttgtgt ggggtgtgag gtattttgtg gggtagttag gtgtttttaa     840
   gaaattttgt tttagaggat ttgtatgagt tgttgttatt ttttgttgaa gtttttgttt     900
   tggggttggt gttttttttt atgattttat taattgttgg tatttttttt tttttttttt     960
   tttttttagg aaggtgttgg ggtgtgtggt ggtaatggtg gtggtgaagg gatttttttg    1020
   tggtattttt ttgtgggttt ttgttttgaa ggattttttt tttgttttaa ttttatgttt    1080
   aagtagtgtt ttgggggttg ggttggtttt tatagttttt atttttggtt tttttttttt    1140
   aaagggtgtg tggagtttag aggatgggtg gagagtgggt ttggttggaa ttgaattggt    1200
   agaggtaaaa ggggaatgga ggattttaag ttttggttat tttggtttag tgaatttatt    1260
   ttatttttgt ttaaggaaaa gttgtagggg ggaggatttt ttttaggaag tttattttgt    1320
   tggtttttga tttttgggtt ggatgtttga gagttttgtt ttttttgttt tgagagtggt    1380
   ttagttgggt ttgaattgat tttttttttt tttgattttt tttttatttt ttagtttgga    1440
   gaaatagggg agtgggggtt taagaatgag aagatgagaa tgtgttgttt tgtgttatgt    1500
   tagaatgggg tgggtgtgag gggaatagtt tttttgtgat tagtttagga gtatgagttt    1560
   tttggaggat ggtatgagtt ttggatattt taggagtttt tagttagtga tatggttggt    1620
   aagtgatttt gggattaata tttatttatt tttatttttt ttgtttagat tttatttttt    1680
   ttttagtttt tttgtttttt                                                1700
<210> 157
   <211> 1700
   <212> DNA
   <213> Artificial Sequence
<220>
   <223> chemically treated genomic DNA (Homo sapiens)
<400> 157
   ggggagtagg gggattggga gggggataga atttagatga aagaggtggg agtgggtggg      60
   tattagtttt agagttattt attgattatg ttattagttg aggatttttg aagtgtttag     120
   aatttatgtt gttttttggg aggtttatat ttttgagttg attgtaagag agttgttttt     180
   tttatatttg ttttattttg atatagtgta gggtgatgtg tttttgtttt tttgtttttg     240
   ggtttttatt tttttatttt tttgggttgg aaaatgagga gggggttgaa aggggaggga     300
   gttgatttag atttagttgg gttatttttg gaataaggga aataaagttt ttaagtattt     360
   aatttaaaaa ttaggggttg gtagagtaga ttttttaaaa agaatttttt tttttgtagt     420
   ttttttttgg ataaaaataa agtgggtttg ttgggttgaa gtggttagag tttaaggttt     480
   tttgtttttt ttttattttt attaatttag ttttggttaa gtttgttttt tatttatttt     540
   ttgaatttta tatatttttt aaaaggagag ggttgggggt gggggttatg aaaattaatt     600
   taatttttaa agtgttattt gaatataaag ttgaggtgga gaagaaattt tttaagatag     660
   gaatttatga ggagatatta taagaggatt tttttattgt tgttgttgtt gttgtatatt     720
   ttagtgtttt tttaaaaaga aagaagggag gaaaaaaatg ttgatgattg gtagaattat 780
   agagaagaat attagtttta gggtaggggt tttagtgaaa aatggtagta atttgtgtgg 840
   gttttttggg gtggggtttt ttggaggtat ttagttgttt tatggggtat tttgtgtttt 900
   atgtgagtta gaatttaaag tgttttttgg gtaatttgag tgggtagagt gattttgttt 960
   ttttttggtt ttgttaggtt taaaggagag gtgtgattat tgtgaggatt ttatttaggg 1020
   tggagttttt taaaaaggat aggtggttgt tagaggagag tgtaaagtgg gaggaggtgg 1080
   agaagggtgg ggttgagaag gaggagggag gaaggagggg gtggggtggg gttttgggtt 1140
   gatagagttg gttttgtttt tggtggttgt tttttttagt ttttagtagt ggtaggtata 1200
   attttaatta attttatttt tttatttgtt ttaaaattaa ggtttgtaag tatattgttt 1260
   gattatttat ttattttggt tatgttaatt ttttagggga gggtggtgaa atgggtattg 1320
   ggattggggt tgggggttgt ttaagaggga aaggagaaaa taaaaaagtt gataattgga 1380
   agtgatattt atttttttat ttttttatgg taatatgaat aaggatagtg aggttatagt 1440
   gtttttttag ggaggaagtt aaaaggaatg gagattggag tttggtgtat tttttagtaa 1500
   gagataagtt aaggaggtga gggtgaagga gtaggtgggg aagaagaagg agttgaagat 1560
   gatttgtgaa tatagtgtgg tttagtaggg agatttaaga aaggtttttt tagtttttta 1620
   gatattagat ttttaagaat agagttttta attttttgag attgtttttt agttaggtat 1680
   tggtgaagat ttttgtaaag 1700
<210> 158
   <211> 1700
   <212> DNA
   <213> Artificial Sequence
<220>
   <223> chemically treated genomic DNA (Homo sapiens)
<400> 158
   ttagatagta gaagtttata ttttttggag gtggaagttg gaggggaagg gtttgaggta 60
   gagataaaag taagagaggg attttttagg tttttttggg gatgggagga tggtgtttgt 120
   ttagttttgt gtttgtaatt tttatgttat atatattata gttatgtttg ggttatgttt 180
   gtttagagtt tattgggtat gtatatatgt gatatgtttg aagttgaggg taagttattt 240
   agggtagagt atgtttttag gttttgtgtt tgttttaggg tagttgtaat gtagtaagtg 300
   tgttattgtt atggaggtaa gaaagttagt gtttttttat gtatgtatat aggttaggtg 360
   gttttttttg tataagatgg gttattaata aatttattta tgttatatat ttgtttttta 420
   ttgtttttat tttatttgta aagtatgggg aaaagaaagt aaaagttttt tttgatttgg 480
   tttttttttg tgtttttttt tggagttgaa gagtttgatt tgggaaaaga tatttaattt 540
   gtttttgttg tagttggttt tgtttttgtt ggggttgttg atatatttgt aggtgttgga 600
   ttttttgtgt ttgtgggttt gttttttttg atggatgagt gagttggtga tagttgttgt 660
   tatggttatg atgtttatta ttattgtttt ttttgttgtt tttttttggg tttgtttttt 720
   tttttttttt ttttgttgtt tgtttgttgt tttgtgattt tgttgttttg gtttttttag 780
   tttgtgtttg tttgggtttt tttgtatttg ggttttagtt aaggaggggg tttagtatgt 840
   tgtttgagtt tttttggtgg tggtttggtt tttgtgtggg ttgttggttg tttgggttgg 900
   agttgatgtt atagtttggg gtttgggatt gtgggtgaga ttggtatgtg gatgttgaat 960
   tgtgtgattg tgtgtggttg gtttttgtgt ttggtggata ttgtgtatgt ttagttgttt 1020
   tggtttgaat tttgttggat ttttttgttt tgttgttttt ttgatggggg ttagaggagg 1080
   gaggtgggtg gagggagtga gtgtgggtgg gagagaggtt gggagtttgg gtggttggat 1140
   ggaggaggga tgaggtagtg tgtgggggag tgtgtttttg ttttggtttt tttgagtttt 1200
   tgattagttt ttgtaatttt ttggtgtgat aattgggaaa agttggtttg tgttaggttt 1260
   ttgataggga ttaaataggt aatggttttg tatttttgtt gttgttgttg ttttgggtgt 1320
   ggtatagttg tagtatagga atttagttgt tgtaggtatt ttttggaata gtgtgatagt 1380
   ttttttgtag tttttttttg tgatggtttt ttttttttgg ggttgagttt gtaggtttgg 1440
   agttgtaggt tgtttttttt atttttattt ttatttttat ttttttaagt ttttggttgt 1500
   ttgttttggt taatatttgt aggggttttt gtattgtttt agttgtttaa attttttttt 1560
   tttttttttt ttgaaaggag ttttgggagg aaatagtgat ggaggaggaa ttgagatgag 1620
   gtagttgggt tgatggagtg ggggtttgtt tggtattggg ggatggagtg gtgggggtgt 1680
   ttttttagtg tttgagttga 1700
<210> 159
   <211> 1700
   <212> DNA
   <213> Artificial Sequence
<220>
   <223> chemically treated genomic DNA (Homo sapiens)
<400> 159
   ttagtttaaa tattgaggaa atatttttat tattttattt tttaatgttg agtaaatttt      60
   tgttttgtta gtttaattat tttattttag tttttttttt attattgttt ttttttgaag     120
   ttttttttgg gaaaaggagg ggagaaggat ttggatgatt ggggtagtgt ggaagttttt     180
   ataggtgtta gttggaatgg gtggttgggg atttgggggg gtgggggtgg gggtgggggt     240
   gggaggggtg gtttgtggtt ttgagtttat gggtttagtt ttgggaggga ggggttgttg     300
   tgggaggggg ttgtaaggag gttgttgtgt tgttttggag ggtgtttgtg gtggttgaat     360
   ttttgtgttg tgattgtgtt gtgtttagag tagtagtaat agtgaagatg tgaggttatt     420
   atttgtttga tttttgttgg aaatttggta tgggttaatt ttttttgatt attatgttaa     480
   gaagttgtaa ggattagttg aagatttgga ggggttaggg tgagggtgtg tttttttgtg     540
   tgttgttttg tttttttttt gtttggttgt ttgagttttt ggtttttttt ttgtttgtgt     600
   ttattttttt tgtttgtttt ttttttttgg tttttattag aagggtaata gggtgagggg     660
   gtttggtgaa atttggattg gagtagttgg atatgtatgg tgtttgttgg gtgtaggggt     720
   tgattatatg tagttgtgta gtttagtatt tgtgtgttag ttttgtttgt gattttgggt     780
   ttggggttgt ggtgttgatt ttgatttagg tagttagtag tttgtgtggg agttggattg     840
   ttgttggagg agtttggatg gtatgttgag tttttttttt ggttgaagtt tgagtgtgga     900
   gaagtttggg taaatgtagg ttaaggagat taaagtggtg aagttgtgag atagtggata     960
   agtagtggag gagaaggagg aggaggtgaa tttagagagg ggtagtaaaa gaagtggtgg    1020
   tggtgggtgt tgtggttatg gtggtggtta ttgttagttt gtttatttgt tagaagaggt    1080
   aagtttgtga gtgtgagaaa tttaatgttt gtaagtgtgt tagtagtttt agtaaaggta    1140
   agattagttg tgataaaaat aagttaaatg tttttttttg ggttaaattt tttggtttta    1200
   agaagaggtg tagaagaaga ttaggttgga aagggttttt gttttttttt ttttatattt    1260
   tatgagtgag gtgggggtgg tggggagtag gtatgtagta taggtgagtt tgttggtggt    1320
   ttattttgtg tagaagaggt tatttagttt gtgtgtgtgt gtgggaaggt gttggttttt    1380
   ttgtttttgt aatagtaata tgtttgttgt attgtaattg ttttgggata ggtgtagagt    1440
   ttgggggtat attttgtttt gggtggtttg tttttagttt tggatatgtt atatatgtgt    1500
   atgtttggtg ggttttgggt aggtgtgatt tagatatggt tgtgatatgt atggtgtgga    1560
   aattgtaggt gtagagttgg gtagatatta tttttttgtt tttaggagag tttggggaat    1620
   tttttttttg tttttatttt tgttttagat tttttttttt tagtttttat ttttaaggga    1680
   tgtgggtttt tgttgtttag                                                 1700
<210> 160
   <211> 1700
   <212> DNA
   <213> Artificial Sequence
<220>
   <223> chemically treated genomic DNA (Homo sapiens)
<400> 160
   tttttttttt tttttttttt tttttttttt tttttttttt tttttttttt ttgtttagtg      60
   aggagtttag attagagagg tggtttagat agtttattaa atggtaagat tagattagtt     120
   tttaaattaa gataatttat tttgtttttt tttttttttg aaatatatat tttttgtttt     180
   tagagatttt aaaatttttt gatgatttat atatttattt gaatttttgt tttttgataa     240
   aagttattat gtgtattttg gaaaaagtga agaaaagtta aaagaaaata tttatttaga     300
   gataatattt tagtggattt ttttgttttt atttttgtat atatgagttt atagggtata     360
   tatgtttgta tatgtttaaa tttatattat aagtatgttt agtttatttt tagaattttt     420
   taaataaata taatttaaaa taggaattta atattttatg ttattgatag attgtagtag     480
   attgtgtttg tatttttaga taatattttt ttttattatt gtaaataaga ttttattttg     540
   tgtttttaat ttttattttt gttaatattt tttgttattt ttttaggata tatatgtaga     600
   agaaatatta attagttaaa tattttaatg tttataagat atgttatttt agggtttttt     660
   agaaaaatag tattattgta ttttgttttt ggaggtgtag gtgagtgttt ggtttatggg     720
   tgtttataat tattattggg tatttataag agattttgaa tttagaatat ttttgtggta     780
   ggagttttgt tttgtttttt taggggatat tgatgatttg ttgggttttt gttttttaag     840
   tgttggaggg gggaagtagg gttatttggg gtagaaattt aatgttagaa attttttagt     900
   gttttattag ttggtggaat ttttgtggta gatagttgtg ttttatttaa gttgtagaag     960
   aaaagatatt ttgtggggga tttggtgttg gaagttttga gtttaaattg tagttttagg    1020
   tttttagttt tgtttttttt ggtttgttgt gaggatgaaa tgagattttt gtatgtgaaa    1080
   atgtttttta attttttttg aggggtggaa atgtttttat ttgattagag tttgggtttg    1140
   tggagtaatt tatttgttgt tttttaagag taggtttttt agtgtagtga gtttttgtta    1200
   tggaaagggt tgttttggtt taggatgtgt gtttttggtg tagatttggg gataggggtt    1260
   tttgttgtgt ttgttttatt tttgtaggga gtaaggttgt ttttttgtag tattgtgtta    1320
   tgtggtggga atttgtagtg ttttgttttt tggttttagt ttttttgttt gtttgggttt    1380
   tttttggtta ttttttggag gggtttggag tttttttttt ttttttttat tttagtgtta    1440
   gaagtattga aagttgtttg tttgttgaga gtggaattgg tattttgaat tttgtttatt    1500
   ttgggttaat tggaggtgta ttagtggttg ttttgtagtg taatagaggt ttgagtgata    1560
   ggttatttgt ttaagtgttg gtgtttagga aagttgggga ggaaattagg aagggaggtg    1620
   atagtagaaa agttagagag tttagagatt tttaggtagg tttttgtgta gttttaatag    1680
   tgtggtgttg ggaggtgtgt                                                 1700
<210> 161
   <211> 1700
   <212> DNA
   <213> Artificial Sequence
<220>
   <223> chemically treated genomic DNA (Homo sapiens)
<400> 161
   atatattttt tggtattatg ttgttggggt tgtatggagg tttgtttagg agtttttgga     60
   ttttttggtt tttttgttgt tgtttttttt tttagttttt tttttggttt ttttgggtgt    120
   tagtgtttag gtaggtggtt tgttatttga gtttttgttg tattgtaagg tagttgttaa    180
   tgtgttttta attgatttgg aatgagtgag atttgaggta ttggttttat ttttggtggg    240
   tgggtggttt ttaatgtttt tggtgttgaa gtaggaggag ggaggaggga ttttaggttt    300
   ttttgagagg tgattgagag agatttaagt gagtgaagaa gttgaaatta gaaagtagga    360
   tattgtaaat ttttgttgtg tggtgtggtg ttgtagaagg atggttttgt tttttgtagg    420
   ggtggggtga gtgtgatagg gatttttatt tttaggttta tgttgggagt gtgtattttg    480
   agttgaaatg atttttttta taataaggat ttgttatgtt ggggagtttg tttttggaaa    540
   gtggtgggta aattgttttg tagatttgga ttttggttga atggagatgt ttttattttt    600
   tgagaggggt tagaaagtat ttttatgtat aaaaatttta ttttattttt gtagtaagtt    660
   aggaagggta aggttgggga tttagggtta tgatttgaat ttaaggtttt tgatgttaag    720
   ttttttataa ggtgtttttt tttttatgat ttaggtagag tgtgattgtt tgttatgaaa    780
   attttattaa ttgataaggt gttggaaggt ttttggtgtt aggtttttat tttgggtggt    840
   tttatttttt ttttttagta tttggaaggt gagaatttag tgagttgtta gtattttttg    900
   gaaggatgag gtggaatttt tattgtggaa atattttaag tttagggttt tttgtgggtg    960
   tttggtgatg attgtaaata tttgtagatt aggtatttgt ttgtattttt gggaataaaa   1020
   tatgataata ttgttttttt aggaagtttt ggaatggtat gttttatgag tattaaaata   1080
   tttgattgat tgatgttttt tttatatatg tattttgaga aaataataga aaatattgat   1140
   aaggatgaaa attgaaggta taaaataaag ttttatttat agtgataaaa aaagatatta   1200
   tttaaaaata taaatatagt ttattatggt ttattaataa tatgaaatat tagattttta   1260
   ttttaaatta tatttgttta aaaaatttta aagatgaatt gggtatgttt atgatgtaaa   1320
   tttaaatata tataaatata tatattttat gagtttatat atatagaaat aaagataaga   1380
   aggtttatta aaatgttgtt tttgggtgga tatttttttt taattttttt ttattttttt   1440
   taaggtgtgt atgatgattt ttattaggaa ataaaaattt aaataaatgt atgggttatt   1500
   agaagatttt gagattttta agagtaaagg atgtatattt taaaaagaaa gaaagatagg   1560
   gtaaattgtt ttggtttaga ggttagtttg gttttgttat ttggtgaatt atttgggtta   1620
   tttttttgat ttggattttt tgttgaataa aagaagaaga aggagaagga gaaggaggag   1680
   gaggaggagg aggggaagaa                                               1700
<210> 162
   <211> 1700
   <212> DNA
   <213> Artificial Sequence
<220>
   <223> chemically treated genomic DNA (Homo sapiens)
<400> 162
   ttattaaaaa agtggagggg gttttgttta atatttgtat atattttttt ttgtagtatt     60
   ttgtttattt tagtttttta gggaaattat agataatgat gttttagaga gtgttagagg      120
   tgaagttgat ttgaaattat attatagaaa ggttttttgt tttaaatagg tttaataaag       180
   ttattaaatt aagtttatga ttataaagaa ttgtgaatta gaaaataatt ttatataggt      240
   gtagtttttt attatttttt tttttatttt tggaaatttt aaagaaaaat tgttatattt      300
   ttaaatatga ggttattatt ttaaaggttg taataatttg gaatgtaaat ttgttaaatt      360
   atgatataat aaggttaatt ttgaggattt ttggtggatt attagtgtat ttttttataa       420
   ttagaaagtg aaaaattggg ttagttttag agttatgtaa taatgttgtg ttatgaaatt      480
   gtgtatttta agagaaattt tttttaggtt tagaattaag ttggagggat ttgttgtgta      540
   gtttaatttt taggaagtat gaaagaatgt attgtaaaga gtaaaattat gtgtaattta      600
   ttttaatata attgtagttg tttgtgggtt tttttgtaga gaaaggttgt ttaattttga      660
   tggggttatg ggtgttgtgg gagggtaaaa gattatagag atttgtgaag ttaaagtaaa      720
   taatggggtt ggggtggtga gaatgattaa tagtgatgtt ttgaaaggat tttgtgatag      780
   gaattgagtg ggaaggattt tttttatttt aatagtttat gggttaagag gttgaagttt      840
   ttttaaatat tatatttttg tgagagaagg gattgagaat aattttttaa attatttttt      900
   tttgaaattg tttttttttt tgaaaatttt atttttgaga tttggatgag gtttttattt      960
   tttttatttt tgttttgtga ttgttgtttg tttagttttt tagttgagtt tgtggggttt     1020
   ggggtgttta ttttgtttat ttaagggatt gtgtaggggt gaattttttt gtgttttatt     1080
   tgtgtttttt agattttggg tgttttggtg ttgttttgag agttttttgt gtggttgttg      1140
   tggtatggat tagtttttat ttttttatat tgggtgttgt tgtgtttgtt gggggttggt      1200
   tttgaggttt ttaaggtttt gtgtgtgtgt ttgttgtggt aattaagatg ttttatgatg     1260
   tgtgtttttg aatgttttgt gttatgtggt tgtgtggttt tgtttgttgg ttttgttttt      1320
   gttatagagt ttgtagtatg ttgttgttgt agtttaggtt atgtgagtat ttatgtgtgt     1380
   gttttgttag tggatttatt attggtttgt ttagattagg ttttgtttat tttgattttt     1440
   taaatggtat gtgggaggat gtttgttttt tttggattta agagttattg taatatttta     1500
   gaaggggaga aaaggagtga gggtggtagg tgatagagaa ttttgtgagt tagtggtttt     1560
   gtgtagattt ttttaggtat ggttttttgt ggttatgttg gttgtttggt gtttgtgtaa      1620
   tttttttttt tttagtgaaa ttgaggtttt tggagagttt agtagagagt gtgggtagtg     1680
   agtgtttgta gttgttagag                                                 1700
<210> 163
   <211> 1700
   <212> DNA
   <213> Artificial Sequence
<220>
   <223> chemically treated genomic DNA (Homo sapiens)
<400> 163
   ttttagtggt tataagtgtt tattgtttat attttttatt aggttttttg gaggttttgg      60
   ttttgttgga gagaaagaga ttgtgtaggt gttgagtagt tgatgtggtt gtaggggatt    120
   gtgtttgggg gggtttgtgt ggggttgttg atttgtgagg ttttttgttg tttgttgttt    180
   ttgttttttt tttttttttt tagagtgtta tggtgatttt tgggtttgaa ggggatggat    240
   gtttttttat gtattattta gaaggttaga gtgggtagaa tttagtttga gtaggttggt    300
   gatgaatttg ttggtaagat gtgtgtgtgg gtatttatgt gatttaggtt gtggtggtgg    360
   tgtgttgtgg gttttgtggt gggagtgagg ttgatgggtg gggttgtgtg gttgtgtgat    420
   gtgaagtgtt tgagagtgtg tgttgtggaa tgttttggtt gttatggtaa gtgtgtgtgt    480
   gaggttttgg gaattttggg attggttttt ggtgagtgta gtggtgttta gtgtaaggga    540
   gtgggagttg gtttgtgttg tggtggttgt gtagggagtt tttgaggtaa tgttggggtg    600
   tttgaggttt ggaaggtgta ggtggggtgt gggggagttt atttttgtgt ggttttttgg    660
   gtgggtgggg tgggtgtttt aggttttgtg gatttggttg ggaggttgag tgggtgatgg    720
   ttatgggata agggtggagg gggtggggat tttatttgag ttttagagat ggaatttttg    780
   aggaagggag tgattttgag agagaatagt ttgagaagtt gtttttagtt tttttttttg    840
   tgggagtgtg gtatttaggg aagttttggt tttttaattt atggattgtt aggatggagg    900
   gaattttttt tgtttgattt ttattgtgga gtttttttgg agtattgtta ttgattattt    960
   ttgttgtttt aattttgttg tttattttgg ttttgtagat ttttgtggtt ttttgttttt   1020
   ttgtagtgtt tatggttttg ttaaggttgg gtagtttttt tttgtaagaa ggtttatgaa   1080
   taattgtggt tatattggga tggattgtgt gtgattttat tttttataat atattttttt   1140
   atgtttttta gggattgagt tgtgtggtgg atttttttga tttaattttg ggtttgagaa   1200
   aagttttttt tggaatgtgt agttttgtga tgtagtatta ttgtgtgatt ttagggttga   1260
   tttagttttt tattttttaa ttgtaaggaa gtgtgttagt agtttattga ggatttttga   1320
   ggttggtttt attgtattat aatttaatga atttgtgttt taggttgtta tagtttttga   1380
   aataatggtt ttatatttaa gaatgtagta attttttttt gaaatttttg aaagtgggga   1440
   aaaaagtaat agaagattgt atttatatgg aattattttt tgatttgtag ttttttataa   1500
   ttgtagattt aatttggtgg ttttattgga tttgtttgaa ataggaagtt tttttgtaat   1560
   gtggttttgg gttggtttta tttttggtat tttttgaaat gttgttgttt atggtttttt   1620
   taagaagtta gaatgagtgg gatgttatgg agggaggtgt atataggtgt tagataaagt   1680
   tttttttgtt tttttggtaa                                                1700
<210> 164
   <211> 1700
   <212> DNA
   <213> Artificial Sequence
<220>
   <223> chemically treated genomic DNA (Homo sapiens)
<400> 164
   aatttaggtt tggtaggtta tttgtatata ttgggtggag gtggaagggg taaagtattt      60
   ttgaggtgat ggttttgatt ttattttata tttttttggg gtttggttgg atttggttga     120
   gtgtgtttga gtagtggttg atttttggaa aataggagga ttttttttta ggaggttgtg     180
   attattaagg ttgtaaatgt tttatttgaa tttgggtgta tgagggattt ttttttttta     240
   tatatatata tttttttatt gtgtaagata aatttttgtt tgtgagttgt ttatattaaa     300
   tgttttgttt ttgatttttt gtgattaaaa tttgttttgt ttgtagggtt tggtgtgttt     360
   ggatttagtg tattttgttg gtatagtaaa aaagagaatt gtttttttat aggtgatttt     420
   tgttttaaag tttttttttt tattaatttt tttttgagaa gagagtaaaa ttgaatttga     480
   gagaaaagag gtagtaatta tttgggagga ggtagattgg agtgggtatt agttttggta     540
   gtaagagttt taatgttttt tttgtttttt ttttgtagag tagtatggaa gtattagttt     600
   ttttttttgg gtttttaaaa gttatttttt ttttgggtgt gatttttgaa tttgaagtgt     660
   ttgagtttta agaaattaga ataagttaga gttaaagagg tttggtgggt gggggtgtgg     720
   gtggagtttg ggttgggtgg tgtggaaggt tgagtatagt tgtgagtggt gagttttggg     780
   agaggaggag tgggagaaag tggggtgggt ttgtggttgg ggtttggttg ggggaggggg     840
   tttttgtttt ttttttttta tttttgtttt ttttggtttt ggtgatgttg tggttttttt     900
   ttttttttag tggagtttta gtgtttgttg tgtttttttt tttgtttttt ttgtttatat     960
   ttatttgttt ttgttggttt ggtttttagt agtgttgttt gaggttgtag tagtgtattt    1020
   tggggtatgg tgtggtgggg gtgtggaggg tttggtttgg agggggttgg gagtttgggt    1080
   gttttggagt gaggaggatt gggagttggt tttggaggtt gtggaggtga tgttggagag    1140
   aatgaagttt tggttgggag tggtaagtgg aggggtgtgt aagggatgag ttggggtgtg    1200
   tggtgtaatt ttttttggga gttttgggtg ggtagttttt ggggttgttt tggtgtgggt    1260
   gggagatgga gagttatgtt tttggttgag agtggagtgt tgtgggttgt gtgtttgggt    1320
   ttggtttggt tggattttga gagagtgttt gatgtgtgaa gtttgagtta tgggtttttt    1380
   tggtggtgga gtagttttga ggggtagttt tttgattttt tttatttttt tgaatgttga    1440
   gtattgagtt gggaatatga tgagaatttg gagattgtat tttgtgtgtt ggatttggtt    1500
   ggtttagttg tgattttttt gatggttttt ggggttttgg ttttattttg tggggttggg    1560
   tggtatattt ttgtgttttt tttttttttt gtattttagt tatttggggg taggggaggt    1620
   ggtggtggtt tgtgtgtaga ttttggtgtt ggtaatttta ttttgtggtt gtagaagttg    1680
   aggaagagtt ttttaagttt                                                1700
<210> 165
   <211> 1700
   <212> DNA
   <213> Artificial Sequence
<220>
   <223> chemically treated genomic DNA (Homo sapiens)
<400> 165
   aggtttgggg agtttttttt tggtttttgt agttgtaaag tagggttgtt agtgttagga      60
   tttgtatata agttgttgtt attttttttg tttttaaatg gttggggtat ggagggaaag     120
   gaggatgtga gggtgtgtta tttaattttg tagagtgaag ttgaggtttt aaggattgtt     180
   gaggaaattg tggttgggtt ggttagattt gatgtatgga atgtgatttt taggttttta     240
   ttgtgttttt ggtttggtgt ttggtgttta agggggtggg gaggattgaa aagttgtttt     300
   ttgaggttgt tttgttgttg gggaagtttg tggtttaggt tttatgtgtt aggtgttttt     360
   ttggagttta gttgagttgg gtttgggtgt gtaatttgtg gtgttttgtt tttagttggg     420
   gatgtggttt tttgtttttt gtttgtgttg ggatagtttt gaaggttatt tgtttgaaat 480
   ttttgaagaa agttgtgttg tgtgttttag tttgtttttt gtgtgttttt ttatttattg 540
   tttttagttg aggttttgtt ttttttggtg ttgtttttgt agtttttaga gttagttttt 600
   ggtttttttt attttagggt gtttgggttt ttggtttttt ttgaattgag ttttttgtgt 660
   ttttgttgtg ttatgttttg ggatgtgttg ttgtagtttt gggtgatgtt attgagagtt 720
   gggttggtgg gggtgggtag gtgtgggtga aaggggtgga gggagggatg tggtgggtgt 780
   tgaggttttg ttggaggggg gaggaaattg taatgttatt agagttagag gagatgagga 840
   tgaggagagg gaaatgggga tttttttttt tggttgaatt ttgattgtag gtttgttttg 900
   tttttttttg tttttttttt tttgggattt gttgtttgta gttgtgtttg gttttttatg 960
   ttatttggtt tggattttat ttgtattttt gtttgttagg tttttttggt tttaatttgt 1020
   tttagttttt taggatttaa atgttttggg tttgagaatt atatttaaag gaaaggtaat 1080
   ttttggaggt ttagggggag gagttgatgt ttttatatta ttttgtgaga ggaaagtgag 1140
   gaagatgtta agatttttat tattgggatt ggtgtttatt ttagtttgtt tttttttgaa 1200
   tagttattgt tttttttttt ttgaatttgg ttttattttt tttttaaaga gaagttagtg 1260
   ggagggggga ttttggggtg ggggttattt atagagaagt agtttttttt tttattgtat 1320
   taataaaata tattaaattt aagtgtattg aattttgtaa atggaataga ttttaattat 1380
   agaaggttag gagtaaagta tttaatgtag gtagtttata aatagaaatt tattttatgt 1440
   ggtggggggg tgtgtgtgtg tgagagagag agatttttta tgtatttaag tttaggtgga 1500
   gtatttgtaa ttttggtagt tatagttttt tggagaagag tttttttgtt ttttaaaaat 1560
   tagttattgt ttaaatatgt ttagttaggt ttaattaggt tttaaggaga tgtgaggtag 1620
   gattagggtt attattttag gaatattttg ttttttttat ttttatttag tgtgtgtaag 1680
   taatttatta gatttagatt 1700
<210> 166
   <211> 1700
   <212> DNA
   <213> Artificial Sequence
<220>
   <223> chemically treated genomic DNA (Homo sapiens)
<400> 166
   aatagaattt agaattttgg ggtagttttt tggagttgga gtttttaggg taaagataat      60
   gttaatgtgt tgtttaaaag ggtttaattt ttaaaatatt tttattggta atatttattt     120
   tgtttattag aaatgtagtt atgtattttt gtgtttggta aagatttttt gttttgtgta     180
   aatttataaa ttaaaataat tagtttagat ttttgatgtg attttgtagt ggaagtattt     240
   gtattagtga atgttgaaaa gtaaataaaa atgaaagttt tgtggaggtt gtgggttgga     300
   tgggtgtggg gtgttttttg ttattttagt gtttgttggg attttttttg tgtttttgtt     360
   gtttgaatta gtttggtttt gggatgtttt ttttaaattt ttataagtta aattgaattt     420
   aggtttgagt ttgttattgt aaaggaattt ggggtgtggg gtgggaggga taggaggaga     480
   gatgggttgg gtgttaaatg gtagaataaa tggttgattt aattaaagtt gttaatttaa     540
   ttttttattt tttttatatt agtttttttt tttttttttt tttttttttt tttttaaatt     600
   tgttgaattt tattattttt gtgttggtaa gaaaatttga atattttggg aattgtgttt     660
   aaattgtatt tgtaattttt ttggaagttt ttgaaatttt ttgagaatga ggtggggtgg     720
   tttaagggga aagggtaggg aggagagatt tttgggaagt tgatgatgtg tttggggtaa     780
   gttagtttgt ttttgagatg tgtgttttat ggttgtagta gtgttgtgtt tattttgttt     840
   ttttagtgat ttagttagtt tttgttgggg gtttttgata atgtttattg ataagttttt     900
   tttgttttta aaaggtgtgt tttttttttt ttttaggtat aaagtagggg tgggaagggg     960
   agagttatat aatgttgtaa aaagaagtgt ggttttattt ggaagatggt tgaatttttt    1020
   ttgttttttt gatttagtta gtaatgataa gaaagatatt gaatattgtt ttttgaaaat    1080
   tgggtgtatt tgaatttggt gaaaagtgtt tttgtgagga agtttaatgt ttatttttgt    1140
   gtaaggataa ttggaaatta aagtgtttag taatgtagag attttattaa gtgagtgttt    1200
   tttttttttt tgtgttagga atgaaataag ttagttagat agaaaatatt tttgggagaa    1260
   gattaattat ttttgggtag gttgaattga ttggaaatta tattaagtaa aggtagtata    1320
   ttttattttg ttttggtgat gttgatttta tattattaga atattgtttt tttagtgtgg    1380
   gggtgttttg tattttttaa agttagggta gattggttat ttgggaaaat gtagtgttag    1440
   tgttttgttt ttattttttt tattgtttaa atatgtttta tattttttta atgttttttg    1500
   agggttttta atttagttat tgagtttttt atttttggtg attgtgatat taataatgtt    1560
   gttatttttt ttgggaaata tggttgtaaa gatgttttgt aggaggtaga taaaaggttg    1620
   tgtttttaga tattgaaggt tgttttgtta tttagatgtt tttaattttt gtatagagaa    1680
   ggagggaaaa tatggggtgt                                                1700
<210> 167
   <211> 1700
   <212> DNA
   <213> Artificial Sequence
<220>
   <223> chemically treated genomic DNA (Homo sapiens)
<400> 167
   gtattttgtg tttttttttt ttttttatgt aggaattgga aatatttaag taataaagta      60
   atttttagta tttgaaagta tagtttttta tttgtttttt gtggagtatt tttgtaatta     120
   tgttttttaa ggagggtgat agtattgttg gtattatagt tgttaagggt gaggaattta     180
   gtggttgggt tgagaatttt tagaaggtat tagaaaaatg tggaatatgt ttaaatagtg     240
   aagaaagtag gagtaaagta ttaatattgt atttttttag gtaattagtt tattttgatt     300
   ttgggaagta taaagtattt ttatattggg ggaataatgt tttaataata tagaattaat     360
   attgttagga tggaatggaa tgtattgttt ttgtttggta taatttttag ttaatttggt     420
   ttatttagaa gtaattagtt tttttttaaa agtgtttttt gtttaattgg tttattttat     480
   ttttggtgtg ggggggaggg gagtgtttgt ttaatgaaat ttttgtatta ttagatgttt     540
   taatttttag ttatttttgt ataaaagtaa atattaggtt tttttatagg gatatttttt      600
   attgagttta aatgtgtttg atttttggag ggtgatattt agtatttttt ttgttgttat     660
   taattgggtt gaggaagtaa aagaaattta attgtttttt aaataaagtt atgttttttt     720
   ttataatatt gtgtagtttt ttttttttta tttttatttt gtatttggag agaaagagaa     780
   gtatgttttt tgggagtaag gagagtttat tgatgagtgt tattagaaat ttttagtaaa     840
   ggttggttgg gttattaaag gggtagggtg ggtgtagtgt tgttatgatt gtgaggtgtg     900
   tgttttggaa ataggttgat ttgttttgga tgtgttatta gttttttgag ggtttttttt     960
   ttttgttttt tttttttgag ttgttttgtt ttatttttgg gaagttttag gaatttttag    1020
   gggagttgta agtgtaattt aagtataatt tttaaggtat ttaggttttt ttattaatgt    1080
   ggagatagtg ggatttagta agtttaagag gaaaaaagaa aaagaaaaaa aaaaagggtt    1140
   ggtgtgagag ggatgggaaa ttagattgat aattttaatt aaattaatta tttgttttat    1200
   tatttggtat ttagtttatt ttttttttta ttttttttat tttgtatttt gggttttttt    1260
   gtagtgatga atttggattt gagtttgatt tgatttgtga ggatttgggg gaaatgtttt    1320
   aagattgagt tggtttgggt agtgaaggtg tgggaaggat tttggtagat gttaagatgg    1380
   taggaggtgt tttgtgtttg tttggtttat ggtttttgta aagtttttat ttttatttgt    1440
   tttttagtat ttattggtgt aaatattttt attgtagggt tatgttaaga atttaaatta    1500
   attattttag tttgtagatt tatatagaat agaaaatttt tattgggtat aaaaatgtat    1560
   ggttgtattt ttgatgaata gaatgggtgt tattaataaa agtattttaa aaattgaatt    1620
   tttttaaata atgtattagt gttatttttg ttttaggagt tttaatttta aagaattgtt    1680
   ttagagtttt gaattttgtt                                                1700
<210> 168
   <211> 1700
   <212> DNA
   <213> Artificial Sequence
<220>
   <223> chemically treated genomic DNA (Homo sapiens)
<400> 168
   aatagaattt agaattttgg ggtagttttt tggagttgga gtttttaggg taaagataat      60
   gttaatgtgt tgtttaaaag ggtttaattt ttaaaatatt tttattggta atatttattt     120
   tgtttattag aaatgtagtt atgtattttt gtgtttggta aagatttttt gttttgtgta     180
   aatttataaa ttaaaataat tagtttagat ttttgatgtg attttgtagt ggaagtattt     240
   gtattagtga atgttgaaaa gtaaataaaa atgaaagttt tgtggaggtt gtgggttgga     300
   tgggtgtggg gtgttttttg ttattttagt gtttgttggg attttttttg tgtttttgtt     360
   gtttgaatta gtttggtttt gggatgtttt ttttaaattt ttataagtta aattgaattt     420
   aggtttgagt ttgttattgt aaaggaattt ggggtgtggg gtgggaggga taggaggaga     480
   gatgggttgg gtgttaaatg gtagaataaa tggttgattt aattaaagtt gttaatttaa     540
   ttttttattt tttttatatt agtttttttt tttttttttt tttttttttt tttttaaatt     600
   tgttgaattt tattattttt gtgttggtaa gaaaatttga atattttggg aattgtgttt     660
   aaattgtatt tgtaattttt ttggaagttt ttgaaatttt ttgagaatga ggtggggtgg     720
   tttaagggga aagggtaggg aggagagatt tttgggaagt tgatgatgtg tttggggtaa     780
   gttagtttgt ttttgagatg tgtgttttat ggttgtagta gtgttgtgtt tattttgttt 840
   ttttagtgat ttagttagtt tttgttgggg gtttttgata atgtttattg ataagttttt 900
   tttgttttta aaaggtgtgt tttttttttt ttttaggtat aaagtagggg tgggaagggg 960
   agagttatat aatgttgtaa aaagaagtgt ggttttattt ggaagatggt tgaatttttt 1020
   ttgttttttt gatttagtta gtaatgataa gaaagatatt gaatattgtt ttttgaaaat 1080
   tgggtgtatt tgaatttggt gaaaagtgtt tttgtgagga agtttaatgt ttatttttgt 1140
   gtaaggataa ttggaaatta aagtgtttag taatgtagag attttattaa gtgagtgttt 1200
   tttttttttt tgtgttagga atgaaataag ttagttagat agaaaatatt tttgggagaa 1260
   gattaattat ttttgggtag gttgaattga ttggaaatta tattaagtaa aggtagtata 1320
   ttttattttg ttttggtgat gttgatttta tattattaga atattgtttt tttagtgtgg 1380
   gggtgttttg tattttttaa agttagggta gattggttat ttgggaaaat gtagtgttag 1440
   tgttttgttt ttattttttt tattgtttaa atatgtttta tattttttta atgttttttg 1500
   agggttttta atttagttat tgagtttttt atttttggtg attgtgatat taataatgtt 1560
   gttatttttt ttgggaaata tggttgtaaa gatgttttgt aggaggtaga taaaaggttg 1620
   tgtttttaga tattgaaggt tgttttgtta tttagatgtt tttaattttt gtatagagaa 1680
   ggagggaaaa tatggggtgt 1700
<210> 169
   <211> 1700
   <212> DNA
   <213> Artificial Sequence
<220>
   <223> chemically treated genomic DNA (Homo sapiens)
<400> 169
   gtattttgtg tttttttttt ttttttatgt aggaattgga aatatttaag taataaagta 60
   atttttagta tttgaaagta tagtttttta tttgtttttt gtggagtatt tttgtaatta 120
   tgttttttaa ggagggtgat agtattgttg gtattatagt tgttaagggt gaggaattta 180
   gtggttgggt tgagaatttt tagaaggtat tagaaaaatg tggaatatgt ttaaatagtg 240
   aagaaagtag gagtaaagta ttaatattgt atttttttag gtaattagtt tattttgatt 300
   ttgggaagta taaagtattt ttatattggg ggaataatgt tttaataata tagaattaat 360
   attgttagga tggaatggaa tgtattgttt ttgtttggta taatttttag ttaatttggt 420
   ttatttagaa gtaattagtt tttttttaaa agtgtttttt gtttaattgg tttattttat 480
   ttttggtgtg ggggggaggg gagtgtttgt ttaatgaaat ttttgtatta ttagatgttt 540
   taatttttag ttatttttgt ataaaagtaa atattaggtt tttttatagg gatatttttt 600
   attgagttta aatgtgtttg atttttggag ggtgatattt agtatttttt ttgttgttat 660
   taattgggtt gaggaagtaa aagaaattta attgtttttt aaataaagtt atgttttttt 720
   ttataatatt gtgtagtttt ttttttttta tttttatttt gtatttggag agaaagagaa 780
   gtatgttttt tgggagtaag gagagtttat tgatgagtgt tattagaaat ttttagtaaa 840
   ggttggttgg gttattaaag gggtagggtg ggtgtagtgt tgttatgatt gtgaggtgtg 900
   tgttttggaa ataggttgat ttgttttgga tgtgttatta gttttttgag ggtttttttt 960
   ttttgttttt tttttttgag ttgttttgtt ttatttttgg gaagttttag gaatttttag 1020
   gggagttgta agtgtaattt aagtataatt tttaaggtat ttaggttttt ttattaatgt 1080
   ggagatagtg ggatttagta agtttaagag gaaaaaagaa aaagaaaaaa aaaaagggtt 1140
   ggtgtgagag ggatgggaaa ttagattgat aattttaatt aaattaatta tttgttttat 1200
   tatttggtat ttagtttatt ttttttttta ttttttttat tttgtatttt gggttttttt 1260
   gtagtgatga atttggattt gagtttgatt tgatttgtga ggatttgggg gaaatgtttt 1320
   aagattgagt tggtttgggt agtgaaggtg tgggaaggat tttggtagat gttaagatgg 1380
   taggaggtgt tttgtgtttg tttggtttat ggtttttgta aagtttttat ttttatttgt 1440
   tttttagtat ttattggtgt aaatattttt attgtagggt tatgttaaga atttaaatta 1500
   attattttag tttgtagatt tatatagaat agaaaatttt tattgggtat aaaaatgtat 1560
   ggttgtattt ttgatgaata gaatgggtgt tattaataaa agtattttaa aaattgaatt 1620
   tttttaaata atgtattagt gttatttttg ttttaggagt tttaatttta aagaattgtt 1680
   ttagagtttt gaattttgtt 1700
<210> 170
   <211> 1700
   <212> DNA
   <213> Artificial Sequence
<220>
   <223> chemically treated genomic DNA (Homo sapiens)
<400> 170
   attggatgag tggggttttt tgttttagtt ggatttttat aggaggatgg aggaaagtat      60
   ttttttgagt aagtttggag agtgaagggg tggggttgta aggttggaga aggaggtgtt     120
   ttttttataa aaatgattgg gttttttttg taatggttgg tataaagttg gaagggtttg     180
   gggatgggag gggtttgaag tgatttttga ggagttgggt tttttttgta aggttttggg     240
   agggaaggta aggtattttg tgtaatgata tattttagtt gttggatgtg aagatttttt     300
   tttgaagata gaataaagaa aggttttttt attaatgtgg gtttgaaatt ggtttgaaat     360
   ggtgtgtttg tagggatttg ggaggaaatg tgatgtttat ttttagattt gggaaaggtg     420
   ggggtaataa ttttttaaga gaattggagt ttgaaagagg aatgaaaagt ttttgtgttg     480
   tgtatatata ttttttttta ttggaaagtt aggagtgagg aaagaatttt gaagtgattt     540
   gggaatgatt tgaaagtgtg taagttggaa gttgggttga agttagtata gtggagtagg     600
   ggttaggttt ttgtgtggtt ggagtgggag agtgtttttg agaataagat ggggtaggat     660
   tgaaggggtg tgaggttgat gttttttagg gatgatttgg agggtgaagg aggtttgggg     720
   gtggttttgg gtttttggag gtgattgagg gagtttttga gttaatgttt gggggtggga     780
   ggtgttattg gtatttattg agttgatttt tagtgttagg gtgtattgta gtgtttttat     840
   ttttggtatt tgtgtgggga ggggtggtgg ggagtgattt tggttatggg tttgagtgag     900
   gtggggagat ttggggttgg gtggggatgg ggaggagatg aggtggtggt ggtggtggta     960
   gtagtggttt ttaggttggg gtttgtggtt tagtttgggt tttgttggag aagtagttga    1020
   gtgtggggtg tagatttgtt gttatggtag ttaggagggg tggggttgga agagaggttg    1080
   tgtggggtat tttagggtta tttgtgttgg gatgagtgtt ggaggtggag tttgatgaat    1140
   gttgagtgag gtggagttgg gtagggggtg gggtttttga aggggtgggg tgaggtaagg    1200
   tagttttttt gtgatgttaa attgtgttta aagttggatg aaagggagtt gttgtttgta    1260
   taatgggatg gtgttttgtt tagggagttg gagtttttgt aaggttgttg gagggaattt    1320
   ttgagttata atgtggtgat ggtattttgg gttagtggtg atttattgtg gttttagttt    1380
   ttttaattgt aaaagaataa taattattgt tagttttttt atttaaattt tatggtatgg    1440
   tttataaagt tagtataagg atattgggaa ttagggaaga gatttgttta aattaaagtt    1500
   ggaattagga ttggaattta ggagttttta agttaaattt tagttgtttt tttgaggaga    1560
   ggggtttgta ttagatggtt tatattttta gttttttatt ttgtgatttt ttttgttagt    1620
   gtgttttttt ttttttttaa atttttttgt ttaatttatt tatgtatgtg ttttaatgta    1680
   tattgtttaa gtggaattgt                                                1700
<210> 171
   <211> 1700
   <212> DNA
   <213> Artificial Sequence
<220>
   <223> chemically treated genomic DNA (Homo sapiens)
<400> 171
   atagttttat ttaagtaata tgtattaaga tgtatatatg ggtgaattag ataggaaaat 60
   ttgaagggaa aagggaatgt gttaataaag ggaattatag aataggagat taggagtatg 120
   gattatttga tgtaagtttt tttttttagg gaggtaattg gggtttaatt tgagagtttt 180
   tgggttttag ttttggtttt gattttgatt tgggtaagtt ttttttttaa tttttagtat 240
   ttttatgttg gttttgtaaa ttgtgttgtg aaatttaaat aggaggatta ataataatta 300
   ttattttttt ataattggga aaattgaggt tatagtgggt tgttattagt ttaaggtgtt 360
   attattatgt tgtaatttag gggttttttt tgatggtttt ataggaattt taattttttg 420
   aatagagtat tgttttatta tataaatagt agtttttttt tgtttggttt tgggtgtagt 480
   ttgatgttat agaagagttg ttttgtttta ttttgttttt ttggaagttt tgttttttgt 540
   ttgattttgt tttgtttggt gtttgttaag ttttgttttt agtgtttgtt ttagtgtggg 600
   tagttttgga gtgttttgtg tggttttttt tttggttttg ttttttttgg ttgttatgat 660
   aatgagtttg tgttttgtgt ttagttgttt ttttggtgga atttaggttg gattgtgggt 720
   tttggtttgg gggttattgt tgttattgtt gttgttattt tgtttttttt ttgtttttgt 780
   ttagttttag gtttttttgt tttatttggg tttgtggttg gggttatttt ttgttgtttt 840
   tttttgtatg gatgttgaag gtgaaggtgt tgtagtgtgt tttggtgttg gagattagtt 900
   tagtgagtat tggtagtgtt ttttattttt gggtattggt ttagggattt ttttagttat 960
   ttttgagagt ttggaattat ttttaaattt tttttatttt ttgaattatt tttagggagt 1020
   gttgatttta tgtttttttg gttttgtttt attttgtttt tagaagtgtt tttttatttt 1080
   ggttatgtag aggtttgatt tttgttttgt tatgttggtt ttaatttgat ttttaatttg 1140
   tgtgttttta gattgttttt aaattatttt agagtttttt ttttgttttt aattttttag 1200
   tgagagagag tgtatgtgtg tggtgtgggg gttttttatt ttttttttgg gttttagttt 1260
   ttttagagaa ttattgtttt tgtttttttt aagtttgggg gtgggtgttg tatttttttt 1320
   tgaatttttg taggtatatt attttagatt agttttaaat ttgtattaat gaaggggttt 1380
   ttttttattt tgtttttgaa ggagaatttt tatatttaat aattgaaatg tgttattgtg 1440
   taaagtattt tgtttttttt tttagggttt tataggaaaa atttaatttt ttagaaatta 1500
   ttttaggttt tttttgtttt tagatttttt tgattttgtg ttagttattg taagaaaggt 1560
   ttagttattt ttatgaggga aatatttttt tttttaattt tgtaatttta tttttttatt 1620
   ttttagattt gtttaagagg atgttttttt ttattttttt gtggaagttt agttggggta 1680
   gagaatttta tttatttagt 1700
<210> 172
   <211> 1700
   <212> DNA
   <213> Artificial Sequence
<220>
   <223> chemically treated genomic DNA (Homo sapiens)
<400> 172
   ttaggagttt agatttgggt aggaaataaa gttttaaagt ggttttgaag tgtttttttt      60
   ttagattttt ttttagtgtg ggttatttat tttagtttag ggaagagatt attttgtttg     120
   aatgtagtta aatgtattgt atgtgggagt taagtgggag atgaaataat ttaatgtgtt     180
   ttttttatta ttattatttt tattaaatta gttattgttg gtttaaattt attgggaatg    240
   tttgtgtatt ttattgatta gggaaataaa agggtgtata taaattagtg gaagtgggtg     300
   ggaaggaata agggatgtag attgttgtga gtaaaatgtt ttgtggtttg gtgagaggtt     360
   gttttttttt taagttattt attttagatt gaaatattaa gttagtttag gttatttttt     420
   gagtagtatt ataagttaaa ataagattta gaaaatttat taatttttaa ttgtgttgtt     480
   tttagattgg gagtataggg ggatagggtg tagggttgtg gggaggttga ggggaggggg     540
   atttgtggaa aagtaaatgt ttgagaaatt ttagtttttg attttagttg ttttttattt    600
   tatttagttt ggttgaggag ttgtttttat attttatggt aattttggaa tttatgatgt     660
   attttttaga gttgggtttt tagtttttta ggttttggag tttaagagtt ggattttata    720
   ggtttaatat ttattgtttg gttttttttg ttatatttta gtttagatag ttatttttat    780
   ttaggatagt tgtgaagtgt aaatggagga tgtaggtatt tttaaaaaga gtagttgtgg     840
   tttgtgtttt atggatgagt tattgttgta ggaggtttgg tggtgtggtg tgtttgtagt     900
   tgtggtagtg gtggagtttg tgtggggatt tttttgtgtg tatttagtgt tttgttttgg     960
   ggtagtgtta ttttgggtgt tgttttgtta tttttatggt gttttgttgt tttttattat    1020
   taggatggag ggtatttttt agtatgagtt gatattttag agggtattgg tgagtttttg    1080
   aaaaaattag ggtggtgggg tgaggaagat ttgtgttttg gattttagag aaggaaagaa    1140
   agttagaggg agttggagtg gagttgtatt ttatgttgaa gttttttttt tgaattgttt    1200
   ggagttgtaa gtggtttttg attgaatata gttgtttagt tgttggttat ggggtgtgtg   1260
   gaggaggtgg ggttagaggg agaaaagggt gggggtgagt agtttggagg ttgagtagag    1320
   gagattgtgt tgggagagta gagtaggtat ttttttagtt ttttgtaata gtaattaagg   1380
   atttgagttt aggaggggga gtagaaatag gaaaagttat ttgaataatt gtgtaggaaa    1440
   ttaaaaaaaa aaaaagaaaa gaaaaagaaa aaaaattttt tttataggag ttgtttttta    1500
   tgatttaata aggttaagat aaatttaaat attgtatatt agatagtatg gtgtattttt    1560
   tatggaatat taagatttta taagggtgta gggtagttat tttttttaat tagtttaaag   1620
   ttaatgttat gggtgtaaat ttaatgttaa attttttttg tttgttattg gggtagagta    1680
   tattttttgt atgtttataa                                                1700
<210> 173
   <211> 1700
   <212> DNA
   <213> Artificial Sequence
<220>
   <223> chemically treated genomic DNA (Homo sapiens)
<400> 173
   ttatggatat gtagaaaatg tattttgttt tagtgataga tagagggagt ttgatattga      60
   atttatattt gtagtgttga ttttagattg attagggaaa atgattgttt tgtatttttg    120
   taaaatttta atgttttgtg agaagtgtat tgtgttgttt gatgtatagt gtttaggttt      180
   gttttgattt tattaaatta tggaaagtgg tttttatgag ggaagttttt tttttttttt       240
   tttttttttt ttttttttaa ttttttgtat gattatttag gtggtttttt ttgtttttat       300
   tttttttttt ggatttgggt ttttaattat tattgtagag aattggaagg atgtttgttt     360
   tgttttttta gtatggtttt ttttgtttgg tttttaggtt atttgttttt attttttttt        420
   ttttttagtt ttgttttttt tgtgtatttt gtggttagta gttagatagt tgtgtttaat     480
   tagaaattat ttataatttt agatgatttg aaggggaaat tttggtgtga agtgtagttt     540
   tgttttggtt ttttttagtt tttttttttt ttttggaatt tgaggtgtgg attttttttg        600
   ttttattgtt ttagtttttt tgggagtttg ttggtgtttt ttagggtgtt ggtttgtgtt     660
   gggaagtgtt ttttattttg gtaatggggg gtggtgaggt attgtaggag tggtgaggtg     720
   gtgtttaggg tggtattgtt ttggaatggg gtgttgggtg tgtgtgggag ggtttttgtg     780
   tgggttttgt tgttgttgta gttgtgagtg tgttgtgtta ttgagttttt tgtagtaatg     840
   gtttgtttgt gaaatgtgag ttatggttgt tttttttaag agtgtttgta ttttttgttt     900
   gtgttttgta attgttttgg gtgaaaatgg ttgtttagat taaaatgtgg tagaagggat     960
   taagtagtgg atattgagtt tgtgaagttt aatttttaag ttttgagatt tgggggattg     1020
   agagtttagt tttgaaaagt gtattatgaa ttttggagtt gttatgaaat atggaaatga     1080
   ttttttggtt gagttgagtg aggtaagaaa tagttgaggt tgggagttag ggttttttgg      1140
   gtgtttgttt ttttataaat tttttttttt ttgatttttt tatagtttta tattttattt     1200
   ttttgtgttt ttagtttgga ggtagtgtaa ttgaagatta gtagattttt tggattttgt     1260
   tttaatttgt agtgttattt aggaaatgat ttgaattggt ttgatatttt agtttgaaat     1320
   gagtggttta aaaaagaaat aattttttat taagttgtga ggtgttttat ttatgatagt     1380
   ttgtattttt tgtttttttt tatttgtttt tattaattta tatatatttt tttatttttt      1440
   tgattggtaa aatatatgga tatttttgat gagtttaagt tgatggtaat taatttggtg     1500
   ggggtggtgg tggtgagggg gatgtattgg attattttat tttttatttg gtttttatgt     1560
   gtgatgtgtt tggttatgtt taggtaaggt ggtttttttt ttaggttgag atgaatgatt     1620
   tgtgttgggg aggggtttgg ggagaaagta ttttaaagtt gttttagaat tttatttttt     1680
   gtttggattt ggatttttga                                                 1700
<210> 174
   <211> 1700
   <212> DNA
   <213> Artificial Sequence
<220>
   <223> chemically treated genomic DNA (Homo sapiens)
<400> 174
   gttggtaagt gaaaagttta tagtttatag tatttttatt gtttagtttt atttttattg      60
   ttgagtttag gaatttgttt ttttgttttt ttttggaagt gattttgtgt gtgtaggtag     120
   gggtagaggg agtaaagtta aatgtaatta tagtatttta gatttgtaga tttttaaatt     180
   agttttatat tttagtgagg tgataatggt taatattttt attttagttt tatagaagtt     240
   tagttatagt aggtgatttg tgtaaagtta tatagttttt ggttttaagg tagggttttt     300
   ttttttgttt gtgttttttt gttattgtag tttttgtttt ttagttgggg gattatttag     360
   aggattattt ggattttgtt gtttttgttg ggttatttgt ttggtggagt agttgggggg     420
   tggggggttt gtaatttagg attttatttg gtgttgtttt agtttattga gtagtgagtt     480
   agtttttttt ttattggtgg tagggggagt tgttgggatt ttagggttgt tttgaggggt     540
   tgtgtagatt tgggggatgt aaaggtttaa atggatttgg aatggttggt gtggttgggt     600
   tttgggaaat gaagggtttg tggtgttttg tattttttgt gggggtggga gtaattatta     660
   ggtggttgtg gagttagttg agggggtggt tgttaatttg ggtggtgatt gtaggtggag     720
   atgttttgtt tgggataggg attgggttgt gttggtgttt gtggatagat atttgtggtt     780
   ttggttgttg ttgtgttttt tgtttttgtt atgttttggt ggttttgttt ggggtgtgtt     840
   tttgttttgt atgtgtatgt gtttgtattt gtattggttt gtttggtttt tttgtgtttg     900
   tggggttgtt ttgggggagg tgttgagatt tggttttggt ttgtgtgtgg tttttggggt     960
   ttggtgtgtt ggggatttat aggtgaggtg agagaaaggg gttggtttgt ggggatattt    1020
   atttgtgtgg tgtggggttg tggtgttggt tattattatt ttgtgttttt ttttttgtta    1080
   gtgtgtatgt agatggtggg gtggtttggg gaggtttttg ggtttttttt tgggaatgta    1140
   gggttaagtt gtgttttgat tttatgtttt ttttatttat gttgggtata tgtagttttg    1200
   agtgagggtt tatgttttgt tttttatgtt gattatagtt tttaggtttg gtgtttgttg    1260
   ggaagggttg taatggttta gggatttttg tttttattat tattttgttt tggggggata   1320
   ggttttagaa tattgtgtgg ggtttagagt gggtttagtt gtaaaaggat aggggttttt    1380
   ttttagttat gtttattttt gggtgttttg gttgttagta gttttttatt ataattgtag   1440
   ggtgagaagt ttagagaata gtttaggagt agtttttttg ttttttatta tgtttgtgtg    1500
   tttagtatgg gtaaattagt agttatgtaa taaatgaagt gtggtgttgt agttttgggt    1560
   ttttttgtat ttgttttgat tttagtttgt ttagtttagt ttggggattt gtatttaggt    1620
   ttgtagtttt ttgggttttt gtttggtgtt ttttagagtt ggagtttgtt tgttgtaggg    1680
   atggtttaga ggatttttta                                                 1700
<210> 175
   <211> 1700
   <212> DNA
   <213> Artificial Sequence
<220>
   <223> chemically treated genomic DNA (Homo sapiens)
<400> 175
   taggaggttt tttaagttat ttttgtggta gataggtttt ggttttgagg agtattgggt      60
   agggatttag gaggttgtaa atttaagtgt agatttttag gttgggttgg ataggttgag     120
   gttagggtag gtgtagagag gtttagagtt gtaatattgt attttattta ttgtgtggtt     180
   attgatttgt ttgtgttggg tatatggatg tggtaaagga tagaagggtt atttttggat     240
   tgttttttgg gttttttatt ttgtggttgt ggtgagaggt tgttgatagt tagggtattt     300
   gggagtgggt gtggttggag aaggattttt gtttttttat agttaggttt attttgagtt     360
   ttatgtagtg ttttagagtt tgttttttta gggtaggatg gtagtggggg taggggtttt     420
   tgagttattg tggttttttt tggtaggtgt taagtttagg ggttgtggtt agtgtgaaag     480
   gtagggtatg gatttttatt tagggttgtg tgtgtttgat gtgggtgggg ggggtgtgga     540
   attggagtat aatttggttt tgtgttttta ggaagggatt tgaagatttt tttaggttat     600
   tttgttattt gtgtgtgtgt tggtaagagg aggggtgtgg ggtaatggtg gttggtgtta     660
   tagttttgtg ttgtgtaggt aggtgttttt gtgagttaat tttttttttt tgttttgttt     720
   gtgggttttt ggtgtgttgg attttggaaa ttgtgtgtgg gttaaggttg ggttttggta     780
   ttttttttgg agtggttttg tgagtgtggg ggaattgggt gggttagtgt gggtgtaggt     840
   gtgtgtgtgt gtgaggtggg ggtgtgtttt aagtgaggtt gttggggtgt ggtgggggtg     900
   ggagatgtgg tagtggttgg gattgtgggt gtttgtttgt gagtgttggt gtggtttggt     960
   ttttgttttg ggtggggtgt ttttgtttgt ggttgttgtt tgggttagta gttgtttttt    1020
   tggttggttt tgtggttatt tggtaattat ttttgttttt ataaaaagtg tgagatattg    1080
   tgagtttttt attttttaga gtttggttgt attggttatt ttgagtttgt ttgggttttt    1140
   atatttttta gatttgtgtg gttttttagg gtagttttga gattttgata gttttttttg    1200
   ttattgatga ggggagagtt ggtttattgt ttggtgggtt ggggtagtgt taggtggggt    1260
   tttaggttgt aggtttttta ttttttaatt attttgttaa gtaaatggtt taatggaggt    1320
   agtagggttt aggtggtttt ttgggtggtt ttttagttgg agaatagggg ttgtggtggt    1380
   gagagggtat aggtagggga gagagttttg ttttggagtt agaggttgtg tagttttgtg    1440
   taggttattt gttgtggttg ggtttttgta aaattgagat gaaaatgtta gttattgtta    1500
   ttttattgag gtgtgaggtt ggtttgagag tttgtaaatt tgagatgtta tagttatgtt    1560
   tggttttatt ttttttgttt ttatttgtat atataggatt gtttttaaaa ggaggtaggg    1620
   aaataaattt ttgagtttaa tagtggaagt ggggttggat agtgaggatg ttgtggattg    1680
   tgggtttttt atttgttagt                                                 1700
<210> 176
   <211> 1700
   <212> DNA
   <213> Artificial Sequence
<220>
   <223> chemically treated genomic DNA (Homo sapiens)
<400> 176
   gtaagatttt attttaaaaa aaaaaaaaaa aaaaagtgat ttgtttagta atagttttaa      60
   aatatggaag agttgggatt ggatttagat tatttggttt tggaattggg ttggtaagtg     120
   aaaagtttat agtttatagt atttttattg tttagtttta tttttattgt tgagtttagg     180
   aatttgtttt tttgtttttt tttggaagtg attttgtgtg tgtaggtagg ggtagaggga      240
   gtaaagttaa atgtaattat agtattttag atttgtagat ttttaaatta gttttatatt     300
   ttagtgaggt gataatggtt aatattttta ttttagtttt atagaagttt agttatagta     360
   ggtgatttgt gtaaagttat atagtttttg gttttaaggt agggtttttt tttttgtttg      420
   tgtttttttg ttattgtagt ttttgttttt tagttggggg attatttaga ggattatttg     480
   gattttgttg tttttgttgg gttatttgtt tggtggagta gttggggggt ggggggtttg     540
   taatttagga ttttatttgg tgttgtttta gtttattgag tagtgagtta gttttttttt    600
   tattggtggt agggggagtt gttgggattt tagggttgtt ttgaggggtt gtgtagattt   660
   gggggatgta aaggtttaaa tggatttgga atggttggtg tggttgggtt ttgggaaatg   720
   aagggtttgt ggtgttttgt attttttgtg ggggtgggag taattattag gtggttgtgg    780
   agttagttga gggggtggtt gttaatttgg gtggtgattg taggtggaga tgttttgttt   840
   gggataggga ttgggttgtg ttggtgtttg tggatagata tttgtggttt tggttgttgt   900
   tgtgtttttt gtttttgtta tgttttggtg gttttgtttg gggtgtgttt ttgttttgta   960
   tgtgtatgtg tttgtatttg tattggtttg tttggttttt ttgtgtttgt ggggttgttt   1020
   tgggggaggt gttgagattt ggttttggtt tgtgtgtggt ttttggggtt tggtgtgttg   1080
   gggatttata ggtgaggtga gagaaagggg ttggtttgtg gggatattta tttgtgtggt   1140
   gtggggttgt ggtgttggtt attattattt tgtgtttttt tttttgttag tgtgtatgta   1200
   gatggtgggg tggtttgggg aggtttttgg gttttttttt gggaatgtag ggttaagttg    1260
   tgttttgatt ttatgttttt tttatttatg ttgggtatat gtagttttga gtgagggttt   1320
   atgttttgtt ttttatgttg attatagttt ttaggtttgg tgtttgttgg gaagggttgt   1380
   aatggtttag ggatttttgt ttttattatt attttgtttt ggggggatag gttttagaat   1440
   attgtgtggg gtttagagtg ggtttagttg taaaaggata ggggtttttt tttagttatg   1500
   tttatttttg ggtgttttgg ttgttagtag ttttttatta taattgtagg gtgagaagtt 1560
   tagagaatag tttaggagta gtttttttgt tttttattat gtttgtgtgt ttagtatggg   1620
   taaattagta gttatgtaat aaatgaagtg tggtgttgta gttttgggtt tttttgtatt   1680
   tgttttgatt ttagtttgtt                                               1700
<210> 177
   <211> 1700
   <212> DNA
   <213> Artificial Sequence
<220>
   <223> chemically treated genomic DNA (Homo sapiens)
<400> 177
   gataggttga ggttagggta ggtgtagaga ggtttagagt tgtaatattg tattttattt    60
   attgtgtggt tattgatttg tttgtgttgg gtatatggat gtggtaaagg atagaagggt   120
   tatttttgga ttgttttttg ggttttttat tttgtggttg tggtgagagg ttgttgatag     180
   ttagggtatt tgggagtggg tgtggttgga gaaggatttt tgttttttta tagttaggtt   240
   tattttgagt tttatgtagt gttttagagt ttgttttttt agggtaggat ggtagtgggg    300
   gtaggggttt ttgagttatt gtggtttttt ttggtaggtg ttaagtttag gggttgtggt   360
   tagtgtgaaa ggtagggtat ggatttttat ttagggttgt gtgtgtttga tgtgggtggg   420
   gggggtgtgg aattggagta taatttggtt ttgtgttttt aggaagggat ttgaagattt   480
   ttttaggtta ttttgttatt tgtgtgtgtg ttggtaagag gaggggtgtg gggtaatggt   540
   ggttggtgtt atagttttgt gttgtgtagg taggtgtttt tgtgagttaa tttttttttt    600
   ttgttttgtt tgtgggtttt tggtgtgttg gattttggaa attgtgtgtg ggttaaggtt   660
   gggttttggt attttttttg gagtggtttt gtgagtgtgg gggaattggg tgggttagtg   720
   tgggtgtagg tgtgtgtgtg tgtgaggtgg gggtgtgttt taagtgaggt tgttggggtg   780
   tggtgggggt gggagatgtg gtagtggttg ggattgtggg tgtttgtttg tgagtgttgg   840
   tgtggtttgg tttttgtttt gggtggggtg tttttgtttg tggttgttgt ttgggttagt   900
   agttgttttt ttggttggtt ttgtggttat ttggtaatta tttttgtttt tataaaaagt   960
   gtgagatatt gtgagttttt tattttttag agtttggttg tattggttat tttgagtttg  1020
   tttgggtttt tatatttttt agatttgtgt ggttttttag ggtagttttg agattttgat  1080
   agtttttttt gttattgatg aggggagagt tggtttattg tttggtgggt tggggtagtg  1140
   ttaggtgggg ttttaggttg taggtttttt attttttaat tattttgtta agtaaatggt    1200
   ttaatggagg tagtagggtt taggtggttt tttgggtggt tttttagttg gagaataggg  1260
   gttgtggtgg tgagagggta taggtagggg agagagtttt gttttggagt tagaggttgt 1320
   gtagttttgt gtaggttatt tgttgtggtt gggtttttgt aaaattgaga tgaaaatgtt  1380
   agttattgtt attttattga ggtgtgaggt tggtttgaga gtttgtaaat ttgagatgtt  1440
   atagttatgt ttggttttat tttttttgtt tttatttgta tatataggat tgtttttaaa  1500
   aggaggtagg gaaataaatt tttgagttta atagtggaag tggggttgga tagtgaggat  1560
   gttgtggatt gtgggttttt tatttgttag tttagtttta gaattagata gtttaagttt  1620
   aattttagtt tttttatatt ttagagttat tattgggtaa gttgtttttt tttttttttt  1680
   tttttgagat ggagttttgt                                               1700
<210> 178
   <211> 1700
   <212> DNA
   <213> Artificial Sequence
<220>
   <223> chemically treated genomic DNA (Homo sapiens)
<400> 178
   aggtgggtag gttttttgga gtagtaggtg gtttttttag tttttatggt tggagttagg 60
   attttttttt gtttataatt tttttaattt ttttaattta atatttttta tttttttttt 120
   tttttttttt tttttttttt tttttttttt tttttttttt tttttttttt tttttttttt 180
   tttttttttt tttttttttt ttgttttttt ttttttttta gttttttttt tttattttgt 240
   tgtgggtggg ttgtagtgtt gattttattg ggatgtttgg ataggtattg tttgtgataa 300
   tggtggtatt ggaaggtttt ggtagttgaa tgtttagttt tttatttttt atgtttttat 360
   attttttatg tataaatttt gtaggaagta ggttagggaa tgtagtttat tgtttttttt 420
   tgattagtag ttttagtttg gttatattgt agttgtggta gaggatatgg tttattttta 480
   tgagttaatt ggggtttatt ttttattttg gtggttttag tgggtttagt aagtgagagt 540
   aggtttttgg agttataggg gtttttttgt ttttgtagtg agggaagtag gaggtggtag 600
   gttttgaggt ttgtgggttg aaggtttagg ttttatgtgg agttatttgt ggaggattta 660
   ggttaggtta atttgtttgt ggggaatttg tttgattttg gtgagttagg ggtttggaag 720
   gttgggtgga gtttggttaa tagtttgggt tttttatttt gggttttgtt ttttttggag 780
   gttttggggg tatttagtgg gtaggttttt ttggtttggt tggttttgga gtaggtgagg 840
   aaggttaggt aggttttgtg gaggtgggtt aggttttggt gggtggtttt gttgatgaag 900
   tagtagagta tggggttggt gatgtagttg aagttggtga gtaggaggga gaagtggtag 960
   gtgttgaaaa tgtttttggt gaagttgtag ttggtttttt agatgttgtg tattagtagt 1020
   aatatgtggt agggtaggaa gtaggttagg aagatgatta tggtgttgag tattagttgt 1080
   tggatttggt ttttgtggtt tttttgggtg ttgtggtttt ggtgtatggt gtgtaggatg 1140
   ttttggtagg atgttagtag taggtagatg gggaagagga agtttattag gaagtggtag 1200
   tagttgatgg tgtgttgtta tgtttggatg gggtagtgtt taaagtatat gtggtgttgg 1260
   tttttgtttt tgatgatttt tttgtgtatt aggaagtaga tgttggttag tagttttttg 1320
   gtttagatga ttatgttgat gttgatggtt gtttttaggg tttggaattg gtggaagtgg 1380
   aagggatggg ttatagttag gtagtggttt atggagatgt agtagaggaa gtttatgttg 1440
   atgtagatgt ttttgtatag gaggatgttg tatatttggt aggataggtt gttgtgagat 1500
   tagttgttgt gttgtagtat gtattgtagt tagaagggta gtgagtagat gtagaagagg 1560
   ttggttattg ttaggttgta taggtatatg tttagtttgt tttgggtttt gatttgtagg 1620
   tagttgaagt agagggatag gtagttggtt gggaagttta ttattagtat ggtaatatag 1680
   attattgggg ttagtgtttg 1700
<210> 179
   <211> 1700
   <212> DNA
   <213> Artificial Sequence
<220>
   <223> chemically treated genomic DNA (Homo sapiens)
<400> 179
   tagatgttgg ttttggtggt ttatgttatt gtgttggtgg tgggtttttt ggttaattgt 60
   ttgttttttt attttggtta tttgtagatt aaggtttgga atgagttggg tgtgtatttg 120
   tgtaatttga tggtggttga ttttttttat atttgtttgt tgtttttttg gttgtagtat 180
   gtgttgtagt atgataattg gttttatggt gatttgtttt gttaggtgtg tggtattttt 240
   ttgtatgaga atatttatat tagtgtgggt tttttttgtt gtatttttgt ggattgttat 300
   ttggttgtgg tttatttttt ttgtttttat tagttttgga ttttgaaggt ggttgttggt 360
   gttagtgtgg ttatttgggt taaggagttg ttgattagta tttatttttt gatgtatgag 420
   gaggttattg aggatgagaa ttagtattgt gtgtgttttg agtattattt tatttaggta 480
   tggtagtgtg ttattaatta ttattgtttt ttggtgggtt tttttttttt tatttgtttg 540
   ttgttggtgt tttattaggg tattttgtgt gttgtgtgtt ggagttatgg tatttagaag 600
   agttgtaagg attagattta gtggttggtg tttagtattg tggttatttt tttggtttgt 660
   tttttgtttt attatgtgtt gttgttggtg tgtagtgttt gggaggttag ttgtgatttt 720
   gttaagggtg tttttaatgt ttattatttt ttttttttgt ttattagttt taattgtgtt 780
   gttgattttg tgttttattg ttttgttagt gagattattt attgggattt ggtttgtttt 840
   tgtggggttt gtttggtttt ttttatttgt tttaggattg gttgggttag ggaggtttat    900
   ttgttgggtg tttttgaggt ttttgggaaa agtggggttt agggtgagga gtttgagttg    960
   ttgattaagt tttatttggt tttttagatt tttaatttgt tagggttggg tgggtttttt   1020
   atgggtaggt tggtttagtt tgggtttttt gtgggtggtt ttatgtgagg tttgagtttt   1080
   tagtttatgg gttttagggt ttgttgtttt ttgttttttt tgttgtggag gtagggaagt   1140
   ttttgtaatt ttggaagttt gtttttgttt gttgagtttg ttgggattgt tgagggtggg   1200
   aataagtttt ggttggtttg tgggaataag ttgtgttttt tgttgtggtt gtgatgtggt   1260
   taggttgggg ttgttggttg ggggaagata gtgaattgtg ttttttggtt tgttttttgt   1320
   agagtttgtg tatggggagt gtgaggatat ggagggtggg aggttgggtg tttagttgtt   1380
   agggtttttt aatgttattg ttgttataga taatgtttgt ttaaatgttt tggtgggatt   1440
   agtattgtag tttgtttata atagggtggg aagggaagat tggaggggga aggaaggtag   1500
   gagggggaag gaagaaagag ggggaaggaa agaaagaggg gggaaggaaa ggaggaaggg   1560
   gagaaggaag ggaggaggaa agaaggaagg tagggggtgt tgggttggga gaattgaggg   1620
   agttataggt agaggaggat tttagtttta gttataagaa ttgggagagt tgtttgttgt   1680
   tttagaagat ttgtttattt                                               1700
<210> 180
   <211> 1700
   <212> DNA
   <213> Artificial Sequence
<220>
   <223> chemically treated genomic DNA (Homo sapiens)
<400> 180
   aggtgggtag gttttttgga gtagtaggtg gtttttttag tttttatggt tggagttagg      60
   attttttttt gtttataatt tttttaattt ttttaattta atatttttta tttttttttt   120
   tttttttttt tttttttttt tttttttttt tttttttttt tttttttttt tttttttttt   180
   tttttttttt tttttttttt ttgttttttt ttttttttta gttttttttt tttattttgt   240
   tgtgggtggg ttgtagtgtt gattttattg ggatgtttgg ataggtattg tttgtgataa   300
   tggtggtatt ggaaggtttt ggtagttgaa tgtttagttt tttatttttt atgtttttat   360
   attttttatg tataaatttt gtaggaagta ggttagggaa tgtagtttat tgtttttttt   420
   tgattagtag ttttagtttg gttatattgt agttgtggta gaggatatgg tttattttta   480
   tgagttaatt ggggtttatt tttatttttg gtggttttag tgggtttagt aagtgagagt   540
   aggtttttgg agttataggg gtttttttgt ttttgtagtg agggaagtag gaggtggtag   600
   gttttgaggt ttgtgggttg aaggtttagg ttttatgtgg agttatttgt ggaggattta   660
   ggttaggtta atttgtttgt ggggaatttg tttgattttg gtgagttagg ggtttggaag   720
   gttgggtgga gtttggttaa tagtttgggt tttttatttt gggttttgtt ttttttggag   780
   gttttggggg tatttagtgg gtaggttttt ttggtttggt tggttttgga gtaggtgagg   840
   aaggttaggt aggttttgtg gaggtgggtt aggttttggt gggtggtttt gttgatgaag   900
   tagtagagta tggggttggt gatgtagttg aagttggtga gtaggaggga gaagtggtag   960
   gtgttgaaaa tgtttttggt gaagttgtag ttggtttttt agatgttgtg tattagtagt  1020
   aatatgtggt agggtaggaa gtaggttagg aagatgatta tggtgttgag tattagttgt  1080
   tggatttggt ttttgtggtt tttttgggtg ttgtggtttt ggtgtatggt gtgtaggatg  1140
   ttttggtagg atgttagtag taggtagatg gggaagagga agtttattag gaagtggtag  1200
   tagttgatgg tgtgttgtta tgtttggatg gggtagtgtt taaagtatat gtggtgttgg  1260
   tttttgtttt tgatgatttt tttgtgtatt aggaagtaga tgttggttag tagttttttg   1320
   gtttagatga ttatgttgat gttgatggtt gtttttaggg tttggaattg gtggaagtgg  1380
   aagggatggg ttatagttag gtagtggttt atggagatgt agtagaggaa gtttatgttg  1440
   atgtagatgt ttttgtatag gaggatgttg tatatttggt aggataggtt gttgtgagat  1500
   tagttgttgt gttgtagtat gtattgtagt tagaagggta gtgagtagat gtagaagagg  1560
   ttggttattg ttaggttgta taggtatatg tttagtttgt tttgggtttt gatttgtagg  1620
   tagttgaagt agagggatag gtagttggtt gggaagttta ttattagtat ggtaatatag  1680
   attattgggg ttagtgtttg                                               1700
<210> 181
   <211> 1700
   <212> DNA
   <213> Artificial Sequence
<220>
   <223> chemically treated genomic DNA (Homo sapiens)
<400> 181
   tagatgttgg ttttggtggt ttatgttatt gtgttggtgg tgggtttttt ggttaattgt      60
   ttgttttttt attttggtta tttgtagatt aaggtttgga atgagttggg tgtgtatttg     120
   tgtaatttga tggtggttga ttttttttat atttgtttgt tgtttttttg gttgtagtat     180
   gtgttgtagt atgataattg gttttatggt gatttgtttt gttaggtgtg tggtattttt     240
   ttgtatgaga atatttatat tagtgtgggt tttttttgtt gtatttttgt ggattgttat     300
   ttggttgtgg tttatttttt ttgtttttat tagttttgga ttttgaaggt ggttgttggt     360
   gttagtgtgg ttatttgggt taaggagttg ttgattagta tttatttttt gatgtatgag     420
   gaggttattg aggatgagaa ttagtattgt gtgtgttttg agtattattt tatttaggta     480
   tggtagtgtg ttattaatta ttattgtttt ttggtgggtt tttttttttt tatttgtttg     540
   ttgttggtgt tttattaggg tattttgtgt gttgtgtgtt ggagttatgg tatttagaag     600
   agttgtaagg attagattta gtggttggtg tttagtattg tggttatttt tttggtttgt     660
   tttttgtttt attatgtgtt gttgttggtg tgtagtgttt gggaggttag ttgtgatttt     720
   gttaagggtg tttttaatgt ttattatttt ttttttttgt ttattagttt taattgtgtt     780
   gttgattttg tgttttattg ttttgttagt gagattattt attgggattt ggtttgtttt     840
   tgtggggttt gtttggtttt ttttatttgt tttaggattg gttgggttag ggaggtttat     900
   ttgttgggtg tttttgaggt ttttgggaaa agtggggttt agggtgagga gtttgagttg     960
   ttgattaagt tttatttggt tttttagatt tttaatttgt tagggttggg tgggtttttt    1020
   atgggtaggt tggtttagtt tgggtttttt gtgggtggtt ttatgtgagg tttgagtttt    1080
   tagtttatgg gttttagggt ttgttgtttt ttgttttttt tgttgtggag gtagggaagt    1140
   ttttgtaatt ttggaagttt gtttttgttt gttgagtttg ttgggattgt tgagggtggg    1200
   aataagtttt ggttggtttg tgggaataag ttgtgttttt tgttgtggtt gtgatgtggt    1260
   taggttgggg ttgttggttg ggggaagata gtgaattgtg ttttttggtt tgttttttgt    1320
   agagtttgtg tatggggagt gtgaggatat ggagggtggg aggttgggtg tttagttgtt    1380
   agggtttttt aatgttattg ttgttataga taatgtttgt ttaaatgttt tggtgggatt    1440
   agtattgtag tttgtttata atagggtggg aagggaagat tggaggggga aggaaggtag    1500
   gagggggaag gaagaaagag ggggaaggaa agaaagaggg gggaaggaaa ggaggaaggg    1560
   gagaaggaag ggaggaggaa agaaggaagg tagggggtgt tgggttggga gaattgaggg    1620
   agttataggt agaggaggat tttagtttta gttataagaa ttgggagagt tgtttgttgt    1680
   tttagaagat ttgtttattt                                                1700
<210> 182
   <211> 22
   <212> DNA
   <213> Artificial Sequence
<220>
   <223> chemically treated genomic DNA (Homo sapiens)
<400> 182
   aaacgactac cgaaaaatac gc 22
<210> 183
   <211> 23
   <212> DNA
   <213> Artificial Sequence
<220>
   <223> chemically treated genomic DNA (Homo sapiens)
<400> 183
   ggtacgtttt ttttacggtc gta 23
<210> 184
   <211> 22
   <212> DNA
   <213> Artificial Sequence
<220>
   <223> chemically treated genomic DNA (Homo sapiens)
<400> 184
   aaatccaacg cgatccgaac gc 22
<210> 185
   <211> 26
   <212> DNA
   <213> Artificial Sequence
<220>
   <223> chemically treated genomic DNA (Homo sapiens)
<400> 185
   gggattagaa tttttttatg cgagtt 26
<210> 186
   <211> 20
   <212> DNA
   <213> Artificial Sequence
<220>
   <223> chemically treated genomic DNA (Homo sapiens)
<400> 186
   cgaatcctac cccgacgata 20
<210> 187
   <211> 23
   <212> DNA
   <213> Artificial Sequence
<220>
   <223> chemically treated genomic DNA (Homo sapiens)
<400> 187
   cgaatcgctc gcgttctcga cat 23
<210> 188
   <211> 22
   <212> DNA
   <213> Artificial Sequence
<220>
   <223> chemically treated genomic DNA (Homo sapiens)
<400> 188
   gtattcgttc ggttcgtatc gt 22
<210> 189
   <211> 17
   <212> DNA
   <213> Artificial Sequence
<220>
   <223> chemically treated genomic DNA (Homo sapiens)
<400> 189
   cgtcccgcga ccaataa 17
<210> 190
   <211> 33
   <212> DNA
   <213> Artificial Sequence
<220>
   <223> chemically treated genomic DNA (Homo sapiens)
<400> 190
   cgctccccta aactataaac gaaccgaaaa acg 33
<210> 191
   <211> 18
   <212> DNA
   <213> Artificial Sequence
<220>
   <223> chemically treated genomic DNA (Homo sapiens)
<400> 191
   caaacacccg aaaaccga 18
<210> 192
   <211> 19
   <212> DNA
   <213> Artificial Sequence
<220>
   <223> chemically treated genomic DNA (Homo sapiens)
<400> 192
   gggaagagga tacggcgta 19
<210> 193
   <211> 23
   <212> DNA
   <213> Artificial Sequence
<220>
   <223> chemically treated genomic DNA (Homo sapiens)
<400> 193
   ctccgcgaac gtcccatacg aaa 23
<210> 194
   <211> 17
   <212> DNA
   <213> Artificial Sequence
<220>
   <223> chemically treated genomic DNA (Homo sapiens)
<400> 194
   cgacgaacgc gacgaaa 17
<210> 195
   <211> 18
   <212> DNA
   <213> Artificial Sequence
<220>
   <223> chemically treated genomic DNA (Homo sapiens)
<400> 195
   gcgtttgcgg cgtttagt 18
<210> 196
   <211> 30
   <212> DNA
   <213> Artificial Sequence
<220>
   <223> chemically treated genomic DNA (Homo sapiens)
<400> 196
   cgactcctcg atatcaaaac gaacgaaacg 30
<210> 197
   <211> 19
   <212> DNA
   <213> Artificial Sequence
<220>
   <223> chemically treated genomic DNA (Homo sapiens)
<400> 197
   tcggtgaggt tttcgtcga 19
<210> 198
   <211> 21
   <212> DNA
   <213> Artificial Sequence
<220>
   <223> chemically treated genomic DNA (Homo sapiens)
<400> 198
   cgctacacga acttatcccg a 21
<210> 199
   <211> 23
   <212> DNA
   <213> Artificial Sequence
<220>
   <223> chemically treated genomic DNA (Homo sapiens)
<400> 199
   atcacgtcca accgacgtac gcc 23
<210> 200
   <211> 17
   <212> DNA
   <213> Artificial Sequence
<220>
   <223> chemically treated genomic DNA (Homo sapiens)
<400> 200
   aaacgtaacc ccgccga 17
<210> 201
   <211> 19
   <212> DNA
   <213> Artificial Sequence
<220>
   <223> chemically treated genomic DNA (Homo sapiens)
<400> 201
   gtagtagcgc gtcggttcg 19
<210> 202
   <211> 22
   <212> DNA
   <213> Artificial Sequence
<220>
   <223> chemically treated genomic DNA (Homo sapiens)
<400> 202
   cgctaccctc tccgctaccc gc 22
<210> 203
   <211> 17
   <212> DNA
   <213> Artificial Sequence
<220>
   <223> chemically treated genomic DNA (Homo sapiens)
<400> 203
   gcgtttggaa ttcggcg 17
<210> 204
   <211> 24
   <212> DNA
   <213> Artificial Sequence
<220>
   <223> chemically treated genomic DNA (Homo sapiens)
<400> 204
   gaatcaaccg tctataaaca acgc 24
<210> 205
   <211> 25
   <212> DNA
   <213> Artificial Sequence
<220>
   <223> chemically treated genomic DNA (Homo sapiens)
<400> 205
   tccgccgacg cttaacgtca atacc 25
<210> 206
   <211> 23
   <212> DNA
   <213> Artificial Sequence
<220>
   <223> chemically treated genomic DNA (Homo sapiens)
<400> 206
   attttcgtta aaaacgtcgg ttt 23
<210> 207
   <211> 21
   <212> DNA
   <213> Artificial Sequence
<220>
   <223> chemically treated genomic DNA (Homo sapiens)
<400> 207
   aacgaaacga cgacgtaacg t 21
<210> 208
   <211> 34
   <212> DNA
   <213> Artificial Sequence
<220>
   <223> chemically treated genomic DNA (Homo sapiens)
<400> 208
   ataattaaaa cacgaaaaaa cgtaaccgaa cgac 34
<210> 209
   <211> 22
   <212> DNA
   <213> Artificial Sequence
<220>
   <223> chemically treated genomic DNA (Homo sapiens)
<400> 209
   tgtcgtacgt agttttaggc gg 22
<210> 210
   <211> 17
   <212> DNA
   <213> Artificial Sequence
<220>
   <223> chemically treated genomic DNA (Homo sapiens)
<400> 210
   cgacgcctcc gcctaat 17
<210> 211
   <211> 23
   <212> DNA
   <213> Artificial Sequence
<220>
   <223> chemically treated genomic DNA (Homo sapiens)
<400> 211
   ctaaaccccg acgaaaaccc cgt 23
<210> 212
   <211> 20
   <212> DNA
   <213> Artificial Sequence
<220>
   <223> chemically treated genomic DNA (Homo sapiens)
<400> 212
   tattttgtag gcgttcgcgg 20
<210> 213
   <211> 20
   <212> DNA
   <213> Artificial Sequence
<220>
   <223> chemically treated genomic DNA (Homo sapiens)
<400> 213
   ccgacaaaaa caaaaatcgc 20
<210> 214
   <211> 31
   <212> DNA
   <213> Artificial Sequence
<220>
   <223> chemically treated genomic DNA (Homo sapiens)
<400> 214
   atcgtccttc tttcgaaata caaaactccg a 31
<210> 215
   <211> 17
   <212> DNA
   <213> Artificial Sequence
<220>
   <223> chemically treated genomic DNA (Homo sapiens)
<400> 215
   gccctaccga acccgaa 17
<210> 216
   <211> 20
   <212> DNA
   <213> Artificial Sequence
<220>
   <223> chemically treated genomic DNA (Homo sapiens)
<400> 216
   gtcgtttaat cgggagtcgg 20
<210> 217
   <211> 24
   <212> DNA
   <213> Artificial Sequence
<220>
   <223> chemically treated genomic DNA (Homo sapiens)
<400> 217
   acaaacgctc gacgcgtacg tcac 24
<210> 218
   <211> 21
   <212> DNA
   <213> Artificial Sequence
<220>
   <223> chemically treated genomic DNA (Homo sapiens)
<400> 218
   actacgccga aaacctacga c 21
<210> 219
   <211> 23
   <212> DNA
   <213> Artificial Sequence
<220>
   <223> chemically treated genomic DNA (Homo sapiens)
<400> 219
   gaggtttcgt attttagacg cgt 23
<210> 220
   <211> 23
   <212> DNA
   <213> Artificial Sequence
<220>
   <223> chemically treated genomic DNA (Homo sapiens)
<400> 220
   cgaccacgcc ctacctcgac gac 23
<210> 221
   <211> 21
   <212> DNA
   <213> Artificial Sequence
<220>
   <223> chemically treated genomic DNA (Homo sapiens)
<400> 221
   gcgaaagttt cgtttcgtag a 21
<210> 222
   <211> 21
   <212> DNA
   <213> Artificial Sequence
<220>
   <223> chemically treated genomic DNA (Homo sapiens)
<400> 222
   acaaaaaccg aaccgaaaat c 21
<210> 223
   <211> 24
   <212> DNA
   <213> Artificial Sequence
<220>
   <223> chemically treated genomic DNA (Homo sapiens)
<400> 223
   tccgaactct aaactcgcga tcgc 24
<210> 224
   <211> 23
   <212> DNA
   <213> Artificial Sequence
<220>
   <223> chemically treated genomic DNA (Homo sapiens)
<400> 224
   ttcgttgagg ttttaaacgc gta 23
<210> 225
   <211> 23
   <212> DNA
   <213> Artificial Sequence
<220>
   <223> chemically treated genomic DNA (Homo sapiens)
<400> 225
   aatccgaact ctaaactcgc gat 23
<210> 226
   <211> 26
   <212> DNA
   <213> Artificial Sequence
<220>
   <223> chemically treated genomic DNA (Homo sapiens)
<400> 226
   tacgaaacga aactttcgcc ccgata 26
<210> 227
   <211> 21
   <212> DNA
   <213> Artificial Sequence
<220>
   <223> chemically treated genomic DNA (Homo sapiens)
<400> 227
   tagtaatcgg agcgatttgc g 21
<210> 228
   <211> 23
   <212> DNA
   <213> Artificial Sequence
<220>
   <223> chemically treated genomic DNA (Homo sapiens)
<400> 228
   caaaatatac gacccgacga ata 23
<210> 229
   <211> 30
   <212> DNA
   <213> Artificial Sequence
<220>
   <223> chemically treated genomic DNA (Homo sapiens)
<400> 229
   cgtacgtatc ttctcgaaat tcccaaccgc 30
<210> 230
   <211> 24
   <212> DNA
   <213> Artificial Sequence
<220>
   <223> chemically treated genomic DNA (Homo sapiens)
<400> 230
   gtattttagg ttttgattcg cgtg 24
<210> 231
   <211> 25
   <212> DNA
   <213> Artificial Sequence
<220>
   <223> chemically treated genomic DNA (Homo sapiens)
<400> 231
   actcgtacga atcctctaaa acgaa 25
<210> 232
   <211> 24
   <212> DNA
   <213> Artificial Sequence
<220>
   <223> chemically treated genomic DNA (Homo sapiens)
<400> 232
   actaccccac gaaatacctc gcgc 24
<210> 233
   <211> 23
   <212> DNA
   <213> Artificial Sequence
<220>
   <223> chemically treated genomic DNA (Homo sapiens)
<400> 233
   gagggggttt agtatgtcgt tcg 23
<210> 234
   <211> 23
   <212> DNA
   <213> Artificial Sequence
<220>
   <223> chemically treated genomic DNA (Homo sapiens)
<400> 234
   cgaaactata acgtcgactc cga 23
<210> 235
   <211> 22
   <212> DNA
   <213> Artificial Sequence
<220>
   <223> chemically treated genomic DNA (Homo sapiens)
<400> 235
   aaaaccgaac cgccgccgaa aa 22
<210> 236
   <211> 24
   <212> DNA
   <213> Artificial Sequence
<220>
   <223> chemically treated genomic DNA (Homo sapiens)
<400> 236
   acgaatattt acaatcatca ccga 24
<210> 237
   <211> 20
   <212> DNA
   <213> Artificial Sequence
<220>
   <223> chemically treated genomic DNA (Homo sapiens)
<400> 237
   gcgagaattt agcgagtcgt 20
<210> 238
   <211> 23
   <212> DNA
   <213> Artificial Sequence
<220>
   <223> chemically treated genomic DNA (Homo sapiens)
<400> 238
   ccgcgataaa aattccgcct cgt 23
<210> 239
   <211> 24
   <212> DNA
   <213> Artificial Sequence
<220>
   <223> chemically treated genomic DNA (Homo sapiens)
<400> 239
   aaacaacgaa attaaaacga cgaa 24
<210> 240
   <211> 22
   <212> DNA
   <213> Artificial Sequence
<220>
   <223> chemically treated genomic DNA (Homo sapiens)
<400> 240
   gagggaattt ttttcgttcg at 22
<210> 241
   <211> 29
   <212> DNA
   <213> Artificial Sequence
<220>
   <223> chemically treated genomic DNA (Homo sapiens)
<400> 241
   acgatactcc gaaaaaactc cgcgataaa 29
<210> 242
   <211> 27
   <212> DNA
   <213> Artificial Sequence
<220>
   <223> chemically treated genomic DNA (Homo sapiens)
<400> 242
   aaaacctaac gaacgaaaat acgaata 27
<210> 243
   <211> 18
   <212> DNA
   <213> Artificial Sequence
<220>
   <223> chemically treated genomic DNA (Homo sapiens)
<400> 243
   tcgggattcg tcgttcgt 18
<210> 244
   <211> 29
   <212> DNA
   <213> Artificial Sequence
<220>
   <223> chemically treated genomic DNA (Homo sapiens)
<400> 244
   tccgaaccga ataacgtaaa aaaccgaac 29
<210> 245
   <211> 19
   <212> DNA
   <213> Artificial Sequence
<220>
   <223> chemically treated genomic DNA (Homo sapiens)
<400> 245
   tttttcgaga atgaggcgg 19
<210> 246
   <211> 23
   <212> DNA
   <213> Artificial Sequence
<220>
   <223> chemically treated genomic DNA (Homo sapiens)
<400> 246
   aaataaacgc aacgctacta cga 23
<210> 247
   <211> 23
   <212> DNA
   <213> Artificial Sequence
<220>
   <223> chemically treated genomic DNA (Homo sapiens)
<400> 247
   cgaacgcgtc atcaacttcc cga 23
<210> 248
   <211> 19
   <212> DNA
   <213> Artificial Sequence
<220>
   <223> chemically treated genomic DNA (Homo sapiens)
<400> 248
   aagttgatga cgcgttcgg 19
<210> 249
   <211> 23
   <212> DNA
   <213> Artificial Sequence
<220>
   <223> chemically treated genomic DNA (Homo sapiens)
<400> 249
   aaataaacgc aacgctacta cga 23
<210> 250
   <211> 29
   <212> DNA
   <213> Artificial Sequence
<220>
   <223> chemically treated genomic DNA (Homo sapiens)
<400> 250
   acgcgcgtct cgaaaacaaa ctaacttac 29
<210> 251
   <211> 24
   <212> DNA
   <213> Artificial Sequence
<220>
   <223> chemically treated genomic DNA (Homo sapiens)
<400> 251
   cgaaaataaa aaacgctacc gata 24
<210> 252
   <211> 20
   <212> DNA
   <213> Artificial Sequence
<220>
   <223> chemically treated genomic DNA (Homo sapiens)
<400> 252
   ttcgtacgga tgtcgaaggt 20
<210> 253
   <211> 23
   <212> DNA
   <213> Artificial Sequence
<220>
   <223> chemically treated genomic DNA (Homo sapiens)
<400> 253
   acgccaaaac gcactacaac gcc 23
<210> 254
   <211> 20
   <212> DNA
   <213> Artificial Sequence
<220>
   <223> chemically treated genomic DNA (Homo sapiens)
<400> 254
   tagttgcgaa gcgtaaacgg 20
<210> 255
   <211> 18
   <212> DNA
   <213> Artificial Sequence
<220>
   <223> chemically treated genomic DNA (Homo sapiens)
<400> 255
   cgccaccgaa cctcctac 18
<210> 256
   <211> 24
   <212> DNA
   <213> Artificial Sequence
<220>
   <223> chemically treated genomic DNA (Homo sapiens)
<400> 256
   tcgtccgtaa aacgcgaacc acga 24
<210> 257
   <211> 21
   <212> DNA
   <213> Artificial Sequence
<220>
   <223> chemically treated genomic DNA (Homo sapiens)
<400> 257
   aaaaaactcg cgatatctcg c 21
<210> 258
   <211> 21
   <212> DNA
   <213> Artificial Sequence
<220>
   <223> chemically treated genomic DNA (Homo sapiens)
<400> 258
   cgtcgttcgg gttagtagtc g 21
<210> 259
   <211> 32
   <212> DNA
   <213> Artificial Sequence
<220>
   <223> chemically treated genomic DNA (Homo sapiens)
<400> 259
   acgaaaataa ttaccaaata accgcgaaac ca 32
<210> 260
   <211> 18
   <212> DNA
   <213> Artificial Sequence
<220>
   <223> chemically treated genomic DNA (Homo sapiens)
<400> 260
   taggcggaga cgtttcgt 18
<210> 261
   <211> 17
   <212> DNA
   <213> Artificial Sequence
<220>
   <223> chemically treated genomic DNA (Homo sapiens)
<400> 261
   aacgcgacaa cgaccga 17
<210> 262
   <211> 24
   <212> DNA
   <213> Artificial Sequence
<220>
   <223> chemically treated genomic DNA (Homo sapiens)
<400> 262
   cgaatatcta tccgcgaacg ccga 24
<210> 263
   <211> 20
   <212> DNA
   <213> Artificial Sequence
<220>
   <223> chemically treated genomic DNA (Homo sapiens)
<400> 263
   aggttttttt ggttcggtcg 20
<210> 264
   <211> 25
   <212> DNA
   <213> Artificial Sequence
<220>
   <223> chemically treated genomic DNA (Homo sapiens)
<400> 264
   ccgtactcta ctacttcgtc aacga 25
<210> 265
   <211> 22
   <212> DNA
   <213> Artificial Sequence
<220>
   <223> chemically treated genomic DNA (Homo sapiens)
<400> 265
   cgaaacctaa cccgcctccg cg 22
<210> 266
   <211> 19
   <212> DNA
   <213> Artificial Sequence
<220>
   <223> chemically treated genomic DNA (Homo sapiens)
<400> 266
   ctccctaacc cgaccgatc 19
<210> 267
   <211> 24
   <212> DNA
   <213> Artificial Sequence
<220>
   <223> chemically treated genomic DNA (Homo sapiens)
<400> 267
   cgtgttttat tgtttcgtta gcga 24
<210> 268
   <211> 23
   <212> DNA
   <213> Artificial Sequence
<220>
   <223> chemically treated genomic DNA (Homo sapiens)
<400> 268
   cgcgaaaacg aaccaaatcc cga 23
<210> 269
   <211> 20
   <212> DNA
   <213> Artificial Sequence
<220>
   <223> chemically treated genomic DNA (Homo sapiens)
<400> 269
   tagtgagtac gcgcggttcg 20